(19) Europäisches Patentamt
European Patent Office
Office européen des brevets

(11) EP 4 238 967 A1

(12) **EUROPEAN PATENT APPLICATION**
published in accordance with Art. 153(4) EPC

(43) Date of publication:
**06.09.2023 Bulletin 2023/36**

(21) Application number: **21884948.7**

(22) Date of filing: **15.10.2021**

(51) International Patent Classification (IPC):
***C07D 401/12*** *(2006.01)* ***C07D 471/02*** *(2006.01)*
***A61K 31/519*** *(2006.01)* ***A61P 35/00*** *(2006.01)*

(52) Cooperative Patent Classification (CPC):
**A61K 31/519; A61P 35/00; C07D 401/12; C07D 471/02**

(86) International application number:
**PCT/CN2021/123949**

(87) International publication number:
**WO 2022/089219 (05.05.2022 Gazette 2022/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.10.2020 CN 202011169409**
**23.06.2021 CN 202110702246**

(71) Applicant: **Sichuan Kelun-Biotech Biopharmaceutical Co., Ltd.**
**Chengdu, Sichuan 611138 (CN)**

(72) Inventors:
- **CHEN, Zhonghui**
**Chengdu, Sichuan 611138 (CN)**
- **HAN, Xiaojun**
**Chengdu, Sichuan 611138 (CN)**
- **LIU, Qian**
**Chengdu, Sichuan 611138 (CN)**
- **TIAN, Qiang**
**Chengdu, Sichuan 611138 (CN)**
- **SONG, Hongmei**
**Chengdu, Sichuan 611138 (CN)**
- **GE, Junyou**
**Chengdu, Sichuan 611138 (CN)**
- **WANG, Jingyi**
**Chengdu, Sichuan 611138 (CN)**

(74) Representative: **Hoffmann Eitle**
**Patent- und Rechtsanwälte PartmbB**
**Arabellastraße 30**
**81925 München (DE)**

(54) **ARYLAMIDE COMPOUND, PHARMACEUTICAL COMPOSITION COMPRISING SAME, AND PREPARATION METHOD THEREFOR AND USE THEREOF**

(57)

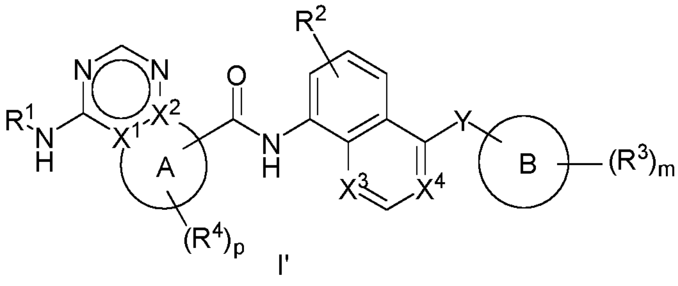

I'

The present invention relates to an aryl amide compound of formula (1'), a pharmaceutical composition comprising same, a preparation method therefor, and the use thereof for preventing or treating tumor-related diseases or conditions.

EP 4 238 967 A1

## Description

## TECHNICAL FIELD

The present invention relates to an arylamide compound, a pharmaceutical composition comprising the same, a preparation method therefor, and use thereof for the prophylaxis or treatment of a disease or disorder associated with RAF and/or RAS kinase activity.

## BACKGROUND ART

Protein kinases are a class of enzymes that catalyze protein phosphorylation. Protein phosphorylation regulates cell physiological activities such as cell survival, proliferation, differentiation, apoptosis and metabolism *etc.* by mediating cell signal transduction. Protein kinase dysfunction is closely related to various diseases, including tumor, autoimmune disease, inflammatory reaction, central nervous system disease, cardiovascular disease, diabetes and the like.

RAF is an ATP kinase and an important part of the RAS-RAF-MEK signaling pathway. It comprises three subtypes (A, B, and C), with a high degree of homology and similar structural domains. RAF exists in the cytoplasm as an inactive monomer. After stimulation of upstream growth factors, RAS is converted from the inactive conformation (GDP binding) to the active conformation (GTP binding), thereby recruiting intracellular RAF to the cell membrane and promoting its dimerization and phosphorylation. Activated RAF in turn phosphorylates and activates MEK and ERK, ultimately regulating cell proliferation, differentiation, apoptosis and metastasis (Karoulia Z et al., Nat Rev Cancer. 2017 Nov; 17(11):676-691). The mutated B-RAF can continuously activate the MAPK signaling pathway in the form of monomer (V600 mutation) or dimer (non-V600 mutation), independent of RAS. An RAF inhibitor inhibits the RAS-RAF-MEK signaling pathway by inhibiting the activity of RAF monomers and dimers, and can be used for the treatment of tumors with RAS or RAF mutants, and the applicable population accounts for about 1/3 of tumor patients. Applicable tumor types mainly comprise melanoma, NSCLC, CRC, ovarian cancer, endometrial cancer, thyroid cancer, pancreatic cancer, *etc.*

The $\alpha$C-OUT RAF inhibitors represented by Vemurafenib can effectively inhibit the kinase activity of V600 point mutant BRAF. However, for RAS-mutated, wild-type B-RAF, and non-V600 point-mutated BRAF-driven tumors, the $\alpha$C-OUT inhibitors cannot effectively inhibit RAF activation, and drug resistance has emerged upon clinical use.

## CONTENTS OF THE INVENTION

The present invention provides novel arylamide compounds, which have a good inhibitory effect on RAF and/or RAS kinase, and have good properties such as pharmacokinetic properties. The compound of the present invention can inhibit the activity of RAF dimer, can overcome the dimer resistance mechanism caused by the existing RAF inhibitors, reduce the abnormal activation toxicity of ERK, and can be applied to the treatment of RAS or RAF mutant tumors.

An aspect of the present invention provides a compound of Formula I' or a pharmaceutically acceptable salt, ester, stereoisomer, tautomer, polymorph, cocrystal, solvate, metabolite, isotopically labeled compound, N-oxide or prodrug thereof:

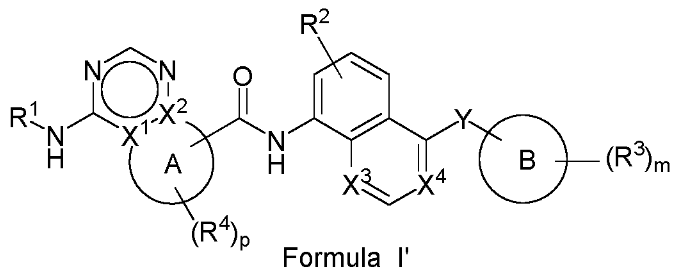

Formula I'

wherein:

- ring A is selected from the group consisting of benzene ring and 5-6-membered heteroaromatic ring;
- ring B is selected from the group consisting of $C_{6-10}$ aromatic ring, 5-10-membered heteroaromatic ring and 4-10-membered heterocycle;
- $X^1$ and $X^2$ are each independently selected from the group consisting of C and N;
- $X^3$ and $X^4$ are each independently selected from the group consisting of CH and N;
- Y is selected from the group consisting of -O-, -NH-, -C(=O)-, $-CR^5R^6-$, $-CR^5R^6O-$ and $-CR^5R^6NH-$; provided that, when ring A is a thiophene ring, Y is not -O- or -NH-;

$R^1$ is selected from the group consisting of H, $C_{1-6}$ alkyl and $C_{3-6}$ cycloalkyl, the alkyl and cycloalkyl are each optionally substituted with one or more halogens;
$R^2$ is selected from the group consisting of H, halogen, $C_{1-6}$ alkyl and $C_{1-6}$ alkoxy, the alkyl and alkoxy are each optionally substituted with one or more halogens;
$R^3$ is L-$R^{3'}$;
L, at each occurrence, is each independently a direct bond or $-(CH_2)_n-$;
$R^{3'}$, at each occurrence, is each independently selected from the group consisting of H, hydroxyl, halogen, CN, NOz, $C_{1-6}$ alkyl, $C_{1-6}$ heteroalkyl (e.g., $C_{1-6}$ alkoxy), $C_{2-6}$ alkenyl, $C_{2-6}$ alkynyl, $C_{3-8}$ cycloalkyl, $C_{3-8}$ cycloalkoxy, 4-10-membered heterocyclyl, $C_{6-12}$ aryl, 5-10-membered heteroaryl, $-NR^{20a}R^{20b}$, $-SR^{21}$, - $S(=O)_2R^{22}$, $-S(=O)_2NR^{20a}R^{20b}$, $-NR^{20a}S(=O)_2R^{20b}$, $-C(=O)R^{21}$, $-C(=O)NR^{23a}R^{23b}$, $-NR^{23a}C(=O)R^{23b}$ and - $NR^{24a}C(=O)NR^{25a}R^{25b}$, the alkyl, heteroalkyl (e.g., alkoxy), alkenyl, alkynyl, cycloalkyl, cycloalkoxy, heterocyclyl, aryl and heteroaryl are each optionally substituted with one or more substituents independently selected from the group consisting of: hydroxyl, halogen, CN, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl and 4-10-membered heterocyclyl; or when L is a direct bond, and m is greater than 1, two $R^{3'}$ together with the group to which they are attached form a 4-10-membered heterocycle;
$R^4$, at each occurrence, is each independently selected from the group consisting of H, hydroxyl, halogen, CN, NOz, $C_{1-6}$ alkyl, $C_{1-6}$ heteroalkyl (e.g., $C_{1-6}$ alkoxy), $C_{2-6}$ alkenyl, $C_{2-6}$ alkynyl, $C_{3-8}$ cycloalkyl, $C_{3-8}$ cycloalkoxy, 4-10-membered heterocyclyl, $C_{6-12}$ aryl, 5-10-membered heteroaryl, $-NR^{20a}R^{20b}$, $-SR^{21}$, - $S(=O)_2R^{22}$, $-S(=O)_2NR^{20a}R^{20b}$, $-NR^{20a}S(=O)_2R^{20b}$, $-C(=O)R^{21}$, $-C(=O)NR^{23a}R^{23b}$, $-NR^{23a}C(=O)R^{23b}$ and - $NR^{24a}C(=O)NR^{25a}R^{25b}$, the alkyl, heteroalkyl (e.g., alkoxy), alkenyl, alkynyl, cycloalkyl, cycloalkoxy, heterocyclyl, aryl and heteroaryl are each optionally substituted with one or more substituents independently selected from the group consisting of: hydroxyl, halogen, CN, $NH_2$, $NO_2$, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl and 4-10-membered heterocyclyl;
$R^5$ and $R^6$ are each independently selected from the group consisting of H, hydroxyl, halogen, $-NH_2$, - $NHCH_3$, $-N(CH_3)_2$, CN, $C_{1-6}$ alkyl, $C_{1-6}$ heteroalkyl (e.g., $C_{1-6}$ alkoxy), $C_{3-8}$ cycloalkyl, $C_{3-8}$ cycloalkoxy and 4-10-membered heterocyclyl, or $R^5$ and $R^6$ together with the atom to which they are attached form $C_{3-8}$ cycloalkyl or 3-8-membered heterocyclyl, the alkyl, heteroalkyl (e.g., alkoxy), cycloalkyl, cycloalkoxy and heterocyclyl are each optionally substituted with one or more halogens;
$R^{20a}$, $R^{20b}$, $R^{23a}$, $R^{23b}$, $R^{24a}$, $R^{25a}$ and $R^{25b}$ are each independently selected from the group consisting of H, OH, $-NHCH_3$, $-N(CH_3)_2$, $C_{1-6}$ alkyl, $C_{1-6}$ alkoxy, $C_{3-8}$ cycloalkyl and 4-10-membered heterocyclyl; the alkyl, alkoxy, cycloalkyl and heterocyclyl are each optionally substituted with one or more substituents independently selected from the group consisting of: halogen, $C_{1-6}$ alkyl and 4-10-membered heterocyclyl;
$R^{21}$ and $R^{22}$ are each independently selected from the group consisting of $C_{1-6}$ alkyl, $C_{1-6}$ alkoxy, $C_{3-8}$ cycloalkyl and 4-10-membered heterocyclyl, the alkyl, alkoxy, cycloalkyl and heterocyclyl are each optionally substituted with one or more halogens;
m is 0, 1, 2, 3, 4 or 5;
n is 1 or 2; and
p is 0, 1, 2 or 3.

Another aspect of the present invention provides a pharmaceutical composition comprising a prophylactically or therapeutically effective amount of the compound of the present invention, or a pharmaceutically acceptable salt, ester, stereoisomer, tautomer, polymorph, cocrystal, solvate, metabolite, isotopically labeled compound, N-oxide or prodrug thereof, and one or more pharmaceutically acceptable carriers.

Another aspect of the present invention provides use of the compound of the present invention or a pharmaceutically acceptable salt, ester, stereoisomer, tautomer, polymorph, cocrystal, solvate, metabolite, isotopically labeled compound, N-oxide or prodrug thereof, or the pharmaceutical composition of the present invention in the manufacture of a medicament for the prophylaxis or treatment of a disease or disorder associated with RAF and/or RAS kinase activity.

Another aspect of the present invention provides the compound of the present invention or a pharmaceutically acceptable salt, ester, stereoisomer, tautomer, polymorph, cocrystal, solvate, metabolite, isotopically labeled compound, N-oxide or prodrug thereof, or the pharmaceutical composition of the present invention, for use in the prophylaxis or treatment of a disease or disorder associated with RAF and/or RAS kinase activity.

Another aspect of the present invention provides a method for the prophylaxis or treatment of a disease or disorder associated with RAF and/or RAS kinase activity, wherein the method comprises administering to a subject in need thereof an effective amount of the compound of the present invention or a pharmaceutically acceptable salt, ester, stereoisomer, tautomer, polymorph, cocrystal, solvate, metabolite, isotopically labeled compound, N-oxide or prodrug thereof, or the pharmaceutical composition of the present invention.

Another aspect of the present invention provides a method for preparing the compound of the present invention.

## Definitions

Unless otherwise defined in the context, all technical and scientific terms used herein are intended to have the same meaning as commonly understood by a person skilled in the art. References to techniques employed herein are intended to refer to the techniques as commonly understood in the art, including variations on those techniques or substitutions of equivalent techniques which would be apparent to a person skilled in the art. While it is believed that the following terms will be readily understood by a person skilled in the art, the following definitions are nevertheless put forth to better illustrate the present invention.

The terms "contain", "include", "comprise", "have", or "relate to", as well as other variations used herein are inclusive or open-ended, and do not exclude additional, unrecited elements or method steps, although the unrecited elements or method steps do not necessarily exist (that is, these terms also contemplate terms "substantially consist of ..." and "consist of ...").

As used herein, the term "alkyl" is defined as a saturated linear or branched aliphatic hydrocarbon. In some embodiments, alkyl has 1-12, e.g., 1-6, carbon atoms. For example, as used herein, the terms "$C_{1-6}$ alkyl" and "$C_{1-4}$ alkyl" respectively refer to linear or branched groups having 1-6 and 1-4 carbon atoms (such as methyl, ethyl, n-propyl, isopropyl, n-butyl, isobutyl, sec-butyl, *tert*-butyl, n-pentyl, isopentyl, neopentyl, or n-hexyl), which is optionally substituted with one or more (e.g., 1 to 3) suitable substituents such as halogen (in which case the group may be referred to as "haloalkyl") (e.g., $CH_2F$, $CHF_2$, $CF_3$, $CCl_3$, $C_2F_5$, $C_2Cl_5$, $CH_2CF_3$, $CH_2Cl$ or $-CH_2CH_2CF_3$ *etc.).* The term "$C_{1-4}$ alkyl" refers to a linear or branched aliphatic hydrocarbon chain having 1-4 carbon atoms (i.e., methyl, ethyl, n-propyl, isopropyl, n-butyl, isobutyl, sec-butyl or *tert*-butyl).

As used herein, the term "heteroalkyl" refers to an alkyl group having backbone carbon atoms wherein one or more backbone atoms independently selected from atoms other than carbon, such as oxygen, nitrogen, sulfur, phosphorus, or a combination thereof. A numerical range (e.g., $C_{1-6}$ heteroalkyl) can be given to indicate the number of carbons in the chain (in this example, 1-6 carbon atoms are comprised). For example, the $-CH_2OCH_2CH_3$ group is referred to as a $C_3$ heteroalkyl group. The linkage with the rest of the molecule can be through the heteroatom or carbon atom in the heteroalkyl chain.

As used herein, the term "haloalkyl" refers to alkyl substituted with one or more (e.g., 1 to 3) same or different halogen atoms. The terms "$C_{1-8}$ haloalkyl", "$C_{1-6}$ haloalkyl" and "$C_{1-4}$ haloalkyl" respectively refer to haloalkyl having 1 to 8 carbon atoms, 1 to 6 carbon atoms and 1 to 4 carbon atoms, e.g., $-CF_3$, $-C_2F_5$, $- CHF_2$, $-CH_2F$, $-CH_2CF_3$, $-CH_2Cl$ or $-CH_2CH_2CF_3$, *etc.*

As used herein, the term "hydroxyalkyl" refers to a group formed by replacing the hydrogen atoms in an alkyl group with one or more hydroxyl groups, such as $C_{1-4}$ hydroxyalkyl or $C_{1-3}$ hydroxyalkyl. Examples thereof include but are not limited to hydroxymethyl, hydroxyethyl, hydroxypropyl, hydroxybutyl, $-CH(OH)CH_3$, *etc.*

As used herein, the term "alkoxy" means a group obtained by inserting an oxygen atom at any suitable position in an alkyl group (as defined above), preferably $C_{1-8}$ alkyloxy, $C_{1-6}$ alkyloxy, $C_{1-4}$ alkyloxy or $C_{1-3}$ alkyloxy. Representative examples of the $C_{1-6}$ alkyloxy group include, but are not limited to, methoxy, ethoxy, propoxy, isopropoxy, n-propoxy, isopropoxy, n-butoxy, isobutoxy, t-butoxy, pentoxy, hexyloxy, $- CH_2-OCH_3$, *etc.,* and the alkyloxy group is optionally substituted with one or more (e.g., 1 to 3) same or different substituents. The term "haloalkoxy" refers to an alkoxy group wherein hydrogen atoms are replaced with one or more (e.g., 1 to 3) same or different halogen atoms.

As used herein, the term "alkenyl" refers to a linear or branched monovalent hydrocarbyl having one or more double bonds. For example, "$C_{2-6}$ alkenyl" refers to alkenyl having 2-6 carbon atoms. The alkenyl is e.g., $CH=CH_2$, $-CH_2CH=CH_2$, $-C(CH_3)=CH_2$, $-CH_2-CH=CH-CH_3$, 2-pentenyl, 3-pentenyl, 4-pentenyl, 2-hexenyl, 3-hexenyl, 4-hexenyl, 5-hexenyl, 2-methyl-2-propenyl and 4-methyl-3-pentenyl. When the compound of the present invention contains an alkenyl group, the compound may exist as the pure E (entgegen) form, the pure Z (zusammen) form, or any mixture thereof.

As used herein, the term "alkynyl" refers to a monovalent hydrocarbyl containing one or more triple bonds, preferably having 2, 3, 4, 5 or 6 carbon atoms, such as ethynyl, 2-propynyl, 2-butynyl, buta-1,3-diynyl, *etc.* The alkynyl group is optionally substituted with one or more (e.g., 1 to 3) same or different substituents.

As used herein, the term "fused ring" refers to a ring system formed by two or more ring structures sharing two adjacent atoms with each other.

As used herein, the term "spiro" refers to a ring system formed by two or more ring structures sharing one ring atom with each other.

As used herein, the term "bridged ring" refers to a ring system formed by two or more ring structures sharing two atoms which are not directly linked with each other.

As used herein, the term "cycloalkyl" refers to a saturated or unsaturated nonaromatic monocyclic or polycyclic (e.g., bicyclic) cyclohydrocarbyl group, including, but not limited to, monocyclic alkyl groups (e.g., cyclopropyl, cyclobutyl, cyclopentyl, cyclohexyl, cycloheptyl, cyclooctyl, cyclononyl, *etc.)* and bicyclic alkyl, including spiro, fused or bridged cyclic systems (i.e., spiro cycloalkyl, fused cycloalkyl and bridged cycloalkyl, such as bicyclo[1.1.1]pentyl, bicyclo[2.2.1]heptyl, *etc.).* In the present invention, the cycloalkyl group is optionally substituted with one or more (e.g., 1 to

3) same or different substituents. The carbon atom in the cycloalkyl group is optionally substituted with an oxo group (i.e., forming C=O). The term "$C_{3-8}$ cycloalkyl" refers to a cycloalkyl group having 3 to 8 ring-forming carbon atoms, such as $C_{3-6}$ cycloalkyl, which can be monocyclic alkyl, such as cyclopropyl, cyclobutyl, cyclopentyl, cyclohexyl, cycloheptyl or cyclooctyl, or bicycloalkyl, such as $C_{5-8}$ spiro cycloalkyl, $C_{5-8}$ bridged cycloalkyl, $C_{5-8}$ fused cycloalkyl, $C_{5-6}$ spiro cycloalkyl, $C_{5-6}$ bridged cycloalkyl or $C_{5-6}$ fused cycloalkyl.

As used herein, the term "cycloalkoxy" refers to -O-cycloalkyl, wherein the cycloalkyl is as defined above. Representative examples of a cycloalkoxy group include, but are not limited to, cyclopropoxy, cyclobutoxy, cyclopentyloxy, cyclohexyloxy, *etc.*

As used herein, the term "heterocyclyl" or "heterocycle" refers to an aliphatic monocyclic or polycyclic (e.g., a fused, a spiro, or bridged ring) group having two or more (e.g., 3, 4, 5, 6, 7, 8, 9, 10, 11, 12, 13, or 14) carbon atoms and one or more (e.g., 1, 2, 3, or 4) heteroatoms, which include, but are not limited to, oxygen, nitrogen and sulfur atoms. The carbon atoms and heteroatoms in the heterocyclyl group are optionally substituted with oxo groups (e.g., forming C=O, S(= O) or $S(=O)_2$), or are optionally substituted with one or more (e.g., 1 to 3) substituents independently selected from halogen and $C_{1-3}$ alkyl.

As used herein, the term "4-11-membered heterocyclyl" refers to a heterocyclyl group comprising 4-11 ring atoms, including, but not limited to, 4-10-membered heterocyclyl, 4-9-membered heterocyclyl, 4-8-membered heterocyclyl, 4-7-membered heterocyclyl, 5-6-membered heterocyclyl, 3-8-membered heterocyclyl, 3-7-membered heterocyclyl, 4-7-membered nitrogen-containing heterocyclyl, 4-7-membered oxygen-containing heterocyclyl, 4-7-membered sulfur-containing heterocyclyl, 5-6-membered nitrogen-containing heterocyclyl, 5-6-membered oxygen-containing heterocyclyl, 5-6-membered sulfur-containing heterocyclyl and the like. The "nitrogen-containing heterocyclyl", "oxygen-containing heterocyclyl" and "sulfur-containing heterocyclyl" respectively optionally comprise one or more additional heteroatoms independently selected from oxygen, nitrogen and sulfur. Examples of 4-11-membered heterocyclyl include, but are not limited to oxiranyl, aziridinyl, azetidinyl, oxetanyl, tetrahydrofuranyl, pyrrolidinyl, pyrrolidonyl (e.g.,

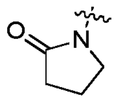

), imidazolidinyl, pyrazolidinyl, tetrahydropyranyl, piperidinyl, morpholinyl, dithianyl, thiomorpholinyl, piperazinyl or trithianyl.

In the present invention, heterocyclyl can form a fused ring structure with heterocyclyl or cycloalkyl, and the attachment point of the fused ring structure with another group can be from any heterocyclyl or cycloalkyl. As such, the heterocyclyl of the present invention also includes but is not limited to heterocyclyl-fused heterocyclyl, heterocyclyl-fused cycloalkyl, monoheterocyclyl-fused monoheterocyclyl, monoheterocyclyl-fused monocycloalkyl, such as 3-7-membered (mono)heterocyclyl-fused 3-7-membered (mono)heterocyclyl, 3-7-membered (mono)heterocyclyl-fused (mono)cycloalkyl, 3-7-membered (mono)heterocyclyl-fused $C_{4-6}$ (mono)cycloalkyl, and the examples include, but are not limited to, pyrrolidinyl-fused cyclopropyl, cyclopentyl-fused azacyclopropyl, pyrrolidinyl-fused cyclobutyl, pyrrolidinyl-fused pyrrolidinyl, pyrrolidinyl-fused piperidinyl, pyrrolidinyl-fused piperazinyl, piperidinyl-fused morpholinyl,

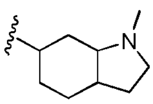, 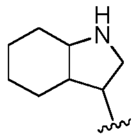 or 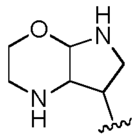.

In the present invention, heterocyclyl further encompasses bridged heterocyclyl and spiro heterocyclyl.

As used herein, the term "bridged heterocycle" refers to a ring structure comprising one or more (e.g., 1, 2, 3 or 4) heteroatoms (e.g., oxygen, nitrogen, and/or sulfur atoms), formed by two saturated rings sharing two ring atoms which are not directly linked, including, but not limited to, 7-10-membered bridged heterocycle, 8-10-membered bridged heterocycle, 7-10-membered nitrogen-containing bridged heterocycle, 7-10-membered oxygen-containing bridged heterocycle, 7-10-membered sulfur-containing bridged heterocycle, and the like, such as

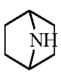, 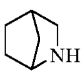, 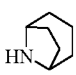, 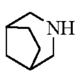, 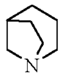, 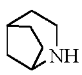, 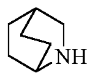, 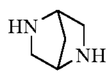,

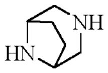, 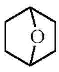, 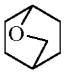, 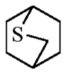, 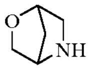, 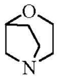, 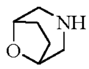, 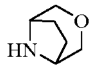, 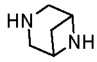, 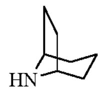, 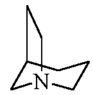,

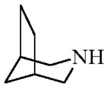, 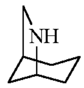,

*etc.* The "nitrogen-containing bridged heterocycle", "oxygen-containing bridged heterocycle" and "sulfur-containing bridged heterocycle" optionally further comprise one or more additional heteroatoms independently selected from oxygen, nitrogen and sulfur.

As used herein, the term "spiro heterocycle" refers to a ring structure comprising one or more (e.g., 1, 2, 3 or 4) heteroatoms (e.g., oxygen atoms, nitrogen atoms, sulfur atoms) formed by two or more saturated rings sharing one ring atom, including, but not limited to, 5-10-membered spiro heterocycle, 6-10-membered spiro heterocycle, 6-10-membered nitrogen-containing spiro heterocycle, 6-10-membered oxygen-containing spiro heterocycle, and 6-10-membered sulfur-containing spiro heterocycle, *etc.* such as

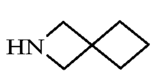, 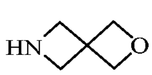, 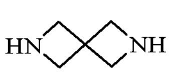, 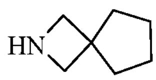, 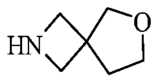, 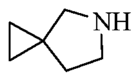, 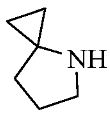,

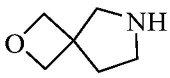, 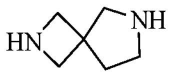, 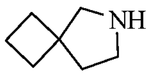, 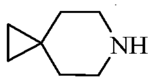, 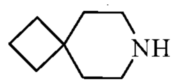, 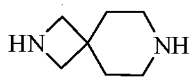,

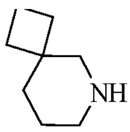, 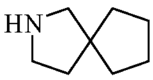, 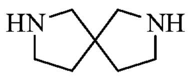, 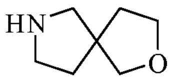, 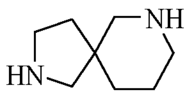 and 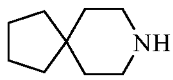.

The "nitrogen-containing spiro heterocycle", "oxygen-containing spiro heterocycle" and "sulfur-containing spiro heterocycle" optionally further comprise one or more additional heteroatoms independently selected from oxygen, nitrogen and sulfur. The term "6-10-membered nitrogen-containing spiro heterocyclyl" refers to a spiro heterocyclyl group comprising a total of 6-10 ring atoms, and at least one of the ring atoms is a nitrogen atom.

Examples of groups obtained by the fuse of heterocyclyl with aryl include, but are not limited to,

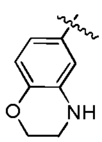, 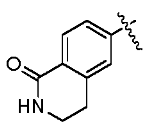, 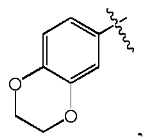, 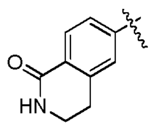, 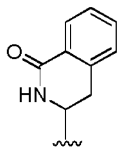.

As used herein, the term "aryl" or "aromatic ring" refers to an all-carbon monocyclic or fused polycyclic aromatic group having a conjugated $\pi$ electron system. As used herein, the term "$C_{6-12}$ aryl (aromatic ring)" refers to an aryl (aromatic ring) group comprising 6 to 12 carbon atoms, preferably $C_{6-10}$ aryl (aromatic ring), preferably phenyl (benzene ring) or naphthyl (naphthalene ring). The aryl group is optionally substituted with one or more (e.g., 1 to 3) same or different substituents (such as halogen, OH, CN, NOz, $C_1$-$C_6$ alkyl, *etc.).*

As used herein, the term "heteroaryl" or "heteroaromatic ring" refers to a monocyclic or polycyclic aromatic group comprising one or more same or different heteroatoms, including a monocyclic heteroaryl group and a bicyclic or polycyclic ring system comprising at least one heteroaromatic ring (an aromatic ring system comprising at least one heteroatom). It can comprise 5, 6, 7, 8, 9, 10, 11, 12, 13 or 14 ring atoms, e.g., 5, 6, 7, 8, 9, or 10 ring atoms. The heteroatom may be oxygen, nitrogen or sulfur. The carbon atoms and heteroatoms in a heteroaryl group are optionally substituted with an oxo group (e.g., forming C=O, S(=O) or S(=O)z).

As used herein, the term "5-10-membered heteroaryl" or "5-10-membered heteroaromatic ring" refers to heteroaryl (heteroaromatic ring) comprising 5 to 10 (e.g., 5 to 6) ring atoms, including 5-10-membered nitrogen-containing heteroaryl, 5-10-membered oxygen-containing heteroaryl, 5-10-membered sulfur-containing heteroaryl, 5-6-membered nitrogen-containing heteroaryl, 5-6-membered oxygen-containing heteroaryl, 5-6-membered sulfur-containing heteroaryl, *etc.* The "nitrogen-containing heteroaryl", "oxygen-containing heteroaryl" and "sulfur-containing heteroaryl" each optionally comprise one or more additional heteroatoms independently selected from oxygen, nitrogen and sulfur. Examples thereof include, but are not limited to, thienyl, furyl, pyrrolyl, oxazolyl, thiazolyl, imidazolyl, pyrazolyl, isoxazolyl, isothiazolyl, triazolyl, tetrazolyl, oxadiazolyl, thiadiazolyl, *etc.*; or pyridinyl, pyridazinyl, pyrimidinyl, pyrazinyl, triazinyl, *etc.*, and 5-10-membered fused ring moieties comprising such groups.

In the present invention, heteroaryl (e.g., monoheteroaryl) and aryl (e.g., monocyclic aryl, e.g., phenyl), heterocyclyl (e.g., monoheterocyclyl), cycloalkyl (e.g., monocycloalkyl) or another heteroaryl group (e.g., another monoheteroaryl group) can share two adjacent atoms with each other, thereby forming a fused ring structure, and the attachment point can be from any heteroaryl ring or another ring. It includes but is not limited to (mono)heteroaryl-fused (mono)heteroaryl, (mono)heteroaryl-fused (monocyclo)aryl, (mono)heteroaryl-fused (mono)heterocyclyl and (mono)heteroaryl-fused (mono)cycloalkyl, e.g., 5-6-membered (mono)heteroaryl-fused 5-6-membered (mono)heteroaryl, 5-6-membered (mono)heteroaryl-fused phenyl, 5-6-membered (mono)heteroaryl-fused 5-6-membered (mono)heterocyclyl or 5-6-membered (mono)heteroaryl-fused $C_{4-6}$ (mono)cycloalkyl (e.g., 5-6-membered heteroaryl-fused cyclobutyl, 5-6-membered heteroaryl-fused cyclopentyl or 5-6-membered heteroaryl-fused cyclohexyl), and the examples include but are not limited to indolyl, isoindolyl, indazolyl, benzoimidazole, quinolinyl, isoquinolinyl,

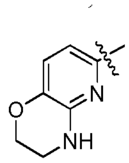, 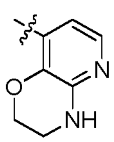, 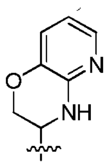, 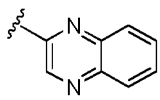, 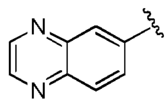, 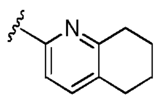, 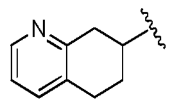,

and the like.

As used herein, the term "halo" or "halogen" are defined to include F, Cl, Br, or I.

The term "substituted" means that one or more (e.g., one, two, three, or four) hydrogens on the designated atom is replaced with a selection from the indicated group, provided that the designated atom's normal valency under the existing circumstances is not exceeded, and that the substitution results in a stable compound. Combinations of substituents and /or variables are permissible only if such combinations result in stable compounds.

If a substituent is described as being "optionally substituted with one or more ...", the substituent may be either (1) not substituted, or (2) substituted. If a carbon of a substituent is described as being optionally substituted with one or more of a list of substituents, one or more of the hydrogens on the carbon (to the extent there are any) may separately and /or together be replaced with an independently selected optional substituent. If a nitrogen of a substituent is described as being optionally substituted with one or more of a list of substituents, one or more of the hydrogens on the nitrogen (to the extent there are any) may each be replaced with an independently selected optional substituent.

If substituents are described as being "independently selected" from a group, each substituent is selected independent of the other(s). Each substituent therefore may be identical to or different from the other substituent(s).

As used herein, the term "one or more" means one or more than one (e.g., 2, 3, 4, 5 or 10) as reasonable.

As used herein, unless specified, the point of attachment of a substituent can be from any suitable position of the substituent.

When a bond to a substituent is shown to cross a bond connecting two atoms in a ring, then such substituent may be bonded to any of the ring-forming atoms in that ring that are substitutable.

The present invention also includes all pharmaceutically acceptable isotopically labeled compounds, which are identical to those of the present invention except that one or more atoms are replaced by an atom having the same atomic number, but an atomic mass or mass number different from the atomic mass or mass number which predominates in nature. Examples of isotopes suitable for inclusion in the compound of the present invention include, but are not limited to, isotopes of hydrogen, such as $^{2}H$, $^{3}H$; carbon, such as $^{11}C$, $^{13}C$, and $^{14}C$; chlorine, such as $^{36}Cl$; fluorine, such as $^{18}F$; iodine, such as $^{123}I$ and $^{125}I$; nitrogen, such as $^{13}N$ and $^{15}N$; oxygen, such as $^{15}O$, $^{17}O$, and $^{18}O$; phosphorus, such as $^{32}P$; and sulfur, such as $^{35}S$. Certain isotopically labeled compounds of the present invention, for example those incorporating a radioactive isotope, are useful in drug and /or substrate tissue distribution studies (e.g., assays). The radioactive isotopes tritium, i.e., $^{3}H$, and carbon-14, i.e., $^{14}C$, are particularly useful for this purpose in view of their ease of incorporation and ready means of detection. Substitution with positron-emitting isotopes, such as $^{11}C$, $^{18}F$, $^{15}O$ and $^{13}N$, can be useful in positron emission tomography (PET) studies for examining substrate receptor occupancy. Isotopically labeled compounds of the present invention can generally be prepared by processes analogous to those described

in the accompanying Schemes and /or in the Examples and Preparations, by using an appropriate isotopically labeled reagent in place of the non-labeled reagent previously employed. Pharmaceutically acceptable solvates in accordance with the invention include those wherein the solvent of crystallization may be isotopically substituted, e.g., $D_2O$, acetone-$d_6$, or DMSO-$d_6$.

The term "stereoisomer" refers to isomers with at least one asymmetric center. A compound having one or more (e.g., one, two, three or four) asymmetric centers can give rise to a racemic mixture, single enantiomer, diastereomer mixture and individual diastereomer. Certain individual molecules may exist as geometric isomers (cis/trans). Similarly, the compound of the present invention may exist as a mixture of two or more structurally different forms in rapid equilibrium (generally referred to as tautomer). Typical examples of a tautomer include a keto-enol tautomer, phenol-keto tautomer, nitroso-oxime tautomer, imine-enamine tautomer and the like. For example, a nitroso-oxime group may exist as the following tautomers in equilibrium in a solution:

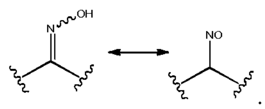

It is to be understood that all such isomers and mixtures thereof in any proportion (such as 60%, 65%, 70%, 75%, 80%, 85%, 90%, 95%, 96%, 97%, 98%, and 99%) are encompassed within the scope of the present invention.

The chemical bonds of the compound of the present invention may be depicted herein using a solid line (), a solid wedge (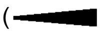), or a dotted wedge (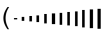). The use of a solid line to depict bonds to asymmetric carbon atoms is meant to indicate that all possible stereoisomers (e.g., specific enantiomers, racemic mixtures, *etc.)* at that carbon atom are included. The use of either a solid or dotted wedge to depict bonds to asymmetric carbon atoms is meant to indicate that the stereoisomer shown is present. When present in racemic compounds, solid and dotted wedges are used to define relative stereochemistry, rather than absolute stereochemistry. Unless stated otherwise, it is intended that the compound of the present invention can exist as stereoisomers, which include cis and trans isomers, optical isomers such as R and S enantiomers, diastereomers, geometric isomers, rotational isomers, conformational isomers, atropisomers, and mixtures thereof. The compound of the present invention may exhibit more than one type of isomerism, and consist of mixtures thereof (such as racemates and diastereomeric pairs).

The present invention includes all possible crystalline forms or polymorphs of the compound of the present invention, either as a single polymorph, or as a mixture of more than one polymorphs, in any ratio.

Co-crystal refers to a combination of a pharmaceutically active molecule and another physiologically acceptable acid, base, salt and non-ionic compound in a same crystal lattice through connection of hydrogen bonds, $\pi$-$\pi$ stacking interaction, van der Waals force and other non-covalent bonds.

It also should be understood that, certain compounds of the present invention can be used for the treatment in a free form, or where appropriate, in a form of a pharmaceutically acceptable derivative. In the present invention, the pharmaceutically acceptable derivative includes, but is not limited to a pharmaceutically acceptable salt, ester, solvate, N-oxide, metabolite or prodrug, which can directly or indirectly provide the compound of the present invention or a metabolite or residue thereof after being administered to a patient in need thereof. Therefore, "the compound of the present invention" mentioned herein also means to encompass various derivative forms of the compound as mentioned above.

A pharmaceutically acceptable salt of the compound of the present invention includes an acid addition salt and a base addition salt thereof.

A pharmaceutically acceptable salt of the compound of the present invention includes an acid addition salt and a base addition salt thereof, such as hexafluorophosphate, meglumine, and the like. For a review on suitable salts, see "Hand book of Pharmaceutical Salts: Properties, Selection, and Use" by Stahl and Wermuth (Wiley-VCH, 2002).

As used herein, the term "ester" refers to those derived from the compounds of the various general formulae in the present application, which include physiologically-hydrolyzable esters (which may be hydrolyzed under physiological conditions to release the compounds of the present invention in the form of free acids or alcohols). The compound of the present invention itself may be an ester as well.

The compound of the present invention can exist as a solvate (preferably a hydrate), wherein the compound of the present invention contains a polar solvent, in particular water, methanol or ethanol for example, as a structural element of the crystal lattice of the compound. The amount of the polar solvent, in particular water, may exist in a stoichiometric or non-stoichiometric ratio.

As can be appreciated by a person skilled in the art, not all nitrogen containing heterocycles can form N-oxides since the nitrogen requires an available lone-pair electron for oxidation to the oxide. A person skilled in the art will recognize those nitrogen containing heterocycles which can form N-oxides. A person skilled in the art will also recognize

that tertiary amines can form N-oxides. Synthetic methods for the preparation of N-oxides of heterocycles and tertiary amines are well known to a person skilled in the art, and they include but are not limited to the oxidation of heterocycles and tertiary amines with peroxy acids such as peracetic acid and m-chloroperbenzoic acid (MCPBA), hydrogen peroxide, alkyl hydroperoxides such as tert-butyl hydroperoxide, sodium perborate, and dioxiranes such as dimethyldioxirane. These methods for the preparation of N-oxides have been extensively described and reviewed in literatures, see e.g., T. L. Gilchrist, Comprehensive Organic Synthesis, vol. 7, pp 748-750; A. R. Katritzky and A. J. Boulton, Eds., Academic Press; and G. W. H. Cheeseman and E. S. G. Werstiuk, Advances in Heterocyclic Chemistry, vol. 22, pp 390-392, A. R. Katritzky and A. J. Boulton, Eds., Academic Press.

The metabolite of the compound of the present invention, namely a substance formed *in vivo* upon administration of the compound of the present invention, is also included within the scope of the present invention. Such a product may result e.g., from the oxidation, reduction, hydrolysis, amidation, deamidation, esterification, enzymolysis, and the like, of the administered compound. Accordingly, the present invention encompasses the metabolite of the compound of the present invention, including a compound produced by a method comprising contacting the compound of the present invention with a mammal for a period of time sufficient to result in a metabolic product thereof.

Also within the scope of the present invention is a prodrug of the compound of the invention, which is certain derivative of the compound of the invention that may have little or no pharmacological activity itself, but can, when administered into or onto the body, be converted into the compound of the invention having the desired activity, for example, by hydrolytic cleavage. In general, such prodrug will be a functional derivative of the compound which is readily converted *in vivo* into the compound with desired therapeutic activity. Further information on the use of the prodrug may be found in "Pro-drugs as Novel Delivery Systems, Vol. 14, ACS Symposium Series (T. Higuchi and V Stella). The prodrug in accordance with the invention can, for example, be produced by replacing appropriate functionalities present in the compound of the present invention with certain moieties known to those skilled in the art as "pro-moieties" as described, for example, in "Design of Prodrugs" by H. Bundgaard (Elsevier, 1985).

The present invention further encompasses the compound of the present invention having a protecting group. During any of the processes for preparation of the compound of the present invention, it may be necessary and /or desirable to protect sensitive or reactive groups on any of the molecules concerned, thereby resulting in the chemically protected form of the compound of the present invention. This may be achieved by means of conventional protecting groups, e.g., those described in T. W. Greene & P. G. M. Wuts, Protective Groups in Organic Synthesis, John Wiley & Sons, 1991, which is incorporated herein by reference. The protecting groups may be removed at a convenient subsequent stage using methods known from the art.

The term "about" refers to a range within ±10%, preferably within ±5%, and more preferably within ±2% of the specified value.

## Compound

In some embodiments, the present invention provides a compound of Formula I' or a pharmaceutically acceptable salt, ester, stereoisomer, tautomer, polymorph, cocrystal, solvate, metabolite, isotopically labeled compound, N-oxide or prodrug thereof:

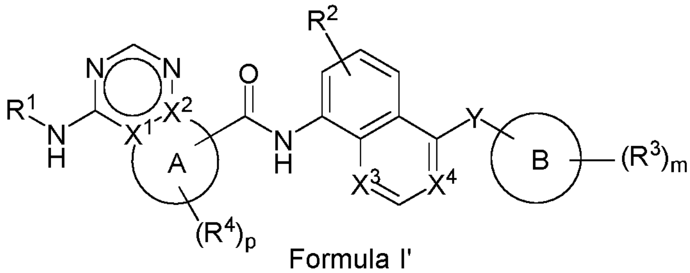

Formula I'

wherein:

ring A is selected from the group consisting of benzene ring and 5-6-membered heteroaromatic ring;
ring B is selected from the group consisting of $C_{6-10}$ aromatic ring, 5-10-membered heteroaromatic ring and 4-10-membered heterocycle;
$X^1$ and $X^2$ are each independently selected from the group consisting of C and N;
$X^3$ and $X^4$ are each independently selected from the group consisting of CH and N;
Y is selected from the group consisting of -O-, -NH-, -C(=O)-, $-CR^5R^6-$, $-CR^5R^6O-$ and $-CR^5R^6NH-$; provided that, when ring A is a thiophene ring, Y is not -O- or -NH-;

$R^1$ is selected from the group consisting of H, $C_{1-6}$ alkyl and $C_{3-6}$ cycloalkyl, the alkyl and cycloalkyl are each optionally substituted with one or more halogens;
$R^2$ is selected from the group consisting of H, halogen, $C_{1-6}$ alkyl and $C_{1-6}$ alkoxy, the alkyl and alkoxy are each optionally substituted with one or more halogens;
$R^3$ is L-$R^{3'}$;
L, at each occurrence, is each independently a direct bond or $-(CH_2)_n-$;
$R^{3'}$, at each occurrence, is each independently selected from the group consisting of H, hydroxyl, halogen, CN, NOz, $C_{1-6}$ alkyl, $C_{1-6}$ heteroalkyl (e.g., $C_{1-6}$ alkoxy), $C_{2-6}$ alkenyl, $C_{2-6}$ alkynyl, $C_{3-8}$ cycloalkyl, $C_{3-8}$ cycloalkoxy, 4-10-membered heterocyclyl, $C_{6-12}$ aryl, 5-10-membered heteroaryl, $-NR^{20a}R^{20b}$, $-SR^{21}$, - $S(=O)_2R^{22}$, $-S(=O)_2NR^{20a}R^{20b}$, $-NR^{20a}S(=O)_2R^{20b}$, $-C(=O)R^{21}$, $-C(=O)NR^{23a}R^{23b}$, $-NR^{23a}C(=O)R^{23b}$ and - $NR^{24a}C(=O)NR^{25a}R^{25b}$, the alkyl, heteroalkyl (e.g., alkoxy), alkenyl, alkynyl, cycloalkyl, cycloalkoxy, heterocyclyl, aryl and heteroaryl are each optionally substituted with one or more substituents independently selected from the group consisting of: hydroxyl, halogen, CN, NOz, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl and 4-10-membered heterocyclyl; or when L is a direct bond, and m is greater than 1, two $R^{3'}$ together with the group to which they are attached form a 4-10-membered heterocycle;
$R^4$, at each occurrence, is each independently selected from the group consisting of H, hydroxyl, halogen, CN, NOz, $C_{1-6}$ alkyl, $C_{1-6}$ heteroalkyl (e.g., $C_{1-6}$ alkoxy), $C_{2-6}$ alkenyl, $C_{2-6}$ alkynyl, $C_{3-8}$ cycloalkyl, $C_{3-8}$ cycloalkoxy, 4-10-membered heterocyclyl, $C_{6-12}$ aryl, 5-10-membered heteroaryl, $-NR^{20a}R^{20b}$, $-SR^{21}$, - $S(=O)_2R^{22}$, $-S(=O)_2NR^{20a}R^{20b}$, $-NR^{20a}S(=O)_2R^{20b}$, $-C(=O)R^{21}$, $-C(=O)NR^{23a}R^{23b}$, $-NR^{23a}C(=O)R^{23b}$ and - $NR^{24a}C(=O)NR^{25a}R^{25b}$, the alkyl, heteroalkyl (e.g., alkoxy), alkenyl, alkynyl, cycloalkyl, cycloalkoxy, heterocyclyl, aryl and heteroaryl are each optionally substituted with one or more substituents independently selected from the group consisting of: hydroxyl, halogen, CN, $NH_2$, NOz, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl and 4-10-membered heterocyclyl;
$R^5$ and $R^6$ are each independently selected from the group consisting of H, hydroxyl, halogen, $-NH_2$, - $NHCH_3$, $-N(CH_3)_2$, CN, $C_{1-6}$ alkyl, $C_{1-6}$ heteroalkyl (e.g., $C_{1-6}$ alkoxy), $C_{3-8}$ cycloalkyl, $C_{3-8}$ cycloalkoxy and 4-10-membered heterocyclyl, or $R^5$ and $R^6$ together with the atom to which they are attached form $C_{3-8}$ cycloalkyl or 3-8-membered heterocyclyl, the alkyl, heteroalkyl (e.g., alkoxy), cycloalkyl, cycloalkoxy and heterocyclyl are each optionally substituted with one or more halogens;
$R^{20a}$, $R^{20b}$, $R^{23a}$, $R^{23b}$, $R^{24a}$, $R^{25a}$ and $R^{25b}$ are each independently selected from the group consisting of H, OH, $-NHCH_3$, $-N(CH_3)_2$, $C_{1-6}$ alkyl, $C_{1-6}$ alkoxy, $C_{3-8}$ cycloalkyl and 4-10-membered heterocyclyl; the alkyl, alkoxy, cycloalkyl and heterocyclyl are each optionally substituted with one or more substituents independently selected from the group consisting of: halogen, $C_{1-6}$ alkyl and 4-10-membered heterocyclyl;
$R^{21}$ and $R^{22}$ are each independently selected from the group consisting of $C_{1-6}$ alkyl, $C_{1-6}$ alkoxy, $C_{3-8}$ cycloalkyl and 4-10-membered heterocyclyl, the alkyl, alkoxy, cycloalkyl and heterocyclyl are each optionally substituted with one or more halogens;
m is 0, 1, 2, 3, 4 or 5;
n is 1 or 2; and
p is 0, 1, 2 or 3.

In some embodiments, the compound of the present invention has the structure of Formula I'-A:

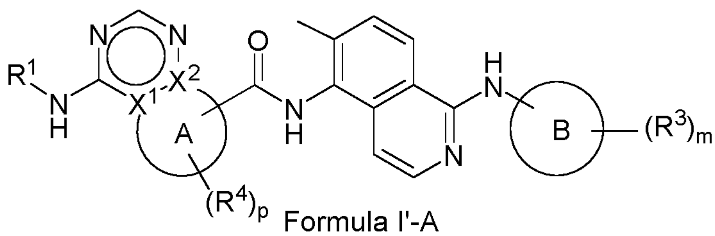

Formula I'-A

wherein:
each group is as defined above for Formula I'.

In certain embodiments, in the compound of Formula I' or Formula I'-A provided in the present invention, $R^4$, at each occurrence, is each independently selected from the group consisting of H, hydroxyl, halogen, CN, NOz, $C_{1-4}$ alkyl, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-6-membered heterocyclyl, $C_{6-10}$ aryl, 5-6-membered heteroaryl, $-NR^{20a}R^{20b}$, $-SR^{21}$, - $S(=O)_2R^{22}$, $-S(=O)_2NR^{20a}R^{20b}$, $-NR^{20a}S(=O)_2R^{20b}$, $-C(=O)R^{21}$, $-C(=O)NR^{23a}R^{23b}$, $-NR^{23a}C(=O)R^{23b}$ and - $NR^{24a}C(=O)NR^{25a}R^{25b}$, the alkyl, heteroalkyl (e.g., alkoxy), alkenyl, alkynyl, cycloalkyl, cycloalkoxy, heterocyclyl, aryl and heteroaryl are each optionally substituted

with one or more substituents independently selected from the group consisting of: hydroxyl, halogen, CN, $NH_2$, NOz, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl and 4-6-membered heterocyclyl.

In certain embodiments, in the compound of Formula I' or Formula I'-A provided in the present invention, $R^4$, at each occurrence, is each independently selected from the group consisting of H, hydroxyl, halogen, CN, NOz, $C_{1-4}$ alkyl, $C_{1-4}$ heteroalkyl (e.g., $C_{1-4}$ alkoxy), $C_{3-6}$ cycloalkyl, $C_{3-6}$ cycloalkoxy, 4-6-membered heterocyclyl, 5-6-membered heteroaryl, $-NR^{20a}R^{20b}$, $-S(=O)_2NR^{20a}R^{20b}$, $-NR^{20a}S(=O)_2R^{20b}$, - $C(=O)R^{21}$, $-C(=O)NR^{23a}R^{23b}$ and $-NR^{23a}C(=O)R^{23b}$, the alkyl, heteroalkyl (e.g., alkoxy), cycloalkyl, cycloalkoxy, heterocyclyl and heteroaryl are each optionally substituted with one or more substituents independently selected from the group consisting of: hydroxyl, halogen, $NH_2$, $C_{1-3}$ alkyl, $C_{1-3}$ haloalkyl, $C_{1-3}$ haloalkoxy and 4-6-membered heterocyclyl.

In certain embodiments, in the compound of Formula I' or Formula I'-A provided in the present invention, $R^4$, at each occurrence, is each independently selected from the group consisting of H, hydroxyl, halogen, CN, NOz, $C_{1-4}$ alkyl, $C_{1-4}$ heteroalkyl (e.g., $C_{1-4}$ alkoxy), $C_{3-6}$ cycloalkyl, $C_{3-6}$ cycloalkoxy, 4-6-membered heterocyclyl, 5-6-membered heteroaryl, $-NR^{20a}R^{20b}$, $-S(=O)_2NR^{20a}R^{20b}$, $-NR^{20a}S(=O)_2R^{20b}$, - $C(=O)R^{21}$, $-C(=O)NR^{23a}R^{23b}$ and $-NR^{23a}C(=O)R^{23b}$, the alkyl, heteroalkyl (e.g., alkoxy), cycloalkyl, cycloalkoxy, heterocyclyl and heteroaryl are each optionally substituted with one or more substituents independently selected from the group consisting of: hydroxyl, halogen, $NH_2$, $C_{1-3}$ alkyl, $C_{1-3}$ haloalkyl and $C_{1-3}$ haloalkoxy.

In certain embodiments, in the compound of Formula I' or Formula I'-A provided in the present invention, $R^4$, at each occurrence, is each independently selected from the group consisting of H, hydroxyl, halogen, $C_{1-4}$ alkyl, $C_{1-4}$ heteroalkyl (e.g., $C_{1-4}$ alkoxy), 4-6-membered heterocyclyl, 5-6-membered heteroaryl and $-NR^{20a}R^{20b}$, the alkyl, heteroalkyl (e.g., alkoxy), heterocyclyl and heteroaryl are each optionally substituted with one or more substituents independently selected from the group consisting of: hydroxyl, halogen, $NH_2$, $C_{1-3}$ alkyl, $C_{1-3}$ haloalkyl, $C_{1-3}$ haloalkoxy and 4-6-membered heterocyclyl.

In certain embodiments, in the compound of Formula I' or Formula I'-A provided in the present invention, $R^4$, at each occurrence, is each independently selected from the group consisting of H, halogen, $C_{1-4}$ alkyl, $C_{1-4}$ heteroalkyl (e.g., $C_{1-4}$ alkoxy), 4-6-membered heterocyclyl, 5-6-membered heteroaryl and - $NR^{20a}R^{20b}$, the alkyl, heteroalkyl (e.g., alkoxy), heterocyclyl and heteroaryl are each optionally substituted with one or more substituents independently selected from the group consisting of: hydroxyl, halogen, $NH_2$, $C_{1-3}$ alkyl, $C_{1-3}$ haloalkyl and $C_{1-3}$ haloalkoxy.

In certain embodiments, in the compound of Formula I' or Formula I'-A provided in the present invention, $R^4$, at each occurrence, is each independently selected from the group consisting of hydroxyl, F, methyl, $-CH_2CH_2NH_2$,

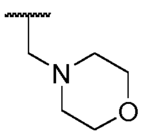, 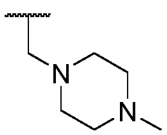, 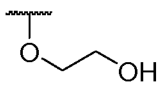, 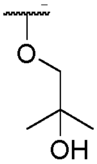, 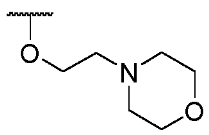,

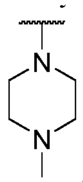, 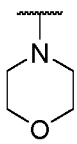, 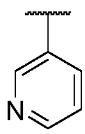, 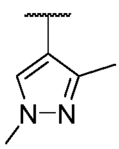, 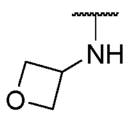 and 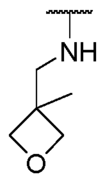.

In certain embodiments, in the compound of Formula I' or Formula I'-A provided in the present invention $R^4$, at each occurrence, is each independently selected from the group consisting of hydroxyl, F, methyl, $-CH_2CH_2NH_2$,

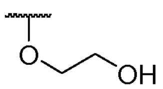, 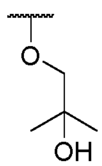, 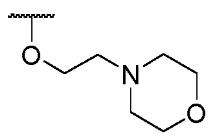, 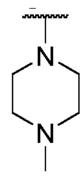, 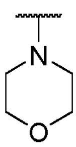, 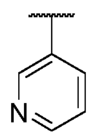,

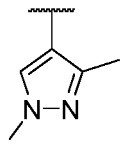, 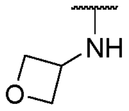 and 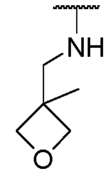.

In certain embodiments, in the compound of Formula I' or Formula I'-A provided in the present invention, $R^4$, at each occurrence, is each independently selected from the group consisting of F, methyl, - $CH_2CH_2NH_2$,

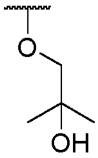, 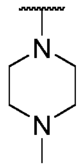, 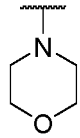, 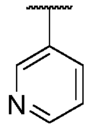, 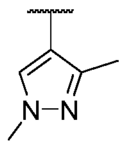, 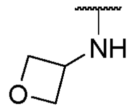 and 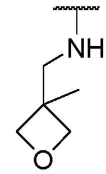.

In certain embodiments, in the compound of Formula I' or Formula I'-A provided in the present invention, p is 0 or 1.

In some embodiments, the present invention provides a compound of Formula I or a pharmaceutically acceptable salt, ester, stereoisomer, tautomer, polymorph, cocrystal, solvate, metabolite, isotopically labeled compound, N-oxide or prodrug thereof:

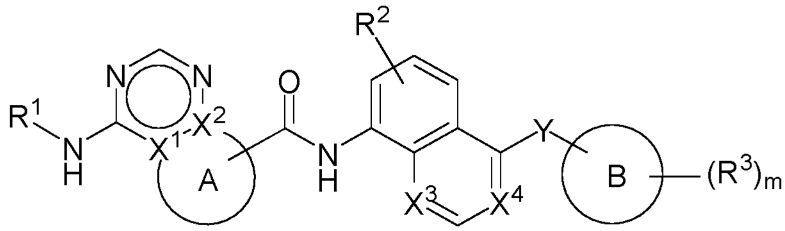

Formula I

wherein:

ring A is selected from the group consisting of benzene ring and 5-6-membered heteroaromatic ring;
ring B is selected from the group consisting of $C_{6-10}$ aromatic ring, 5-10-membered heteroaromatic ring and 4-10-membered heterocycle;
$X^1$ and $X^2$ are each independently selected from the group consisting of C and N;
$X^3$ and $X^4$ are each independently selected from the group consisting of CH and N;
Y is selected from the group consisting of -O-, -NH-, -C(=O)-, $-CR^5R^6-$, $-CR^5R^6O-$ and $-CR^5R^6NH-$; provided that, when ring A is a thiophene ring, Y is not -O- or -NH-;
$R^1$ is selected from the group consisting of H, $C_{1-6}$ alkyl and $C_{3-6}$ cycloalkyl, the alkyl and cycloalkyl are each optionally substituted with one or more halogens;
$R^2$ is selected from the group consisting of H, halogen, $C_{1-6}$ alkyl and $C_{1-6}$ alkoxy, the alkyl and alkoxy are each optionally substituted with one or more halogens;
$R^3$ is $L-R^{3'}$;
L, at each occurrence, is each independently a direct bond or $-(CH_2)_n-$;
$R^{3'}$, at each occurrence, is each independently selected from the group consisting of H, hydroxyl, halogen, CN, NOz, $C_{1-6}$ alkyl, $C_{1-6}$ heteroalkyl (e.g., $C_{1-6}$ alkoxy), $C_{2-6}$ alkenyl, $C_{2-6}$ alkynyl, $C_{3-8}$ cycloalkyl, $C_{3-8}$ cycloalkoxy, 4-10-membered heterocyclyl, $C_{6-12}$ aryl, 5-10-membered heteroaryl, $-NR^{20a}R^{20b}$, $-SR^{21}$, - $S(=O)_2R^{22}$, $-S(=O)_2NR^{20a}R^{20b}$, $-NR^{20a}S(=O)_2R^{20b}$, $-C(=O)R^{21}$, $-C(=O)NR^{23a}R^{23b}$, $-NR^{23a}C(=O)R^{23b}$ and - $NR^{24a}C(=O)NR^{25a}R^{25b}$, the alkyl, heteroalkyl (e.g., alkoxy), alkenyl, alkynyl, cycloalkyl, cycloalkoxy, heterocyclyl, aryl and heteroaryl are each optionally substituted with one or more substituents independently selected from the group consisting of: hydroxyl, halogen, CN, NOz, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl and 4-10-membered heterocyclyl; or when L is a direct bond, and m is greater than 1, two $R^{3'}$ together with the group to which they are attached form a 4-10-membered heterocycle;

$R^5$ and $R^6$ are each independently selected from the group consisting of H, hydroxyl, halogen, $-NH_2$, $-NHCH_3$, $-N(CH_3)_2$, CN, $C_{1-6}$ alkyl, $C_{1-6}$ heteroalkyl (e.g., $C_{1-6}$ alkoxy), $C_{3-8}$ cycloalkyl, $C_{3-8}$ cycloalkoxy and 4-10-membered heterocyclyl, or $R^5$ and $R^6$ together with the atom to which they are attached form $C_{3-8}$ cycloalkyl or 3-8-membered heterocyclyl, the alkyl, heteroalkyl (e.g., alkoxy), cycloalkyl, cycloalkoxy and heterocyclyl are each optionally substituted with one or more halogens;

$R^{20a}$, $R^{20b}$, $R^{23a}$, $R^{23b}$, $R^{24a}$, $R^{25a}$ and $R^{25b}$ are each independently selected from the group consisting of H, OH, $-NHCH_3$, $-N(CH_3)_2$, $C_{1-6}$ alkyl, $C_{1-6}$ alkoxy, $C_{3-8}$ cycloalkyl and 4-10-membered heterocyclyl; the alkyl, alkoxy, cycloalkyl and heterocyclyl are each optionally substituted with one or more substituents independently selected from the group consisting of: halogen, $C_{1-6}$ alkyl and 4-10-membered heterocyclyl;

$R^{21}$ and $R^{22}$ are each independently selected from the group consisting of $C_{1-6}$ alkyl, $C_{1-6}$ alkoxy, $C_{3-8}$ cycloalkyl and 4-10-membered heterocyclyl, the alkyl, alkoxy, cycloalkyl and heterocyclyl are each optionally substituted with one or more halogens;

m is 0, 1, 2, 3, 4 or 5; and

n is 1 or 2.

In some embodiments, the compound of the present invention has the structure of Formula I-A:

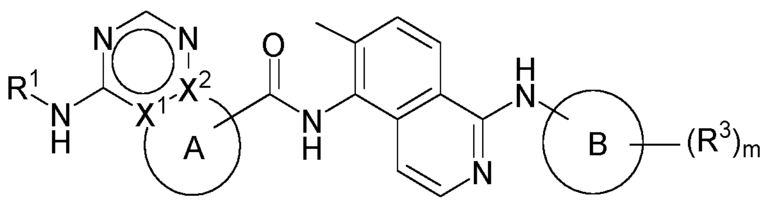

I-A

wherein:

each group is as defined above for Formula I;

In some embodiments, the compound of the present invention has the structure of Formula I-B:

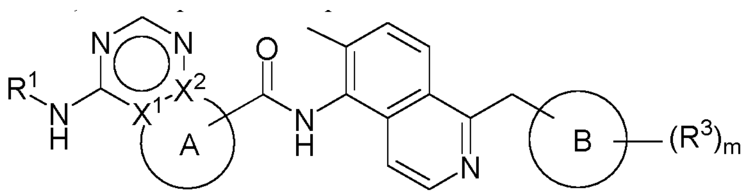

I-B

wherein:

each group is as defined above for Formula I;

In some embodiments, the compound of the present invention has the structure of Formula I-C:

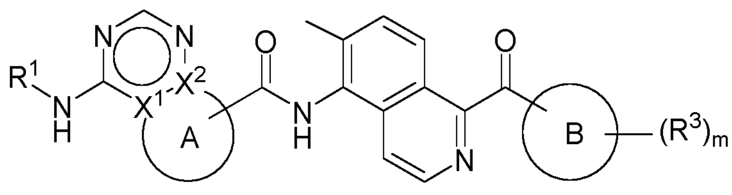

I-C

wherein:

each group is as defined above for Formula I;

In some embodiments, the compound of the present invention has the structure of Formula I-D:

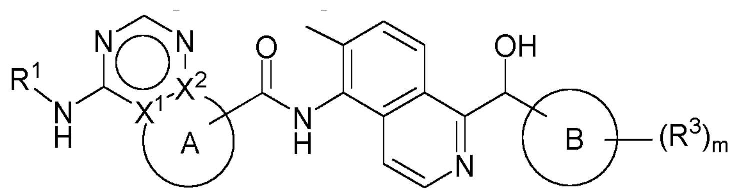

I-D

wherein:
each group is as defined above for Formula I.

In certain embodiments, in the compound of Formula I', Formula I'-A, Formula I, Formula I-A to Formula I-D provided in the present invention, ring A is a benzene ring, thiophene ring, pyrrole ring, pyrazole ring, imidazole ring or pyridine ring.

In certain embodiments, in the compound of Formula I', Formula I'-A, Formula I, Formula I-A to Formula I-D provided in the present invention, ring A is a benzene ring, thiophene ring, pyrazole ring, imidazole ring or pyridine ring.

In certain embodiments, in the compound of Formula I', Formula I'-A, Formula I, Formula I-A to Formula I-D provided in the present invention, ring A is a benzene ring, thiophene ring, pyrrole ring, imidazole ring or pyridine ring.

In certain embodiments, in the compound of Formula I', Formula I'-A, Formula I, Formula I-A to Formula I-D provided in the present invention, ring A is a benzene ring, thiophene ring, imidazole ring or pyridine ring.

In certain embodiments, in the compound of Formula I', Formula I'-A, Formula I, Formula I-A to Formula I-D provided in the present invention, ring A is a benzene ring or 5-membered heteroaromatic ring; preferably, ring A is a benzene ring, thiophene ring, pyrrole ring, pyrazole ring or imidazole ring; more preferably, ring A is a benzene ring, thiophene ring, pyrrole ring or imidazole ring.

In certain embodiments, in the compound of Formula I', Formula I'-A, Formula I, Formula I-A to Formula I-D provided in the present invention, ring A is a benzene ring or 5-membered heteroaromatic ring; preferably, ring A is a benzene ring, thiophene ring, pyrazole ring or imidazole ring; more preferably, ring A is a benzene ring, thiophene ring or imidazole ring.

In certain embodiments, in the compound of Formula I', Formula I'-A, Formula I, Formula I-A to Formula I-D provided in the present invention, ring B is a $C_{6-10}$ aromatic ring or 5-10-membered heteroaromatic ring; preferably, ring B is a $C_6$ aromatic ring or 6-membered heteroaromatic ring; more preferably, ring B is a benzene ring or pyridine ring.

In certain embodiments, in the compound of Formula I', Formula I'-A, Formula I, Formula I-A to Formula I-D provided in the present invention, $X^1$ is C and $X^2$ is C; or $X^1$ is C and $X^2$ is N; or $X^1$ is N and $X^2$ is C; preferably, $X^1$ is C and $X^2$ is C; or $X^1$ is C and $X^2$ is N.

In certain embodiments, in the compound of Formula I' or Formula I provided in the present invention, $X^3$ is CH and $X^4$ is N.

In certain embodiments, in the compound of Formula I' or Formula I provided in the present invention, Y is selected from the group consisting of -NH-, -C(=O)-, $-CR^5R^6-$, $-CR^5R^6O-$ and $-CR^5R^6NH-$; provided that, when ring A is a thiophene ring, Y is not -NH-.

In certain embodiments, in the compound of Formula I' or Formula I provided in the present invention, Y is selected from the group consisting of -NH-, -C(=O)-, $-CH_2-$, -CHOH- and $-CH(CH_3)O-$; provided that, when ring A is a thiophene ring, Y is not -NH-.

In certain embodiments, in the compound of Formula I' or Formula I provided in the present invention, Y is selected from the group consisting of -NH-, -C(=O)-, $-CH_2-$ and -CHOH-; provided that, when ring A is a thiophene ring, in the compound of Formula I' or Formula I provided in the present invention, Y is not - NH-.

In certain embodiments, in the compound of Formula I', Formula I'-A, Formula I, Formula I-A to Formula I-D provided in the present invention, $R^1$ is H or $C_{1-3}$ alkyl; preferably, $R^1$ is H.

In certain embodiments, in the compound of Formula I' or Formula I provided in the present invention, $R^2$ is selected from the group consisting of H, halogen, $C_{1-3}$ alkyl and $C_{1-3}$ alkoxy, the alkyl and alkoxy are each optionally substituted with one or more halogens; preferably, $R^2$ is selected from the group consisting of halogen and $C_{1-3}$ alkyl; preferably, $R^2$ is F or $-CH_3$; more preferably, $R^2$ is $-CH_3$.

In certain embodiments, in the compound of Formula I', Formula I'-A, Formula I, Formula I-A to Formula I-D provided in the present invention, L, at each occurrence, is each independently a direct bond or $-CH_2-$.

In certain embodiments, in the compound of Formula I', Formula I'-A, Formula I, Formula I-A to Formula I-D provided in the present invention, $R^{3'}$, at each occurrence, is each independently selected from the group consisting of H, hydroxyl, halogen, CN, NOz, $C_{1-4}$ alkyl, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-6-membered heterocyclyl, $C_{6-10}$ aryl, 5-6-membered heteroaryl, $-NR^{20a}R^{20b}$, $-SR^{21}$, $-S(=O)_2R^{22}$, $-S(=O)_2NR^{20a}R^{20b}$, $-NR^{20a}S(=O)_2R^{20b}$, $-C(=O)R^{21}$, $-C(=O)NR^{23a}R^{23b}$, $-NR^{23a}C(=O)R^{23b}$ and $-NR^{24a}C(=O)NR^{25a}R^{25b}$,

the alkyl, heteroalkyl (e.g., alkoxy), alkenyl, alkynyl, cycloalkyl, cycloalkoxy, heterocyclyl, aryl and heteroaryl are each optionally substituted with one or more substituents independently selected from the group consisting of: hydroxyl, halogen, CN, NOz, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl and 4-6-membered heterocyclyl.

In certain embodiments, in the compound of Formula I', Formula I'-A, Formula I, Formula I-A to Formula I-D provided in the present invention, $R^{3'}$, at each occurrence, is each independently selected from the group consisting of H, hydroxyl, halogen, CN, NOz, $C_{1-4}$ alkyl, $C_{1-4}$ heteroalkyl (e.g., $C_{1-4}$ alkoxy), $C_{3-6}$ cycloalkyl, $C_{3-6}$ cycloalkoxy, 4-6-membered heterocyclyl, $C_{6-10}$ aryl, 5-6-membered heteroaryl, $-NR^{20a}R^{20b}$, $-S(=O)_2R^{22}$, $-S(=O)_2NR^{20a}R^{20b}$, $-NR^{20a}S(=O)_2R^{20b}$, $-C(=O)R^{21}$, $-C(=O)NR^{23a}R^{23b}$ and $-NR^{23a}C(=O)R^{23b}$, the alkyl, heteroalkyl (e.g., alkoxy), cycloalkyl, cycloalkoxy, heterocyclyl, aryl and heteroaryl are each optionally substituted with one or more substituents independently selected from the group consisting of: hydroxyl, halogen, CN, NOz, $C_{1-3}$ alkyl, $C_{1-3}$ haloalkyl, $C_{1-3}$ hydroxyalkyl, $C_{1-3}$ haloalkoxy, $C_{1-3}$ heteroalkyl (e.g., $C_{1-3}$ alkoxy), $C_{3-6}$ cycloalkyl and 4-6-membered heterocyclyl.

In certain embodiments, in the compound of Formula I', Formula I'-A, Formula I, Formula I-A to Formula I-D provided in the present invention, $R^{3'}$, at each occurrence, is each independently selected from the group consisting of H, hydroxyl, halogen, CN, NOz, $C_{1-4}$ alkyl, $C_{1-4}$ heteroalkyl (e.g., $C_{1-4}$ alkoxy), $C_{3-6}$ cycloalkyl, $C_{3-6}$ cycloalkoxy, 4-6-membered heterocyclyl, $C_{6-10}$ aryl, 5-6-membered heteroaryl, $-NR^{20a}R^{20b}$, $-S(=O)_2NR^{20a}R^{20b}$, $-NR^{20a}S(=O)_2R^{20b}$, $-C(=O)R^{21}$, $-C(=O)NR^{23a}R^{23b}$ and $-NR^{23a}C(=O)R^{23b}$, the alkyl, heteroalkyl (e.g., alkoxy), cycloalkyl, cycloalkoxy, heterocyclyl, aryl and heteroaryl are each optionally substituted with one or more substituents independently selected from the group consisting of: hydroxyl, halogen, CN, NOz, $C_{1-3}$ alkyl, $C_{1-3}$ haloalkyl, $C_{1-3}$ hydroxyalkyl, $C_{1-3}$ haloalkoxy, $C_{1-3}$ heteroalkyl (e.g., $C_{1-3}$ alkoxy), $C_{3-6}$ cycloalkyl and 4-6-membered heterocyclyl.

In certain embodiments, in the compound of Formula I', Formula I'-A, Formula I, Formula I-A to Formula I-D provided in the present invention, $R^{3'}$, at each occurrence, is each independently selected from the group consisting of H, hydroxyl, halogen, CN, NOz, $C_{1-4}$ alkyl, $C_{1-4}$ heteroalkyl (e.g., $C_{1-4}$ alkoxy), $C_{3-6}$ cycloalkyl, $C_{3-6}$ cycloalkoxy, 4-6-membered heterocyclyl, $-NR^{20a}R^{20b}$, $-S(=O)_2R^{22}$, $-S(=O)_2NR^{20a}R^{20b}$, $-NR^{20a}S(=O)_2R^{20b}$, $-C(=O)R^{21}$, $-C(=O)NR^{23a}R^{23b}$ and $-NR^{23a}C(=O)R^{23b}$, the alkyl, heteroalkyl (e.g., alkoxy), cycloalkyl, cycloalkoxy and heterocyclyl are each optionally substituted with one or more substituents independently selected from the group consisting of: hydroxyl, halogen, $C_{1-3}$ alkyl, $C_{1-3}$ haloalkyl, $C_{1-3}$ haloalkoxy, $C_{1-3}$ heteroalkyl and 4-6-membered heterocyclyl.

In certain embodiments, in the compound of Formula I', Formula I'-A, Formula I, Formula I-A to Formula I-D provided in the present invention, $R^{3'}$, at each occurrence, is each independently selected from the group consisting of H, hydroxyl, halogen, CN, NOz, $C_{1-4}$ alkyl, $C_{1-4}$ heteroalkyl (e.g., $C_{1-4}$ alkoxy), $C_{3-6}$ cycloalkyl, $C_{3-6}$ cycloalkoxy, 4-6-membered heterocyclyl, $-NR^{20a}R^{20b}$, $-S(=O)_2NR^{20a}R^{20b}$, $-NR^{20a}S(=O)_2R^{20b}$, $-C(=O)R^{21}$, $-C(=O)NR^{23a}R^{23b}$ and $-NR^{23a}C(=O)R^{23b}$, the alkyl, heteroalkyl (e.g., alkoxy), cycloalkyl, cycloalkoxy and heterocyclyl are each optionally substituted with one or more substituents independently selected from the group consisting of: hydroxyl, halogen, $C_{1-3}$ alkyl, $C_{1-3}$ haloalkyl and $C_{1-3}$ haloalkoxy.

In certain embodiments, in the compound of Formula I', Formula I'-A, Formula I, Formula I-A to Formula I-D provided in the present invention, $R^{3'}$, at each occurrence, is each independently selected from the group consisting of H, halogen, CN, $C_{1-4}$ alkyl, $C_{1-4}$ heteroalkyl (e.g., $C_{1-4}$ alkoxy), $-NR^{20a}R^{20b}$, $- S(=O)_2R^{22}$ and $C(=O)R^{21}$, the alkyl and heteroalkyl (e.g., alkoxy) are each optionally substituted with one or more substituents independently selected from the group consisting of halogen, $C_{1-3}$ heteroalkyl and 4-6-membered heterocyclyl.

In certain embodiments, in the compound of Formula I', Formula I'-A, Formula I, Formula I-A to Formula I-D provided in the present invention, $R^{3'}$, at each occurrence, is each independently selected from the group consisting of H, halogen, CN, $C_{1-4}$ alkyl, $C_{1-4}$ heteroalkyl (e.g., $C_{1-4}$ alkoxy), $-NR^{20a}R^{20b}$ and $C(=O)R^{21}$, the alkyl and heteroalkyl (e.g., alkoxy) are each optionally substituted with one or more halogens.

In certain embodiments, in the compound of Formula I', Formula I'-A, Formula I, Formula I-A to Formula I-D provided in the present invention, $R^{3'}$, at each occurrence, is each independently selected from the group consisting of H, halogen, $C_{1-4}$ alkyl, $C_{1-4}$ heteroalkyl (e.g., $C_{1-4}$ alkoxy) and $-NR^{20a}R^{20b}$, the alkyl and heteroalkyl (e.g., alkoxy) are each optionally substituted with one or more halogens.

In certain embodiments, in the compound of Formula I', Formula I'-A, Formula I, Formula I-A to Formula I-D provided in the present invention, $R^{3'}$, at each occurrence, is each independently F, Cl, Br, CN, methyl, trifluoromethyl, methoxy, ethoxy, $-C(=O)CH_3$, $-N(CH_3)_2$, $-S(=O)_2CH_3$,

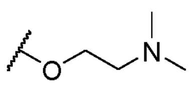
,

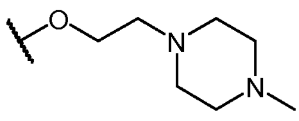, 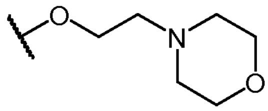 or 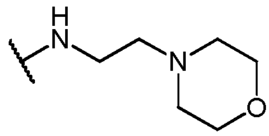.

In certain embodiments, in the compound of Formula I', Formula I'-A, Formula I, Formula I-A to Formula I-D provided in the present invention, $R^{3'}$, at each occurrence, is each independently F, Cl, Br, CN, methyl, trifluoromethyl, methoxy, ethoxy, $-C(=O)CH_3$, $-N(CH_3)_2$, $-S(=O)_2CH_3$,

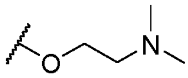,

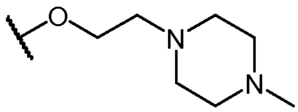 or 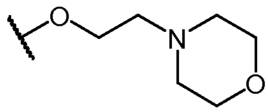.

In certain embodiments, in the compound of Formula I', Formula I'-A, Formula I, Formula I-A to Formula I-D provided in the present invention, $R^{3'}$, at each occurrence, is each independently F, Cl, CN, methyl, trifluoromethyl. methoxy, ethoxy, $-C(=O)CH_3$, $-N(CH_3)_2$ or

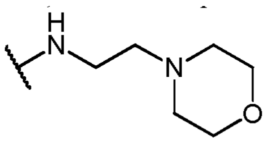.

In certain embodiments, in the compound of Formula I', Formula I'-A, Formula I, Formula I-A to Formula I-D provided in the present invention, $R^{3'}$, at each occurrence, is each independently F, Cl, trifluoromethyl, methoxy, $-N(CH_3)_2$ or

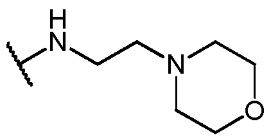.

In certain embodiments, when L is a direct bond, and m is greater than 1, any two $R^{3'}$ together with the group to which they are attached form a 4-6-membered heterocycle, preferably, together form

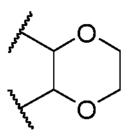

or

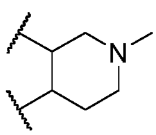.

In certain embodiments, in the compound of Formula I' or Formula I provided in the present invention, $R^5$ and $R^6$ are each independently selected from the group consisting of H, hydroxyl, $-NH_2$, $-NHCH_3$, $-N(CH_3)_2$, CN, $C_{1-4}$ alkyl,

$C_{1-4}$ heteroalkyl (e.g., $C_{1-4}$ alkoxy) and $C_{3-6}$ cycloalkyl, or $R^5$ and $R^6$ together with the atom to which they are attached form $C_{3-6}$ cycloalkyl or 3-6-membered heterocyclyl, the alkyl, heteroalkyl (e.g., alkoxy), cycloalkyl and heterocyclyl are each optionally substituted with one or more halogens.

In certain embodiments, in the compound of Formula I' or Formula I provided in the present invention, $R^5$ and $R^6$ are each independently selected from the group consisting of H, hydroxyl, $-NH_2$ and $C_{1-4}$ alkyl, or $R^5$ and $R^6$ together with the atom to which they are attached form $C_{3-6}$ cycloalkyl or 3-6-membered heterocyclyl, the alkyl, cycloalkyl and heterocyclyl are each optionally substituted with one or more halogens.

In certain embodiments, in the compound of Formula I' or Formula I provided in the present invention, $R^5$ and $R^6$ are each independently selected from the group consisting of H, hydroxyl and methyl.

In certain embodiments, in the compound of Formula I', Formula I'-A, Formula I, Formula I-A to Formula I-D provided in the present invention, $R^{20a}$, $R^{20b}$, $R^{23a}$, $R^{23b}$, $R^{24a}$, $R^{25a}$ and $R^{25b}$ are each independently selected from the group consisting of H, $-NHCH_3$, $-N(CH_3)_2$, $C_{1-4}$ alkyl, $C_{1-4}$ alkoxy, $C_{3-6}$ cycloalkyl and 4-6-membered heterocyclyl; the alkyl, alkoxy, cycloalkyl and heterocyclyl are each optionally substituted with one or more substituents independently selected from the group consisting of: halogen, $C_{1-6}$ alkyl and 4-6-membered heterocyclyl.

In certain embodiments, in the compound of Formula I', Formula I'-A, Formula I, Formula I-A to Formula I-D provided in the present invention, $R^{20a}$, $R^{20b}$, $R^{23a}$, $R^{23b}$, $R^{24a}$, $R^{25a}$ and $R^{25b}$ are each independently selected from the group consisting of H, $C_{1-4}$ alkyl, $C_{1-4}$ alkoxy and 4-6-membered heterocyclyl; the alkyl, alkoxy and heterocyclyl are each optionally substituted with one or more substituents independently selected from the group consisting of: halogen, $C_{1-6}$ alkyl and 4-6-membered heterocyclyl.

In certain embodiments, in the compound of Formula I', Formula I'-A, Formula I, Formula I-A to Formula I-D provided in the present invention, $R^{20a}$, $R^{20b}$, $R^{23a}$, $R^{23b}$, $R^{24a}$, $R^{25a}$ and $R^{25b}$ are each independently H, methyl, ethyl, propyl or oxetanyl, which are each optionally substituted with one or more substituents independently selected from the group consisting of: F, Cl, Br, methyl and oxetanyl.

In certain embodiments, in the compound of Formula I', Formula I'-A, Formula I, Formula I-A to Formula I-D provided in the present invention, $R^{21}$ and $R^{22}$ are each independently selected from the group consisting of $C_{1-4}$ alkyl, $C_{1-4}$ alkoxy and $C_{3-6}$ cycloalkyl, the alkyl, alkoxy and cycloalkyl are each optionally substituted with one or more halogens.

In certain embodiments, in the compound of Formula I', Formula I'-A, Formula I, Formula I-A to Formula I-D provided in the present invention, $R^{21}$ and $R^{22}$ are each independently $C_{1-4}$ alkyl. In preferred embodiments, $R^{21}$ and $R^{22}$ are each independently methyl.

In certain embodiments, in the compound of Formula I', Formula I'-A, Formula I, Formula I-A to Formula I-D provided in the present invention, m is 0, 1, 2 or 3.

In certain embodiments, in the compound of Formula I' or Formula I'-A provided in the present invention, $R^4$, at each occurrence, is each independently selected from the group consisting of H, hydroxyl, halogen, CN, NOz, $C_{1-4}$ alkyl, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-6-membered heterocyclyl, $C_{6-10}$ aryl, 5-6-membered heteroaryl, $-NR^{20a}R^{20b}$, $-SR^{21}$, $-S(=O)_2R^{22}$, $-S(=O)_2NR^{20a}R^{20b}$, $-NR^{20a}S(=O)_2R^{20b}$, $-C(=O)R^{21}$, $-C(=O)NR^{23a}R^{23b}$, $-NR^{23a}C(=O)R^{23b}$ and $-NR^{24a}C(=O)NR^{25a}R^{25b}$, the alkyl, heteroalkyl (e.g., alkoxy), alkenyl, alkynyl, cycloalkyl, cycloalkoxy, heterocyclyl, aryl and heteroaryl are each optionally substituted with one or more substituents independently selected from the group consisting of: hydroxyl, halogen, CN, $NH_2$, NOz, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl and 4-6-membered heterocyclyl;

$R^{20a}$, $R^{20b}$, $R^{23a}$, $R^{23b}$, $R^{24a}$, $R^{25a}$ and $R^{21b}$ are each independently selected from the group consisting of H, $-NHCH_3$, $-N(CH_3)_2$, $C_{1-4}$ alkyl, $C_{1-4}$ alkoxy, $C_{3-6}$ cycloalkyl and 4-6-membered heterocyclyl; the alkyl, alkoxy, cycloalkyl and heterocyclyl are each optionally substituted with one or more substituents independently selected from the group consisting of: halogen, $C_{1-6}$ alkyl and 4-6-membered heterocyclyl; and

$R^{21}$ and $R^{22}$ are each independently selected from the group consisting of $C_{1-4}$ alkyl, $C_{1-4}$ alkoxy and $C_{3-6}$ cycloalkyl, the alkyl, alkoxy and cycloalkyl are each optionally substituted with one or more halogens.

In certain embodiments, in the compound of Formula I' or Formula I'-A provided in the present invention, $R^4$, at each occurrence, is each independently selected from the group consisting of H, hydroxyl, halogen, CN, NOz, $C_{1-4}$ alkyl, $C_{1-4}$ heteroalkyl (e.g., $C_{1-4}$ alkoxy), $C_{3-6}$ cycloalkyl, $C_{3-6}$ cycloalkoxy, 4-6-membered heterocyclyl, 5-6-membered heteroaryl, $-NR^{20a}R^{20b}$, $-S(=O)_2NR^{20a}R^{20b}$, $-NR^{20a}S(=O)_2R^{20b}$, $-C(=O)R^{21}$, $-C(=O)NR^{23a}R^{23b}$ and $-NR^{23a}C(=O)R^{23b}$, the alkyl, heteroalkyl (e.g., alkoxy), cycloalkyl, cycloalkoxy, heterocyclyl and heteroaryl are each optionally substituted with one or more substituents independently selected from the group consisting of: hydroxyl, halogen, $NH_2$, $C_{1-3}$ alkyl, $C_{1-3}$ haloalkyl, $C_{1-3}$ haloalkoxy and 4-6-membered heterocyclyl;

$R^{20a}$, $R^{20b}$, $R^{23a}$ and $R^{23b}$ are each independently selected from the group consisting of H, $C_{1-4}$ alkyl, $C_{1-4}$ alkoxy and 4-6-membered heterocyclyl; the alkyl, alkoxy and heterocyclyl are each optionally substituted with one or more substituents independently selected from the group consisting of: halogen, $C_{1-6}$ alkyl and 4-6-membered heterocyclyl; and

$R^{21}$ is $C_{1-4}$ alkyl.

In certain embodiments, in the compound of Formula I' or Formula I'-A provided in the present invention, $R^4$, at each occurrence, is each independently selected from the group consisting of H, halogen, $C_{1-4}$ alkyl, $C_{1-4}$ heteroalkyl (e.g., $C_{1-4}$ alkoxy), hydroxyl, 4-6-membered heterocyclyl, 5-6-membered heteroaryl and $-NR^{20a}R^{20b}$, the alkyl, heteroalkyl (e.g., alkoxy), heterocyclyl and heteroaryl are each optionally substituted with one or more substituents independently selected from the group consisting of: hydroxyl, halogen, $NH_2$, $C_{1-3}$ alkyl, $C_{1-3}$ haloalkyl, $C_{1-3}$ haloalkoxy, 4-6-membered heterocyclyl; and

$R^{20a}$ and $R^{20b}$ are each independently H, methyl, ethyl, propyl or oxetanyl, which are each optionally substituted with one or more substituents independently selected from the group consisting of: F, Cl, Br, methyl and oxetanyl.

In certain embodiments, in the compound of Formula I' or Formula I'-A provided in the present invention, $R^4$, at each occurrence, is each independently selected from the group consisting of H, halogen, $C_{1-4}$ alkyl, $C_{1-4}$ heteroalkyl (e.g., $C_{1-4}$ alkoxy), 4-6-membered heterocyclyl, 5-6-membered heteroaryl and - $NR^{20a}R^{20b}$, the alkyl, heteroalkyl (e.g., alkoxy), heterocyclyl and heteroaryl are each optionally substituted with one or more substituents independently selected from the group consisting of: hydroxyl, halogen, $NH_2$, $C_{1-3}$ alkyl, $C_{1-3}$ haloalkyl and $C_{1-3}$ haloalkoxy; and

$R^{20a}$ and $R^{20b}$ are each independently H, methyl, ethyl, propyl or oxetanyl, which are each optionally substituted with one or more substituents independently selected from the group consisting of: F, Cl, Br, methyl and oxetanyl.

In certain embodiments, in the compound of Formula I', Formula I'-A, Formula I, Formula I-A to Formula I-D provided in the present invention, $R^{3}$', at each occurrence, is each independently selected from the group consisting of H, hydroxyl, halogen, CN, NOz, $C_{1-4}$ alkyl, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-6-membered heterocyclyl, $C_{6-10}$ aryl, 5-6-membered heteroaryl, $-NR^{20a}R^{20b}$, $-SR^{21}$, $-S(=O)_2R^{22}$, $-S(=O)_2NR^{20a}R^{20b}$, $-NR^{20a}S(=O)_2R^{20b}$, $-C(=O)R^{21}$, - $C(=O)NR^{23a}R^{23b}$, $-NR^{23a}C(=O)R^{23b}$ and $-NR^{24a}C(=O)NR^{25a}R^{25b}$, the alkyl, heteroalkyl (e.g., alkoxy), alkenyl, alkynyl, cycloalkyl, cycloalkoxy, heterocyclyl, aryl and heteroaryl are each optionally substituted with one or more substituents independently selected from the group consisting of: hydroxyl, halogen, CN, NOz, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl and 4-6-membered heterocyclyl;

$R^{20a}$, $R^{20b}$, $R^{23a}$, $R^{23b}$, $R^{24a}$, $R^{25a}$ and $R^{25b}$ are each independently selected from the group consisting of H, $-NHCH_3$, $-N(CH_3)_2$, $C_{1-4}$ alkyl, $C_{1-4}$ alkoxy, $C_{3-6}$ cycloalkyl and 4-6-membered heterocyclyl; the alkyl, alkoxy, cycloalkyl and heterocyclyl are each optionally substituted with one or more substituents independently selected from the group consisting of: halogen, $C_{1-6}$ alkyl and 4-6-membered heterocyclyl; and

$R^{21}$ and $R^{22}$ are each independently selected from the group consisting of $C_{1-4}$ alkyl, $C_{1-4}$ alkoxy and $C_{3-6}$ cycloalkyl, the alkyl, alkoxy and cycloalkyl are each optionally substituted with one or more halogens.

In certain embodiments, in the compound of Formula I', Formula I'-A, Formula I, Formula I-A to Formula I-D provided in the present invention, $R^{3}$', at each occurrence, is each independently selected from the group consisting of H, hydroxyl, halogen, CN, NOz, $C_{1-4}$ alkyl, $C_{1-4}$ heteroalkyl (e.g., $C_{1-4}$ alkoxy), $C_{3-6}$ cycloalkyl, $C_{3-6}$ cycloalkoxy, 4-6-membered heterocyclyl, $C_{6-10}$ aryl, 5-6-membered heteroaryl, $-NR^{20a}R^{20b}$, $-S(=O)_2NR^{20a}R^{20b}$, $-NR^{20a}S(=O)_2R^{20b}$, $-C(=O)R^{21}$, $-C(=O)NR^{23a}R^{23b}$ and $-NR^{23a}C(=O)R^{23b}$, the alkyl, heteroalkyl (e.g., alkoxy), cycloalkyl, cycloalkoxy, heterocyclyl, aryl and heteroaryl are each optionally substituted with one or more substituents independently selected from the group consisting of: hydroxyl, halogen, CN, NOz, $C_{1-3}$ alkyl, $C_{1-3}$ haloalkyl, $C_{1-3}$ hydroxyalkyl, $C_{1-3}$ haloalkoxy, $C_{1-3}$ heteroalkyl (e.g., $C_{1-3}$ alkoxy), $C_{3-6}$ cycloalkyl and 4-6-membered heterocyclyl;

$R^{20a}$, $R^{20b}$, $R^{23a}$ and $R^{23b}$ are each independently selected from the group consisting of H, $C_{1-4}$ alkyl, $C_{1-4}$ alkoxy and 4-6-membered heterocyclyl; the alkyl, alkoxy and heterocyclyl are each optionally substituted with one or more substituents independently selected from the group consisting of: halogen, $C_{1-6}$ alkyl and 4-6-membered heterocyclyl; and

$R^{21}$ is $C_{1-4}$ alkyl.

In certain embodiments, in the compound of Formula I', Formula I'-A, Formula I, Formula I-A to Formula I-D provided in the present invention, $R^{3}$', at each occurrence, is each independently selected from the group consisting of H, hydroxyl, halogen, CN, NOz, $C_{1-4}$ alkyl, $C_{1-4}$ heteroalkyl (e.g., $C_{1-4}$ alkoxy), $C_{3-6}$ cycloalkyl, $C_{3-6}$ cycloalkoxy, 4-6-membered heterocyclyl, $-NR^{20a}R^{20b}$, $-S(=O)_2NR^{20a}R^{20b}$, $-NR^{20a}S(=O)_2R^{20b}$, $-C(=O)R^{21}$, $-C(=O)NR^{23a}R^{23b}$ and $-NR^{23a}C(=O)R^{23b}$, the alkyl, heteroalkyl (e.g., alkoxy), cycloalkyl, cycloalkoxy and heterocyclyl are each optionally substituted with one or more substituents independently selected from the group consisting of: hydroxyl, halogen, $C_{1-3}$ alkyl, $C_{1-3}$ haloalkyl and $C_{1-3}$ haloalkoxy;

$R^{20a}$, $R^{20b}$, $R^{23a}$ and $R^{23b}$ are each independently H, methyl, ethyl, propyl or oxetanyl, which are each optionally substituted with one or more substituents independently selected from the group consisting of: F, Cl, Br, methyl and oxetanyl; and

$R^{21}$ is $C_{1-4}$ alkyl.

The present invention encompasses any combinations of the above embodiments.

In some embodiments, the compound of the present invention includes but is not limited to:

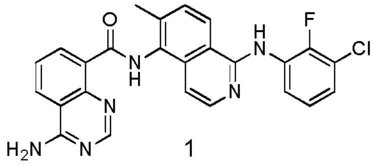
1

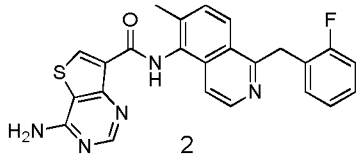
2

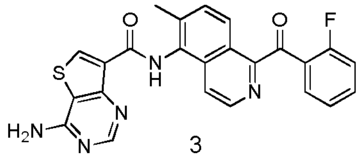
3

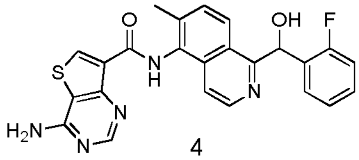
4

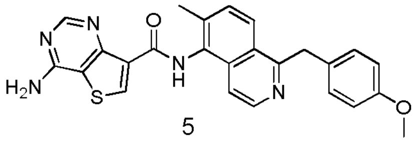
5

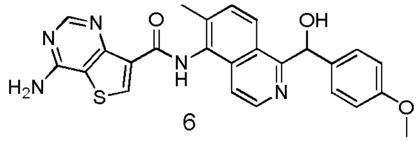
6

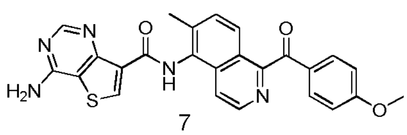
7

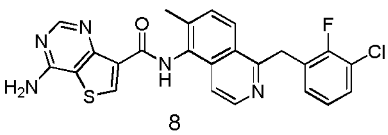
8

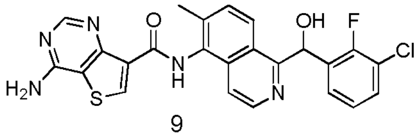
9

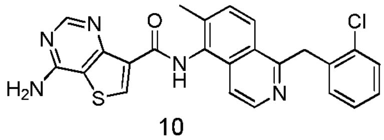
10

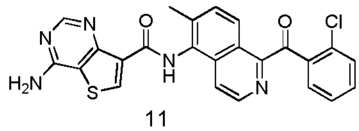
11

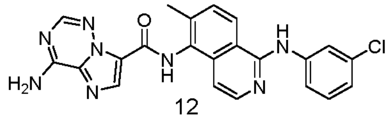
12

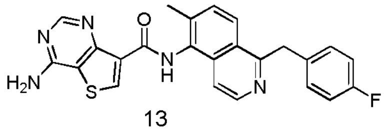
13

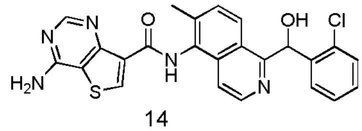
14

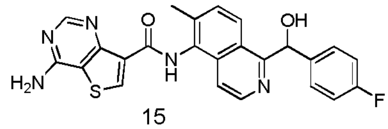
15

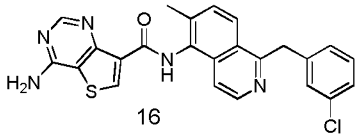
16

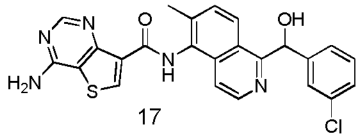
17

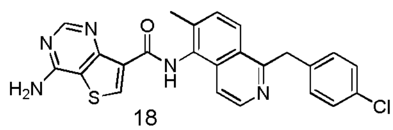
18

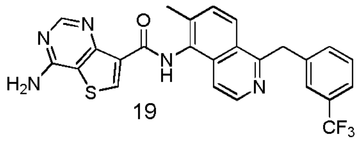
19

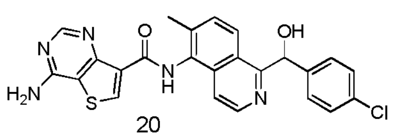
20

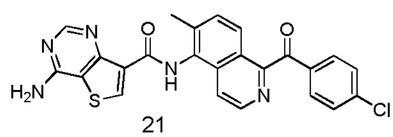
21

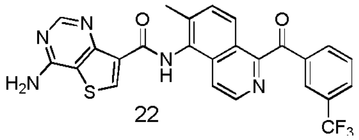
22

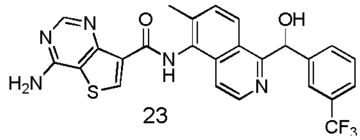
23

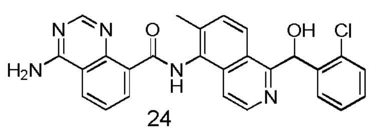
24

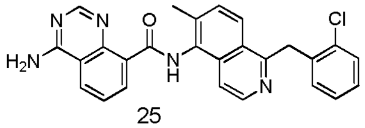
25

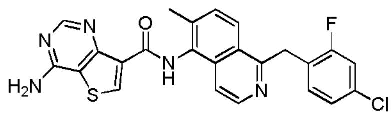
26

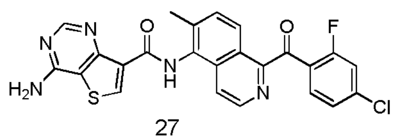
27

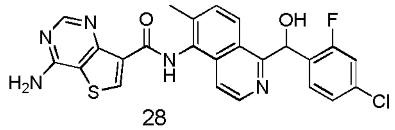
28

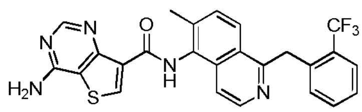
29

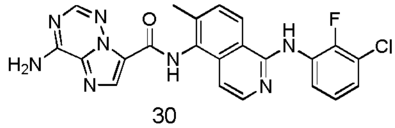
30

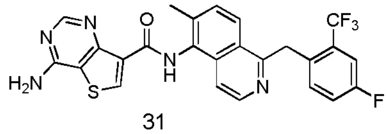
31

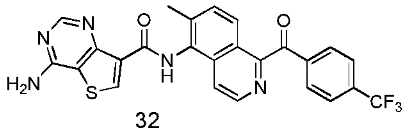
32

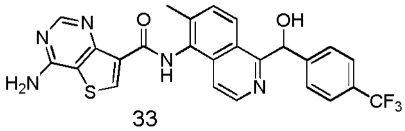
33

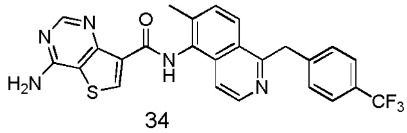
34

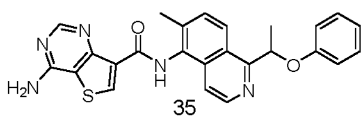
35

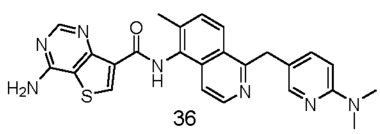
36

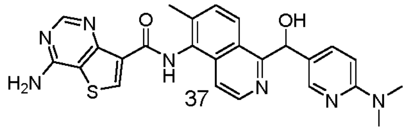
37

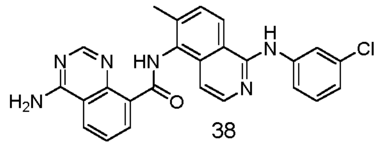
38

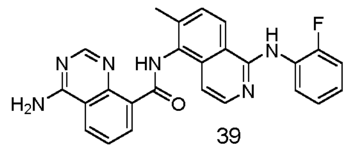
39

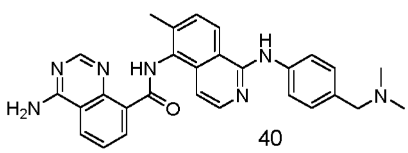
40

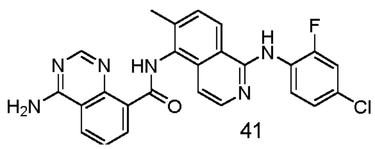
41

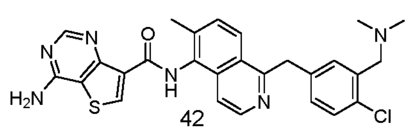
42

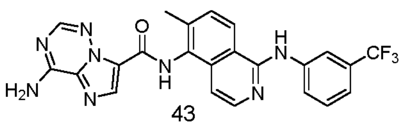
43

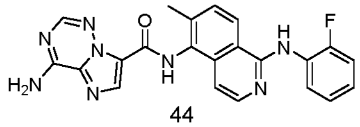
44

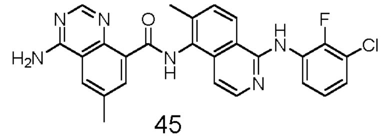
45

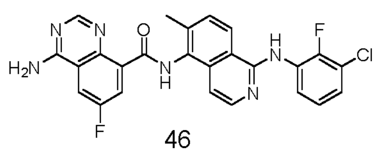
46

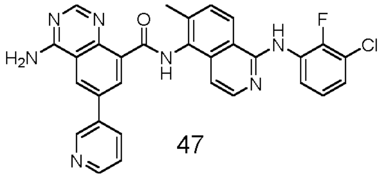
47

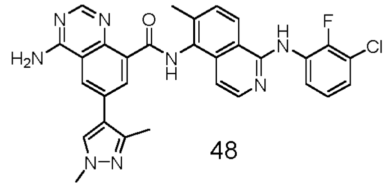
48

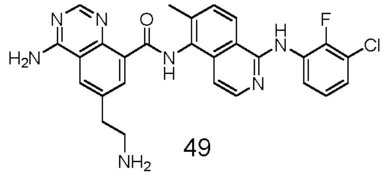
49

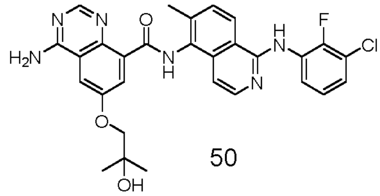
50

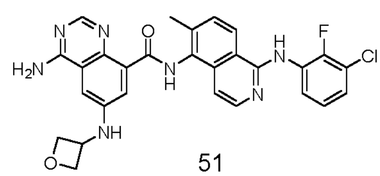
51

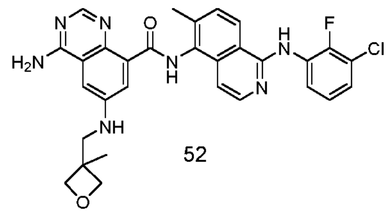
52

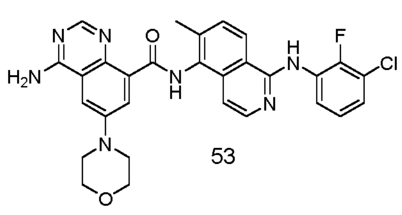
53

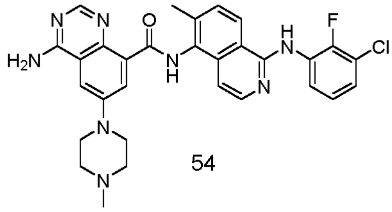
54

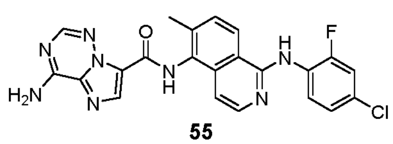
**55**

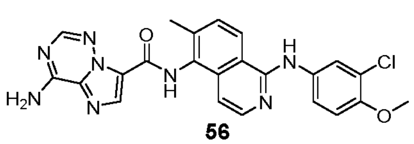
**56**

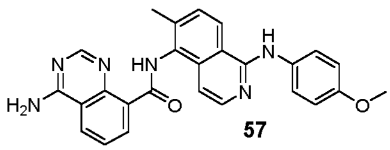
**57**

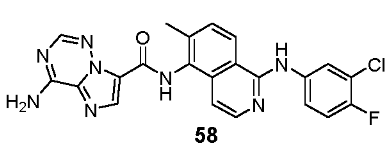
**58**

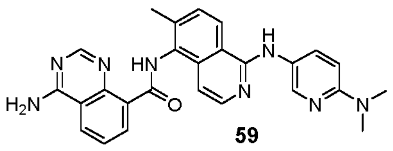
**59**

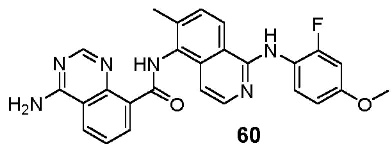
**60**

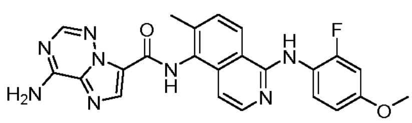
**61**

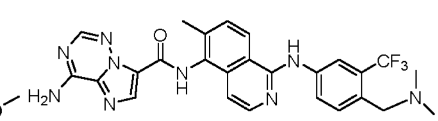
**62**

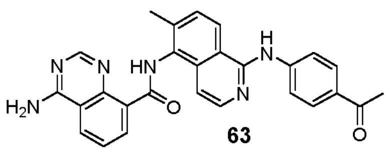
**63**

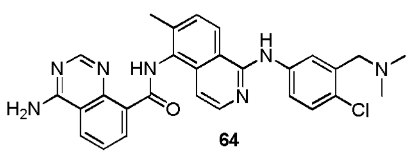
**64**

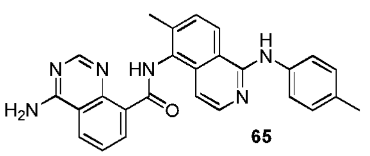
**65**

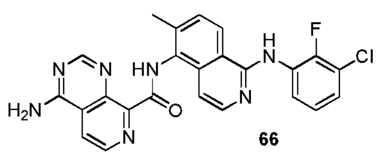
**66**

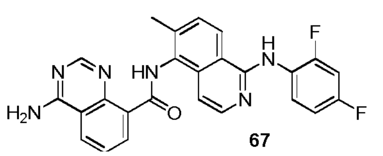
**67**

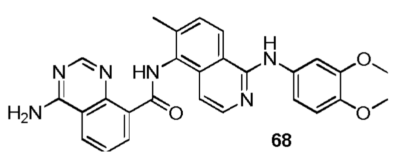
**68**

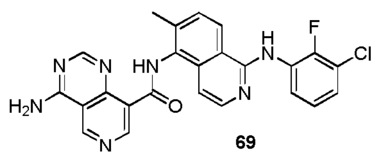
**69**

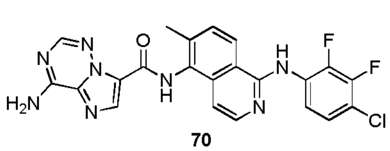
**70**

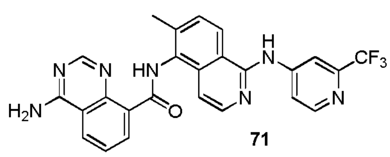
**71**

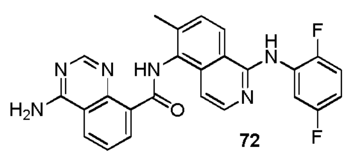
**72**

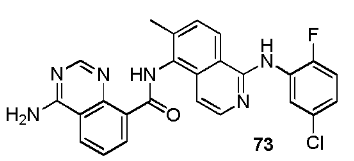
**73**

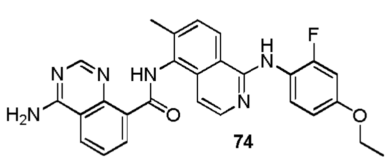
**74**

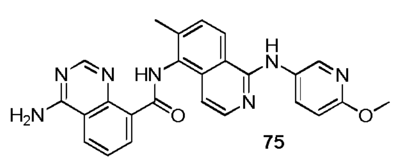
**75**

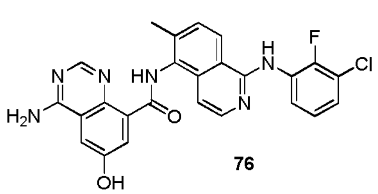
**76**

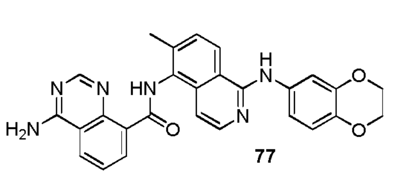
**77**

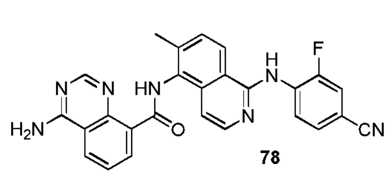
**78**

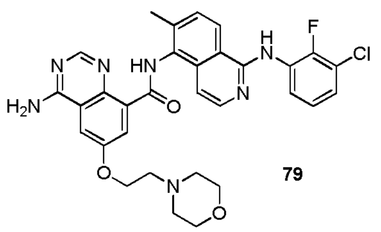

79

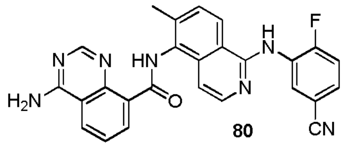

80

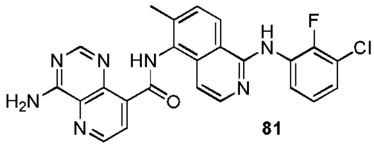

81

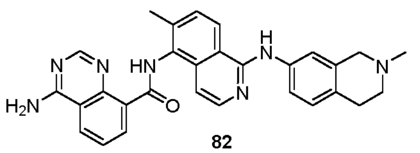

82

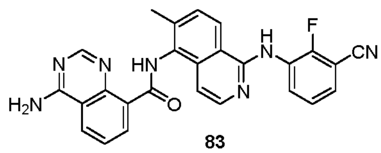

83

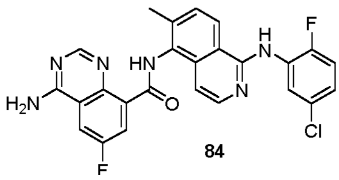

84

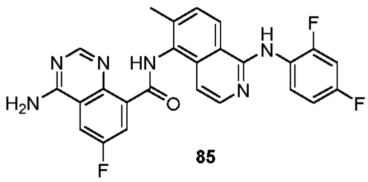

85

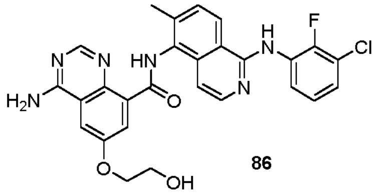

86

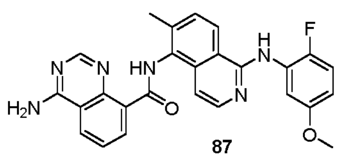

87

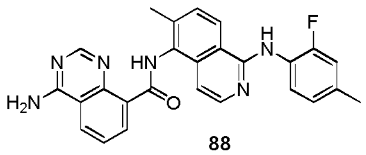

88

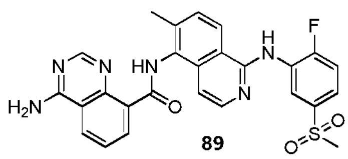

89

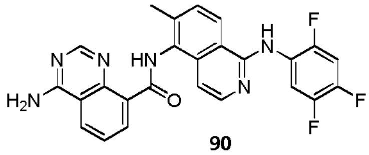

90

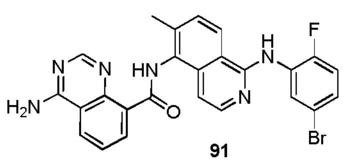

91

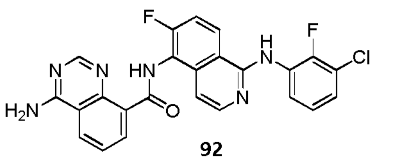

92

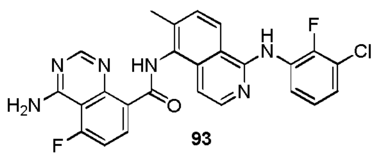

93

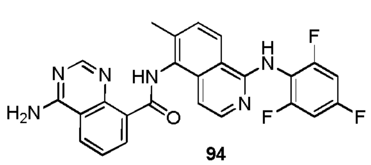

94

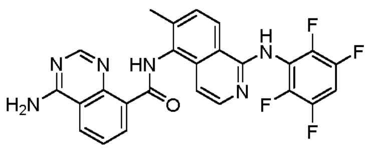

95

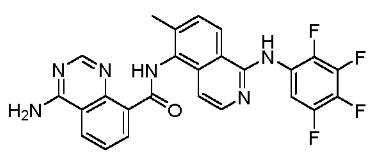

96

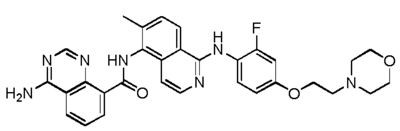

97

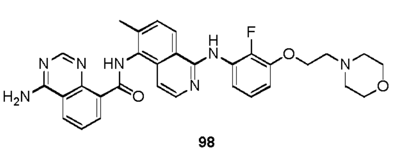

98

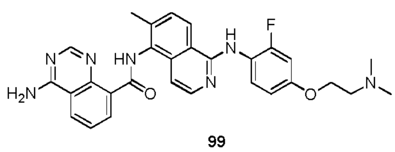

99

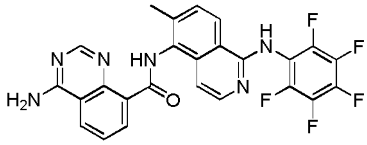

100

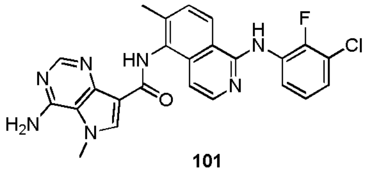

101

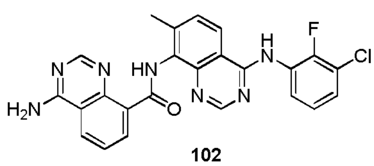

102

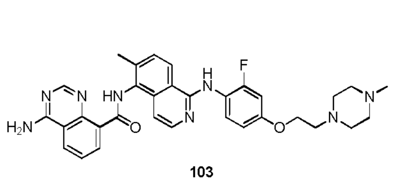

103

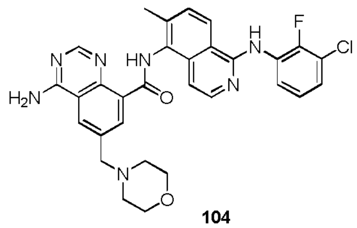

104

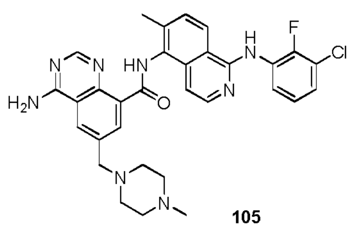

105

.

Preparation Method

In certain embodiments, the present invention provides a method for preparing a compound of Formula I-A, comprising the following steps:

Scheme A

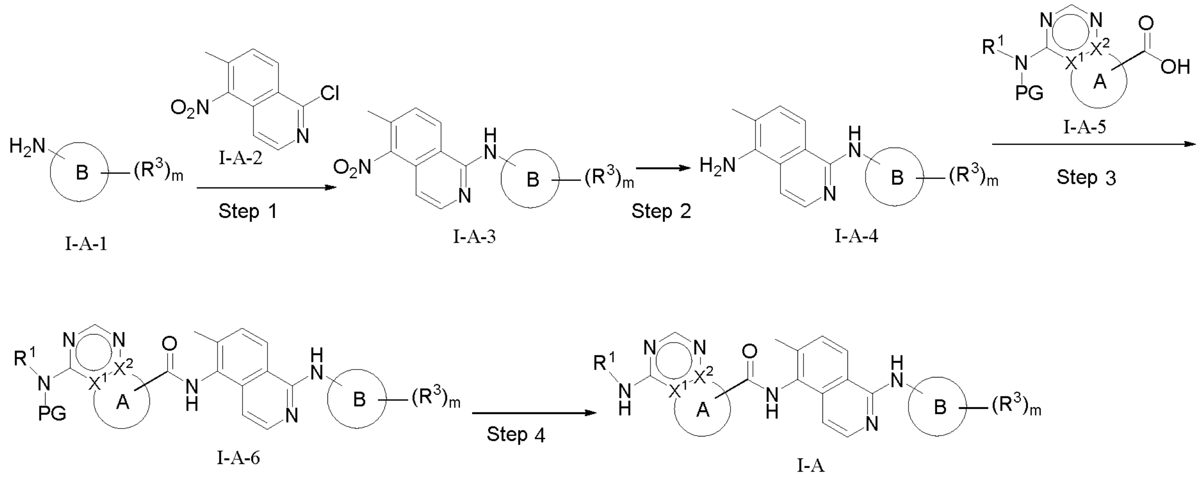


wherein:

PG is an amino-protecting group, preferably 2,4-dimethoxybenzyl;
the remaining groups are each as defined above;
the reaction conditions of each step are as follows:

Step 1: subjecting compounds I-A-1 and I-A-2 to a substitution reaction or a coupling reaction (e.g., a Buchwald or Ullman reaction, *etc.)* to afford compound I-A-3;
For the substitution reaction, it can be performed in the presence of an acid (e.g., trifluoroacetic acid, hydrochloric acid, *etc.)* or a base (e.g., $^tBuONa$, $^tBuOK$, $^tBuOLi$, $Cs_2CO_3$, DIPEA, LiHMDS, LDA, NaHMDS, KHMDS, $K_3PO_4$, $Na_2CO_3$, KOAc, $NaHCO_3$ or $K_2CO_3$); the solvent that can be employed is e.g., isopropanol, tert-butanol, toluene, xylene, THF, DME, 1,4-dioxane, DMF, DMSO or NMP; and the reaction temperature is 40°C to 140°C.

For the Buchwald reaction, the catalyst that can be employed is e.g., $Pd(OAc)_2$, $Pd_2(dba)_3$, $Pd(dba)_2$, $PdCl_2$, $Pd(PPh_3)_4$, $Pd(dppf)Cl_2$, $Pd(acac)_2$ or $Pd(allyl)_2$; the ligand that can be employed is $PPh_3$, XPhos, SPhos, RuPhos, XantPhos, dppf, BINOL, BINAP or $PCy_3$, *etc.;* the base that can be employed is e.g., $^tBuONa$, $^tBuOK$, $^tBuOLi$, $Cs_2CO_3$, LiHMDS, LDA, NaHMDS, KHMDS, $K_3PO_4$, $Na_2CO_3$, KOAc, $NaHCO_3$ or $K_2CO_3$; the solvent that can be employed is e.g., toluene, xylene, THF, DME, 1,4-dioxane, DMF, DMSO or NMP; and the reaction temperature is 40°C to 140°C.

For the Ullmann reaction, the catalyst that can be employed is e.g., CuCl, CuBr, CuI or $Cu_2O$; the ligand that can be employed is e.g., salicylaldoxime, diaminocyclohexane, N,N'-dimethylethylenediamine, TMEDA or ethylenediamine; the base that can be employed is e.g., $^tBuONa$, $^tBuOK$, $^tBuOLi$, $Cs_2CO_3$, LiHMDS, LDA, NaHMDS, KHMDS, $K_3PO_4$, $Na_2CO_3$, KOAc, $NaHCO_3$ or $K_2CO_3$; the solvent that can be employed is e.g., toluene, xylene, THF, DME, 1,4-dioxane, DMF, DMSO or NMP; and the reaction temperature is 40°C to 140°C.

Step 2: subjecting compound I-A-3 to a reduction reaction to afford compound I-A-4;
The reducing agent that can be employed in the reduction reaction is e.g., zinc powder/acetic acid, iron powder/ammonium chloride solution, iron powder/hydrochloric acid solution, palladium on carbon/hydrogen, etc.; the solvent that can be

employed is e.g., water, ethanol, methanol or a mixture thereof, *etc.;* the reaction temperature is 0°C to 90°C, e.g., at room temperature, 60°C, 70°C, 80°C or 90°C.

Step 3: subjecting compounds I-A-4 and I-A-5 to a condensation reaction to afford compound I-A-6;

The condensation reaction is preferably performed in the presence of a condensation reagent and a base. The condensation reagent that can be employed is $T_3P$, HATU, CDI, HOBt, DMAP, DCC, DIC, EDC, HBTU, HCTU or PyBOP, *etc.* The base that can be employed is pyridine, TEA, DIPEA, $^tBuOK$, $^tBuONa$, $^tBuOLi$, NaH, NaOH, $Cs_2CO_3$, $K_3PO_4$ or $Na_2CO_3$, *etc.* The solvent that can be employed is THF, DCM, DCE, MeOH, EtOH, DMF, DMSO, acetone, $CH_3CN$, 1,4-dioxane or toluene, *etc.* The reaction temperature is 0 °C to 120 °C, e.g., at room temperature.

Alternatively, compound I-A-5 is firstly prepared as an acyl halide, the acylation reagent that can be employed is e.g., thionyl chloride, oxalyl chloride, *etc.* The reaction can be catalyzed by a small amount of DMF, and can also be performed in a system free of DMF; the reaction temperature is 0 °C to 120 °C; the resulting acyl halide compound is then reacted with compound I-A-4 in the presence of a base, to afford compound I-A-6. The base that can be employed is TEA or DIPEA, *etc.;* The solvent that can be employed is THF, DCM, DCE, $CH_3CN$, 1,4-dioxane or toluene, *etc.;* the reaction can be performed at 0 °C to 120 °C.

Step 4: removing the protecting group from compound I-A-6 under an acidic condition to afford a compound of Formula I-A;

The reaction can be performed in a solvent such as trifluoroacetic acid, and the reaction temperature is 25 °C to 120 °C, e.g., 70 °C or 100 °C.

In certain embodiments, the present invention provides a method for preparing a compound of Formula I-A, comprising the following steps:

Scheme A-1

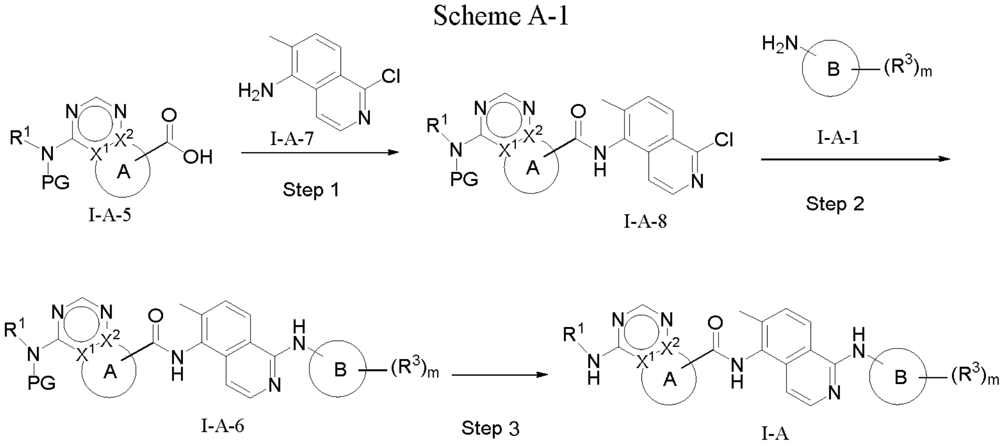


I-A-5, I-A-8, I-A-6, I-A

wherein:

PG is an amino-protecting group, preferably 2,4-dimethoxybenzyl;
the remaining groups are each as defined above;
the reaction conditions of each step are as follows:

Step 1: subjecting compounds I-A-5 and I-A-7 to a condensation reaction to afford compound I-A-8;
The condensation reaction is preferably performed in the presence of a condensation reagent and a base. The condensation reagent that can be employed is $T_3P$, HATU, CDI, HOBt, DMAP, DCC, DIC, EDC, HBTU, HCTU or PyBOP, *etc.* The base that can be employed is pyridine, TEA, DIPEA, $^tBuOK$, $^tBuONa$, $^tBuOLi$, NaH, NaOH, $Cs_2CO_3$, $K_3PO_4$ or $Na_2CO_3$, *etc.* The solvent that can be employed is THF, DCM, DCE, MeOH, EtOH, DMF, DMSO, acetone, $CH_3CN$, 1,4-dioxane or toluene, *etc.* The reaction temperature is 0 °C to 120 °C, e.g., at room temperature.

Alternatively, compound I-A-5 is firstly prepared as an acyl halide, the acylation reagent that can be employed is e.g., thionyl chloride, oxalyl chloride, *etc.* The reaction can be catalyzed by a small amount of DMF, and can also be performed in a system free of DMF; the reaction temperature is 0 °C to 120 °C; the resulting acyl halide compound is then reacted with compound I-A-4 in the presence of a base, to afford compound I-A-6. The base that can be employed is TEA or DIPEA, *etc.;* The solvent that can be employed is THF, DCM, DCE, $CH_3CN$, 1,4-dioxane or toluene, *etc.;* the reaction can be performed at 0 °C to 120 °C.

Step 2: subjecting compounds I-A-1 and I-A-8 to a substitution reaction or a coupling reaction (e.g., a Buchwald

or Ullman reaction, *etc.)* to afford compound I-A-6;

For the substitution reaction, it can be performed in the presence of an acid (e.g., trifluoroacetic acid, hydrochloric acid, *etc.)* or a base (e.g., $^t$BuONa, $^t$BuOK, $^t$BuOLi, $Cs_2CO_3$, DIPEA, LiHMDS, LDA, NaHMDS, KHMDS, $K_3PO_4$, $Na_2CO_3$, KOAc, $NaHCO_3$ or $K_2CO_3$); the solvent that can be employed is e.g., isopropanol, *tert*-butanol, toluene, xylene, THF, DME, 1,4-dioxane, DMF, DMSO or NMP; and the reaction temperature is 40°C to 140°C.

For the Buchwald reaction, the catalyst that can be employed is e.g., $Pd(OAc)_2$, $Pd_2(dba)_3$, $Pd(dba)_2$, $PdCl_2$, $Pd(PPh_3)_4$, $Pd(dppf)Cl_2$, $Pd(acac)_2$ or $Pd(allyl)_2$; the ligand that can be employed is $PPh_3$, XPhos, SPhos, RuPhos, XantPhos, dppf, BINOL, BINAP or $PCy_3$, *etc.;* the base that can be employed is e.g., $^t$BuONa, $^t$BuOK, $^t$BuOLi, $Cs_2CO_3$, LiHMDS, LDA, NaHMDS, KHMDS, $K_3PO_4$, $Na_2CO_3$, KOAc, $NaHCO_3$ or $K_2CO_3$; the solvent that can be employed is e.g., toluene, xylene, THF, DME, 1,4-dioxane, DMF, DMSO or NMP; and the reaction temperature is 40°C to 140°C.

For the Ullmann reaction, the catalyst that can be employed is e.g., CuCl, CuBr, CuI or $Cu_2O$; the ligand that can be employed is e.g., salicylaldoxime, diaminocyclohexane, N,N'-dimethylethylenediamine, TMEDA or ethylenediamine; the base that can be employed is e.g., $^t$BuONa, $^t$BuOK, $^t$BuOLi, $Cs_2CO_3$, LiHMDS, LDA, NaHMDS, KHMDS, $K_3PO_4$, $Na_2CO_3$, KOAc, $NaHCO_3$ or $K_2CO_3$; the solvent that can be employed is e.g., toluene, xylene, THF, DME, 1,4-dioxane, DMF, DMSO or NMP; and the reaction temperature is 40°C to 140°C.

Step 3: removing the protecting group from compound I-A-6 under an acidic condition to afford a compound of Formula I-A.

The reaction can be performed in a solvent such as trifluoroacetic acid, and the reaction temperature is 25 °C to 120 °C, e.g., 70 °C or 100 °C.

In certain embodiments, the present invention provides a method for preparing a compound of Formula I-B, comprising the following steps:

Scheme B-1

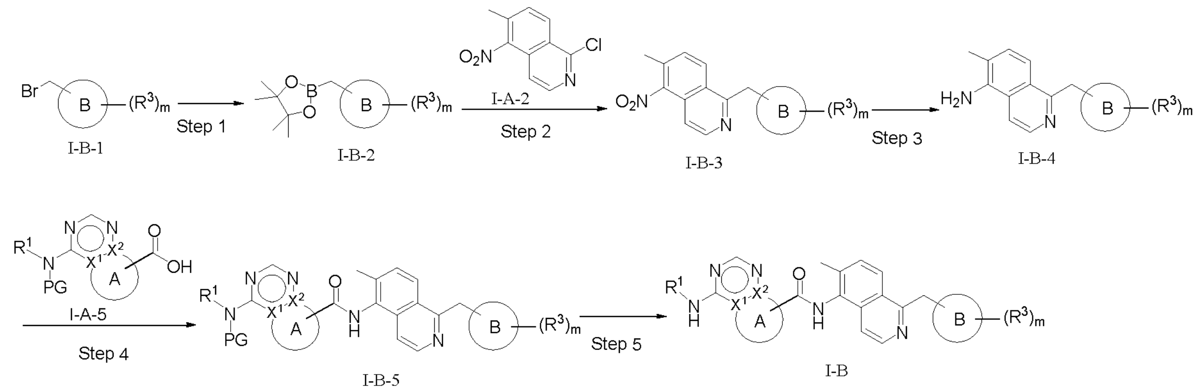


wherein:

PG is an amino-protecting group, preferably 2,4-dimethoxybenzyl;
the remaining groups are each as defined above;
the reaction conditions of each step are as follows:

Step 1: reacting compound I-B-1 with a boron-containing reagent to afford compound I-B-2;
The boron-containing reagent that can be employed is e.g., $B_2(pin)_2$. The catalyst that can be employed is e.g., $Pd(OAc)_2$, $Pd(PPh_3)_4$, $Pd(dppf)Cl_2$, *etc.*, the ligand that can be employed is e.g., $PPh_3$, dppf, BINOL, BINAP or $PCy_3$, *etc.*, the base that can be employed is e.g., $Cs_2CO_3$, $K_3PO_4$, $Na_2CO_3$, KOAc, $NaHCO_3$ or $K_2CO_3$, *etc.* The solvent that can be employed is e.g., 1,4-dioxane, DMF, DMSO or $CH_3CN$. The reaction temperature can be 50°C to 120°C.
Step 2: subjecting compounds I-B-2 and I-A-2 to a coupling reaction to afford compound I-B-3;
The catalyst that can be employed in the coupling reaction is e.g., $Pd(OAc)_2$, $Pd(PPh_3)_4$ or $Pd(dppf)Cl_2$, *etc.;* the base that can be employed is e.g., $Cs_2CO_3$, $K_3PO_4$, $Na_2CO_3$, AcOK, $NaHCO_3$ or $K_2CO_3$, the ligand that can be employed is e.g., $PPh_3$, DPPF, BINOL, BINAP or $PCy_3$, *etc.*, the solvent that can be employed is e.g., toluene/$H_2O$, 1,4-dioxane/$H_2O$, DMF/$H_2O$, DMSO/$H_2O$ or $CH_3CN$/$H_2O$, and the reaction temperature can be 60°C to 120°C.
Step 3: subjecting compound I-B-3 to a reduction reaction to afford compound I-B-4;
The reaction conditions are as described for Step 2 in the method for preparing a compound of Formula I-A (Scheme A).

Step 4: subjecting compounds I-B-4 and I-A-5 to a condensation reaction to afford compound I-B-5;
The reaction conditions are as described for Step 3 in the method for preparing a compound of Formula I-A (Scheme A).
Step 5: removing the protecting group from compound I-B-5 under an acidic condition to afford a compound of Formula I-B;
The reaction conditions are as described for Step 4 in the method for preparing a compound of Formula I-A (Scheme A).

In certain embodiments, the present invention provides a method for preparing a compound of Formula I-B comprising the following steps:

Scheme B-2

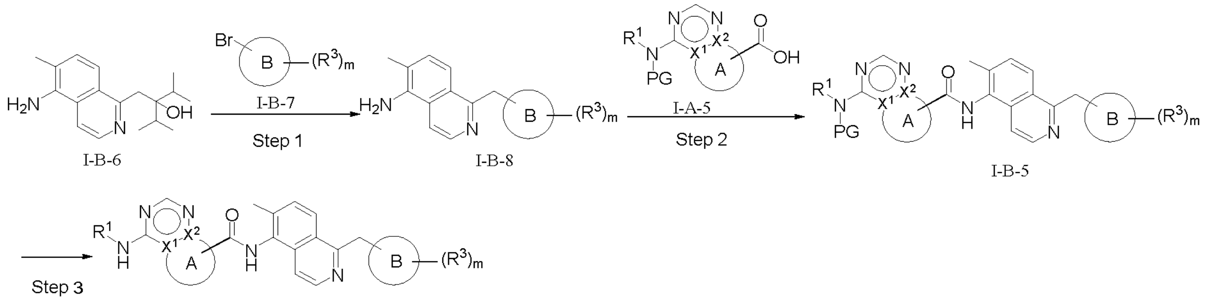


I-B-6 I-B-8 I-B-5

I-B

wherein:

PG is an amino-protecting group, preferably 2,4-dimethoxybenzyl;
the remaining groups are each as defined above;
the reaction conditions of each step are as follows:

Step 1: subjecting compounds I-B-6 and I-B-7 to a coupling reaction to afford compound I-B-8;
The catalyst that can be employed in the coupling reaction is e.g., $Pd(TFA)_2$, $Pd(OAc)_2$, $Pd(PPh_3)_4$ or $Pd(dppf)Cl_2$, *etc.;* the ligand that can be employed is e.g., $PPh_3$, DPPF, BINOL, BINAP or $PCy_3$, *etc.;* the base that can be employed is e.g., $Cs_2CO_3$, $K_3PO_4$, $Na_2CO_3$, AcOK, $NaHCO_3$ or $K_2CO_3$; the solvent that can be employed is e.g., toluene, xylene, 1,4-dioxane, DMF, DMSO or $CH_3CN$, *etc.;* and the reaction temperature can be 60°C to 120°C.
Step 2: subjecting compounds I-B-8 and I-A-5 to a condensation reaction to afford compound I-B-5;
The reaction conditions are as described for Step 3 in the method for preparing a compound of Formula I-A (Scheme A).
Step 3: removing the protecting group from compound I-B-5 under an acidic condition to afford a compound of Formula I-B;
The reaction conditions are as described for Step 4 in the method for preparing a compound of Formula I-A (Scheme A).

In certain embodiments, the present invention provides a method for preparing a compound of Formula I-C, comprising the following steps:

Scheme C

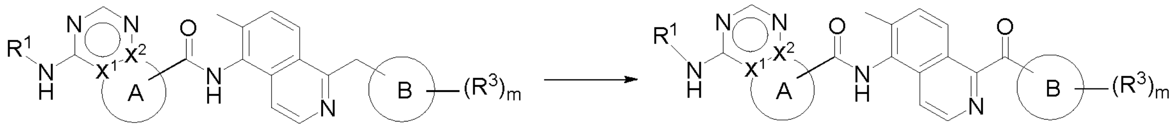

I-B I-C

wherein:

the groups are each as defined above;
the method comprises subjecting compound I-B to a catalytic oxidation reaction to afford a compound of Formula I-C;

The catalyst that can be employed is e.g., NIS, NBS, $I_2$, *etc.;* the solvent that can be employed is e.g., DMSO; and the reaction temperature can be 25°C to 120°C, e.g., 100°C.

In certain embodiments, the present invention provides a method for preparing a compound of Formula I-D, comprising the following steps:

Scheme D-1

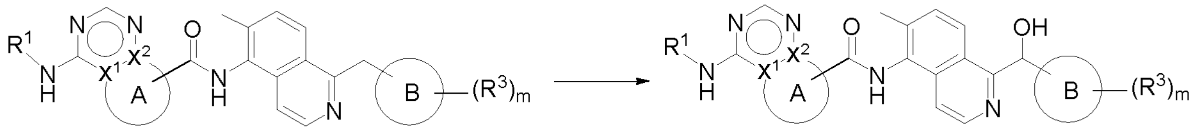

I-B I-D

wherein:

the groups are each as defined above;
the method comprises subjecting compound I-B to a catalytic oxidation reaction to afford a compound of Formula I-D;
The catalyst that can be employed is e.g., NIS, NBS, $I_2$, *etc.;* the solvent that can be employed is e.g., DMSO; and the reaction temperature can be 25°C to 120°C, e.g., 100°C.

In certain embodiments, the present invention provides a method for preparing a compound of Formula I-D, comprising the following steps:

Scheme D-2

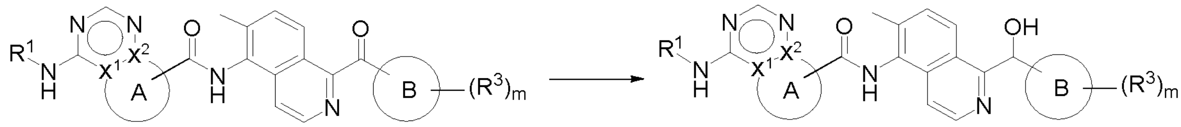

I-C I-D

wherein:

the groups are each as defined above;
the method comprises subjecting compound I-C to a reduction reaction to afford a compound of Formula I-D;
The reducing agent that can be employed is e.g., sodium borohydride or borane, *etc.;* the solvent that can be employed is THF, DCM, DCE or MeOH, *etc.;* and the reaction temperature can be -20°C to 60°C, e.g., at room temperature.

**Pharmaceutical composition, formulation and therapeutic method**

In some embodiments, the present invention provides a pharmaceutical composition comprising a prophylactically or therapeutically effective amount of the compound of the present invention, or a pharmaceutically acceptable salt, ester, stereoisomer, tautomer, polymorph, cocrystal, solvate, metabolite, isotopically labeled compound, N-oxide or prodrug thereof, and one or more pharmaceutically acceptable carriers.
In some embodiments, the present invention provides a pharmaceutical formulation, and the pharmaceutical formulation is preferably a solid formulation, a semi-solid formulation, a liquid formulation, or a gas formulation.
In some embodiments, the pharmaceutical composition or the pharmaceutical formulation is preferably administered via an oral, intravenous, intraarterial, subcutaneous, intraperitoneal, intramuscular or transdermal route.
In some embodiments, the present invention provides use of the compound of the present invention or a pharmaceutically acceptable salt, ester, stereoisomer, tautomer, polymorph, cocrystal, solvate, metabolite, isotopically labeled compound, N-oxide or prodrug thereof, or the pharmaceutical composition of the present invention, or the pharmaceutical formulation of the present invention in the manufacture of a medicament for the prophylaxis or treatment of a disease or disorder associated with RAF and/or RAS kinase activity.
In some embodiments, the present invention provides use of the compound of the present invention or a pharmaceutically acceptable salt, ester, stereoisomer, tautomer, polymorph, cocrystal, solvate, metabolite, isotopically labeled compound, N-oxide or prodrug thereof, or the pharmaceutical composition of the present invention, or the

pharmaceutical formulation of the present invention in the manufacture of a medicament for modulating (e.g., reducing or inhibiting) the RAF and/or RAS kinase activity.

In some embodiments, the present invention provides the compound of the present invention or a pharmaceutically acceptable salt, ester, stereoisomer, tautomer, polymorph, cocrystal, solvate, metabolite, isotopically labeled compound, N-oxide or prodrug thereof, or the pharmaceutical composition of the present invention, or the pharmaceutical formulation of the present invention, for use in the prophylaxis or treatment of a disease or disorder associated with RAF and/or RAS kinase activity.

In some embodiments, the present invention provides a method for the prophylaxis or treatment of a disease or disorder associated with RAF and/or RAS kinase activity, wherein the method comprises administering to a subject in need thereof an effective amount of the compound of the present invention or a pharmaceutically acceptable salt, ester, stereoisomer, tautomer, polymorph, cocrystal, solvate, metabolite, isotopically labeled compound, N-oxide or prodrug thereof, or the pharmaceutical composition of the present invention, or the pharmaceutical formulation of the present invention.

In some embodiments, the disease or disorder associated with RAF and/or RAS kinase activity is preferably cancer or tumor.

In some embodiments, the cancer or tumor is preferably lung cancer (e.g., non-small cell lung cancer), breast cancer, ovarian cancer, gastric cancer, liver cancer, kidney cancer, bone cancer, colorectal cancer, intestinal cancer, pancreatic cancer, head and neck cancer, uterine cancer, esophageal cancer, thyroid cancer, bladder cancer, blood cancer, lymphoma, multiple myeloma, melanoma, glioma, brain tumor or sarcoma.

The term "pharmaceutically acceptable carrier" in the present invention refers to a diluent, auxiliary material, excipient, or vehicle with which a therapeutic is administered, and it is, within the scope of sound medical judgment, suitable for contact with the tissues of human beings and animals without excessive toxicity, irritation, allergic response, or other problem or complication, commensurate with a reasonable benefit/risk ratio.

The pharmaceutically acceptable carrier which can be employed in the pharmaceutical composition of the present invention includes, but is not limited to, sterile liquids. Examples of suitable pharmaceutical carriers are described in e.g., Remington's Pharmaceutical Sciences (1990).

The pharmaceutical composition of the present invention can act systemically and/or topically. To this end, it can be administered through a suitable route.

For these routes of administration, the pharmaceutical composition of the present invention can be administered in a suitable dosage form.

As used herein, the term "effective amount" refers to the amount of a compound being administered which will relieve to some extent one or more of the symptoms of the disorder being treated.

Dosage regimens may be adjusted to provide the optimum desired response. For example, a single bolus may be administered, several divided doses may be administered over time, or the dose may be proportionally reduced or increased as indicated by the exigencies of the therapeutic situation. It is to be noted that dosage values may vary with the type and severity of the condition to be alleviated, and may include single or multiple doses. It is to be further understood that for any particular subject, specific dosage regimens should be adjusted over time according to the individual need and the professional judgment of the person administering or supervising the administration of the composition.

The amount of the compound of the present invention administered will be dependent on the subject being treated, the severity of the disorder or condition, the rate of administration, the disposition of the compound and the discretion of the prescribing physician. Generally, an effective dosage is in the range of about 0.0001 to about 50 mg per kg body weight per day. In some instances, dosage levels below the lower limit of the aforesaid range may be more than adequate, while in other cases, still larger doses may be employed without causing any harmful side effect, provided that such larger doses are first divided into several small doses for administration throughout the day.

The content or dosage of the compound of the present invention in the pharmaceutical composition or pharmaceutical formulation is about 0.01 mg to about 1000 mg.

Unless otherwise indicated, the term "treating" or "treatment", as used herein, means reversing, alleviating, inhibiting the disorder or condition to which such term applies, or the progress of one or more symptoms of such disorder or condition.

The term "prophylaxis" refers to the fact of preventing or delaying the onset of, or of decreasing the intensity of, the clinical or biochemical manifestations associated with the disease, and it encompasses not only prophylaxis before a disease develops, but also the prophylaxis of the recurrence of a disease after treatment.

As used herein, the term "subject" includes a human or non-human animal. An exemplary human subject includes a human subject having a disease (such as one described herein) (referred to as a patient), or a normal subject. The term "non-human animal" as used herein includes all vertebrates, such as non-mammals (e.g., birds, amphibians, reptiles) and mammals, such as non-human primates, livestock and/or domesticated animals (such as sheep, dog, cat, cow, pig and the like).

In some embodiments, the pharmaceutical composition or pharmaceutical formulation of the present invention can further comprise one or more additional therapeutic agents or prophylactic agents (e.g., additional agents for treating cancer or tumor). In some embodiments, the therapeutic method of the present invention can further comprise administering one or more additional therapeutic agents or prophylactic agents (e.g., additional agents for treating cancer or tumor).

## MODE OF CARRYING OUT THE INVENTION

### Examples

The present invention is further described with reference to the following examples, which are not provided to limit the scope of the present invention.

The abbreviations as used herein have the following meanings:

| Abbrevia tion | Meaning |
|---|---|
| AcOH/H OAc | acetic acid |
| BINAP | 1,1'-binaphthalene-2,2'-bisdiphenylphosphine |
| BINOL | 1,1'-binaphthol |
| Boc | *tert*-butoxycarbonyl |
| BOP | Benzotriazol-1-yloxytris(dimethylamino)phosphoniu m hexafluorophosphate |
| $B_2(pin)_2$ | bis(pinacolato)diboron |
| CDI | carbonyldiimidazole |
| CO | carbon monoxide |
| $Cs_2CO_3$ | cesium carbonate |
| Davephos | 2-dicyclohexylphosphino-2'-(N,N-dimethylamino)-biphenyl |
| DBU | 1,8-diazabicyclo[5.4.0]undec-7-ene |
| DCC | dicyclohexylcarbodiimide |
| DCE | 1,2-dichloroethane |
| DCM | dichloromethane |
| DIAD | diisopropyl azodicarboxylate |
| DIC | N,N' -diisopropylcarbodiimide |
| DIPEA | N,N-diisopropylethylamine |
| DMAP | 4-dimethylaminopyridine |
| DME | ethylene glycol dimethyl ether |
| DMF | *N,N*-dimethylformamide |
| DMSO-$d_6$ | deuterated dimethyl sulfoxide |
| DMSO | dimethyl sulfoxide |
| dppf | 1,1' -bis(diphenylphosphino)ferrocene |
| EA | ethyl acetate |
| EDC | 1-ethyl-(3-dimethylaminopropyl)carbodiimide |
| Et | ethyl |
| Fe | iron |
| HATU | O-(7-azabenzotriazolyl)-N,N,N',N'-tetramethyluronium hexafluorophosphate |
| HBTU | benzotriazol-N,N,N',N'-tetramethyluronium hexafluorophosphate |

(continued)

| Abbrevia tion | Meaning |
|---|---|
| HCTU | O-(6-chloro-1 -benzotriazol-1 -yl)-N,N,N',N'-tetramethyluronium hexafluorophosphate |
| $^1$H NMR | nuclear magnetic resonance hydrogen spectrum |
| $H_2O$ | water |
| HOBt | 1 -hydroxybenzotriazole |
| HPLC | high performance liquid chromatography |
| hr | hour |
| $^i$Pr | isopropyl |
| $K_2CO_3$ | potassium carbonate |
| KHMDS | potassium bis(trimethylsilyl)amide |
| KOAc | potassium acetate |
| $K_3PO_4$ | potassium phosphate |
| LC-MS | liquid chromatography-mass spectrum |
| LDA | lithium diisopropylamide |
| LiHMDS | lithium bis(trimethylsilyl)amide |
| mCPBA | m-chloroperbenzoic acid |
| Me | methyl |
| MeCN | acetonitrile |
| MeOH | methanol |
| min | minutes |
| MS | mass spectrum |
| MTBE | methyl tert-butyl ether |
| $NaBH_4$ | sodium borohydride |
| NBS | N-bromosuccinimide |
| NaHMDS | sodium bis(trimethylsilyl)amide |
| NaOH | sodium hydroxide |
| $NH_4Cl$ | ammonium chloride |
| NIS | N-iodosuccinimide |
| NMP | N-methylpyrrolidone |
| NMR | nuclear magnetic resonance |
| $PCy_3$ | tricyclohexylphosphine |
| Pd | palladium |
| Pd/C | palladium on carbon |
| $Pd(acac)_2$ | bis(acetylacetonato)palladium |
| $Pd(dba)_2$ | bis(dibenzylideneacetone)palladium |
| $Pd_2(dba)_3$ | tris(dibenzylideneacetone)dipalladium |
| Pd(dppf)C $l_2$ | [1,1'-bis(diphenylphosphino)ferrocene]dichl oropalladium |
| $Pd(OAc)_2$ | Palladium acetate |
| $Pd(PPh_3)_4$ | tetrakis(triphenylphosphine)palladium |

(continued)

| Abbrevia tion | Meaning |
|---|---|
| $Pd(PPh_3)_2Cl_2$ | bis(triphenylphoshine)palladium chloride |
| $Pd(TFA)_2$ | palladium trifluoroacetate |
| PE | petroleum ether |
| $PPh_3$ | triphenylphosphine |
| Prep-HPLC | preparative high performance liquid chromatography |
| PTSA/p-TsOH | p-toluenesulfonic acid |
| PyBOP | 1H-benzotriazol-1-yloxytripyrrolidinophosphonium hexafluorophosphate |
| PyBrOP | bromotrispyrrolidinophosphonium hexafluorophosphate |
| rt | room temperature |
| RuPhos | 2-dicyclohexylphosphino-2',6'-diisopropoxy-1,1'-biphenyl |
| SPhos | 2-dicyclohexylphosphino-2',6'-dimethoxy-biphenyl |
| $^tBuOK$ | potassium *tert*-butoxide |
| $^tBuOLi$ | lithium *tert*-butoxide |
| $^tBuONa$ | sodium *tert*-butoxide |
| TBAF | tetrabutylammonium fluoride |
| TEA | triethylamine |
| TFA | trifluoroacetic acid |
| $Tf_2O$ | trifluoromethanesulfonic anhydride |
| THF | tetrahydrofuran |
| TLC | thin layer chromatography |
| $T_3P$ | 1-propylphosphonic anhydride |
| XantPhos | 4,5-bisdiphenylphosphino-9,9-dimethylxanthene |
| XPhos | 2-dicyclohexylphosphino-2 ' ,4' ,6'-triisopropylbiphenyl |
| Zn | zinc |

The compounds of the present invention were separated and purified by preparative TLC, silica gel column chromatography, Prep-HPLC and/or Flash column chromatography, and the structures thereof were verified by $^1H$ NMR and/or MS. The reaction was monitored by TLC or LC-MS.

$^1H$ NMR spectrum was recorded on a Bruker nuclear magnetic resonance spectrometer with superconducting magnet (Model: AVACE III HD 400 MHz).

LC/MS was conducted on Aglient 1260 Infinity/Aglient 6120 Quadrupole.

Silica gel GF 254 was used as the stationary phase in TLC.

200 ~ 300 mesh silica gel (Qingdao Haiyang) was generally used as the stationary phase in column chromatography.

Flash column chromatography was conducted on Biotage Flash column chromatograph.

Prep-HPLC was conducted on Agilent Model 1260, and Waters Model 2489.

The microwave reaction was conducted with BiotageInitiatormicrowave reactor.

In the following examples, unless otherwise specified, the reaction temperature was room temperature (15-30°C).

The reagents employed in the present application were purchased from companies such as Acros Organics, Aldrich Chemical Company or Topbiochem, *etc.*

**Example 1: 4-amino-N-(1-((3-chloro-2-fluorophenyl)amino)-6-methylisoquinolin-5-yl)quinazoline-8-carboxamide (compound 1)**

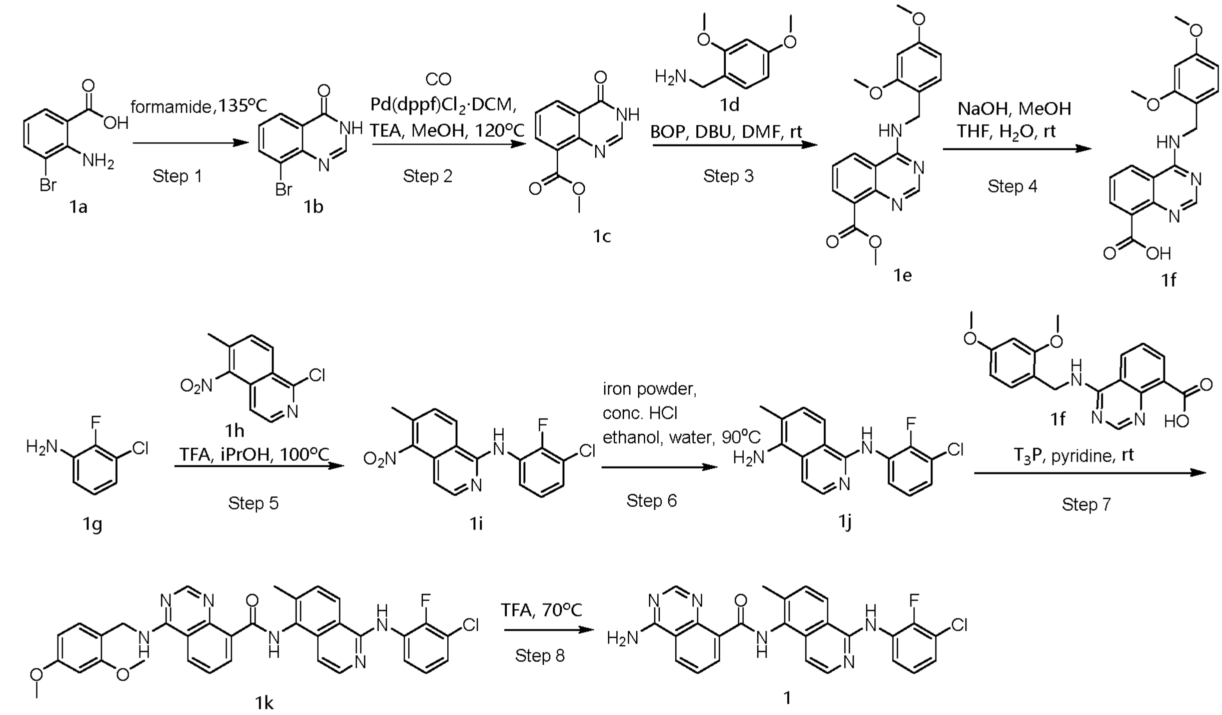


**Step 1: preparation of 8-bromoquinazolin-4(3*H*)-one (compound 1b)**

Compound 1a (1.0 g, 4.63 mmol) was added to formamide (15 mL), protection of nitrogen was applied, and the reaction was heated to 135°C for 2 hours. After the reaction was complete, a large amount of solid precipitated, which was diluted by adding 60 ml water, and filtered under reduced pressure. The filter cake was washed with water, and the resulting solid was dried under reduced pressure at 50°C, to afford compound 1b (800 mg). MS m/z (ESI): 224.9 $[M+H]^+$.

**Step 2: preparation of methyl 4-oxo-3,4-dihydroquinazoline-8-carboxylate (compound 1c)**

Compound 1b (780 mg, 3.47 mmol), TEA (1.75 g, 17.33 mmol, 2.41 mL), Pd(dppf)$Cl_2$·DCM (283.05 mg, 346.60 μmol) and MeOH (25 mL) were added to an autoclave, after being sealed, carbon monoxide was pumped in to 1.0-1.2 Mpa, and then the reaction was heated to 120°C for 5 hours. After the reaction was complete, the reaction was diluted by adding 60 ml water, and filtered under reduced pressure. The filter cake was washed with water, and the resulting solid was dried under reduced pressure at 50°C. The solid was then slurried with methanol (5 mL), filtered with suction and dried to afford compound 1c (400 mg). MS m/z (ESI): 205.0 $[M+H]^+$.

**Step 3: preparation of methyl 4-((2,4-dimethoxybenzyl)amino)quinazoline-8-carboxylate (compound 1e)**

Compound 1c (430.0 mg, 2.11 mmol) and BOP (558.8 mg, 2.74 mmol) were dissolved in DMF (10 mL), followed by dropwise addition of DBU (1.06 g, 4.21 mmol, 1.04 mL). The reaction was stirred for 10 min, then added with compound 1d (528.19 mg, 3.16 mmol, 474.56 μL), and the reaction was then stirred at 25°C for 16 hours. After LC-MS indicated the starting material underwent a complete reaction, the reaction was quenched by adding water, and extracted with EA for three times. The organic phases were combined, washed with water for three times, washed with saturated brine once, dried over anhydrous sodium sulfate, filtered, and concentrated to afford compound 1e (570 mg, 1.61 mmol). MS m/z (ESI): 354.1 $[M+H]^+$.

**Step 4: preparation of 4-((2,4-dimethoxybenzyl)amino)quinazoline-8-carboxylic acid (compound 1f)**

Compound 1e (570 mg, 1.61 mmol) was dissolved in THF (15 mL) and MeOH (5 mL), a solution of sodium hydroxide (193.56 mg, 4.84 mmol) in water (5 mL) was added, and the reaction was stirred at 25°C for 16 hours. After LC-MS indicated the starting material underwent a complete reaction, the solvent was removed under reduced pressure, the resulting solid residue was dissolved by adding 20 ml water, and washed with EA twice. The aqueous phase was adjusted to a pH of about 3 with 2M hydrochloric acid, concentrated under reduced pressure, the resulting solid residue was dissolved in methanol, the insoluble solid was filtered off, and the filtrate was concentrated to afford compound 1f (460 mg). MS m/z (ESI): 340.1 $[M+H]^+$.

**Step 5: preparation of N-(3-chloro-2-fluorophenyl)-6-methyl-5-nitroisoquinolin-1-amine (compound li)**

Compounds 1g (3.92 g, 26.95 mmol) and 1h (5 g, 22.46 mmol) were added to isopropanol (25 mL), followed by addition of TFA (3.07 g, 26.95 mmol, 2.00 mL), and the reaction was stirred under sealing at 100°C for 18 hours. LC-MS indicated the starting material underwent a complete reaction, the reaction solution was naturally cooled to room temperature, filtered with suction, the filter cake was rinsed with isopropanol, and the resulting solid was dried under reduced pressure to afford compound 1i (7 g), MS m/z (ESI): 332.0 $[M+H]^+$.

**Step 6: preparation of $N^1$-(3-chloro-2-fluorophenyl)-6-methylisoquinoline-1,5-diamine (compound lj)**

Compound 1i (3.5 g, 10.55 mmol) was added to ethanol (50 mL) and water (15 mL), followed by addition of iron powder (2.95 g, 52.75 mmol) and concentrated hydrochloric acid (12M, 3 mL), and the reaction was heated to 90°C and stirred for 3.5 hours. LC-MS indicated the starting material underwent a complete reaction, the reaction was immediately filtered through diatomaceous earth, the filtrate was concentrated under reduced pressure to remove most of the solvent, then added with a mixed solvent of chloroform and isopropanol (chloroform /isopropanol=4/1, 200 mL) and a saturated solution of sodium carbonate (50 mL), and the solution was thoroughly stirred. After left standing for phase separation, the lower organic phase was collected, the aqueous phase was then extracted with chloroform/isopropanol=4/1 twice, the organic phases were combined, dried over anhydrous sodium sulfate, filtered, concentrated, and the crude product was separated and purified by column chromatography on silica gel (eluant: 100% DCM), to afford compound 1j (1.7 g). MS m/z (ESI): 302 $[M+H]^+$.

**Step 7: preparation of N-(1-((3-chloro-2-fluorophenyl)amino)-6-methylisoquinolin-5-yl)-4-((2,4-dimethoxybenzyl)amino)quinazoline-8-carboxamide (compound lk)**

Compounds 1f (130 mg, 383.09 μmol) and 1j (115.60 mg, 383.09 μmol) were added to pyridine (9 mL), $T_3P$ (3 mL, 50% in DMF) was then added, and the reaction was allowed to proceed under the protection of nitrogen at room temperature overnight. After the reaction was complete, the reaction solution was directly rotary evaporated to dryness, the mixture was diluted by adding 60 ml water, and extracted with EA (30 mL x3). The organic phases were combined, washed with brine, dried over anhydrous sodium sulfate, filtered, concentrated, and the crude product was separated and purified by column chromatography on silica gel (DCM/MeOH=10/1), to afford compound 1k (110.0 mg). MS m/z (ESI): 623.2 $[M+H]^+$.

**Step 8: preparation of 4-amino-N-(1-((3-chloro-2-fluorophenyl)amino)-6-methylisoquinolin-5-yl)quinazoline-8-carboxamide (compound 1)**

Compound 1k (125 mg, 200.62 μmol) was added to trifluoroacetic acid (5.0 mL), and the reaction was allowed to proceed at 70°C for 2 hours. After the reaction was complete, the reaction solution was concentrated to dryness, added with a saturated solution of sodium bicarbonate for alkalization, and extracted with ethyl acetate. The organic phase was dried over anhydrous sodium sulfate, filtered, concentrated, and the crude product was separated and purified by Prep-HPLC, to afford compound 1 (35.0 mg). MS m/z (ESI): 472.6 $[M+H]^+$.

$^1H$ NMR (400 MHz, DMSO-$d_6$) δ 13.20 (s, 1H), 9.24 (s, 1H), 8.66 (dd, *J* = 7.6, 1.6 Hz, 1H), 8.62 (s, 1H), 8.53 (dd, *J* = 8.4, 1.6 Hz, 1H), 8.44 - 8.32 (m, 2H), 8.23 (br, 1H), 7.90 (d, *J* = 6.0 Hz, 1H), 7.69 (t, *J* = 7.6 Hz, 1H), 7.62 (d, *J* = 8.8 Hz, 1H), 7.59 - 7.53 (m, 1H), 7.40 - 7.31 (m, 1H), 7.28 - 7.20 (m, 2H), 2.45 (s, 3H).

**Example 2: 4-amino-N-(1-(2-fluorobenzyl)-6-methylisoquinolin-5-yl)thieno[3,2-d]pyrimidine-7-carboxamide (compound 2)**

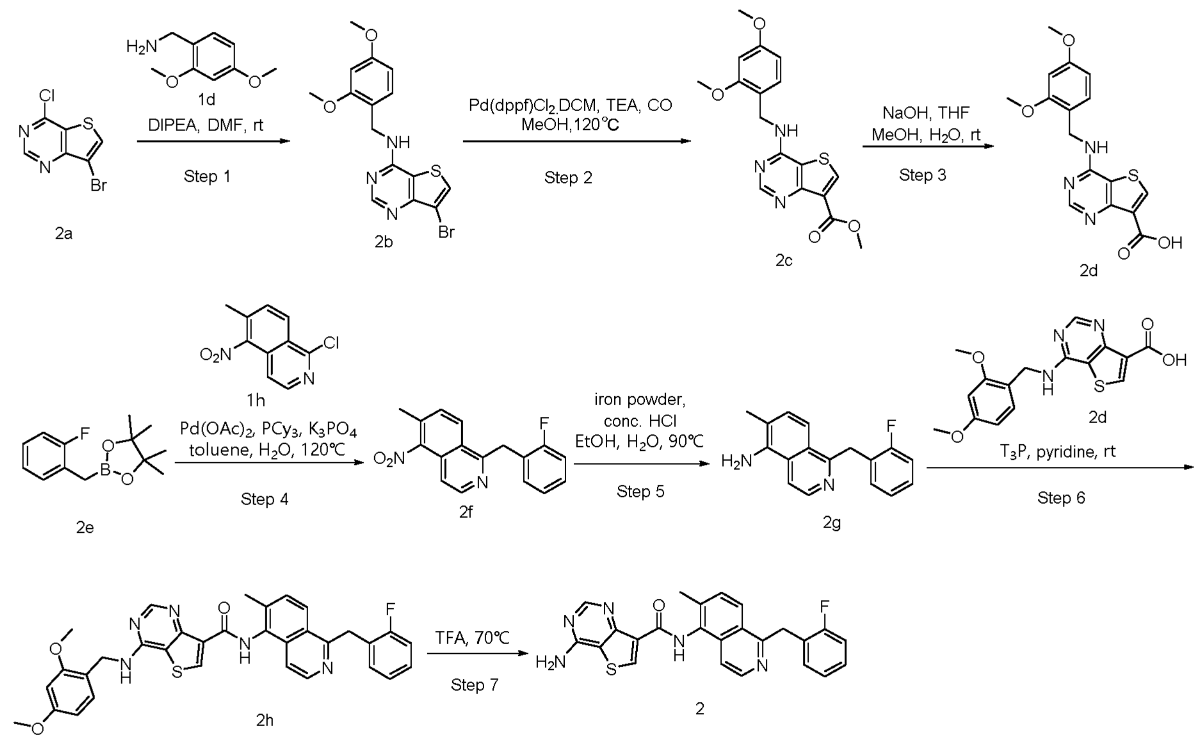


**Step 1: preparation of 7-bromo-N-(2,4-dimethoxybenzyl)thieno[3,2-d]pyrimidin-4-amine (compound 2b)**

Compound 2a (3 g, 12.02 mmol) was dissolved in DMF (15 mL), compound 1d (2.11 g, 12.62 mmol) and DIPEA (2.33 g, 18.03 mmol) were sequentially added, and after the addition, the reaction was stirred at 20°C for 16 hours. After LC-MS indicated the starting material underwent a complete reaction, the reaction solution was diluted by adding ethyl acetate, and washed with water for 3 times. The organic phase was dried over anhydrous sodium sulfate and then filtered, concentrated under reduced pressure to afford compound 2b (4.46 g). MS m/z (ESI): 379.9 $[M+H]^+$.

**Step 2: preparation of methyl 4-((2,4-dimethoxybenzyl)amino)thieno[3,2-d]pyrimidine-7-carboxylate (compound 2c)**

Compound 2b (2 g, 5.26 mmol), Pd(dppf)$Cl_2$·DCM (429.52 mg, 525.96 μmol), MeOH (25 mL) and TEA (2.66 g, 26.30 mmol, 3.66 mL) were sequentially added to an autoclave, purge with nitrogen was performed for three times, and then carbon monoxide was pumped in to 2.3 MPa, the reaction was then stirred at 120°C for 5 hours. LC-MS indicated the starting material underwent a complete reaction, the reaction solution was separated and purified by Flash column chromatography on silica gel (DCM/MeOH=93/7) after cooling, to afford compound 2c (1.82 g). MS m/z (ESI): 360.0 $[M+H]^+$.

**Step 3: preparation of 4-((2,4-dimethoxybenzyl)amino)thieno[3,2-d]pyrimidine-7-carboxylic acid (compound 2d)**

Compound 2c (1.82 g, 5.06 mmol) was added to a mixed solvent of THF (60 mL), MeOH (15 mL) and water (15 mL), NaOH (607.68 mg, 15.19 mmol) was then added, and the reaction was stirred at 25°C for 4 hours after the addition. LC-MS indicated the starting material underwent a complete reaction, the reaction solution was diluted by adding a small amount of water, and extracted with ethyl acetate. The aqueous phases were collected, adjusted to pH=3 with dilute hydrochloric acid, and the solvent was removed under reduced pressure. The resulting solid crude product was re-dissolved in dichloromethane and methanol (10:1), the insoluble solid was filtered off, the filtrate was concentrated under reduced pressure, and then slurried and purified with MTBE and methanol, to afford compound 2d (1.4 g). MS m/z (ESI): 346.0 $[M+H]^+$.

**Step 4: preparation of 1-(2-fluorobenzyl)-6-methyl-5-nitroisoquinoline (compound 2f)**

Compounds 2e (1.06 g, 4.49 mmol) and 1h (200 mg, 898.36 μmol) were dissolved in toluene (10.0 mL), palladium acetate (40.34 mg, 179.67 μmol), potassium phosphate (667.43 mg, 3.14 mmol), tricyclohexylphosphane

(50.39 mg, 179.67 μmol) and water (1.5 mL) were then added, purge with nitrogen was performed for three times, and the reaction was warmed to 120°C and allowed to proceed for 12 hours. After LC-MS indicated the reaction was complete, the reaction was diluted by adding ethyl acetate, washed with water once, washed with saturated brine once, and the organic phase was dried over anhydrous sodium sulfate, filtered, and concentrated. The crude product was separated and purified by column chromatography on silica gel (DCM/MeOH=98/2), to afford compound 2f (220.0 mg). MS m/z (ESI): 297.0 $[M+H]^+$.

**Step 5: preparation of 1-(2-fluorobenzyl)-6-methylisoquinolin-5-amine (compound 2g)**

Compound 2f (50 mg, 168.75 μmol) was added to ethanol (6.0 mL) and water (2.0 mL), iron powder (47.12 mg, 843.75 μmol) and concentrated hydrochloric acid (12M, 0.5 mL) were then added, and the reaction was warmed to 90°C and allowed to proceed for 3.5 hours. After LC-MS indicated the reaction was complete, the reaction was immediately filtered through diatomaceous earth, alkalized and diluted by adding a saturated solution of sodium carbonate, extracted with a mixed solvent of chloroform/isopropanol =4/1 (100 mL), and the organic phase was dried over anhydrous sodium sulfate, filtered, and concentrated, to afford compound 2g (44 mg). MS m/z (ESI): 267.0 $[M+H]^+$.

**Step 6: preparation of 4-((2,4-dimethoxybenzyl)amino)-N-(1-(2-fluorobenzyl)-6-methylisoquinolin-5-yl)thieno[3,2-d]pyrimidine-7-carboxamide (compound 2h)**

Compounds 2g (44.0 mg, 165.2 μmol) and 2d (60.0 mg, 173.72 μmol) were dissolved in pyridine (4.0 mL), $T_3P$ (3.0 mL, 50% in DMF) was dropwise added, and the reaction was allowed to proceed at room temperature for 16 hours. After LC-MS indicated the reaction was complete, the solvent was removed by concentration under reduced pressure, the reaction solution was diluted with ethyl acetate, adjusted to pH of about 13 with a saturated aqueous solution of sodium bicarbonate, and extracted with ethyl acetate for three times. The organic phases were combined, washed with water once, washed with saturated brine once, dried over anhydrous sodium sulfate, filtered, and concentrated, to afford compound 2h (100 mg). MS m/z (ESI): 594.0 $[M+H]^+$.

**Step 7: preparation of 4-amino-N-(1-(2-fluorobenzyl)-6-methylisoquinolin-5-yl)thieno[3,2-d]pyrimidine-7-carboxamide (compound 2)**

Compound 2h (100 mg, 168.44 μmol) was added to trifluoroacetic acid (3.0 mL), and the reaction was allowed to proceed at 70°C for 3 hours. After LC-MS indicated the reaction was complete, the reaction solution was concentrated to dryness, added with a saturated solution of sodium bicarbonate, and extracted with EA for three times. The organic phases were combined, washed with saturated brine once, dried over anhydrous sodium sulfate, filtered, and concentrated to afford a crude product 50 mg, among which 20 mg was separated and purified through Prep-HPLC to afford compound 2 (6.0 mg). MS m/z (ESI): 444.0 $[M+H]^+$.

$^1H$ NMR (400 MHz, DMSO-$d_6$) δ 11.64 (s, 1H), 8.95 (s, 1H), 8.58 (s, 1H), 8.38 (d, *J* = 6.0 Hz, 1H), 8.22 (d, *J* = 8.7 Hz, 1H), 7.97 (s, 2H), 7.68 (s, 1H), 7.66 (d, *J* = 2.5 Hz, 1H), 7.31-7.24 (m, 1H), 7.22-7.13 (m, 2H), 7.09 (td, *J* = 7.5, 1.2 Hz, 1H), 4.69 (s, 2H), 2.44 (s, 3H).

**Example 3: 4-amino-N-(1-(2-fluorobenzoyl)-6-methylisoquinolin-5-yl)thieno[3,2-d]pyrimidine-7-carboxamide (compound 3)**

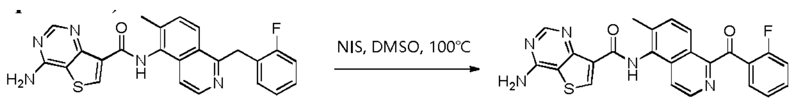


2 3

Compound 2 (30 mg, 67.64 μmol) was dissolved in DMSO (2.0 mL), NIS (15.22 mg, 67.64 μmol) was added, and the reaction was allowed to proceed at 100°C for 4 hours. After LC-MS indicated the reaction was complete, the reaction solution was cooled to room temperature, and was directly separated and purified by Prep-HPLC, to afford compound 3 (7.57 mg). MS m/z (ESI): 458.1 $[M+H]^+$.

$^1$HNMR (400 MHz, DMSO-$d_6$) δ 11.74 (s, 1H), 8.98 (s, 1H), 8.60 (s, 1H), 8.54 (d, *J* = 5.8 Hz, 1H), 8.31 *(d, J*= 8.7 Hz, 1H), 8.01 (d, *J* = 5.9 Hz, 1H), 7.98 (s, 2H), 7.85 (td, *J* = 7.5, 1.7 Hz, 1H), 7.78 *(d, J*= 8.8 Hz, 1H), 7.76 - 7.70 (m, 1H), 7.43 (td, *J* = 7.7, 0.7 Hz, 1H), 7.31 (dd, *J* = 10.5, 8.6 Hz, 1H), 2.49 (s, 3H).

**Example 4: 4-amino-N-(1-((2-fluorophenyl)(hydroxy)methyl)-6-methylisoquinolin-5-yl)thieno[3,2-d]pyrimidine-7-carboxamide (compound 4)**

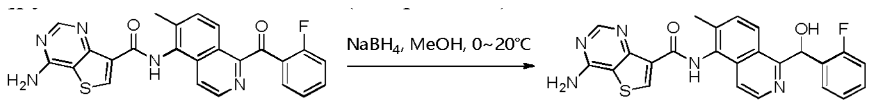


3 4

Compound 3 (7 mg, 15.30 μmol) was added to MeOH (4 mL), $NaBH_4$ (8.68 mg, 229.52 μmol) was added under ice-bath cooling, the reaction was naturally warmed to room temperature, and was continuously stirred at room temperature for 1 hour. After LC-MS indicated the reaction was complete, the solvent was concentrated to dryness, the residue was diluted by adding water, and extracted with EA for three times. The organic phases were combined, washed with water once, and washed with saturated brine once, dried over anhydrous sodium sulfate, filtered, concentrated, the residue was separated and purified through Prep-HPLC, to afford compound 4 (2.27 mg). MS m/z (ESI): 460.1 $[M+H]^+$.

$^1$HNMR (400 MHz, DMSO-$d_6$) δ 11.64 (s, 1H), 8.95 (s, 1H), 8.58 (s, 1H), 8.41 (d, *J* = 5.9 Hz, 1H), 8.32 (d, *J* = 8.7 Hz, 1H), 7.97 (s, 2H), 7.73 (d, *J* = 5.9 Hz, 1H), 7.68 - 7.58 (m, 2H), 7.35-7.31 (m, 1H), 7.22 (t, *J*= 7.4 Hz, 1H), 7.14 - 7.06 (m, 1H), 6.74 (d, *J*= 6.4 Hz, 1H), 6.40 (d, *J*= 6.4 Hz, 1H), 2.43 (s, 3H).

**Example 5: 4-amino-N-(1-(4-methoxybenzyl)-6-methylisoquinolin-5-yl)thieno[3,2-d]pyrimidine-7-carboxamide (compound 5)**

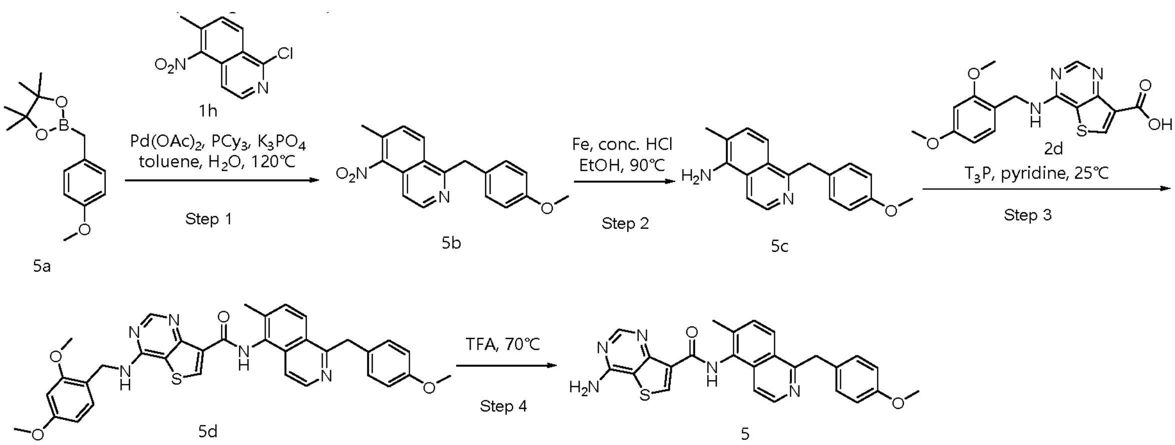


**Step 1: preparation of 1-(4-methoxybenzyl)-6-methyl-5-nitroisoquinoline (compound 5b)**

Compound 1h (100 mg, 449.18 μmol), 5a (668.72 mg, 2.70 mmol), palladium acetate (20.17 mg, 89.84 μmol), potassium phosphate (333.72 mg, 1.57 mmol) and tricyclohexylphosphane (25.19 mg, 89.84 μmol) were added to toluene (5.0 mL) and water (1.0 mL), purge with nitrogen was performed for three times, and the reaction was stirred at 120°C for 12 hours. After the reaction was complete, the reaction was diluted by adding EA, washed with saturated brine, dried over anhydrous sodium sulfate, filtered, concentrated, and the crude product was separated and purified by column chromatography on silica gel (DCM/MeOH=98/2), to afford compound 5b (110.0 mg). MS m/z (ESI): 309.1 $[M+H]^+$.

**Step 2: preparation of 1-(4-methoxybenzyl)-6-methylisoquinolin-5-amine (compound 5c)**

Compound 5b (260 mg, 843.25 μmol) was added to EtOH (5 mL), and stirred uniformly, concentrated hydrochloric acid (12 M, 1.05 mL) was then added. After the reaction was warmed to 60°C, iron powder (235.48 mg, 4.22 mmol) was slowly added, and the reaction was warmed to 90°C and allowed to proceed for 2 hours after the addition. After the reaction was complete, the reaction solution was rotary evaporated to dryness, diluted by adding 60 ml water, and extracted with EA. The organic phases were combined, washed with saturated brine, dried over anhydrous sodium sulfate, filtered, and concentrated, to afford compound 5c (250 mg). MS m/z (ESI): 279.1 $[M+H]^+$.

**Step 3: preparation of 4-((2,4-dimethoxybenzyl)amino)-N-(1-(4-methoxybenzyl)-6-methylisoquinolin-5-yl)thieno[3,2-d]pyrimidine-7-carboxamide (compound 5d)**

Compounds 2d (49.63 mg, 143.71 μmol) and 5c (50 mg, 143.71 μmol) were added to pyridine (4 mL), and dissolved with stirring, $T_3P$ (3 mL, 50% in EA) was then added, and the reaction was allowed to proceed under the protection of nitrogen at room temperature overnight. After the reaction was complete, the reaction solution was directly rotary evaporated to dryness, added with 60 ml water, and extracted with EA (30 mL x 3). The organic phases were combined, washed with brine, dried over anhydrous sodium sulfate, filtered, concentrated, and the crude product was separated and purified by column chromatography on silica gel (EA/PE=1/1), to afford compound 5d (51.0 mg). MS m/z (ESI): 606.3 $[M+H]^+$.

**Step 4: preparation of 4-amino-N-(1-(4-methoxybenzyl)-6-methylisoquinolin-5-yl)thieno[3,2-d]pyrimidine-7-carboxamide (compound 5)**

Compound 5d (35 mg, 57.78 μmol) was added to trifluoroacetic acid (3.0 mL), and the reaction was allowed to proceed at 70°C for 3 hours. After the reaction was complete, the reaction solution was concentrated to dryness, added with a saturated solution of sodium bicarbonate for alkalization, and extracted with ethyl acetate. The organic phase was dried over anhydrous sodium sulfate, filtered, concentrated, and the crude product was separated and purified by Prep-HPLC, to afford compound 5 (15.0 mg). MS m/z (ESI): 456.1 $[M+H]^+$.

$^1$H NMR (400 MHz, DMSO-$d_6$) δ 11.62 (s, 1H), 8.94 (s, 1H), 8.57 (s, 1H), 8.42 (d, $J$ = 5.6 Hz, 1H), 8.23 (d, $J$ = 8.4 Hz, 1H), 7.97 (s, 2H), 7.65 (d, $J$ = 6.0 Hz, 1H), 7.60 (d, $J$ = 8.8 Hz, 1H), 7.24 - 7.18 (m, 2H), 6.84 - 6.78 (m, 2H), 4.58 (s, 2H), 3.68 (s, 3H), 2.41 (s, 3H).

**Example 6: 4-amino-N-(1-(4-(methoxybenzoyl)-6-methylisoquinolin-5-yl)thieno[3,2-d]pyrimidine-7-carboxamide (compound 7)**

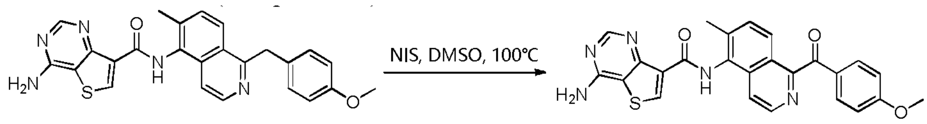


5 7

Compound 5 (10.0 mg, 21.95 μmol) and NIS (4.94 mg, 21.95 μmol) were dissolved in DMSO (2.0 mL), and the reaction was allowed to proceed at 100°C for 3 hours. After the reaction was complete, the reaction solution was cooled to room temperature, and then directly separated and purified by Prep-HPLC, to afford compound 7 (5.0 mg). MS m/z (ESI): 470.1 $[M+H]^+$.

$^1$H NMR (400 MHz, DMSO-$d_6$) δ 11.74 (s, 1H), 8.98 (s, 1H), 8.61 (s, 1H), 8.58 (d, $J$ = 6.0 Hz, 1H), 7.99 (s, 2H), 7.97 - 7.94 (m, 1H), 7.87 (d, $J$ = 8.8 Hz, 1H), 7.84 - 7.79 (m, 2H), 7.68 (d, $J$ = 8.8 Hz, 1H), 7.11 - 7.06 (m, 2H), 3.86 (s, 3H), 2.47 (s, 3H).

**Example 7: 4-amino-N-(1-(hydroxy(4-methoxyphenyl)methyl)-6-methylisoquinolin-5-yl)thieno[3,2-d]pyrimidine-7-carboxamide (compound 6)**

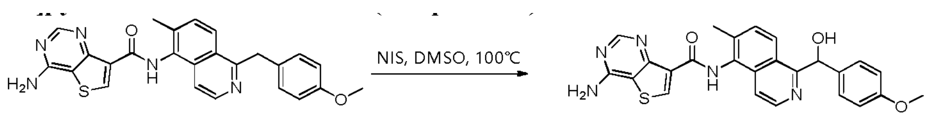


5 6

Compound 5 (35 mg, 76.83 μmol) and NIS (17.29 mg, 76.83 μmol) were dissolved in DMSO (3.0 mL), and the reaction was allowed to proceed at 100°C for 3 hours. After the reaction was complete, the reaction solution was cooled to room temperature, and then directly separated and purified by Prep-HPLC to afford compound 6 (8.0 mg). MS m/z (ESI): 472.1 $[M+H]^+$;

$^1$H NMR (400 MHz, DMSO-$d_6$) δ 11.61 (s, 1H), 8.94 (s, 1H), 8.57 (s, 1H), 8.47 (d, $J$ = 6.0 Hz, 1H), 8.31 (d, $J$ =

8.8 Hz, 1H), 7.96 (s, 2H), 7.72 (d, *J* = 6.0 Hz, 1H), 7.53 (d, *J* = 8.8 Hz, 1H), 7.32 - 7.27 (m, 2H), 6.86 - 6.82 (m, 2H), 6.36 (d, *J* = 5.6 Hz, 1H), 6.30 (d, *J* = 5.2 Hz, 1H), 3.69 (s, 3H), 2.39 (s, 3H).

**Example 8: 4-amino-N-(1-(2-chlorobenzyl)-6-methylisoquinolin-5-yl)thieno[3,2-d]pyrimidine-7-carboxamide (compound 10)**

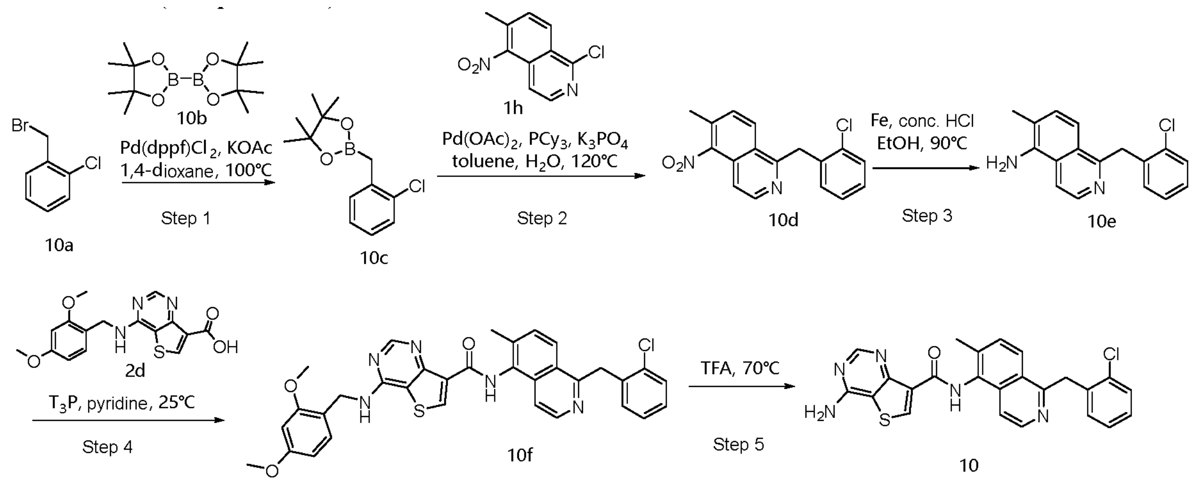


**Step 1: preparation of 2-(2-chlorobenzyl)-4,4,5,5-tetramethyl-1,3,2-dioxaborolane (compound 10c)**

Compounds 10a (1.25 g, 6.08 mmol), 10b (1.70 g, 6.69 mmol), Pd(dppf)$Cl_2$·DCM (496.40 mg, 608.33 μmol) and potassium acetate (1.49 g, 15.21 mmol) were added to 1,4-dioxane (4 mL), purge with nitrogen was performed for three times, and the reaction was heated to 100°C and allowed to proceed for 3 hours. After the reaction was complete, the reaction solution was filtered through diatomaceous earth, diluted by adding 100 ml water, and extracted with EA (50 ml x 3). The organic phases were combined, washed with brine, dried over anhydrous sodium sulfate, filtered, concentrated, and the crude product was separated and purified by column chromatography on silica gel (PE/EA=95/5), to afford compound 10c (800 mg).

**Step 2: preparation of 1-(2-chlorobenzyl)-6-methyl-5-nitroisoquinoline (compound 10d)**

Compounds 1h (100 mg, 449.18 μmol), 10c (340.31 mg, 1.35 mmol), palladium acetate (20.17 mg, 89.84 μmol), potassium phosphate (333.72 mg, 1.57 mmol) and tricyclohexylphosphane (25.19 mg, 89.84 μmol) were added to toluene (5 mL) and water (1 mL), purge with nitrogen was performed for three times, and then the reaction was stirred at 120°C for 12 hours. After the reaction was complete, the reaction was diluted by adding EA, washed with saturated brine, dried over anhydrous sodium sulfate, filtered, concentrated, and the crude product was separated and purified by column chromatography on silica gel (PE/EA=95/5), to afford compound 10d (120.0 mg). MS m/z (ESI): 313.0 $[M+H]^+$.

**Step 3: preparation of 1-(2-chlorobenzyl)-6-methylisoquinolin-5-amine (compound 10c)**

Compound 10d (90 mg, 287.77 μmol) was added to EtOH (5 mL), and stirred uniformly before addition of HCl (12 M, 0.36 mL), the reaction was then warmed to 60°C, iron powder (80.36 mg, 1.44 mmol) was slowly added, and the reaction was warmed to 90°C and allowed to proceed for 2 hours after the addition. After the reaction was complete, the reaction solution was rotary evaporated to dryness, the residue was diluted by adding 60 ml water, and extracted with EA. The organic phase was washed with a saturated saline solution, dried over anhydrous sodium sulfate, filtered, and concentrated, to afford compound 10e (95 mg). MS m/z (ESI): 283.1 $[M+H]^+$.

**Step 4: preparation of N-(1-(2-chlorobenzyl)-6-methylisoquinolin-5-yl)-4-((2,4-dimethoxybenzyl)amino)thieno[3,2-d]pyrimidine-7-carboxamide (compound 10f)**

Compounds 2d (103.82 mg, 300.60 μmol), and 10e (85 mg, 300.60 μmol) were added to pyridine (3 mL), and dissolved with stirring before the addition of $T_3P$ (2 mL, 50% in EA). The reaction was allowed to proceed under the

protection of nitrogen at room temperature overnight. After the reaction was complete, the reaction solution was directly rotary evaporated to dryness, the residue was diluted by adding 60 ml water, and extracted with EA (30 ml x 3). The organic phases were combined, washed with brine, dried over anhydrous sodium sulfate, filtered, concentrated, and the crude product was separated and purified by column chromatography on silica gel (EA/PE=1/1), to afford compound 10f (127 mg). MS m/z (ESI): 610.2 $[M+H]^+$.

**Step 5: preparation of 4-amino-N-(1-(2-chlorobenzyl)-6-methylisoquinolin-5-yl)thieno[3,2-d]pyrimidine-7-carboxamide (compound 10)**

Compound 10f (125 mg, 204.88 μmol) was added to trifluoroacetic acid (5.0 mL), and the reaction was allowed to proceed at 70°C for 3 hours. After the reaction was complete, the reaction solution was concentrated to dryness, the residue was added with a saturated solution of sodium bicarbonate for alkalization, and extracted with ethyl acetate. The organic phase was dried over anhydrous sodium sulfate, filtered, and concentrated, to afford a crude product 85 mg, among which 20 mg was separated and purified through Prep-HPLC, to afford compound 10 (4.04 mg). MS m/z (ESI): 460.0 $[M+H]^+$.

$^1$H NMR (400 MHz, DMSO-$d_6$) δ 11.66 (s, 1H), 8.95 (s, 1H), 8.58 (s, 1H), 8.36 (d, *J* = 6.0 Hz, 1H), 8.19 (d, *J*= 8.8 Hz, 1H), 7.97 (s, 2H), 7.70 - 7.64 (m, 2H), 7.51 - 7.46 (m, 1H), 7.30 - 7.20 (m, 2H), 7.13 - 7.08 (m, 1H), 4.77 (s, 2H), 2.44 (s, 3H).

**Example 9: 4-amino-N-(1-(2-chlorobenzoyl)-6-methylisoquinolin-5-yl)thieno[3,2-d]pyrimidine-7-carboxamide (compound 11)**

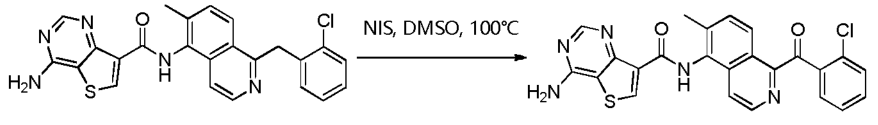


10 11

Compound 10 (20.0 mg, 43.48 μmol) and NIS (24.5 mg, 108.71 μmol) were dissolved in DMSO (2.0 mL), and the reaction was allowed to proceed at 100°C for 3 hours. After the reaction was complete, the reaction solution was cooled to room temperature, and then directly separated and purified by Prep-HPLC, to afford compound 11 (8.0 mg). MS m/z (ESI): 474.0 $[M+H]^+$.

$^1$H NMR (400 MHz, DMSO-$d_6$) δ 11.74 (s, 1H), 8.97 (s, 1H), 8.60 - 8.56 (m, 2H), 8.53 (d, *J* = 5.6 Hz, 1H), 8.05 - 8.02 (m, 1H), 7.98 (s, 2H), 7.84 (d, *J* = 8.8 Hz, 1H), 7.71 (dd, *J* = 7.6, 1.6 Hz, 1H), 7.64 - 7.58 (m, 1H), 7.56 - 7.50 (m, 2H), 2.50 (s, 3H).

**Example 10: 4-amino-N-(1-((2-chlorophenyl)(hydroxy)methyl)-6-methylisoquinolin-5-yl)thieno[3,2-d]pyrimidine-7-carboxamide (compound 14)**

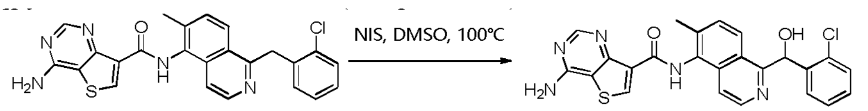


10 14

Compound 10 (25.0 mg, 54.35 μmol) and NIS (13.5 mg, 59.79 μmol) were dissolved in DMSO (2.0 mL), and the reaction was allowed to proceed at 100°C for 3 hours. After the reaction was complete, the reaction solution was cooled to room temperature, and then directly separated and purified by Prep-HPLC, to afford compound 14 (4.0 mg). MS m/z (ESI): 476.0 $[M+H]^+$.

$^1$H NMR (400 MHz, DMSO-$d_6$) δ 11.65 (s, 1H), 8.95 (s, 1H), 8.58 (s, 1H), 8.38 (d, *J* = 8.4 Hz, 1H), 8.34 (d, *J* = 6.0 Hz, 1H), 7.97 (s, 2H), 7.75 - 7.65 (m, 3H), 7.44 - 7.36 (m, 2H), 7.34 - 7.29 (m, 1H), 6.82 (d, *J* = 5.6 Hz, 1H), 6.40 (d, *J* = 5.6 Hz, 1H), 2.45 (s, 3H).

**Example 11: 4-amino-N-(1-(2-chlorobenzyl)-6-methylisoquinolin-5-yl)quinazoline-8-carboxamide (compound 25)**

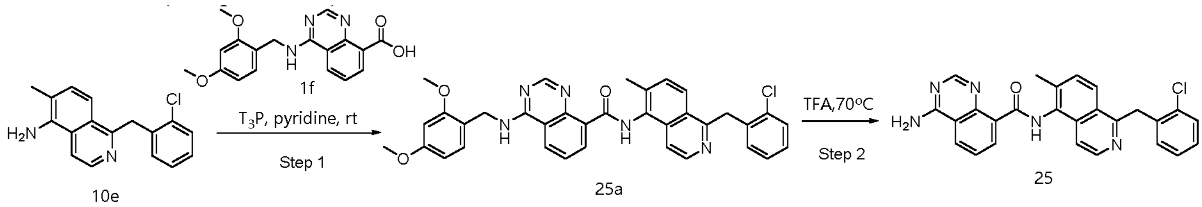


**Step 1: preparation of N-(1-(2-chlorobenzyl)-6-methylisoquinolin-5-yl)-4-((2,4-dimethoxybenzyl)amino)quinazoline-8-carboxamide (compound 25a)**

Compounds 10e (60 mg, 212.19 μmol) and 1f (72 mg, 212.19 μmol) were added to pyridine (3 mL), and dissolved with stirring before addition of $T_3P$ (2 mL, 50% in DMF), and the reaction was allowed to proceed under the protection of nitrogen at room temperature overnight. After the reaction was complete, the reaction solution was directly rotary evaporated to dryness, the residue was diluted by adding 60 ml water, extracted with EA (30 mL x3), the organic phases were combined, washed with brine, dried over anhydrous sodium sulfate, filtered, concentrated, and the crude product was separated and purified by column chromatography on silica gel (DCM/MeOH=10/1), to afford compound 25a (45 mg). MS m/z (ESI): 604.1 $[M+H]^+$.

**Step 2: preparation of 4-amino-N-(1-(2-chlorobenzyl)-6-methylisoquinolin-5-yl)quinazoline-8-carboxamide (compound 25)**

Compound 25a (45 mg, 74.49 μmol) was added to trifluoroacetic acid (5.0 mL), and the reaction was allowed to proceed at 70°C for 2 hours. After the reaction was complete, the reaction solution was concentrated to dryness, added with a saturated solution of sodium bicarbonate for alkalization, and extracted with ethyl acetate. The organic phase was dried over anhydrous sodium sulfate, filtered, concentrated, and the crude product was separated and purified by Prep-HPLC, to afford compound 25 (3.21 mg). MS m/z (ESI): 454.1 $[M+H]^+$.

$^1$H NMR (400 MHz, DMSO-$d_6$) δ 13.26 (s, 1H), 8.67 - 8.60 (m, 2H), 8.53 (dd, *J* = 8.0, 1.6 Hz, 1H), 8.46 - 8.13 (m, 4H), 7.76 (d, *J* = 6.0 Hz, 1H), 7.72 - 7.64 (m, 2H), 7.48 (dd, *J* = 7.6, 1.6 Hz, 1H), 7.31 - 7.19 (m, 2H), 7.10 (dd, *J* = 7.2, 2.0 Hz, 1H), 4.77 (s, 2H), 2.46 (s, 3H).

**Example 12: 4-amino-N-(1-((2-chlorophenyl)(hydroxy)methyl)-6-methylisoquinolin-5-yl)quinazoline-8-carboxamide (compound 24)**

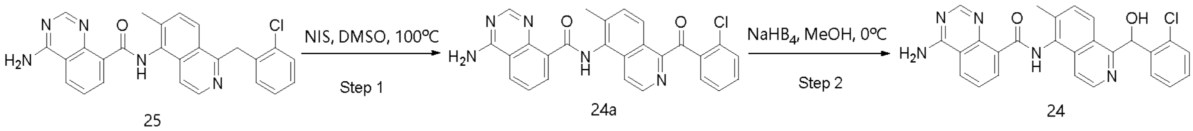


**Step 1: preparation of 4-amino-N-(1-(2-chlorobenzoyl)-6-methylisoquinolin-5-yl)quinazoline-8-carboxamide (compound 24a)**

Compound 25 (20 mg, 44.06 μmol) and NIS (10.41 mg, 46.26 μmol) were dissolved in DMSO (3.0 mL), and the reaction was allowed to proceed at 100°C for 3 hours. The reaction was diluted by adding 20 ml water, extracted with EA (15 ml x3), the organic phases were combined, washed with brine, dried over anhydrous sodium sulfate, filtered, and concentrated, to afford compound 24a (15 mg). MS m/z (ESI): 468.0 $[M+H]^+$.

**Step 2: preparation of 4-amino-N-(1-((2-chlorophenyl)(hydroxy)methyl)-6-methylisoquinolin-5-yl)quinazoline-8-carboxamide (compound 24)**

Compound 24a (20 mg, 42.74 μmol) was dissolved in methanol (3.0 mL), $NaBH_4$ (14.37 mg, 213.72 μmol) was added under ice-bath cooling, and the reaction was allowed to proceed at 0°C for 2 hours after the addition. After the reaction was complete, the reaction solution was directly separated and purified by Prep-HPLC, to afford compound 24 (8 mg). MS m/z (ESI): 470.0 $[M+H]^+$.

$^1H$ NMR (400 MHz, DMSO-$d_6$) δ 13.24 (s, 1H), 8.66 - 8.60 (m, 2H), 8.55 - 8.50 (m, 1H), 8.42 - 8.32 (m, 3H), 8.30 - 8.16 (m, 1H), 7.80 (d, *J* = 6.0 Hz, 1H), 7.73 - 7.66 (m, 3H), 7.43 - 7.36 (m, 2H), 7.34 - 7.28 (m, 1H), 6.82 (d, *J* = 5.6 Hz, 1H), 6.39 (d, *J* = 6.8 Hz, 1H), 2.47 (s, 3H).

**Example 13: 4-amino-N-(1-((3-chloro-2-fluorophenyl)amino)-6-methylisoquinolin-5-yl)imidazo[2,1-f][1,2,4]triazine-7-carboxamide (compound 30)**

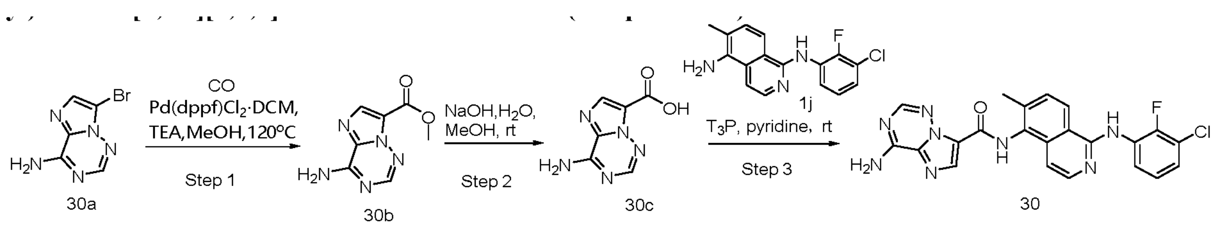


**Step 1: preparation of methyl 4-aminoimidazo[2,1-f][1,2,4]triazine-7-carboxylate (compound 30b)**

Compound 30a (200 mg, 0.934 mmol), TEA (472.8 mg, 4.67 mmol), Pd(dppf)$Cl_2$·DCM (76.31 mg, 93.45 μmol) and MeOH (5 mL) were added to an autoclave, after being sealed, carbon monoxide was pumped in to 1.0-1.2 Mpa, and the reaction was heated to 120°C and allowed to proceed for 5 hours. After the reaction was complete, the reaction solution was cooled to room temperature, a large amount of solid precipitated, which was filtered under reduced pressure. The filter cake was washed with water, and then dried at 50°C under reduced pressure. The solid was then slurried with 5 mL methanol, filtered and dried to afford compound 30b (160 mg). MS m/z (ESI): 194.0 $[M+H]^+$.

**Step 2: preparation of 4-aminoimidazo[2,1-f][1,2,4]triazine-7-carboxylic acid (compound 30c)**

30b (50 mg, 258.85 μmol) was added to MeOH (3 mL), followed by addition of a solution of NaOH (51.77 mg, 1.29 mmol) in water (0.5 mL), the reaction was allowed to proceed at room temperature overnight. After the reaction was complete, the reaction solution was adjusted to pH of 5-6 with dilute hydrochloric acid, and evaporated under reduced pressure to remove MeOH. Solid precipitated, which was filtered, and the filter cake was washed with water, dried under reduced pressure to afford compound 30c (40 mg). MS m/z (ESI): 180.1 $[M+H]^+$.

**Step 3: preparation of 4-amino-N-(1-((3-chloro-2-fluorophenyl)amino)-6-methylisoquinolin-5-yl)imidazo[2,1-f][1,2,4]triazine-7-carboxamide (compound 30)**

Compounds 1j (50 mg, 165.70 μmol) and 30c (78.11 mg, 165.70 μmol) were added to pyridine (3 mL), and dissolved with stirring before addition of $T_3P$ (2 mL, 50% in DMF). The reaction was allowed to proceed under the protection of nitrogen at room temperature overnight. After the reaction was complete, the reaction solution was directly rotary evaporated to dryness, the residue was diluted by adding 60 ml water, extracted with EA (30 mL x3), the organic phases were combined, washed with brine, dried over anhydrous sodium sulfate, filtered, concentrated, and the crude product was separated and purified through Pre-HPLC to afford compound 30 (55.0 mg). MS m/z (ESI): 463.0 $[M+H]^+$.

$^1H$ NMR (400 MHz, DMSO-$d_6$) δ 10.38 (s, 1H), 9.25 (s, 1H), 8.69 (s, 1H), 8.61 (s, 1H), 8.39 - 8.34 (m, 2H), 8.24 (s, 1H), 7.90 (d, *J* = 6.0 Hz, 1H), 7.62 (d, *J* = 8.8 Hz, 1H), 7.58 - 7.51 (m, 1H), 7.39 - 7.33 (m, 1H), 7.26 - 7.18 (m, 2H), 2.44 (s, 3H).

**Example 14: 4-amino-N-(1-(3-chloro-2-fluorobenzyl)-6-methylisoquinolin-5-yl)thieno[3,2-d]pyrimidine-7-carboxamide (compound 8)**

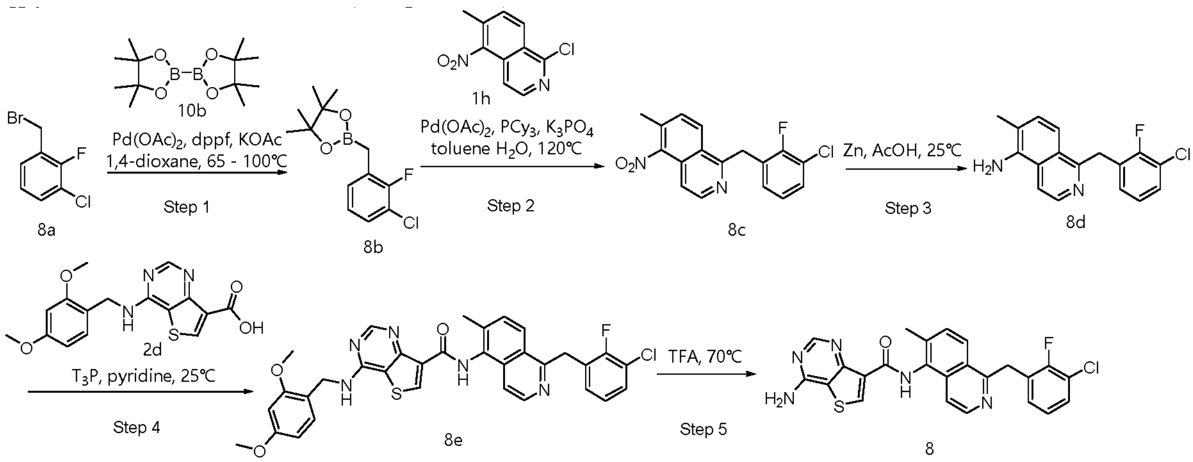


**Step 1: preparation of 2-(3-chloro-2-fluorobenzyl)-4,4,5,5-tetramethyl-1,3,2-dioxaborolane (compound 8b)**

Compounds 8a (2.0 g, 8.9 mmol), 10b (3.4 g, 13.3 mmol), palladium acetate (121.8 mg, 531.6 μmol), 1,1'-bis(diphenylphosphino)ferrocene (312.8 mg, 531.6 μmol) and potassium acetate (1.33 g, 13.3 mmol) were added to 1,4-dioxane (30.0 mL), and the reaction was allowed to proceed at 100°C under the protection of nitrogen for 4 hours, then proceed at 65°C for 16 hours. After the reaction was complete, the reaction solution was concentrated to dryness, and the crude product was separated and purified by column chromatography on silica gel (PE/EA=19/1), to afford compound 8b (1.0 g).

**Step 2: preparation of 1-(3-chloro-2-fluorobenzyl)-6-methyl-5-nitroisoquinoline (compound 8c)**

Compounds 8b (721.8 mg, 2.7 mmol), 1h (200 mg, 889.4 μmol), palladium acetate (40.3 mg, 177.9 μmol), potassium phosphate (667.4 mg, 3.1 mmol) and tricyclohexylphosphane (50.4 mg, 177.9 μmol), water (1.5 mL) and toluene (10.0 mL) were added to a reaction flask, purge with nitrogen was performed, and the reaction was allowed to proceed at 120°C for 12 hours. After the reaction was complete, the reaction solution was concentrated to dryness, the crude product was separated and purified by column chromatography on silica gel (PE/EA=4/1), to afford compound 8c (140 mg). MS m/z (ESI): 331.1 $[M+H]^+$.

**Step 3: preparation of 1-(3-chloro-2-fluorobenzyl)-6-methylisoquinolin-5-amine (compound 8d)**

Compound 8c (130 mg, 393.1 μmol) was dissolved in glacial acetic acid (10.0 mL), purge with nitrogen was performed, zinc powder (311.5 mg, 4.72 mmol) was then added in batches, and the reaction was allowed to proceed at 25°C for 30 minutes. After the reaction was complete, the solution was filtered with suction, concentrated, alkalized with a saturated aqueous solution of sodium bicarbonate, and extracted with dichloromethane. The organic phase was dried over anhydrous sodium sulfate, filtered with suction, concentrated, and the crude product was separated and purified by column chromatography on silica gel (PE/EA=1/1), to afford compound 8d (118 mg). MS m/z (ESI): 301.1 $[M+H]^+$.

**Step 4: preparation of N-(1-(3-chloro-2-fluorobenzyl)-6-methylisoquinolin-5-yl)-4-((2,4-dimethoxybenzyl)amino)thieno[3,2-d]pyrimidine-7-carboxamide (compound 8e)**

Compounds 8d (110 mg, 365.7 μmol) and 2d (126.3 mg, 365.7 μmol) were dissolved in pyridine (5.0 mL), purge with nitrogen was performed, followed by addition of $T_3P$ (2.1 g, 3.34 mmol), and the reaction was allowed to proceed at 25°C for 16 hours. After the reaction was complete, the solution was concentrated, alkalized with a saturated aqueous solution of sodium bicarbonate, and extracted with ethyl acetate. The organic phase was dried over anhydrous sodium sulfate, filtered with suction, concentrated, and the crude product was separated and purified by column chromatography on silica gel (DCM/MeOH=19/1), to afford compound 8e (176 mg). MS m/z (ESI): 628.2 $[M+H]^+$.

**Step 5: preparation of 4-amino-N-(1-(3-chloro-2-fluorobenzyl)-6-methylisoquinolin-5-yl)thieno[3,2-d]pyrimidine-7-carboxamide (compound 8)**

Compound 8e (176 mg, 280.2 μmol) was added to trifluoroacetic acid (5.0 mL), and the reaction was allowed to proceed at 70°C for 3 hours. After the reaction was complete, the reaction solution was concentrated to dryness, then

added with methanol and potassium carbonate and stirred for 10 minutes for alkalization, filtered with suction, concentrated, and the crude product was separated and purified by Prep-HPLC, to afford compound 8 (120.0 mg). MS m/z (ESI): 478.0 $[M+H]^+$.

$^1$H NMR (400 MHz, DMSO-$d_6$) δ 11.65 (br, 1H), 8.95 (s, 1H), 8.58 (s, 1H), 8.36 (d, *J* = 6.0 Hz, 1H), 8.26 (d, *J* = 8.8 Hz, 1H), 7.97 (s, 2H), 7.70 (d, *J* = 8.8 Hz, 1H), 7.67 (d, *J* = 6.0 Hz, 1H), 7.46 (td, *J* = 7.6, 1.6 Hz, 1H), 7.21 - 7.12 (m, 2H), 4.76 (s, 2H), 2.45 (s, 3H).

**Example 15: 4-amino-N-(1-((3-chloro-2-fluorophenyl)(hydroxy)methyl)-6-methylisoquinolin-5-yl)thieno[3,2-d]pyrimidine-7-carboxamide (compound 9)**

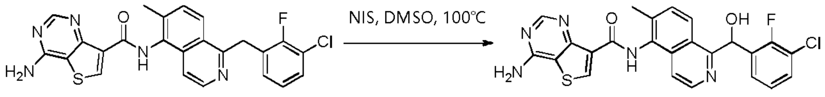


8 9

Compound 8 (100 mg, 209.2 μmol) and NIS (47.6 mg, 209.2 μmol) were dissolved in DMSO (4.0 mL), and the reaction was allowed to proceed at 100°C for 4 hours. After the reaction was complete, the reaction solution was cooled to room temperature, and then directly separated and purified by Prep-HPLC, to afford compound 9 (1.7 mg). MS m/z (ESI): 494.0 $[M+H]^+$.

$^1$H NMR (400 MHz, DMSO-$d_6$) δ 11.65 (s, 1H), 8.95 (s, 1H), 8.58 (s, 1H), 8.38 (d, *J*= 6.0 Hz, 2H), 7.97 (s, 2H), 7.73 (d, *J*= 5.9 Hz, 1H), 7.69 (d, *J*= 8.7 Hz, 1H), 7.64 (t, *J*= 6.9 Hz, 1H), 7.50 (t, *J*= 7.0 Hz, 1H), 7.28 (t, *J*= 7.9 Hz, 1H), 6.79 (s, 1H), 6.57 (s, 1H), 2.45 (s, 3H).

**Example 16: 4-amino-N-(1-((4-((dimethylamino)methyl)phenyl)amino)-6-methylisoquinolin-5-yl)quinazoline-8-carboxamide (compound 40)**

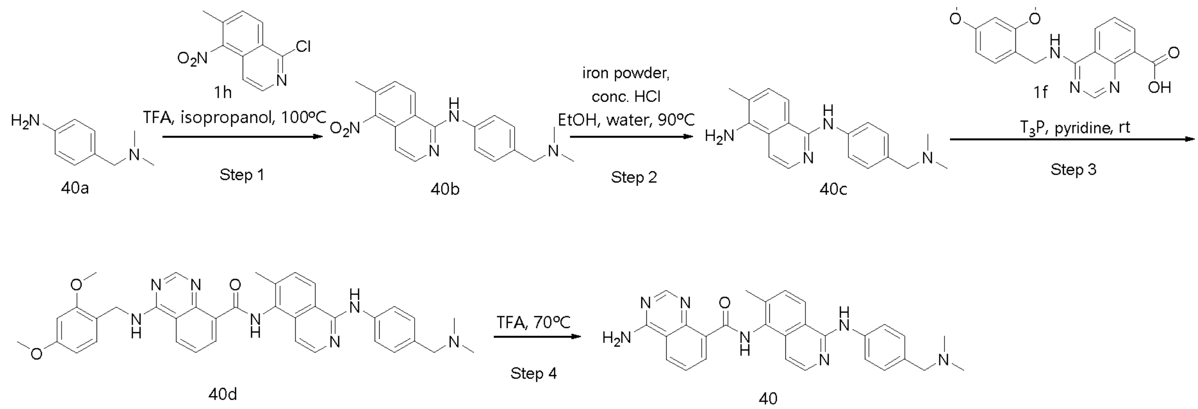


40a 40b 40c 40d 40

**Step 1: preparation of N-(4-((dimethylamino)methyl)phenyl)-6-methyl-5-nitroisoquinolin-1-amine (compound 40b)**

Compounds 40a (121.46 mg, 808.53 μmol) and 1h (150 mg, 673.77 μmol) were added to isopropanol (5 mL), followed by addition of TFA (92 mg, 808.53 μmol), and the reaction was stirred at a temperature of 100°C for 18 hours. After the reaction was complete, the reaction solution was naturally cooled to room temperature, filtered with suction, the filter cake was rinsed with isopropanol, and the resulting solid was dried under reduced pressure to afford compound 40b (170 mg). MS m/z (ESI): 337.2 $[M+H]^+$.

**Step 2: preparation of $N^1$-(4-((dimethylamino)methyl)phenyl)-6-methylisoquinoline-1,5-diamine (compound 40c)**

Compound 40b (170 mg, 505.37 μmol) was added to ethanol (8 mL) and water (0.5 mL), followed by addition of iron powder (141.12 mg, 2.53 mmol) and concentrated hydrochloric acid (12M, 630 μL), and after the addition the reaction was heated to 90°C and stirred for 2 hours. After the reaction was complete, the reaction solution was concentrated under reduced pressure to remove most of the solvent, then diluted by adding water, extracted with EA for three times, the organic phases were combined, dried over anhydrous sodium sulfate, filtered, and concentrated, to afford compound 40c (120 mg). MS m/z (ESI): 307.2 $[M+H]^+$.

**Step 3: preparation of 4-((2,4-dimethoxybenzyl)amino)-N-(1-((4-((dimethylamino)methyl)phenyl)amino)-6-methylisoquinolin-5-yl)quinazoline-8-carboxamide (compound 40d)**

Compounds 1f (50 mg, 147.34 μmol) and 40c (45.15 mg, 147.34 μmol) were added to pyridine (3 mL), then added with $T_3P$ (2 mL, 50% in DMF), and the reaction was allowed to proceed under the protection of nitrogen at room temperature overnight. After the reaction was complete, the reaction solution was directly rotary evaporated to dryness, added with a saturated solution of sodium bicarbonate for alkalization, and extracted with EA. The organic phase was washed with brine, dried over anhydrous sodium sulfate, filtered, and concentrated, to afford compound 40d (110.0 mg). MS m/z (ESI): 628.2 $[M+H]^+$.

**Step 4: preparation of 4-amino-N-(1-((4-((dimethylamino)methyl)phenyl)amino)-6-methylisoquinolin-5-yl)quinazoline-8-carboxamide (compound 40)**

Compound 40d (100 mg, 159.30 μmol) was added to trifluoroacetic acid (5.0 mL), and the reaction was allowed to proceed at 70°C for 2 hours. After the reaction was complete, the reaction solution was concentrated to dryness, added with a saturated solution of sodium bicarbonate for alkalization, and extracted with ethyl acetate. The organic phase was dried over anhydrous sodium sulfate, filtered, concentrated, and the crude product was separated and purified by Prep-HPLC, to afford a trifluoroacetate salt of compound 40 (8.0 mg). MS m/z (ESI): 478.2 $[M+H]^+$.

$^1H$ NMR (400 MHz, DMSO-$d_6$) δ 13.27 - 12.46 (br, 1H), 10.21 - 9.56 (m, 2H), 8.94 - 8.21 (m, 5H), 8.00 - 7.67 (m, 5H), 7.50 (d, *J* = 8.4 Hz, 2H), 7.34 (d, *J* = 6.4 Hz, 1H), 4.28 (d, *J* = 4.8 Hz, 2H), 2.76 (d, *J* = 4.8 Hz, 6H), 2.48 (s, 3H).

**Example 17: 4-amino-N-(1-(4-fluorobenzyl)-6-methylisoquinolin-5-yl)thieno[3,2-d]pyrimidine-7-carboxamide (compound 13)**

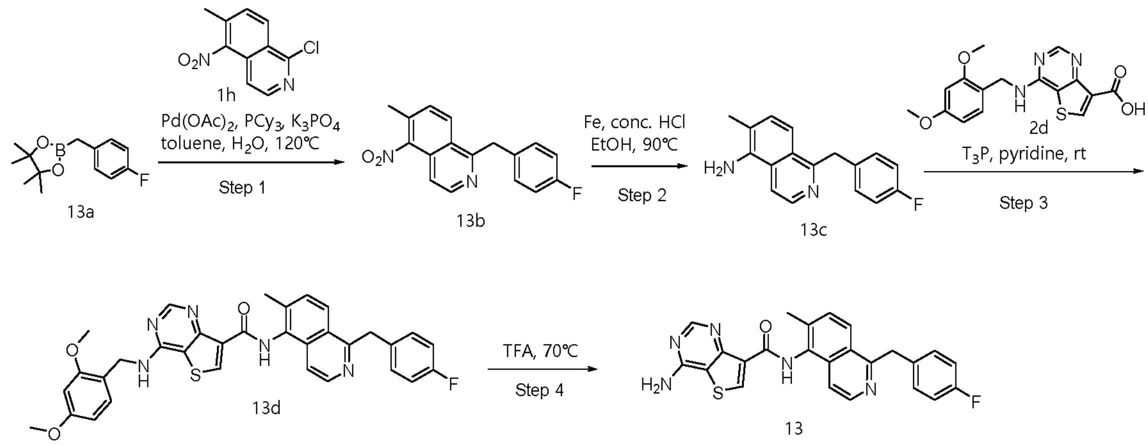


**Step 1: preparation of 1-(4-fluorobenzyl)-6-methyl-5-nitroisoquinoline (compound 13b)**

Compounds 1h (200 mg, 898.36 μmol), 13a (1.7 g, 7.19 mmol), palladium acetate (40.34 mg, 179.67 μmol), tricyclohexylphosphane (25.19 mg, 89.84 μmol) and potassium phosphate (667.43 mg, 3.14 mmol) were added to toluene (3.0 mL) and water (1 mL), purge with nitrogen was performed for three times, and the reaction was allowed to proceed at 120°C for 12 hours. After the reaction was complete, the reaction solution was filtered through diatomaceous earth, added with 200 ml water, and extracted with EA. The organic phase was dried over anhydrous sodium sulfate, filtered, concentrated, separated and purified by Flash column chromatography on silica gel (DCM:MeOH=20:1), to afford compound 13b (165 mg). MS m/z (ESI): 297.1 $[M+H]^+$.

**Step 2: preparation of 1-(4-fluorobenzyl)-6-methylisoquinolin-5-amine (compound 13c)**

Compound 13b (210 mg, 708.75 μmol) was added to ethanol (3.0 mL) and water (1 mL), followed by addition of iron powder (197.92 mg, 3.54 mmol) and concentrated hydrochloric acid (12M, 2 mL), purge with nitrogen was performed for three times, and the reaction was allowed to proceed at 90°C for 3.5 hours. After the reaction was complete, the reaction solution was filtered through diatomaceous earth, added with 200 ml water, and extracted with EA. The organic phase was dried over anhydrous sodium sulfate, filtered, concentrated, separated and separated and purified by Flash column chromatography on silica gel (DCM/MeOH=20/1), to afford compound 13c (165 mg). MS m/z (ESI): 267.2 $[M+H]^+$.

**Step 3: preparation of 4-((2,4-dimethoxybenzyl)amino)-N-(1-(4-fluorobenzyl)-6-methylisoquinolin-5-yl)thieno[3,2-d]pyrimidine-7-carboxamide (compound 13d)**

Compounds 13c (36 mg, 135.18 μmol), 2d (56.02 mg, 162.22 μmol) were added to pyridine (3.0 mL), followed by addition of $T_3P$ (129.03 mg, 405.54 μmol), the reaction was allowed to proceed at room temperature for 1 hour. After the reaction was complete, the mixture was added with 50 ml water, and extracted with EA. The organic phase was dried over anhydrous sodium sulfate, filtered, concentrated, separated and purified by Flash column chromatography on silica gel (DCM/MeOH=20:1), to afford compound 13d (51 mg). MS m/z (ESI): 594.2 $[M+H]^+$.

**Step 4: preparation of 4-amino-N-(1-(4-fluorobenzyl)-6-methylisoquinolin-5-yl)thieno[3,2-d]pyrimidine-7-carboxamide (compound 13)**

Compound 13d (101 mg, 170.13 μmol) was added to trifluoroacetic acid (3.0 mL), and the reaction was allowed to proceed at 70°C for 3 hours. After the reaction was complete, the reaction solution was concentrated to dryness, added with a saturated solution of sodium bicarbonate for alkalization, and extracted with ethyl acetate. The organic phase was dried over anhydrous sodium sulfate, filtered, concentrated, separated and purified through Prep-HPLC, to afford a trifluoroacetate salt of compound 13 (1.3 mg). MS m/z (ESI): 444.1 $[M+H]^+$.

$^1H$ NMR (400 MHz, DMSO-$d_6$) δ 11.61 (s, 1H), 9.04 (s, 1H), 8.59 (s, 1H), 8.51 (d, *J* = 4 Hz, 1H), 8.45 (d, *J* = 8.8 Hz, 1H), 8.24 (brs, 2H), 7.97 (d, *J* = 6.4 Hz, 1H), 7.82 (d, *J* = 8.8 Hz, 1H), 7.42-7.36 (m, 2H), 7.17-7.10 (m, 2H), 4.82 (s, 2H), 2.49 (s, 3H).

**Example 18: 4-amino-N-(1-((4-fluorophenyl)(hydroxy)methyl)-6-methylisoquinolin-5-yl)thieno[3,2-d]pyrimidine-7-carboxamide (compound 15)**

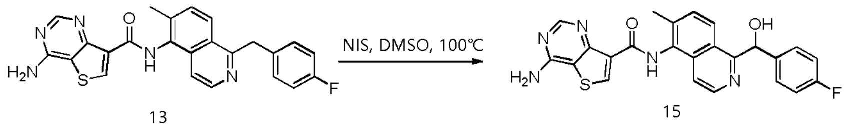


13 15

Compound 13 (20 mg, 45.1 μmol) and NIS (10.15 mg, 45.10 μmol) were dissolved in DMSO (3.0 mL), and the reaction was allowed to proceed at 100°C for 3 hours. After the reaction was complete, the reaction solution was cooled to room temperature, and then directly separated and purified by Prep-HPLC to afford compound 15 (2.5 mg). MS m/z (ESI): 460.1 $[M+H]^+$.

$^1H$ NMR (400 MHz, DMSO-$d_6$) δ 11.62 (s, 1H), 8.94 (s, 1H), 8.57 (s, 1H), 8.47 (d, *J* = 6.0 Hz, 1H), 8.34 (d, *J* = 8.8 Hz, 1H), 7.97 (s, 2H), 7.74 (d, *J* = 5.6 Hz, 1H), 7.56 (d, *J* = 4.8 Hz, 1H), 7.47-7.40 (m, 2H), 7.15-7.07 (m, 2H), 6.51-6.39 (m, 2H), 2.40 (s, 3H).

**Example 19: 4-amino-N-(1-(3-chlorobenzyl)-6-methylisoquinolin-5-yl)thieno[3,2-d]pyrimidine-7-carboxamide (compound 16)**

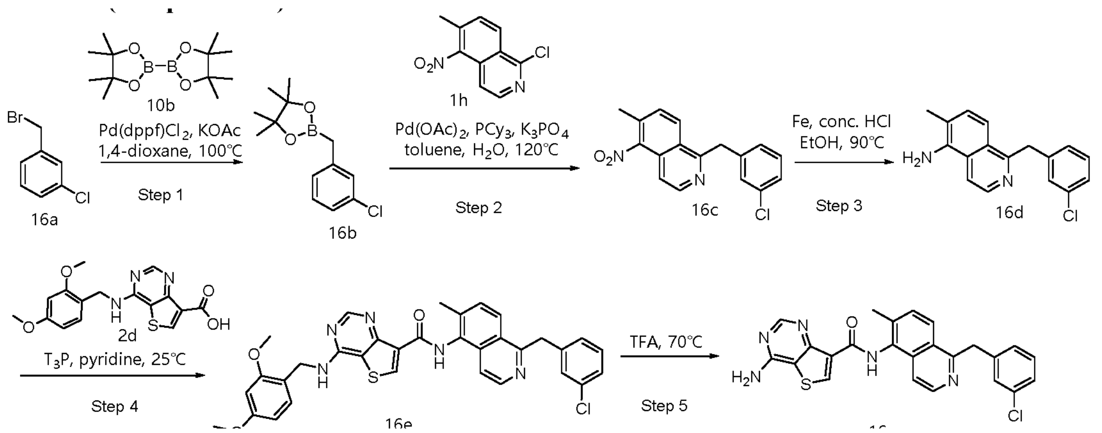


**Step 1: preparation of 2-(3-chlorobenzyl)-4,4,5,5-tetramethyl-1,3,2-dioxaborolane (compound 16b)**

Compounds 16a (1 g, 4.87 mmol), 10b (1.36 g, 5.35 mmol), Pd(dppf)$Cl_2$ (397.12 mg, 486.67 $\mu$mol) and KOAc (1.19 g, 12.17 mmol) were added to 1,4-dioxane (3.0 mL), purge with nitrogen was performed for three times, and the reaction was allowed to proceed at 100°C for 3 hours. After the reaction was complete, the reaction solution was filtered through diatomaceous earth, added with 200 ml water, and extracted with EA. The organic phase was dried over anhydrous sodium sulfate, filtered, concentrated, separated and purified by Flash column chromatography on silica gel (DCM/MeOH=20/1), to afford compound 16b (623 mg).

**Step 2: preparation of 1-(3-chlorobenzyl)-6-methyl-5-nitroisoquinoline (compound 16c)**

Compounds 16b (1.36 g, 5.39 mmol), 1h (400 mg, 1.80 mmol), Pd$(OAc)_2$ (80.68 mg, 359.34 $\mu$mol), tricyclohexylphosphane (50.39 mg, 179.67 $\mu$mol) and $K_3PO_4$ (1.33 g, 6.29 mmol) were added to toluene (3.0 mL) and water (1 mL), purge with nitrogen was performed for three times, and the reaction was allowed to proceed at 120°C for 12 hours. After the reaction was complete, the reaction solution was filtered through diatomaceous earth, added with 200 ml water, extracted with EA, and washed with a saturated solution of sodium bicarbonate. The organic phase was dried over anhydrous sodium sulfate, filtered, concentrated, separated and purified by Flash column chromatography on silica gel (DCM/MeOH=60/1), to afford compound 16c (412 mg). MS m/z (ESI): 313.1 $[M+H]^+$.

**Step 3: preparation of 1-(3-chlorobenzyl)-6-methylisoquinolin-5-amine (compound 16d)**

Compound 16c (400 mg, 1.28 mmol) was added to ethanol (3.0 mL) and water (1 mL), followed by addition of iron powder (357.15 mg, 6.39 mmol) and concentrated hydrochloric acid (12M, 4 mL), purge with nitrogen was performed for three times, and the reaction was allowed to proceed at 90°C for 3 hours. After the reaction was complete, the reaction solution was filtered through diatomaceous earth, added with 200 ml water, and extracted with a mixed solvent of (volume ratio 4:1) chloroform and isopropanol. The organic phase was dried over anhydrous sodium sulfate, filtered, and concentrated, to afford compound 16d (325 mg). MS m/z (ESI): 283.1 $[M+H]^+$.

**Step 4: preparation of N-(1-(3-chlorobenzyl)-6-methylisoquinolin-5-yl)-4-((2,4-dimethoxybenzyl)amino)thieno[3,2-d]pyrimidine-7-carboxamide (compound 16e)**

Compounds 16d (91 mg, 321.82 $\mu$mol) and 2d (133.38 mg, 386.18 $\mu$mol) were added to pyridine (2.0 mL), followed by addition of $T_3P$ (307.19 mg, 965.46 $\mu$mol), and the reaction was allowed to proceed at 25°C for 2 hours. After the reaction was complete, the mixture was added with 100 ml water, and extracted with EA. The organic phase was dried over anhydrous sodium sulfate, filtered, concentrated, separated and purified by Flash column chromatography on silica gel (DCM/MeOH=40/1), to afford compound 16e (85 mg). MS m/z (ESI): 610.0 $[M+H]^+$.

**Step 5: preparation of 4-amino-N-(1-(3-chlorobenzyl)-6-methylisoquinolin-5-yl)thieno[3,2-d]pyrimidine-7-carboxamide (compound 16)**

Compound 16e (80 mg, 131.12 $\mu$mol) was added to trifluoroacetic acid (2.0 mL), and the reaction was allowed to proceed at 70°C for 3 hours. After the reaction was complete, the reaction solution was concentrated to dryness, added with a saturated solution of sodium bicarbonate for alkalization, and extracted with ethyl acetate. The organic

phase was dried over anhydrous sodium sulfate, filtered, concentrated, separated and purified through Prep-HPLC, to afford compound 16 (6.1 mg). MS m/z (ESI): 460.2 [M+H]+.

$^1$H NMR (400 MHz, DMSO-$d_6$) δ 11.70 (s, 1H), 8.95 (s, 1H), 8.58 (s, 1H), 8.44 (d, *J* = 6.0 Hz, 1H), 8.29 (d, *J* = 8.8 Hz, 1H), 7.98 (s, 2H), 7.69 (d, *J* = 6.0 Hz, 1H), 7.64 (d, *J* = 12.4 Hz, 1H), 7.49 (s, 1H), 7.32 - 7.19 (m, 3H), 4.68 (s, 2H), 2.42 (s, 3H).

**Example 20: 4-amino-N-(1-((3-chlorophenyl)(hydroxy)methyl)-6-methylisoquinolin-5-yl)thieno[3,2-d]pyrimidine-7-carboxamide (compound 17)**

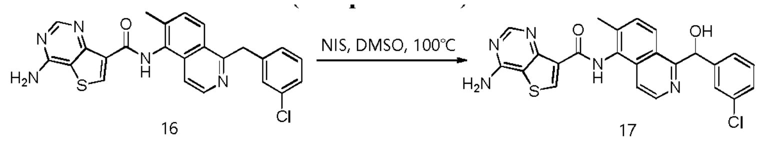


16 17

Compound 16 (54 mg, 117.40 μmol) and NIS (26.41 mg, 117.40 μmol) were dissolved in DMSO (2.0 mL), and the reaction was allowed to proceed at 100°C for 1 hour. After the reaction was complete, the reaction solution was cooled to room temperature, and then directly separated and purified by Prep-HPLC, to afford compound 17 (13.3 mg). MS m/z (ESI): 476.0 [M+H]+.

$^1$H NMR (400 MHz, DMSO-$d_6$) δ 11.62 (s, 1H), 8.94 (s, 1H), 8.57 (s, 1H), 8.47 (d, *J* =6 Hz, 1H), 8.38 (d, *J* = 4.8 Hz, 1H), 7.97 (s, 2H), 7.74 (d, *J* = 6.0 Hz, 1H), 7.59 (d, *J* = 8.8 Hz, 1H), 7.50 (s, 1H), 7.36 - 7.32 (m, 2H), 7.30 - 7.26 (m, 1H), 6.58 (d, *J* = 5.4 Hz, 1H), 6.45 (d, *J*= 4.4 Hz, 1H), 2.40 (s, 3H).

**Example 21: 4-amino-N-(6-methyl-1-(2-(trifluoromethyl)benzyl)isoquinolin-5-yl)thieno[3,2-d]pyrimidine-7-carboxamide (compound 29)**

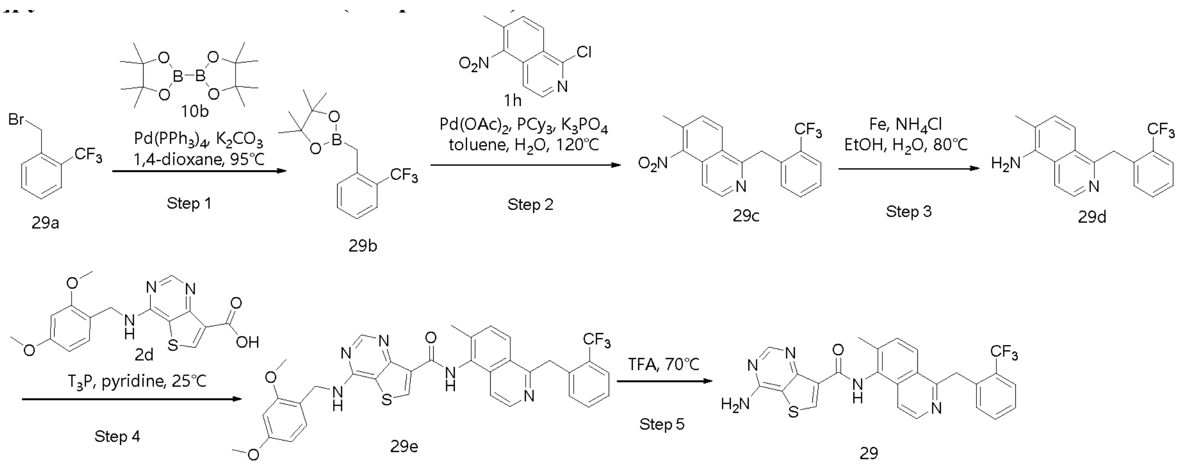


29a 29b 29c 29d 29e 29

**Step 1: preparation of 4,4,5,5-tetramethyl-2-(2-(trifluoromethyl)benzyl)-1,3,2-dioxaborolane (compound 29b)**

Compounds 29a (2.0 g, 8.2 mmol), 10b (2.55 g, 9.8 mmol), potassium carbonate (3.47 g, 24.6 mmol) and tetrakis(triphenylphosphine)palladium (478.6 mg, 410.0 μmol) were added to 1,4-dioxane (50.0 mL), purge with nitrogen was performed, and the reaction was allowed to proceed at 95°C for 16 hours. After the reaction was complete, the reaction solution was concentrated to dryness, the crude product was separated and purified by column chromatography on silica gel (PE/EA=19/1), to afford compound 29b (1.3 g).

**Step 2: preparation of 6-methyl-5-nitro-1-(2-(trifluoromethyl)benzyl)isoquinoline (compound 29c)**

Compounds 29b (1.1 g, 3.8 mmol), 1h (200 mg, 889.4 μmol), palladium acetate (40.3 mg, 177.9 μmol), potassium phosphate (667.4 mg, 3.1 mmol) and tricyclohexylphosphane (50.4 mg, 177.9 μmol) were added to a mixed solvent

of water (1.5 mL) and toluene (10.0 mL), purge with nitrogen was performed, and the reaction was allowed to proceed at 120°C for 12 hours. After the reaction was complete, the reaction solution was concentrated to dryness, the crude product was separated and purified by column chromatography on silica gel (PE/EA=4/1), to afford compound 29c (91 mg). MS m/z (ESI): 347.0 $[M+H]^+$.

**Step 3: preparation of 6-methyl-1-(2-(trifluoromethyl)benzyl)isoquinolin-5-amine (compound 29d)**

Compound 29c (91 mg, 262.8 μmol), iron powder (44.9 mg, 788.3 μmol) and ammonium chloride (28.7 mg, 525.3 μmol) were added to a mixed solution of ethanol (6.0 mL) and water (2.0 mL), purge with nitrogen was performed, and the reaction was allowed to proceed at 80°C for 3 hours. After the reaction was complete, the mixture was filtered with suction, concentrated, alkalized with a saturated aqueous solution of sodium bicarbonate, and extracted with dichloromethane. The organic phase was dried over anhydrous sodium sulfate, filtered with suction, concentrated, and the crude product was separated and purified through reversed-phase column chromatography on a C18 column (0.05% ammonium bicarbonate in water/acetonitrile=60/40), to afford compound 29d (20 mg). MS m/z (ESI): 317.1 $[M+H]^+$.

**Step 4: preparation of 4-((2,4-dimethoxybenzyl)amino)-N-(6-methyl-1-(2-(trifluoromethyl)benzyl)isoquinolin-5-yl)thieno[3,2-d]pyrimidine-7-carboxamide (compound 29e)**

Compounds 29d (20.0 mg, 63.2 μmol) and 2d (21.8 mg, 63.2 μmol) were dissolved in pyridine (2.0 mL), purge with nitrogen was performed, $T_3P$ (0.5 mL) was then added, and after the addition, the reaction was allowed to proceed at 25°C for 16 hours. After the reaction was complete, the reaction solution was concentrated to dryness, alkalized with a saturated aqueous solution of sodium bicarbonate, and extracted with ethyl acetate. The organic phase was dried over anhydrous sodium sulfate, filtered with suction, concentrated, and the crude product was separated and purified through reversed-phase chromatography on a C18 column (0.05% ammonium bicarbonate in water/acetonitrile=60/40), to afford compound 29e (18 mg). MS m/z (ESI): 644.0 $[M+H]^+$.

**Step 5: preparation of 4-amino-N-(6-methyl-1-(2-(trifluoromethyl)benzyl)isoquinolin-5-yl)thieno[3,2-d]pyrimidine-7-carboxamide (compound 29)**

Compound 29e (18 mg, 28.0 μmol) was added to trifluoroacetic acid (2.0 mL), and the reaction was allowed to proceed at 70°C for 3 hours. After the reaction was complete, the reaction solution was concentrated to dryness, then added with methanol and potassium carbonate, the solution was thoroughly stirred, filtered with suction, concentrated, and the crude product was separated and purified by Prep-HPLC, to afford compound 29 (3.0 mg). MS m/z (ESI): 494.1 $[M+H]^+$.

$^1H$ NMR (400 MHz, DMSO-$d_6$) δ 11.67 (s, 1H), 8.95 (s, 1H), 8.58 (s, 1H), 8.36 (d, $J$= 6.0 Hz, 1H), 8.13 (d, $J$= 8.7 Hz, 1H), 7.97 (s, 2H), 7.77 (d, $J$= 7.5 Hz, 1H), 7.68 (t, $J$= 6.9 Hz, 2H), 7.54 (t, $J$= 7.5 Hz, 1H), 7.46 (t, $J$= 7.6 Hz, 1H), 7.11 (d, $J$= 7.7 Hz, 1H), 4.86 (s, 2H), 2.44 (s, 3H).

**Example 22: 4-amino-N-(1-(4-fluoro-2-(trifluoromethyl)benzyl)-6-methylisoquinolin-5-yl)thieno[3,2-d]pyrimidine-7-carboxamide (compound 31)**

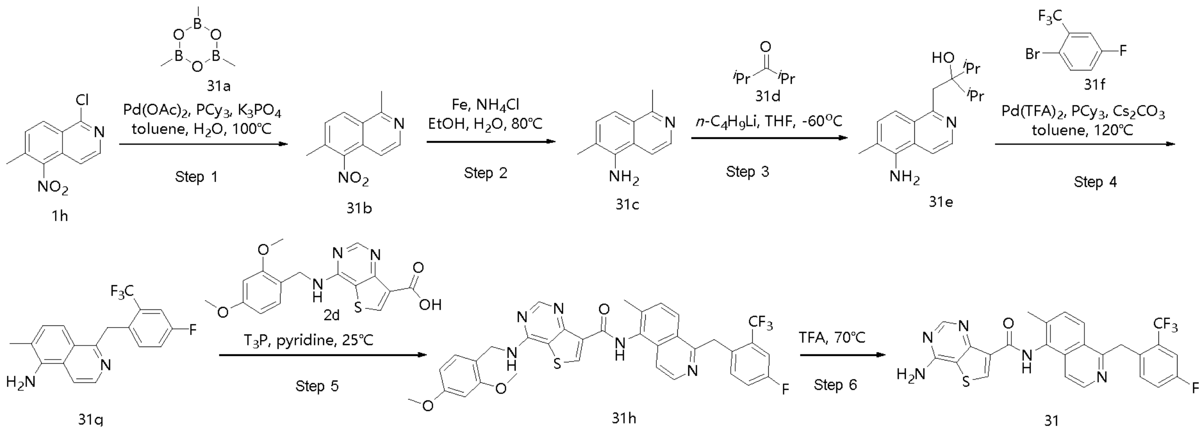

**Step 1: preparation of 1,6-dimethyl-5-nitroisoquinoline (compound 31b)**

Compounds 1h (3.0 g, 13.3 mmol), 31a (16.8 g, 66.7 mmol), palladium acetate (605.1 mg, 2.7 mmol), potassium phosphate (10.0 g, 46.7 mmol) and tricyclohexylphosphane (755.8 mg, 2.7 mmol) were added to a mixed solvent of water (15.0 mL) and toluene (85.0 mL), purge with nitrogen was performed, and the reaction was allowed to proceed at 100°C for 16 hours. After the reaction was complete, the reaction solution was concentrated to dryness, the crude product was separated and purified by column chromatography on silica gel (PE/EA=40/60), to afford compound 31b (2.69 g). MS m/z (ESI): 203.1 $[M+H]^+$.

**Step 2: preparation of 1,6-dimethylisoquinolin-5-amine (compound 31c)**

Compound 31b (2.69 g, 13.3 mmol), iron powder (2.27 g, 39.9 mmol) and ammonium chloride (1.45 g, 26.6 mmol) were added to a mixed solution of ethanol (90.0 mL) and water (30.0 mL), purge with nitrogen was performed, and the reaction was allowed to proceed at 80°C for 3 hours. After the reaction was complete, the mixture was filtered with suction, concentrated, alkalized with a saturated aqueous solution of sodium bicarbonate, and extracted with dichloromethane. The organic phase was dried over anhydrous sodium sulfate, filtered with suction, concentrated, and the crude product was separated and purified by column chromatography on silica gel (100% EA), to afford compound 31c (2.25 g). MS m/z (ESI): 173.1 $[M+H]^+$.

**Step 3: preparation of 3-((5-amino-6-methylisoquinolin-1-yl)methyl)-2,4-dimethylpentan-3-ol (compound 31e)**

Compound 31c (2.69 g, 13.3 mmol) was dissolved in tetrahydrofuran (50.0 mL), the reaction was cooled to -60°C under the protection of nitrogen, n-butyllithium (20 mL, 49.6 mmol, 2.5 M solution in n-hexane) was slowly dropwise added at this temperature, and after the dropwise addition, the reaction was allowed to proceed at -60°C for 1 hour. Compound 31d (4.87g, 41.8 mmol) was then slowly dropwise added, and after the dropwise addition, the reaction was allowed to proceed at -60°C for 3 hours. After the reaction was complete, the reaction was quenched by slowly dropwise addition of ice water under ice-bath cooling, and extracted with ethyl acetate. The organic phase was dried over anhydrous sodium sulfate, filtered with suction, concentrated, and the crude product was separated and purified by column chromatography on silica gel (100% EA), to afford compound 31e (3.33 g). MS m/z (ESI): 287.1 $[M+H]^+$.

**Step 4: preparation of 1-(4-fluoro-2-(trifluoromethyl)benzyl)-6-methylisoquinolin-5-amine (compound 31g)**

Compounds 31e (100 mg, 349.2 μmol), 31f (173.2 mg, 698.3 μmol), palladium trifluoroacetate (11.8 mg, 34.9 μmol), cesium carbonate (229.8 mg, 698.3 μmol) and tricyclohexylphosphane (19.8 mg, 69.8 μmol) were added to toluene (10.0 mL), purge with nitrogen was performed, and the reaction was allowed to proceed at 120°C for 3 hours. After the reaction was complete, the reaction solution was concentrated to dryness, the crude product was separated and purified by column chromatography on silica gel (PE/EA=1/1), to afford compound 31g (100 mg). MS m/z (ESI): 335.1 $[M+H]^+$.

**Step 5: preparation of 4-((2,4-dimethoxybenzyl)amino)-N-(1-(4-fluoro-2-(trifluoromethyl)benzyl)-6-methylisoquinolin-5-yl)thieno[3,2-d]pyrimidine-7-carboxamide (compound 31h)**

Compounds 31g (115.0 mg, 344.0 μmol) and 2d (118.8 mg, 344.0 μmol) were dissolved in pyridine (5.0 mL), purge with nitrogen was performed, $T_3P$ (3.0 mL, 50% in DMF) was then added, and after the addition, the reaction was allowed to proceed at 25°C for 16 hours. After the reaction was complete, the reaction solution was concentrated to dryness, alkalized with a saturated aqueous solution of sodium bicarbonate, and extracted with ethyl acetate. The organic phase was dried over anhydrous sodium sulfate, filtered with suction, concentrated, and the crude product was separated and purified through reversed-phase column chromatography on a C18 column (0.05% ammonium bicarbonate in water/acetonitrile=60/40), to afford compound 31h (200 mg). MS m/z (ESI): 662.1 $[M+H]^+$.

**Step 6: preparation of 4-amino-N-(1-(4-fluoro-2-(trifluoromethyl)benzyl)-6-methylisoquinolin-5-yl)thieno[3,2-d]pyrimidine-7-carboxamide (compound 31)**

Compound 31h (200 mg, 302.3 μmol) was added to trifluoroacetic acid (5.0 mL), and the reaction was allowed to proceed at 70°C for 3 hours. After the reaction was complete, the reaction solution was concentrated to dryness, then added with methanol and potassium carbonate and stirred for 10 minutes, filtered with suction, concentrated, and the crude product was separated and purified by Prep-HPLC, to afford compound 31 (130.0 mg). MS m/z (ESI): 512.1 $[M+H]^+$.

$^1H$ NMR (400 MHz, DMSO-$d_6$) δ 11.66 (s, 1H), 8.95 (s, 1H), 8.58 (s, 1H), 8.33 (d, *J*= 6.0 Hz, 1H), 8.17 (d, *J*=

8.7 Hz, 1H), 7.96 (s, 2H), 7.71 - 7.63 (m, 3H), 7.45 (td, *J*= 8.5, 2.7 Hz, 1H), 7.24 (dd, *J*= 8.5, 5.6 Hz, 1H), 4.85 (s, 2H), 2.45 (s, 3H).

**Example 23: 4-amino-N-(6-methyl-1-(3-(trifluoromethyl)benzyl)isoquinolin-5-yl)thieno[3,2-d]pyrimidine-7-carboxamide (compound 19)**

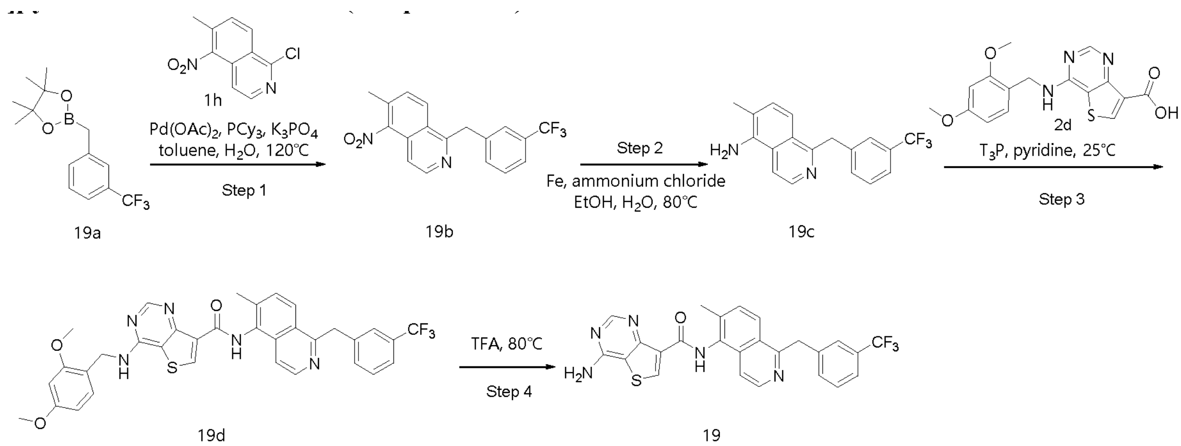


**Step 1: preparation of 6-methyl-5-nitro-1-(3-(trifluoromethyl)benzyl)isoquinoline (compound 19b)**

Compounds 19a (300.0 mg, 1.35 mmol), 1h (424.08 mg, 1.48 mmol), $Pd(OAc)_2$ (60.51 mg, 269.51 μmol), tricyclohexylphosphane (75.58 mg, 269.51 μmol) and $K_3PO_4$ (858.13 mg, 4.04 mmol) were added to toluene (5.0 mL) and water (1.0 mL), purge with nitrogen was performed for three times, and then the reaction was allowed to proceed at 120°C for 12 hours. After the reaction was complete, the reaction solution was concentrated to dryness, added with water, and extracted with ethyl acetate. The organic phase was dried over anhydrous sodium sulfate, filtered, concentrated, and the crude product was separated and purified by column chromatography on silica gel (PE/EA=5/1), to afford compound 19b (322.0 mg). MS m/z (ESI): 347.0 $[M+H]^+$.

**Step 2: preparation of 6-methyl-1-(3-(trifluoromethyl)benzyl)isoquinolin-5-amine (compound 19c)**

Compound 19b (300.0 mg, 866.29 μmol) was added to ethanol (6.0 mL) and water (2.0 mL), iron powder (145.15 mg, 2.60 mmol) and ammonium chloride (92.68 mg, 1.73 mmol) were sequentially added, and after the addition, the reaction was allowed to proceed at 80°C for 3 hours. After the reaction was complete, the reaction solution was filtered, the filtrate was concentrated, to afford compound 19c (270.0 mg). MS m/z (ESI): 317.1 $[M+H]^+$.

**Step 3: preparation of 4-((2,4-dimethoxybenzyl)amino)-N-(6-methyl-1-(3-(trifluoromethyl)benzyl)isoquinolin-5-yl)thieno[3,2-d]pyrimidine-7-carboxamide (compound 19d)**

Compound 19c (16.5 mg, 108.0 μmol) was added to pyridine (5.0 mL), compound 2d (324.28 mg, 938.92 μmol) and $T_3P$ (2.0 mL, 50% in DMF) were sequentially added, and after the addition, the reaction was allowed to proceed at 25°C for 16 hours. After the reaction was complete, the reaction solution was concentrated to dryness, added with a saturated solution of sodium bicarbonate for alkalization, and extracted with ethyl acetate. The organic phase was dried over anhydrous sodium sulfate, filtered, and concentrated, to afford compound 19d (370.0 mg). MS m/z (ESI): 644.1 $[M+H]^+$.

**Step 4: preparation of 4-amino-N-(6-methyl-1-(3-(trifluoromethyl)benzyl)isoquinolin-5-yl)thieno[3,2-d]pyrimidine-7-carboxamide (compound 19)**

Compound 19d (50.0 mg, 77.68 μmol) was added to trifluoroacetic acid (2.0 mL), and the reaction was allowed to proceed at 80°C for 3 hours. After the reaction was complete, the reaction solution was concentrated to dryness, added with a saturated solution of sodium bicarbonate for alkalization, and extracted with ethyl acetate. The organic phase was dried over anhydrous sodium sulfate, filtered, concentrated, and the crude product was separated and purified by Prep-HPLC, to afford compound 19 (12.3 mg). MS m/z (ESI): 494.1 $[M+H]^+$.

$^1H$ NMR (400 MHz, DMSO-$d_6$) δ 11.64 (s, 1H), 8.94 (s, 1H), 8.58 (s, 1H), 8.44 (d, *J* = 5.92 Hz, 1H), 8.32 (d, *J* = 8.72 Hz, 1H), 7.97 (s, 2H), 7.73 (s, 1H), 7.70 - 7.64 (m, 2H), 7.62-7.59 (m, 1H), 7.57-7.48 (m, 2H), 4.79 (s, 2H), 2.43 (s, 3H).

**Example 24: 4-amino-N-(6-methyl-1-(3-(trifluoromethyl)benzoyl)isoquinolin-5-yl)thieno[3,2-d]pyrimidine-7-carboxamide (compound 22) and 4-amino-N-(1-(hydroxy(3-(trifluoromethyl)phenyl)methyl)-6-methylisoquinolin-5-yl)thieno[3,2-d]pyrimidine-7-carboxamide (compound 23)**

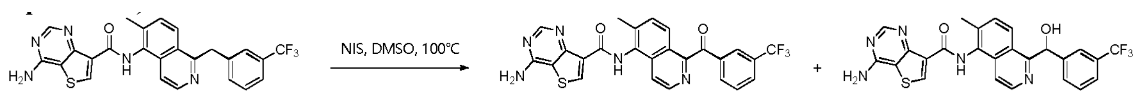

19 22 + 23

Compound 19 (60.0 mg, 121.58 μmol) and NIS (27.35 mg, 121.58 μmol) were dissolved in DMSO (1.0 mL), and the reaction was allowed to proceed at 100°C for 1.5 hours. After the reaction was complete, the reaction solution was cooled to room temperature, added with water, and extracted with ethyl acetate. The organic phase was dried over anhydrous sodium sulfate, filtered, concentrated, and the crude product was firstly roughly purified by column chromatography on silica gel (DCM/MeOH=95/5), to afford crude products of compound 22 and compound 23, respectively, and then separated and purified through Prep-HPLC, to afford compound 22 (10.26 mg), MS m/z (ESI): 508.1 [M+H]$^+$; and compound 23 (10.35 mg), MS m/z (ESI): 510.1 [M+H]$^+$.

compound 22: $^1H$ NMR (400 MHz, DMSO-$d_6$) δ 11.76 (s, 1H), 8.98 (s, 1H), 8.64-8.61 (m, 2H), 8.21-8.14 (m, 3H), 8.12-8.08 (m, 1H), 8.05 (dd, *J* = 5.8, 0.92 Hz, 1H), 7.99 (s, 2H), 7.82 (t, *J* = 7.84 Hz, 1H), 7.75 (d, *J* = 8.76 Hz, 1H), 2.49 (s, 3H).

compound 23: $^1H$ NMR (400 MHz, DMSO-$d_6$) δ 11.62 (s, 1H), 8.94 (s, 1H), 8.57 (s, 1H), 8.47 (d, *J* =5.88 Hz, 1H), 8.40 (d, *J* =8.76 Hz, 1H), 7.97 (s, 2H), 7.85 (s, 1H), 7.74 (dd, *J* = 5.84, 0.84 Hz, 1H), 7.65 (d, *J* =7.72 Hz, 1H), 7.62-7.58 (m, 2H), 7.53 (t, *J* = 7.68 Hz, 1H), 6.66 (d, *J* = 5.68 Hz, 1H), 6.57 (d, *J* = 5.68 Hz, 1H), 2.41 (s, 3H).

**Example 25: 4-amino-N-(1-(4-chloro-2-fluorobenzyl)-6-methylisoquinolin-5-yl)thieno[3,2-d]pyrimidine-7-carboxamide (compound 26)**

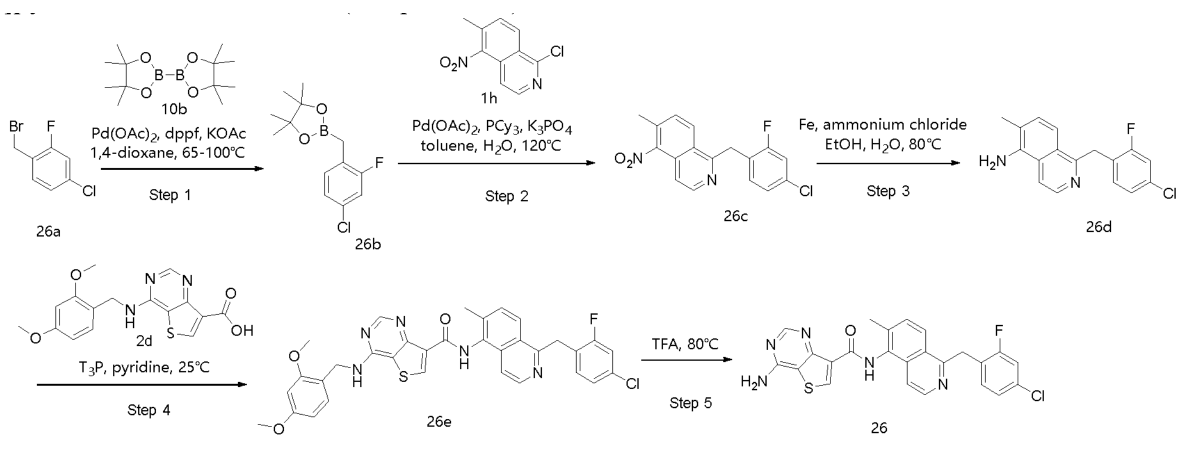


**Step 1: preparation of 2-(4-chloro-2-fluorobenzyl)-4,4,5,5-tetramethyl-1,3,2-dioxaborolane (compound 26b)**

Compounds 26a (1.5 g, 6.71 mmol), 10b (2.05 g, 8.05 mmol), Pd(OAc)$_2$ (150.70 mg, 671.23 μmol), dppf (387.0 mg, 671.23 μmol) and KOAc (988.12 mg, 10.07 mmol) were added to 1,4-dioxane (20.0 mL), purge with nitrogen was performed for three times, the reaction was allowed to proceed at 100°C for 4 hours, and the reaction was then continued at 65°C for 16 hours. After the reaction was complete, the reaction solution was concentrated to dryness, added with water, and extracted with ethyl acetate. The organic phase was dried over anhydrous sodium sulfate, filtered, concentrated, and the crude product was separated and purified by column chromatography on silica gel (PE/EA=10/1), to afford compound 26b (850.0 mg).

**Step 2: preparation of 1-(4-chloro-2-fluorobenzyl)-6-methyl-5-nitroisoquinoline (compound 26c)**

Compounds 26b (850.0 mg, 3.14 mmol), 1h (350.0 mg, 1.57 mmol), $Pd(OAc)_2$ (70.59 mg, 314.43 μmol), tricyclohexylphosphane (88.17 mg, 314.43 μmol) and $K_3PO_4$ (1.0 g, 4.72 mmol) were added to toluene (10.0 mL) and water (2.0 mL), purge with nitrogen was performed for three times, and then the reaction was allowed to proceed at 120°C for 12 hours. After the reaction was complete, the reaction solution was concentrated to dryness, added with water, and extracted with ethyl acetate. The organic phase was dried over anhydrous sodium sulfate, filtered, concentrated, and the crude product was separated and purified by column chromatography on silica gel (PE/EA=5/1), to afford compound 26c (270.0 mg). MS m/z (ESI): 331.0 $[M+H]^+$.

**Step 3: preparation of 1-(4-chloro-2-fluorobenzyl)-6-methylisoquinolin-5-amine (compound 26d)**

Compound 26c (270.0 mg, 816.35 μmol) was added to ethanol (6.0 mL) and water (2.0 mL), iron powder (136.78 mg, 2.45 mmol) and ammonium chloride (43.67 mg, 816.35 μmol) were sequentially added, and after the addition, the reaction was allowed to proceed at 80°C for 2 hours. After the reaction was complete, the reaction solution was filtered, and concentrated, to afford compound 26d (200.0 mg). MS m/z (ESI): 301.1 $[M+H]^+$.

**Step 4: preparation of N-(1-(4-chloro-2-fluorobenzyl)-6-methylisoquinolin-5-yl)-4-((2,4-dimethoxybenzyl)amino)thieno[3,2-d]pyrimidine-7-carboxamide (compound 26e)**

Compound 26d (200.0 mg, 664.99 μmol) was added to pyridine (10.0 mL), compound 2d (229.67 mg, 664.99 μmol) and $T_3P$ (3.0 mL, 50% in DMF) were sequentially added, and after the addition, the reaction was allowed to proceed at 25°C for 16 hours. After the reaction was complete, the reaction solution was concentrated to dryness, added with a saturated solution of sodium bicarbonate for alkalization, and extracted with ethyl acetate. The organic phase was dried over anhydrous sodium sulfate, filtered, and concentrated, to afford compound 26e (400.0 mg). MS m/z (ESI): 628.0 $[M+H]^+$.

**Step 5: preparation of 4-amino-N-(1-(4-chloro-2-fluorobenzyl)-6-methylisoquinolin-5-yl)thieno[3,2-d]pyrimidine-7-carboxamide (compound 26)**

Compound 26e (400.0 mg, 636.83 μmol) was added to trifluoroacetic acid (5.0 mL), and the reaction was allowed to proceed at 80°C for 2 hours. After the reaction was complete, the reaction solution was concentrated to dryness, added with a saturated solution of sodium bicarbonate for alkalization, and extracted with ethyl acetate. The organic phase was dried over anhydrous sodium sulfate, filtered, concentrated, and the crude product was separated and purified by Prep-HPLC, to afford compound 26 (240.0 mg). MS m/z (ESI): 478.0 $[M+H]^+$.

$^1H$ NMR (400 MHz, DMSO-$d_6$) δ 11.65 (s, 1H), 8.95 (s, 1H), 8.58 (s, 1H), 8.36 (d, *J* = 6.0 Hz, 1H), 8.24 (d, *J* = 8.64 Hz, 1H), 7.97 (s, 2H), 7.69 - 7.66 (m, 2H), 7.42 (dd, *J* = 10.32, 2.0 Hz, 1H), 7.25-7.18 (m, 2H), 4.69 (s, 2H), 2.45 (s, 3H).

**Example 26: 4-amino-N-(1-(4-chloro-2-fluorobenzoyl)-6-methylisoquinolin-5-yl)thieno[3,2-d]pyrimidine-7-carboxamide (compound 27) and 4-amino-N-(1-((4-chloro-2-fluorophenyl)(hydroxy)methyl)-6-methylisoquinolin-5-yl)thieno[3,2-d]pyrimidine-7-carboxamide (compound 28)**

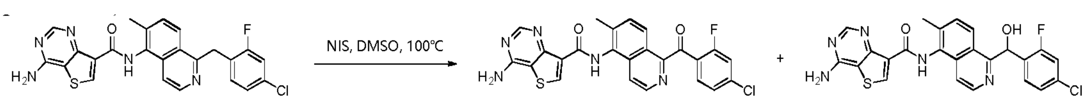


26 27 28

Compound 26 (80.0 mg, 167.38 μmol) and NIS (37.66 mg, 167.38 μmol) were dissolved in DMSO (2.0 mL), and the reaction was allowed to proceed at 100°C for 1.5 hours. After the reaction was complete, the reaction solution was cooled to room temperature, added with water, and extracted with ethyl acetate. The organic phase was dried over anhydrous sodium sulfate, filtered, concentrated, separated and purified through Prep-HPLC to afford compound 27 (19.26 mg), MS m/z (ESI): 492.0 $[M+H]^+$; and compound 28 (15.38 mg), MS m/z (ESI): 494.0 $[M+H]^+$.

compound 27: $^1H$ NMR (400 MHz, DMSO-$d_6$) δ 11.74 (s, 1H), 8.98 (s, 1H), 8.60 (s, 1H), 8.55 (d, *J* = 5.8 Hz, 1H), 8.37 (d, *J* = 8.76 Hz, 1H), 8.03 (d, *J* = 5.84 Hz, 1H), 7.99 (s, 2H), 7.87 (t, *J* = 8.08 Hz, 1H), 7.80 (d, *J* = 8.84 Hz, 1H), 7.60 (dd, *J* = 10.36, 1.96 Hz, 1H), 7.52 (dd, *J* = 8.36, 1.96 Hz, 1H), 2.50 (s, 3H).

compound 28: $^{1}H$ NMR (400 MHz, DMSO-$d_6$) δ 11.64 (s, 1H), 8.96 (s, 1H), 8.58 (s, 1H), 8.39-8.33(m, 2H), 7.98(s, 2H), 7.75 (d, $J$ = 5.92 Hz, 1H), 7.70-7.65 (m, 2H), 7.36-7.31 (m, 2H), 6.75 (s, 1H), 6.55 (s, 1H), 2.45 (s, 3H).

**Example 27: 4-amino-N-(1-(4-chlorobenzyl)-6-methylisoquinolin-5-yl)thieno[3,2-d]pyrimidine-7-carboxamide (compound 18)**

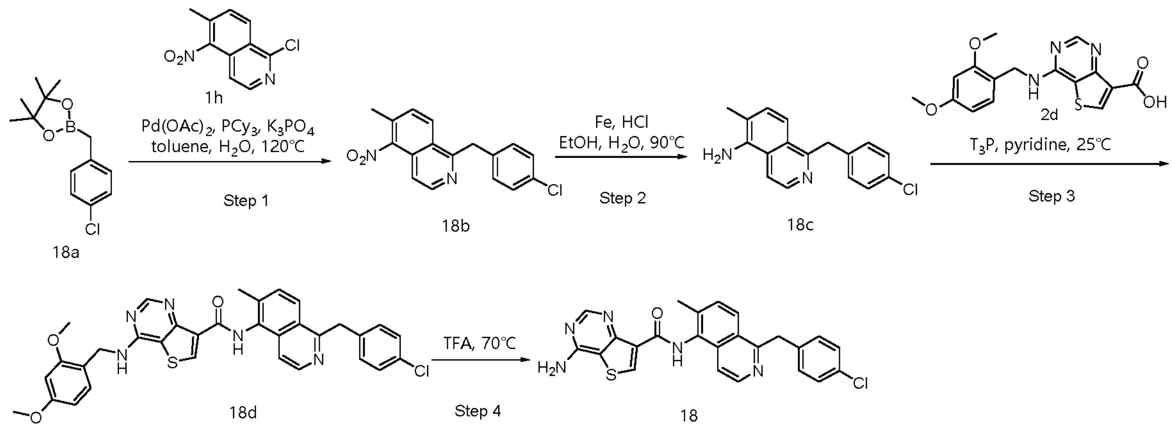


**Step 1: preparation of 1-(4-chlorobenzyl)-6-methyl-5-nitroisoquinoline (compound 18b)**

Compounds 18a (510.5 mg, 2020.0 μmol), 1h (300.0 mg, 1350.0 μmol), palladium acetate (60.5 mg, 269.5 μmol), potassium phosphate (1000.0 mg, 4720.0 μmol) and tricyclohexylphosphane (75.6 mg, 269.5 μmol) were added to toluene (12.0 mL) and water (2.0 mL), purge with nitrogen was performed for three times, and the reaction was warmed to 120°C and allowed to proceed for 12 hours. After the reaction was complete, the reaction solution was diluted with ethyl acetate, and washed with water for 3 times. The organic phase was dried over anhydrous sodium sulfate, filtered, concentrated, and the crude product was separated and purified by column chromatography on silica gel (PE/EA=3/1), to afford compound 18b (310.0 mg). MS m/z (ESI): 313.0 [M+H]+.

**Step 2: preparation of 1-(4-chlorobenzyl)-6-methylisoquinolin-5-amine (compound 18c)**

Compound 18b (310.0 mg, 991.2 μmol) was dissolved in ethanol (12.0 mL), iron powder (276.8 mg, 4960.0 μmol) and concentrated hydrochloric acid (12M, 0.6 mL) and water (3.0 mL) were then sequentially added, and the reaction was warmed to 90°C and allowed to proceed for 3.5 hours. After the reaction was complete, a saturated aqueous solution of sodium bicarbonate was dropwise added to adjust to a basic pH, the solution was filtered through diatomaceous earth to remove the solid, the filter cake was washed with ethyl acetate, the liquid was concentrated to afford a crude product, and the crude product was separated and purified by column chromatography on silica gel (PE/EA=1/1), to afford compound 18c (190.0 mg). MS m/z (ESI): 283.1 [M+H]+.

**Step 3: preparation of N-(1-(4-chlorobenzyl)-6-methylisoquinolin-5-yl)-4-((2,4-dimethoxybenzyl)amino)thieno[3,2-d]pyrimidine-7-carboxamide (compound 18d)**

Compounds 18c (190.0 mg, 671.9 μmol) and 2d (232.1 mg, 671.9 μmol) were dissolved in pyridine (10.0 mL), 1-propylphosphonic anhydride (427.6 mg, 1340.0 μmol) was dropwise added, and after the addition, the reaction was allowed to proceed at 25°C for 12 hours. After the reaction was complete, the solvent was removed by concentration under reduced pressure, the reaction solution was diluted with ethyl acetate, washed with a saturated aqueous solution of sodium bicarbonate for 3 times, the organic phase was dried over anhydrous sodium sulfate, filtered and then concentrated, and the crude product was separated and purified by column chromatography on silica gel (PE/EA=3/2), to afford compound 18d (215.0 mg). MS m/z (ESI): 610.2 [M+H]+.

**Step 4: preparation of 4-amino-N-(1-(4-chlorobenzyl)-6-methylisoquinolin-5-yl)thieno[3,2-d]pyrimidine-7-carboxamide (compound 18)**

Compound 18d (215.0 mg, 352.4 μmol) was added to trifluoroacetic acid (10.0 mL), and the reaction was

allowed to proceed at 70°C for 4 hours. After the reaction was complete, the reaction solution was concentrated to dryness, redissolved with dichloromethane, added with triethylamine for alkalization, and concentrated to afford a crude product, the crude product was separated and purified by Prep-HPLC, to afford compound 18 (66.0 mg). MS m/z (ESI): 460.0 [M+H]+.

$^1$HNMR (400 MHz, DMSO-$d_6$) δ 11.63 (s, 1H), 8.94 (s, 1H), 8.57 (s, 1H), 8.43 (d, *J* = 6.0 Hz, 1H), 8.24 (d, *J* = 8.7 Hz, 1H), 7.97 (s, 2H), 7.68 (d, *J* = 6.0 Hz, 1H), 7.63 (d, *J* = 8.8 Hz, 1H), 7.32 (s, 4H), 4.66 (s, 2H), 2.42 (s, 3H).

**Example 28: 4-amino-N-(1-((4-chlorophenyl)(hydroxy)methyl)-6-methylisoquinolin-5-yl)thieno[3,2-d]pyrimidine-7-carboxamide (compound 20) and 4-amino-N-(1-(4-chlorobenzoyl)-6-methylisoquinolin-5-yl)thieno[3,2-d]pyrimidine-7-carboxamide (compound 21)**

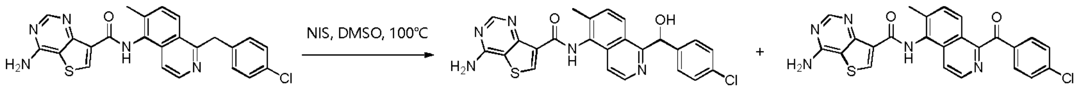


18 20 + 21

Compound 18 (57.0 mg, 123.9 μmol) and NIS (30.7 mg, 136.3 μmol) were dissolved in DMSO (3.0 mL), and the reaction was allowed to proceed at 100°C for 3 hours. After the reaction was complete, the reaction solution was cooled to room temperature, and then directly separated and purified by Prep-HPLC, to afford compound 20 (16.0 mg), MS m/z (ESI): 476.0 [M+H]+; and compound 21 (10.0 mg), MS m/z (ESI): 474.0 [M+H]+.

compound 20: $^1$HNMR (400 MHz, DMSO-$d_6$) δ 11.62 (s, 1H), 8.94 (s, 1H), 8.57 (s, 1H), 8.47 (d, *J* = 5.7 Hz, 1H), 8.33 (d, *J* = 8.8 Hz, 1H), 7.97 (s, 2H), 7.75 (d, *J* = 5.6 Hz, 1H), 7.57 (d, *J* = 8.7 Hz, 1H), 7.42 (d, *J* = 8.2 Hz, 2H), 7.36 (d, *J* = 8.3 Hz, 2H), 6.55 (s, 1H), 6.43 (s, 1H), 2.40 (s, 3H).

compound 21: $^1$HNMR (400 MHz, DMSO-$d_6$) δ 11.75 (s, 1H), 8.98 (s, 1H), 8.61 (s, 1H), 8.60 (d, *J* = 6.0 Hz, 1H), 8.02 (d, *J* = 3.6 Hz, 1H), 8.00 (s, 1H), 7.99 (s, 2H), 7.88 (d, *J* = 8.4 Hz, 2H), 7.72 (d, *J* = 8.8 Hz, 1H), 7.65 (d, *J* = 8.6 Hz, 2H), 2.48 (s, 3H).

**Example 29: 4-amino-N-(6-methyl-1-(4-(trifluoromethyl)benzyl)isoquinolin-5-yl)thieno[3,2-d]pyrimidine-7-carboxamide (compound 34)**

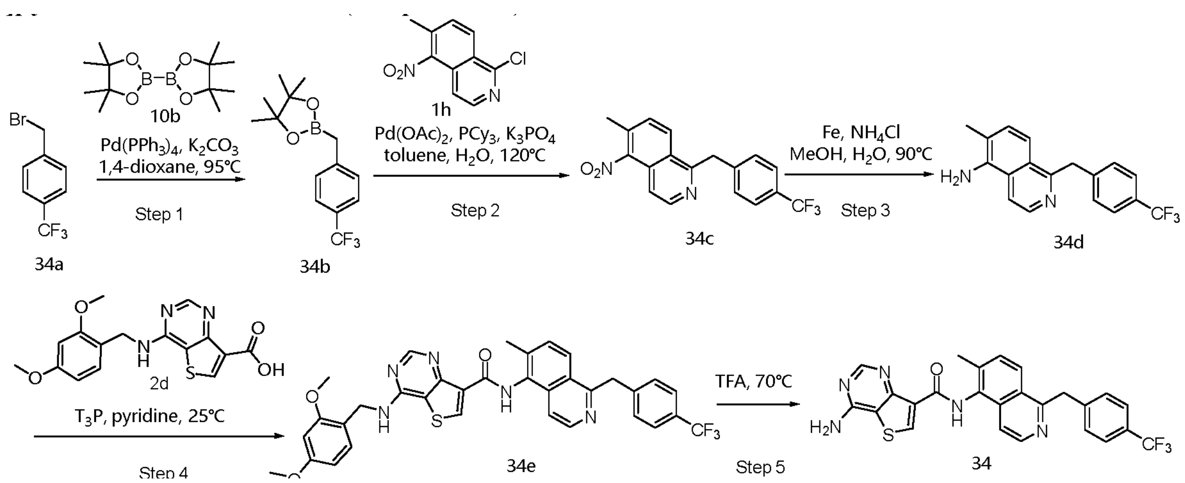


**Step 1: preparation of 4,4,5,5-tetramethyl-2-(4-(trifluoromethyl)benzyl)-1,3,2-dioxaborolane (compound 34b)**

Compounds 34a (3000.0 mg, 12550.0 μmol), 10b (3820.0 mg, 15060.0 μmol), potassium carbonate (5200.0 mg, 37650.0 μmol) and tetrakis(triphenylphosphine)palladium (725.2 mg, 627.5 μmol) were added to 1,4-dioxane (50.0 mL), purge with nitrogen was performed for three times, the reaction was warmed to 95°C and allowed to proceed for 16 hours. After the reaction was complete, the reaction solution was filtered to remove the solid, the filter cake was washed with ethyl acetate, the filtrates were combined, concentrated, separated and purified through silica gel column

chromatography (PE/EA=19/1), to afford compound 34b (470.0 mg). MS m/z (ESI): 287.0 $[M+H]^+$.

**Step 2: preparation of 6-methyl-5-nitro-1-(4-(trifluoromethyl)benzyl)isoquinoline (compound 34c)**

Compound 34c (165.0 mg) was prepared according to the method for preparing compound 18b in Step 1 of Example 27, except that compound 18a was replaced with compound 34b. MS m/z (ESI): 347.0 $[M+H]^+$.

**Step 3: preparation of 6-methyl-1-(4-(trifluoromethyl)benzyl)isoquinolin-5-amine (compound 34d)**

Compound 34c (165.0 mg, 476.5 μmol) was dissolved in methanol (10.0 mL), iron powder (133.1 mg, 2380.0 μmol), ammonium chloride (63.7 mg, 1190.0 μmol) and water (5.0 mL) were then added, and after the addition, the reaction was warmed to 90°C and allowed to proceed for 6 hours. After the reaction was complete, the mixture was filtered through diatomaceous earth to remove the solid, the filter cake was washed with ethyl acetate, the filtrate was concentrated, and then separated and purified by column chromatography on silica gel (PE/EA=1/1), to afford compound 34d (100.0 mg). MS m/z (ESI): 317.1 $[M+H]^+$.

**Step 4: preparation of 4-((2,4-dimethoxybenzyl)amino)-N-(6-methyl-1-(4-(trifluoromethyl)benzyl)isoquinolin-5-yl)thieno[3,2-d]pyrimidine-7-carboxamide (compound 34e)**

Compound 34e (148.0 mg) was prepared according to the method for preparing compound 18d in Step 3 of Example 27, except that compound 18c was replaced with compound 34d. MS m/z (ESI): 644.3 $[M+H]^+$.

**Step 5: preparation of 4-amino-N-(6-methyl-1-(4-(trifluoromethyl)benzyl)isoquinolin-5-yl)thieno[3,2-d]pyrimidine-7-carboxamide (compound 4)**

The preparation was performed according to the method for preparing compound 18 in Step 4 of Example 27, except that compound 18d was replaced with compound 34e. The crude product was separated and purified through reversed-phase column chromatography on a C18 column (acetonitrile/0.05% formic acid in water=60/40), to afford compound 34 (85.0 mg). MS m/z (ESI): 494.1 $[M+H]^+$.

$^1$HNMR (400 MHz, DMSO-*d*$_6$) δ 11.63 (s, 1H), 8.94 (s, 1H), 8.57 (s, 1H), 8.44 (d, *J* = 6.0 Hz, 1H), 8.26 (d, *J* = 8.7 Hz, 1H), 7.96 (s, 2H), 7.69 (d, *J* = 6.0 Hz, 1H), 7.64 (d, *J* = 8.7 Hz, 3H), 7.52 (d, *J* = 8.1 Hz, 2H), 4.78 (s, 2H), 2.42 (s, 3H).

**Example 30: 4-amino-N-(1-(hydroxy(4-(trifluoromethyl)phenyl)methyl)-6-methylisoquinolin-5-yl)thieno[3,2-d]pyrimidine-7-carboxamide (compound 33) and 4-amino-N-(6-methyl-1-(4-(trifluoromethyl)benzoyl)isoquinolin-5-yl)thieno[3,2-d]pyrimidine-7-carboxamide (compound 32)**

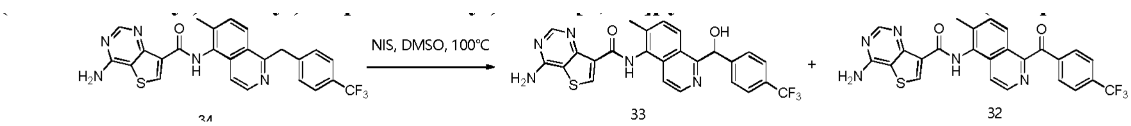


34 33 32

Compound 34 (80.0 mg, 162.1 μmol) and NIS (40.1 mg, 178.3 μmol) were dissolved in DMSO (3.0 mL), and the reaction was allowed to proceed at 100°C for 3 hours. After the reaction was complete, the reaction solution was cooled to room temperature, and then directly separated and purified through reversed-phase column chromatography on a C18 column (acetonitrile /0.05% formic acid in water=40/60), to afford compound 33 (32.0 mg), MS m/z (ESI): 510.1 $[M+H]^+$; and compound 32 (12.0 mg), MS m/z (ESI): 508.1 $[M+H]^+$

compound 33: $^1$HNMR (400 MHz, DMSO-*d*$_6$) δ 11.62 (s, 1H), 8.94 (s, 1H), 8.57 (s, 1H), 8.47 (d, *J* = 5.9 Hz, 1H), 8.36 (d, *J*= 8.8 Hz, 1H), 7.96 (s, 2H), 7.75 (d, *J* = 6.1 Hz, 1H), 7.69-7.63 (m, 4H), 7.57 *(d, J*= 8.9 Hz, 1H), 6.68 (d, *J*= 5.5 Hz, 1H), 6.52 (d, *J*= 5.1 Hz, 1H), 2.40 (s, 3H).

compound 32: $^1$HNMR (400 MHz, DMSO-*d*$_6$) δ 11.76 (s, 1H), 8.98 (s, 1H), 8.62 (d, *J* = 5.9 Hz, 2H), 8.14 (d, *J* = 8.7 Hz, 1H), 8.07 (d, *J* = 8.1 Hz, 2H), 8.04 (d, *J* = 5.9 Hz, 1H), 7.99 (s, 2H), 7.94 (d, *J* = 8.3 Hz, 2H), 7.75 *(d, J*= 8.8 Hz, 1H), 2.49 (s, 3H).

**Example 31: 4-amino-N-(6-methyl-1-(1-phenoxyethyl)isoquinolin-5-yl)thieno[3,2-d]pyrimidine-7-carboxamide (compound 35)**

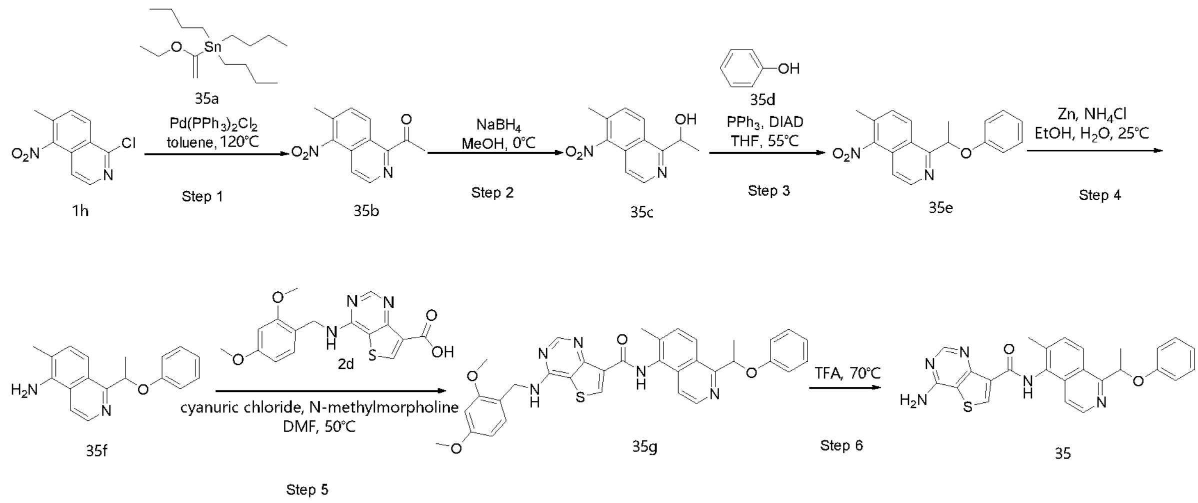


**Step 1: preparation of 1-(6-methyl-5-nitroisoquinolin-1-yl)ethanone (compound 35b)**

Compounds 1h (300 mg, 1.35 mmol) and 35a (632.66 mg, 1.75 mmol) were dissolved in toluene (40 mL), bis(triphenylphoshine)palladium chloride (94.58 mg, 134.75 μmol) was then added, the reaction was heated to 120°C under the protection of nitrogen and allowed to proceed for 16 hours. The reaction solution was cooled to room temperature, concentrated under reduced pressure to remove toluene, added with ethyl acetate to dilute the remaining reaction solution, and washed with water for 3 times. The organic phase was dried over anhydrous sodium sulfate, filtered, and then concentrated under reduced pressure to remove the solvent. The residue was then dissolved by adding tetrahydrofuran (40 mL), followed by slow dropwise addition of 6N hydrochloric acid (6 mL), and after the addition, the reaction was allowed to proceed at room temperature for 2 hours, and then warmed to 40°C and allowed to proceed for 1 hour. The reaction solution was diluted by adding 40 ml water, added with sodium carbonate under ice-bath cooling to adjust the pH to weak alkaline, and extracted with ethyl acetate. The organic phase was dried over anhydrous sodium sulfate, filtered and then concentrated under reduced pressure, the crude product was separated and purified by column chromatography on silica gel (petroleum ether /ethyl acetate =3/1), to afford compound 35b (310 mg). MS m/z (ESI): 231.0 $[M+H]^+$.

**Step 2: preparation of 1-(6-methyl-5-nitroisoquinolin-1-yl)ethanol (compound 35c)**

Compound 35b (150 mg, 651.55 μmol) was dissolved in methanol (10 mL), sodium borohydride (24.65 mg, 651.55 μmol) was added at 0°C in batches, and the reaction was stirred for 5 minutes. The reaction was quenched with 10 mL of a saturated solution of ammonium chloride, and extracted with ethyl acetate. The organic phase was dried over anhydrous sodium sulfate, filtered and then concentrated under reduced pressure, the crude product was separated and purified by column chromatography on silica gel (dichloromethane /methanol =19/1), to afford compound 35c (120 mg). MS m/z (ESI): 233.1 $[M+H]^+$.

**Step 3: preparation of 6-methyl-5-nitro-1-(1-phenoxyethyl)isoquinoline (compound 35e)**

Compounds 35c (120 mg, 516.72 μmol), 35d (58.35 mg, 620.06 μmol) were dissolved in anhydrous tetrahydrofuran (10 mL), the reaction was stirred at room temperature for 10 minutes, triphenylphosphine (176.19 mg, 671.73 μmol) was then added, the reaction was allowed to proceed under the protection of nitrogen for 10 minutes, and diisopropyl azodicarboxylate (135.83 mg, 671.73 μmol, 131.87 μL) was added in the end. The reaction was warmed to 55°C under the protection of nitrogen, and stirred for 16 hours. The reaction was quenched with 10 mL of a saturated solution of ammonium chloride, and extracted with ethyl acetate. The organic phase was dried over anhydrous sodium sulfate, filtered and then concentrated under reduced pressure, the crude product was separated and purified by column chromatography on silica gel (petroleum ether /ethyl acetate =4/1), to afford compound 35e (76 mg). MS m/z (ESI): 309.2 $[M+H]^+$.

**Step 4: preparation of 6-methyl-1-(1-phenoxyethyl)isoquinolin-5-amine (compound 35f)**

Compound 35e (76 mg, 0.25 μmol) was dissolved in ethanol (10 mL), zinc powder (80.59 mg, 1.23 μmol), ammonium chloride (32.96 mg, 0.62 μmol) and water (2 mL) were then added, and after the addition, the reaction was allowed to proceed at 25°C under the protection of nitrogen for 16 hours. After the reaction was complete, the mixture was filtered, concentrated, and the crude product was separated and purified by column chromatography on silica gel (dichloromethane /methanol =9/1), to afford compound 35f (32 mg). MS m/z (ESI): 279.0 $[M+H]^+$.

**Step 5: preparation of 4-((2,4-dimethoxybenzyl)amino)-N-(6-methyl-1-(1-phenoxyethyl)isoquinolin-5-yl)thieno[3,2-d]pyrimidine-7-carboxamide (compound 35g)**

Compound 2d (20 mg, 57.91 μmol), cyanuric chloride (21.36 mg, 115.82 μmol), N-methylmorpholine (17.57 mg, 173.73 μmol) were dissolved in anhydrous DMF (10 mL), the reaction was warmed to 50°C and stirred for 30 minutes. Compound 35f (20 mg, 57.91 μmol) was then added, and the reaction was allowed to proceed under the protection of nitrogen at 50°C for 16 hours. The reaction solution was diluted with ethyl acetate, and washed with water for 3 times. The organic phase was dried over anhydrous sodium sulfate, filtered and then concentrated under reduced pressure, the crude product was purified on a preparative silica gel plate (dichloromethane/methanol=15/1), to afford compound 35g (10 mg). MS m/z (ESI): 606.0 $[M+H]^+$.

**Step 6: preparation of 4-amino-N-(6-methyl-1-(1-phenoxyethyl)isoquinolin-5-yl)thieno[3,2-d]pyrimidine-7-carboxamide (compound 35)**

The preparation was performed according to a method same as that in Step 8 of Example 1, except that compound 1k was replaced with compound 35g. The crude product was separated and purified through reversed-phase column chromatography on a C18 column (acetonitrile/0.05% ammonium bicarbonate in water=56/44), to afford compound 35 (7.5 mg). MS m/z (ESI): 455.9 $[M+H]^+$.

$^1H$ NMR (400 MHz, DMSO-$d_6$) δ 11.64 (s, 1H), 8.94 (s, 1H), 8.56 (s, 1H), 8.51 (d, *J* = 8.8 Hz, 1H), 8.46 (d, *J* = 5.9 Hz, 1H), 7.97 (s, 2H), 7.72 (d, *J* = 5.9 Hz, 1H), 7.67 (d, *J* = 8.9 Hz, 1H), 7.18 (t, *J* = 8.0 Hz, 2H), 6.92 (d, *J* = 8.0 Hz, 2H), 6.83 (t, *J* = 7.3 Hz, 1H), 6.20 (q, *J* = 6.5 Hz, 1H), 2.42 (s, 3H), 1.80 (d, *J* = 6.5 Hz, 3H).

**Example 32: 4-amino-N-(1-((3-chlorophenyl)amino)-6-methylisoquinolin-5-yl)quinazoline-8-carboxamide (compound 38)**

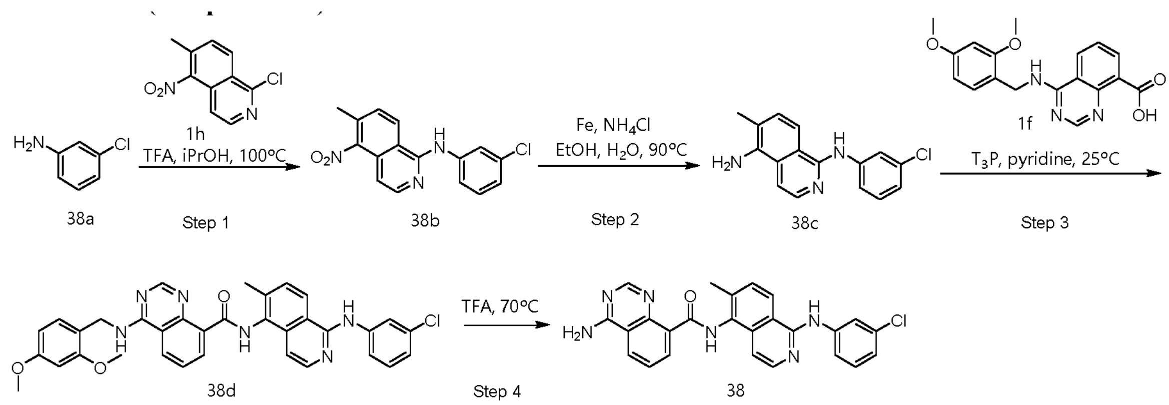


**Step 1: preparation of N-(3-chlorophenyl)-6-methyl-5-nitroisoquinolin-1-amine (compound 38b)**

Compound 38b (280 mg) was prepared according to a method same as that in Step 5 of Example 1, except that compound 1g was replaced with compound 38a. MS m/z (ESI): 314.1 $[M+H]^+$.

**Step 2: preparation of N1-(3-chlorophenyl)-6-methylisoquinoline-1,5-diamine (compound 38c)**

Compound 38c (108 mg) was prepared according to a method same as that in Step 3 of Example 29, except that compound 34c was replaced with compound 38b and methanol was replaced with ethanol. MS m/z (ESI): 284.0

$[M+H]^+$.

**Step 3: preparation of N-(1-((3-chlorophenyl)amino)-6-methylisoquinolin-5-yl)-4-((2,4-dimethoxybenzyl)amino)quinazoline-8-carboxamide (compound 38d)**

Compound 38d (18 mg) was prepared according to a method same as that in Step 7 of Example 1, except that compound 1j was replaced with compound 38c. MS m/z (ESI): 605.2 $[M+H]^+$.

**Step 4: preparation of 4-amino-N-(1-((3-chlorophenyl)amino)-6-methylisoquinolin-5-yl)quinazoline-8-carboxamide (compound 38)**

The preparation was performed according to a method same as that in Step 8 of Example 1, except that compound 1k was replaced with compound 38d. The crude product was separated and purified through reversed-phase column chromatography on a C18 column (acetonitrile/0.05% ammonium bicarbonate in water=65/35), to afford compound 38 (5 mg). MS m/z (ESI): 455.1 $[M+H]^+$.

$^1$H NMR (400 MHz, DMSO-$d_6$) δ 13.21 (s, 1H), 9.37 (s, 1H), 8.66 (dd, *J* = 7.4, 1.4 Hz, 1H), 8.63 (s, 1H), 8.53 (dd, *J* = 8.3, 1.4 Hz, 1H), 8.45 (d, *J* = 8.7 Hz, 1H), 8.38 (brs, 1H), 8.23 (brs, 1H), 8.17 (t, *J* = 2.0 Hz, 1H), 8.05 (d, *J* = 6.0 Hz, 1H), 7.85 (dd, *J* = 8.3, 1.2 Hz, 1H), 7.70 (t, *J* = 8.0 Hz, 1H), 7.64 (d, *J* = 8.7 Hz, 1H), 7.35 (t, *J* = 8.1 Hz, 1H), 7.31 (d, *J* = 6.0 Hz, 1H), 7.02 (dd, *J* = 8.0, 1.2 Hz, 1H), 2.45 (s, 3H).

**Example 33: 4-amino-N-(1-((3-chlorophenyl)amino)-6-methylisoquinolin-5-yl)imidazo[2,1-f][1,2,4]triazine-7-carboxamide (compound 12)**

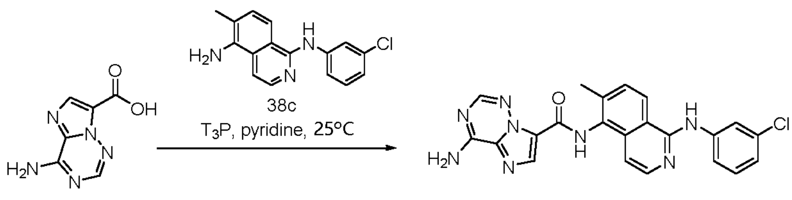


30c

12

The preparation was performed according to a method same as that in Step 3 of Example 13, except that compound 1j was replaced with compound 38c. The crude product was separated and purified through reversed-phase column chromatography on a C18 column (acetonitrile/0.05% ammonium bicarbonate in water=62/38), to afford compound 12 (10 mg). MS m/z (ESI): 445.0 $[M+H]^+$.

$^1$H NMR (400 MHz, DMSO-$d_6$) δ 10.39 (s, 1H), 9.38 (s, 1H), 8.70 (s, 1H), 8.62 (s, 1H), 8.48 (d, *J* = 8.7 Hz, 1H), 8.38 (s, 1H), 8.25 (s, 1H), 8.16 (t, *J* = 2.0 Hz, 1H), 8.06 (d, *J* = 6.0 Hz, 1H), 7.85 (dd, *J* = 8.3, 1.2 Hz, 1H), 7.64 (d, *J* = 8.8 Hz, 1H), 7.35 (t, *J* = 8.1 Hz, 1H), 7.25 (d, *J* = 6.0 Hz, 1H), 7.02 (dd, *J* = 7.9, 1.3 Hz, 1H), 2.44 (s, 3H).

**Example 34: 4-amino-N-(1-((2-fluorophenyl)amino)-6-methylisoquinolin-5-yl)quinazoline-8-carboxamide (compound 39)**

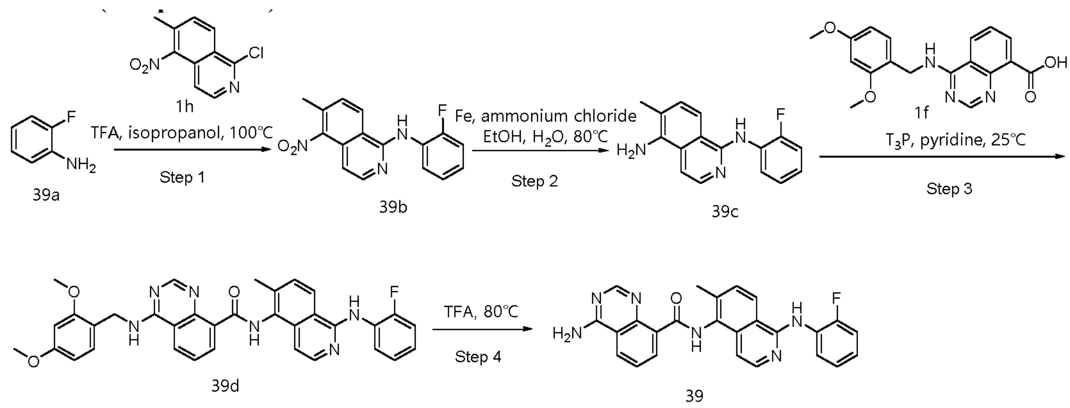


39a 39b 39c 39d 39

**Step 1: preparation of N-(2-fluorophenyl)-6-methyl-5-nitroisoquinolin-1-amine (compound 39b)**

Compounds 39a (89.8 mg, 808.5 μmol), 1h (150.0 mg, 673.8 μmol) and TFA (76.8 mg, 673.8 μmol) were added to isopropanol (5.0 mL), the reaction was warmed to 100°C and allowed to proceed for 16 hours. After the reaction was complete, the reaction solution was adjusted to a basic pH with triethylamine, concentrated, and the crude product was separated and purified by column chromatography on silica gel (PE/EA=5/1), to afford compound 39b (195.0 mg). MS m/z (ESI): 298.1 $[M+H]^+$.

**Step 2: preparation of $N^1$-(2-fluorophenyl)-6-methylisoquinoline-1,5-diamine (compound 39c)**

Compound 39b (100.0 mg, 336.4 μmol) was added to ethanol (3.0 mL) and water (1.0 mL), iron powder (93.9 mg, 1680.0 μmol) and ammonium chloride (18.0 mg, 336.4 μmol) were then sequentially added, and after the addition, the reaction was warmed to 80°C and allowed to proceed for 2 hours. After the reaction was complete, the mixture was filtered through diatomaceous earth, the filtrate was concentrated, to afford compound 39c (85.0 mg). MS m/z (ESI): 268.1 $[M+H]^+$.

**Step 3: preparation of 4-((2,4-dimethoxybenzyl)amino)-N-(1-((2-fluorophenyl)amino)-6-methylisoquinolin-5-yl)quinazoline-8-carboxamide (compound 39d)**

Compounds 39c (20.0 mg, 74.8 μmol) and 1f (38.1 mg, 112.2 μmol) were dissolved in pyridine (2.0 mL), $T_3P$ (1 mL, 50% in DMF) was dropwise added, and after the addition, the reaction was allowed to proceed at 25°C for 3 hours. After the reaction was complete, the solvent was removed by concentration under reduced pressure, the reaction solution was diluted with ethyl acetate, washed with a saturated aqueous solution of sodium bicarbonate for 3 times, the organic phase was dried over anhydrous sodium sulfate, filtered and then concentrated, and the crude product was separated and purified by column chromatography on silica gel (PE/EA=3/2), to afford compound 39d (35.0 mg). MS m/z (ESI): 589.2 $[M+H]^+$.

**Step 4: preparation of 4-amino-N-(1-((2-fluorophenyl)amino)-6-methylisoquinolin-5-yl)quinazoline-8-carboxamide (compound 39)**

Compound 39d (35.0 mg, 59.5 μmol) was added to trifluoroacetic acid (2.0 mL), and the reaction was allowed to proceed at 80°C for 2 hours. After the reaction was complete, the reaction solution was concentrated to dryness, alkalized by adding a saturated aqueous solution of sodium bicarbonate, extracted with dichloromethane, and concentrated to afford a crude product, the crude product was separated and purified by Prep-HPLC, to afford compound 39 (5.3 mg). MS m/z (ESI): 439.1 $[M+H]^+$.

$^1$H NMR (400 MHz, DMSO-$d_6$+$D_2O$) δ 8.67 (dd, *J* = 7.5 Hz, *J* = 1.6 Hz, 1H), 8.62 (s, 1H), 8.50 (dd, *J* = 8.3Hz, *J* =1.5 Hz, 1H), 8.32 (d, *J* = 8.6 Hz, 1H), 7.87 (d, *J* = 6.0 Hz, 1H), 7.74 (t, *J* = 7.8 Hz, 1H), 7.67 - 7.51 (m, 2H), 7.32 - 7.17 (m, 4H), 2.45 (s, 3H).

**Example 35: 4-amino-N-(1-((4-chloro-2-fluorophenyl)amino)-6-methylisoquinolin-5-yl)quinazoline-8-carboxamide (compound 41)**

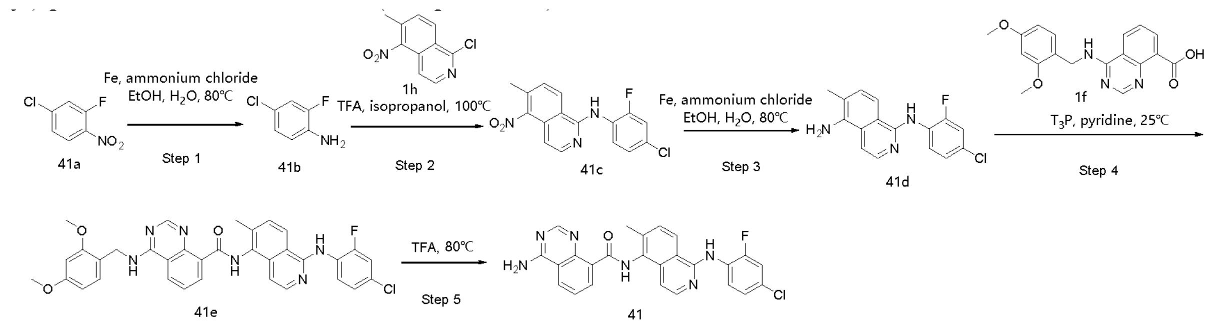


**Step 1: preparation of 4-chloro-2-fluoroaniline (compound 41b)**

Compound 41a (1000.0 mg, 5700.0 μmol) was dissolved in ethanol (12.0 mL) and water (4.0 mL), iron powder (1590.0 mg, 28480.0 μmol) and ammonium chloride (304.7 mg, 5700.0 μmol) were then sequentially added, and the reaction was warmed to 80°C and allowed to proceed for 2 hours. After the reaction was complete, the mixture was filtered through diatomaceous earth, the filtrate was concentrated, to afford compound 41b (800.0 mg). MS m/z (ESI): 146.1 $[M+H]^+$.

**Step 2: preparation of N-(4-chloro-2-fluorophenyl)-6-methyl-5-nitroisoquinolin-1-amine (compound 41c)**

Compounds 1h (200.0 mg, 898.4 μmol), 41b (156.9 mg, 1080.0 μmol) and TFA (102.4 mg, 898.4 μmol) were added to isopropanol (5.0 mL), and the reaction was warmed to 100°C and allowed to proceed for 16 hours. After the reaction was complete, the reaction solution was adjusted to a basic pH with triethylamine, concentrated, and the crude product was separated and purified by column chromatography on silica gel (PE/EA=5/1), to afford compound 41c (240.0 mg). MS m/z (ESI): 332.0 $[M+H]^+$.

**Step 3: preparation of $N^1$-(4-chloro-2-fluorophenyl)-6-methylisoquinoline-1,5-diamine (compound 41d)**

Compound 41c (100.0 mg, 301.5 μmol) was added to ethanol (3.0 mL) and water (1.0 mL), iron powder (84.2 mg, 1510.0 μmol) and ammonium chloride (16.1 mg, 301.5 μmol) were then sequentially added, and the reaction was warmed to 80°C and allowed to proceed for 2 hours. After the reaction was complete, the mixture was filtered through diatomaceous earth, the filtrate was concentrated to dryness, to afford compound 41d (85.0 mg). MS m/z (ESI): 302.1 $[M+H]^+$.

**Step 4: preparation of N-(1-((4-chloro-2-fluorophenyl)amino)-6-methylisoquinolin-5-yl)-4-((2,4-dimethoxybenzyl)amino)quinazoline-8-carboxamide (compound 41e)**

Compounds 41d (20.0 mg, 66.3 μmol) and 1f (33.7 mg, 99.4 μmol) were dissolved in pyridine (2.0 mL), $T_3P$ (1 mL, 50% in DMF) was dropwise added, and after the addition, the reaction was allowed to proceed at 25°C for 3 hours. After the reaction was complete, the solvent was removed by concentration under reduced pressure, the reaction solution was diluted with ethyl acetate, washed with a saturated aqueous solution of sodium bicarbonate for 3 times, the organic phase was dried over anhydrous sodium sulfate, filtered and then concentrated, and the crude product was separated and purified by column chromatography on silica gel (PE/EA=3/2), to afford compound 41e (20.0 mg). MS m/z (ESI): 623.2 $[M+H]^+$.

**Step 5: preparation of 4-amino-N-(1-((4-chloro-2-fluorophenyl)amino)-6-methylisoquinolin-5-yl)quinazoline-8-carboxamide (compound 41)**

Compound 41e (20.0 mg, 32.1 μmol) was added to trifluoroacetic acid (2.0 mL), and the reaction was allowed to proceed at 80°C for 2 hours. After the reaction was complete, the reaction solution was concentrated to dryness, alkalized by adding a saturated aqueous solution of sodium bicarbonate, extracted with dichloromethane, the organic phase was concentrated to afford a crude product, and the crude product was separated and purified by Prep-HPLC, to afford compound 41 (4.4 mg). MS m/z (ESI): 473.0 $[M+H]^+$.

$^1H$ NMR (400 MHz, DMSO-$d_6$) δ 13.19 (s, 1H), 9.13 (s, 1H), 8.65 (dd, *J* = 7.4, 1.3 Hz, 1H), 8.62 (s, 1H), 8.52 (dd, *J* = 8.2, 1.2 Hz, 1H), 8.44 - 8.14 (m, 3H), 7.88 (d, *J* = 6.0 Hz, 1H), 7.74 - 7.57 (m, 3H), 7.47 (dd, *J* = 10.5, 2.3 Hz, 1H), 7.29 (dd, *J* = 8.6, 1.4 Hz, 1H), 7.23 (d, *J* = 5.9 Hz, 1H), 2.44 (s, 3H).

**Example 36: 4-amino-N-(1-((2-fluorophenyl)amino)-6-methylisoquinolin-5-yl)imidazo[2,1-f][1,2,4]triazine-7-carboxamide (compound 44)**

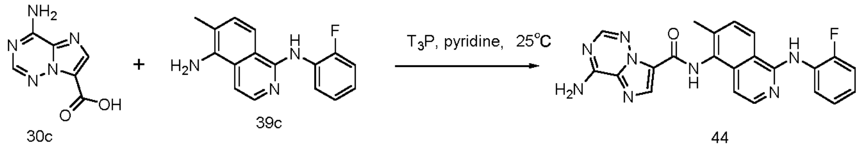


30c 39c 44

Compounds 30c (20 mg, 111.65 μmol) and 39c (29.84 mg, 111.65 μmol) were dissolved in pyridine (3 mL), $T_3P$ (2 mL, 50% in DMF) was then added, and the reaction was stirred at 25°C for 16 hours. After the reaction was complete, pyridine was removed by rotary evaporation, the reaction solution was adjusted to a pH of about 8 by dropwise addition of a saturated solution of sodium bicarbonate, extracted with EA, washed with water and saturated brine, dried over anhydrous sodium sulfate, filtered, concentrated, and separated and purified through Prep-TLC (MeOH/DCM=1/15), to afford a crude product, which was then separated and purified through Prep-HPLC, to afford compound 44 (3 mg). MS m/z (ESI): 429.1 $[M+H]^+$.

$^1H$ NMR (400 MHz, DMSO-$d_6$) δ 10.37 (s, 1H), 9.07 (s, 1H), 8.70 (s, 1H), 8.62 (s, 1H), 8.38 (d, *J* = 10.0Hz, 1H), 8.37 (s, 1H), 8.24 (s, 1H), 7.87 (d, *J* = 6.0 Hz, 1H), 7.62 - 7.56 (m, 2H), 7.28 - 7.18 (m, 3H), 7.15 (d, *J* = 6.0 Hz, 1H), 2.43 (s, 3H).

**Example 37: 4-amino-N-(6-methyl-1-((3-(trifluoromethyl)phenyl)amino)isoquinolin-5-yl)imidazo[2,1-f][1,2,4]triazine-7-carboxamide (comnound 43)**

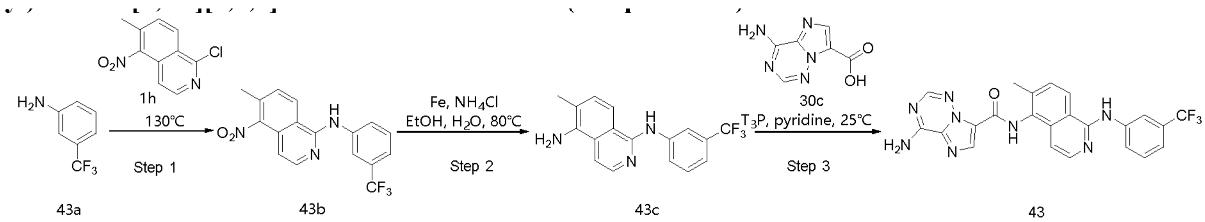


43a 43b 43c 43

**Step 1: preparation of 6-methyl-5-nitro-N-(3-(trifluoromethyl)phenyl)isoquinolin-1-amine (compound 43b)**

Compounds 43a (731.1 mg, 4.45 mmol), 1h (200.0 mg, 889.4 μmol) were warmed to 130°C under neat condition for 5 hours. After the reaction was complete, the mixture was directly separated and purified by column chromatography on silica gel (PE/EA=4/1), to afford compound 43b (120.0 mg). MS m/z (ESI): 348.1 $[M+H]^+$.

**Step 2: preparation of 6-methyl-$N^1$-(3-(trifluoromethyl)phenyl)isoquinoline-1,5-diamine (compound 43c)**

Compound 43b (120.0 mg, 345.5 μmol), reduced iron powder (59.1 mg, 1.04 mmol), ammonium chloride (37.7 mg, 691.1 μmol) were added to a mixed solution of ethanol (24.0 mL) and water (8.0 mL), purge with nitrogen was performed for 3 times, and the reaction was warmed to 80°C and allowed to proceed for 3 hours. After the reaction was complete, the mixture was filtered with suction to remove the solid, the filtrate was concentrated, and the crude product was separated and purified by column chromatography on silica gel (PE/EA=4/1), to afford compound 43c (90.0 mg). MS m/z (ESI): 318.1 $[M+H]^+$.

**Step 3: preparation of 4-amino-N-(6-methyl-1-((3-(trifluoromethyl)phenyl)amino)isoquinolin-5-yl)imidazo[2,1-f][1,2,4]triazine-7-carboxamide (compound 43)**

Compounds 43c (26.6 mg, 83.7 μmol), 30c (15.0 mg, 83.7 μmol) were dissolved in pyridine (5.0 mL), $T_3P$ (159.9 mg, 251.2 μmol) was then added, and after the addition, the reaction was allowed to proceed at 25°C for 16 hours. After the reaction was complete, the reaction was concentrated to remove pyridine, dissolved in ethyl acetate, added with saturated sodium bicarbonate for alkalization, extracted with ethyl acetate for three times, dried over anhydrous sodium sulfate, and filtered with suction. The filtrate was concentrated, and then separated and purified through Prep-HPLC to afford compound 43 (3.0 mg). MS m/z (ESI): 479.1 [M+H]+.

$^1$H NMR (400 MHz, DMSO-$d_6$) δ 10.41 (s, 1H), 9.67 (br, 1H), 8.70 (s, 1H), 8.62 (s, 1H), 8.52 (d, *J*= 8.8 Hz, 1H), 8.38 (s, 1H), 8.35 (s, 1H), 8.25 (s, 1H), 8.22 (d, *J*= 8.8 Hz, 1H), 8.02 (d, *J*= 6.0 Hz, 1H), 7.68 (d, *J*= 8.8 Hz, 1H), 7.58 (t, *J*= 7.6 Hz, 1H), 7.36 (d, *J*= 6.8 Hz, 1H), 7.28 (d, *J*= 6.0 Hz, 1H), 2.45 (s, 3H).

**Example 38: 4-amino-N-(1-((6-(dimethylamino)pyridin-3-yl)methyl)-6-methylisoquinolin-5-yl)thieno[3,2-d]pyrimidine-7-carboxamide (compound 36)**

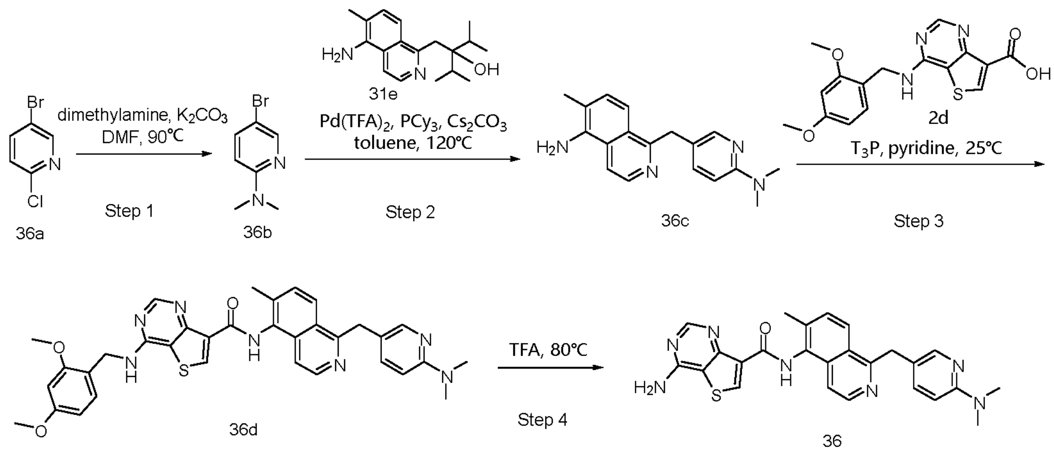


**Step 1: preparation of 5-bromo-N,N-dimethylpyridin-2-amine (compound 36b)**

Compound 36a (650 mg, 3.38 mmol), dimethylamine hydrochloride (550.86 mg, 6.76 mmol) and potassium carbonate (1.4 g, 10.13 mmol) were added to DMF (10.0 mL), and the reaction was allowed to proceed at 90°C for 12 hours. After the reaction was complete, the reaction was added with 200 ml water, and extracted with EA. The organic phase was dried over anhydrous sodium sulfate, filtered, concentrated, separated and purified by column chromatography on silica gel (DCM/MeOH=10/1), to afford compound 36b (512 mg). MS m/z (ESI): 201.0 [M+H]+.

**Step 2: preparation of 1-((6-(dimethylamino)pyridin-3-yl)methyl)-6-methylisoquinolin-5-amine (compound 36c)**

Compounds 36b (280.8 mg, 1.40 mmol), 31e (200 mg, 698.30 mmol), palladium trifluoroacetate (23.21 mg, 69.83 μmol), tricyclohexylphosphane (39.16 mg, 139.66 μmol) and cesium carbonate (455.04 mg, 1.40 mmol) were added to toluene (10.0 mL), purge with nitrogen was performed for three times, and the reaction was allowed to proceed at 120°C for 3 hours. After the reaction was complete, the reaction solution was filtered through diatomaceous earth, added with 200 ml water, and extracted with EA. The organic phase was washed with a saturated solution of sodium bicarbonate, dried over anhydrous sodium sulfate, filtered, concentrated, separated and purified by Flash column chromatography on silica gel (PE/EA=1/1), to afford compound 36c (121 mg). MS m/z (ESI): 293.2 [M+H]+.

**Step 3: preparation of 4-((2,4-dimethoxybenzyl)amino)-N-(1-((6-(dimethylamino)pyridin-3-yl)methyl)-6-methylisoquinolin-5-yl)thieno[3,2-d]pyrimidine-7-carboxamide (compound 36d)**

Compounds 36c (150 mg, 513.04 μmol) and 2d (212.63 mg, 615.54 μmol) were added to pyridine (10.0 mL), $T_3P$ (489.71 mg, 1.54 mmol) was then added, and the reaction was allowed to proceed at 25°C for 3 hours. After the reaction was complete, the reaction was added with 50 ml water, and extracted with EA. The organic phase was dried over anhydrous sodium sulfate, filtered, concentrated, separated and purified by Flash column chromatography on silica gel (DCM/MeOH=20/1), to afford compound 36d (180 mg). MS m/z (ESI): 620.1 [M+H]+.

**Step 4: preparation of 4-amino-N-(1-((6-(dimethylamino)pyridin-3-yl)methyl)-6-methylisoquinolin-5-yl)thieno[3,2-d]pyrimidine-7-carboxamide (compound 36)**

Compound 36d (180 mg, 290.45 μmol) was added to trifluoroacetic acid (5.0 mL), and the reaction was allowed to proceed at 80°C for 12 hours. After the reaction was complete, the reaction solution was concentrated to dryness,

added with a saturated solution of sodium bicarbonate for alkalization, and extracted with ethyl acetate. The organic phase was dried over anhydrous sodium sulfate, filtered, concentrated, and the crude product was separated and purified by Prep-HPLC, to afford compound 36 (120 mg). MS m/z (ESI): 470.1 $[M+H]^+$.

$^1H$ NMR (400 MHz, DMSO-$d_6$) δ 11.62 (s, 1H), 8.94 (s, 1H), 8.57 (s, 1H), 8.40 (d, *J* = 6.0 Hz, 1H), 8.28 (d, *J* = 0.8 Hz, 1H), 8.11 (d, *J* = 2.8 Hz, 1H), 7.97 (s, 2H), 7.63 (t, *J* = 7.8 Hz, 2H), 7.36 (dd, *J* = 8.8, 2.4 Hz, 1H), 6.52 (d, *J* = 8.4 Hz, 1H), 4.50 (s, 2H), 2.94 (s, 6H), 2.42 (s, 3H).

**Example 39: 4-amino-N-(1-((6-(dimethylamino)pyridin-3-yl)(hydroxy)methyl)-6-methylisoquinolin-5-yl)thieno[3,2-d]pyrimidine-7-carboxamide (compound 37)**

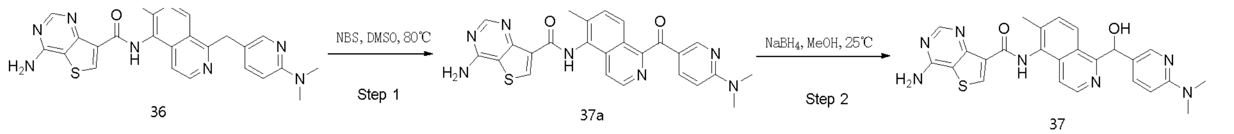


36 37a 37

**Step 1: preparation of 4-amino-N-(1-(6-(dimethylamino)nicotinoyl)-6-methylisoquinolin-5-yl)thieno[3,2-d]pyrimidine-7-carboxamide (compound 37a)**

Compound 36 (50 mg, 106.48 μmol) and NBS (3.79 mg, 21.30 μmol) were dissolved in DMSO (3.0 mL), and the reaction was allowed to proceed at 80°C for 2 hours. After the reaction was complete, the reaction solution was cooled to room temperature, and then directly separated and purified by Prep-HPLC, to afford compound 37a (26 mg). MS m/z (ESI): 484.1 $[M+H]^+$.

**Step 2: preparation of 4-amino-N-(1-((6-(dimethylamino)pyridin-3-yl)(hydroxy)methyl)-6-methylisoquinolin-5-yl)thieno[3,2-d]pyrimidine-7-carboxamide (compound 37)**

Compound 37a (50 mg, 106.48 μmol) was dissolved in methanol (3.0 mL), sodium borohydride (7.82 mg, 206.81 μmol) was then added at 0°C, and after the addition, the reaction was allowed to proceed at 25°C for 12 hours. After the reaction was complete, the reaction was quenched by adding saturated ammonium chloride, and extracted with ethyl acetate. The organic phase was dried over anhydrous sodium sulfate, filtered, concentrated, and the crude product was separated and purified by Prep-HPLC to afford compound 37 (26 mg), MS m/z (ESI): 486.1 $[M+H]^+$.

$^1H$ NMR (400 MHz, DMSO-$d_6$) δ 11.62 (s, 1H), 8.94 (s, 1H), 8.57 (s, 1H), 8.47 (d, *J* = 6.0 Hz, 1H), 8.32 (d, *J* = 8.8 Hz, 1H), 8.16 (d, *J* = 2.0 Hz, 1H), 7.97 (s, 2H), 7.72 (d, *J* = 6.0 Hz, 1H), 7.57 (d, *J* = 8.8 Hz, 1H), 7.38 (dd, *J* = 8.8, 2.4 Hz, 1H), 6.53 (d, *J* = 8.4 Hz, 1H), 6.36 (d, *J* = 5.2 Hz, 1H), 6.21 (d, *J* = 5.6 Hz, 1H), 2.95 (s, 6H), 2.40 (s, 3H).

**Example 40: 4-amino-N-(1-(4-chloro-3-((dimethylamino)methyl)benzyl)-6-methylisoquinolin-5-yl)thieno[3,2-d]pyrimidine-7-carboxamide (compound 42)**

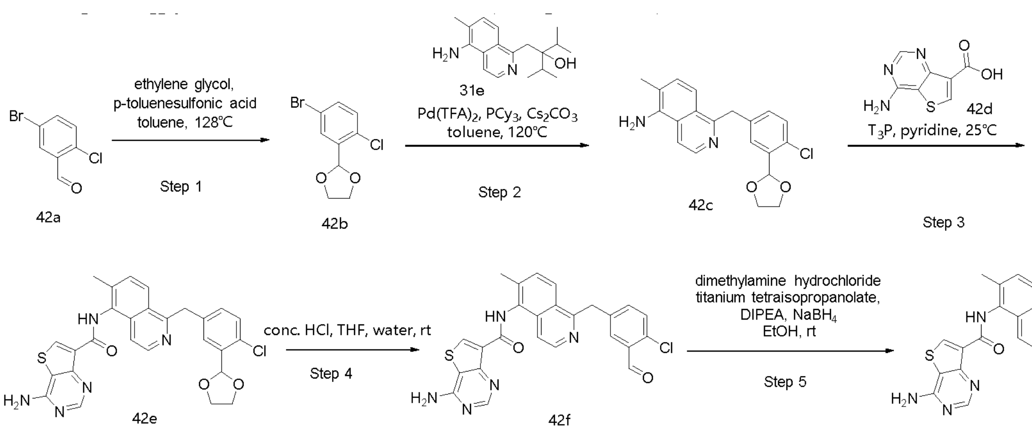


42a 42b 42c 42e 42f 42

**Step 1: preparation of 2-(5-bromo-2-chlorophenyl)-1,3-dioxolane (compound 42b)**

Compound 42a (2 g, 9.11 mmol) and ethylene glycol (2.83 g, 45.57 mmol) were added to toluene (22 mL), p-toluenesulfonic acid (156.93 mg, 911.32 mmol) was then added, and after the addition, the reaction was heated to 128°C, and allowed to proceed under reflux for 16 hours with water being separated. After the reaction was complete, the reaction was added with 200 ml water, and extracted with EA. The organic phase was dried over anhydrous sodium sulfate, filtered, and concentrated, to afford compound 42b (1.5 g).

**Step 2: preparation of 1-(4-chloro-3-(1,3-dioxolan-2-yl)benzyl)-6-methylisoquinolin-5-amine (compound 42c)**

Compounds 42b (607.74 mg, 2.30 mmol), 31e (300 mg, 1.15 mmol), palladium trifluoroacetate (38.3 mg, 115.22 μmol), tricyclohexylphosphane (64.62 mg, 230.44 μmol) and cesium carbonate (750.51 mg, 2.30 mmol) were added to toluene (10.0 mL), purge with nitrogen was performed for three times, and the reaction was allowed to proceed at 120°C for 3 hours. After the reaction was complete, the reaction solution was filtered through diatomaceous earth, added with 200 ml water, and extracted with EA. The organic phase was washed with a saturated solution of sodium bicarbonate, dried over anhydrous sodium sulfate, filtered, concentrated, separated and purified by Flash column chromatography on silica gel (PE/EA=1/1), to afford compound 42c (245 mg). MS m/z (ESI): 355.1 $[M+H]^+$.

**Step 3: preparation of 4-amino-N-(1-(4-chloro-3-(1,3-dioxolan-2-yl)benzyl)-6-methylisoquinolin-5-yl)thieno[3,2-d]pyrimidine-7-carboxamide (compound 42e)**

Compounds 42c (100 mg, 281.83 μmol) and 42d (95.86 mg, 310.01 μmol) were added to pyridine (2.0 mL), $T_3P$ (269.01 mg, 845.48 μmol) was then added, and the reaction was allowed to proceed at 25°C for 3 hours. After the reaction was complete, the reaction was added with 100 ml water, the precipitated solid was filtered and collected, to afford compound 42e (125 mg). MS m/z (ESI): 533.1 $[M+H]^+$.

**Step 4: preparation of 4-amino-N-(1-(4-chloro-3-formylbenzyl)-6-methylisoquinolin-5-yl)thieno[3,2-d]pyrimidine-7-carboxamide (compound 42f)**

Compound 42e (180 mg, 290.45 μmol) was added to THF (3.0 mL) and water (1.00 mL), concentrated hydrochloric acid (12M, 0.5 mL) was then added, and after the addition, the reaction was allowed to proceed at room temperature for 12 hours. After the reaction was complete, the reaction solution was concentrated to dryness, added with a saturated solution of sodium bicarbonate for alkalization, and extracted with ethyl acetate. The organic phase was dried over anhydrous sodium sulfate, filtered, and concentrated, to afford compound 42f (30 mg). MS m/z (ESI): 488.2 $[M+H]^+$.

**Step 5: preparation of 4-amino-N-(1-(4-chloro-3-((dimethylamino)methyl)benzyl)-6-methylisoquinolin-5-yl)thieno[3,2-d]pyrimidine-7-carboxamide (compound 42)**

DIPEA (16.42 mg, 127.06 μmol) was added to a solution of dimethylamine hydrochloride (10.36 mg, 127.06 μmol) and titanium tetraisopropanolate (36.11 mg, 127.06 μmol) in ethanol (3.00 mL), 42f (31 mg, 63.53 μmol) was then added, and after the addition, the reaction was allowed to proceed at room temperature for 12 hours. $NaBH_4$ (3.61 mg, 95.29 μmol) was then slowly added, and after the addition, the reaction was continuously stirred at room temperature for 1 hour. After the reaction was complete, the reaction solution was concentrated, roughly purified through silica gel column chromatography (DCM/MeOH=10/1), and then separated and purified through Prep-HPLC, to afford compound 42 (3.75 mg). MS m/z (ESI): 517.0 $[M+H]^+$.

$^1H$ NMR (400 MHz, DMSO-$d_6$) δ 11.62 (s, 1H), 8.94 (s, 1H), 8.57 (s, 1H), 8.43 (d, *J* = 6.8 Hz, 1H), 8.24 (d, *J* = 8.8 Hz, 1H), 7.97 (s, 2H), 7.67 (d, *J* = 6.0 Hz, 1H), 7.61 (d, *J* = 8.4 Hz, 1H), 7.46 (s, 1H), 7.30 (d, *J* = 9.2 Hz, 1H), 7.17 (d, *J* = 8.0 Hz, 1H), 4.66 (s, 2H), 3.40 (s, 2H), 2.41 (s, 3H), 2.14 (s, 6H).

**Example 41: preparation of 4-amino-N-(1-((3-chloro-2-fluorophenyl)amino)-6-methylisoquinolin-5-yl)-6-methylquinazoline-8-carboxamide (compound 45)**

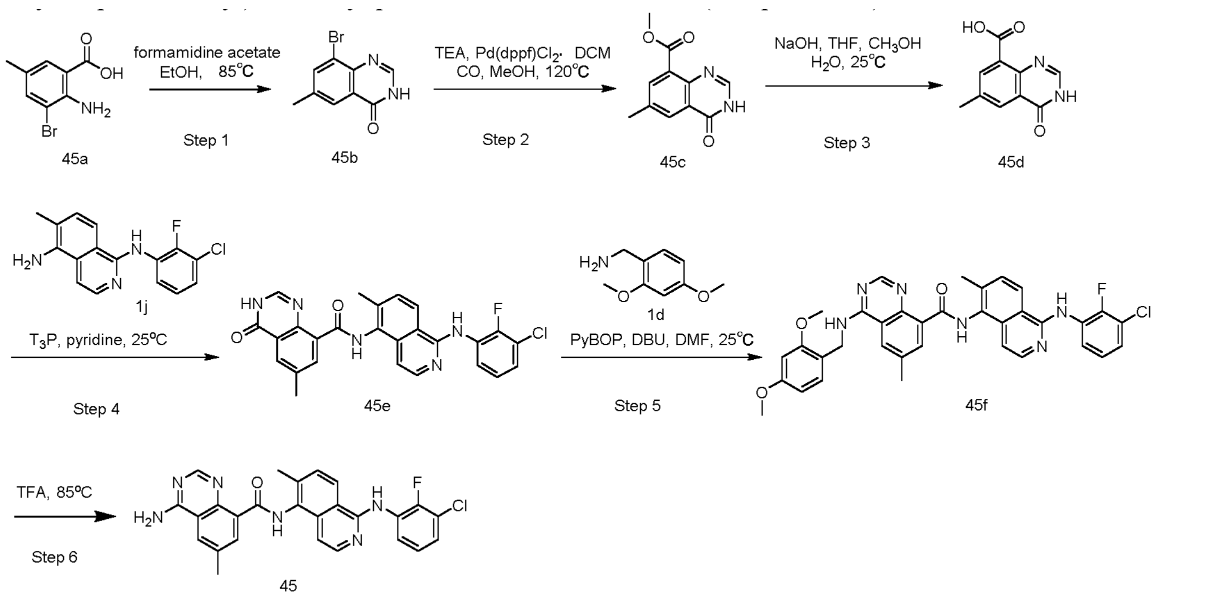


**Step 1: 8-bromo-6-methylquinazolin-4(3H)-one (compound 45b)**

Compound 45a (1 g, 4.35 mmol) and formamidine acetate (1.41 g, 13.56 mmol) were dissolved in anhydrous ethanol (10 mL), and the reaction was stirred at 85°C for 16hr. After the reaction was complete, the reaction solution was cooled to room temperature, filtered with suction, washed with ethanol, and the filter cake was dried to afford compound 45b (800 mg). MS m/z (ESI): 238.1 $[M+H]^+$.

**Step 2: preparation of methyl 6-methyl-4-oxo-3,4-dihydroquinazoline-8-carboxylate (compound 45c)**

45b (900 mg, 3.76 mmol), $Pd(dppf)Cl_2$·DCM (153.72 mg, 188.23 μmol), methanol (15 mL) and TEA (1.90 g, 18.82 mmol, 2.62 mL) were sequentially added to a reaction kettle, purge with nitrogen was performed before CO was pumped in to 1.5 MPa, and then the reaction was heated to 120°C and allowed to proceed for 5hr. After the reaction was complete, the reaction was cooled to room temperature, concentrated, filtered with suction, and the filter cake was dried to afford compound 45c (800 mg). MS m/z (ESI): 218.9 $[M+H]^+$.

**Step 3: preparation of 6-methyl-4-oxo-3,4-dihydroquinazoline-8-carboxylic acid (compound 45d)**

Compound 45c (800 mg, 3.67 mmol) was dissolved in THF (10 mL) and methanol (5 mL), a solution of NaOH (439.95 mg, 11.00 mmol) in water (5 mL) was added, and the reaction was stirred at 25°C for 16hr. After the reaction was complete, the reaction was evaporated under reduced pressure to remove the organic solvent, diluted by adding water, the solution was adjusted to a pH of about 5 with 2M dilute hydrochloric acid, the precipitated solid was filtered with suction, and dried to afford compound 45d (710 mg). MS m/z (ESI): 205.0 $[M+H]^+$.

**Step 4: preparation of N-(1-((3-chloro-2-fluorophenyl)amino)-6-methylisoquinolin-5-yl)-6-methyl-4-oxo-3,4-dihydroquinazoline-8-carboxamide (compound 45e)**

Compounds 45d (67.67 mg, 331.41 μmol) and 1j (100 mg, 331.41 μmol) were dissolved in pyridine (4 mL), $T_3P$ (2 mL, a 50% solution in EA) was then added, and the reaction was stirred at 25°C for 16hr. After the reaction was complete, the solvent was removed under reduced pressure, the reaction was quenched by dropwise addition of a saturated solution of sodium bicarbonate, and extracted with EA. The organic phase was washed with water and saturated brine, dried over anhydrous sodium sulfate, filtered, concentrated, and the crude product was purified by Flash column chromatography on silica gel (DCM:MeOH=90:10), to afford compound 45e (80 mg). MS m/z (ESI): 487.9 $[M+H]^+$.

**Step 5: preparation of N-(1-((3-chloro-2-fluorophenyl)amino)-6-methylisoquinolin-5-yl)-4-((2,4-dimethoxybenzyl)amino)-6-methylquinazoline-8-carboxamide (compound 45f)**

Compound 45e (40 mg, 81.98 μmol) and PyBOP (46.93 mg, 90.18 μmol) were dissolved in DMF (4 mL), and DBU (30.97 mg, 122.97 μmol) was added at 25°C. The reaction was stirred for 10 min, then added with compound 1d

(41.12 mg, 245.95 μmol), and the reaction was kept at 25°C and stirred for 3hr. After the reaction was complete, the reaction was diluted by adding water, and extracted with ethyl acetate. The organic phase was dried over anhydrous sodium sulfate, filtered, and concentrated, to afford compound 45f (36 mg). MS m/z (ESI): 637.0 $[M+H]^+$.

**Step 6: preparation of 4-amino-N-(1-(((3-chloro-2-fluorophenyl)amino)-6-methylisoquinolin-5-yl)-6-methylquinazoline-8-carboxamide (compound 45)**

Compound 45f (36 mg, 56.51 μmol) was dissolved in TFA (3 mL), and the reaction was stirred at 85°C for 3hr. After the reaction was complete, the reaction solution was concentrated to dryness, adjusted to a pH of about 8 by dropwise addition of a saturated solution of sodium bicarbonate, and extracted with EA. The organic phase was washed with a saturated saline solution, dried over anhydrous sodium sulfate, filtered, concentrated, and the crude product was separated and purified by Prep-HPLC, to afford compound 45 (7.58 mg). MS m/z (ESI): 486.9 $[M+H]^+$.

$^1$H NMR (400 MHz, DMSO-$d_6$) δ 13.22 (s, 1H), 9.28 (s, 1H), 8.57 (s, 1H), 8.51 (s, 1H), 8.36 (s, 1H), 8.34 (s, 1H),8.21-7.95 (m, 2H),7.89 (d, *J* = 5.6 Hz, 1H), 7.62 (d, *J* = 8.8Hz, 1H), 7.55 (t, *J* = 7.2 Hz, 1H), 7.37 (t, *J* = 6.8 Hz, 1H), 7.28-7.18 (m, 2H), 2.55 (s, 3H), 2.44 (s, 3H).

**Example 42: 4-amino-N-(1-((3-chloro-2-fluorophenyl)amino)-6-methylisoquinolin-5-yl)-6-fluoroquinazoline-8-carboxamide (compound 46)**

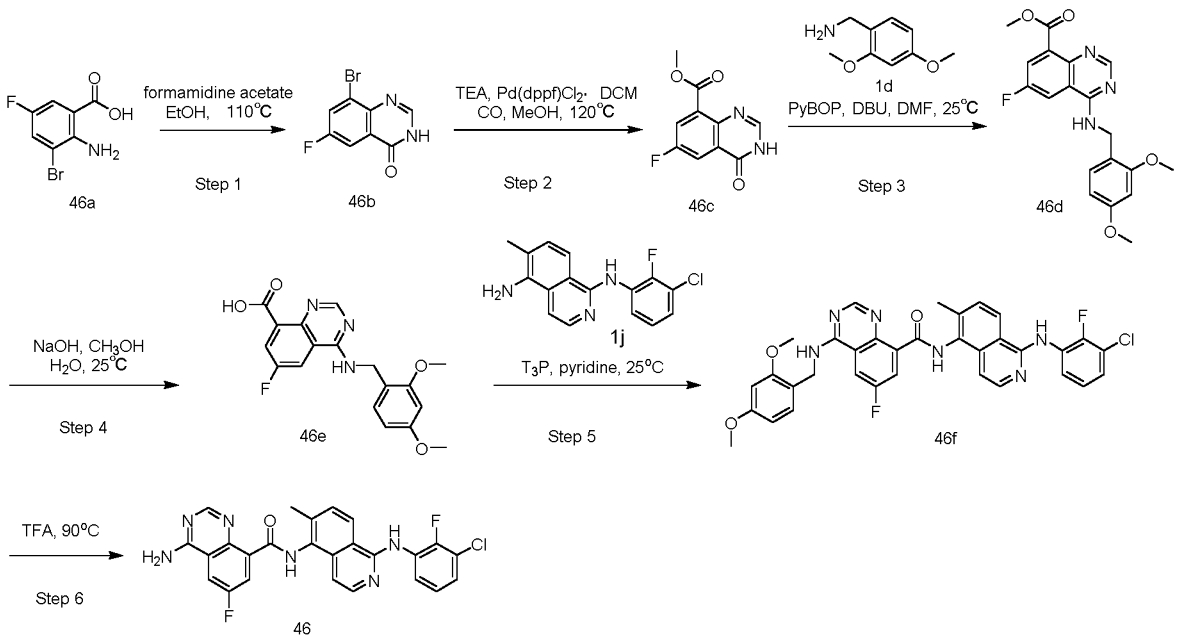


**Step 1: preparation of 8-bromo-6-fluoroquinazolin-4(3H)-one (compound 46b)**

Compound 46a (2 g, 8.55 mmol) and formamidine acetate (3.56 g, 34.18 mmol) were dissolved in anhydrous ethanol (15 mL), the reaction was heated to 110°C and stirred for 16hr. After the reaction was complete, the reaction solution was cooled to room temperature, the precipitated solid was filtered with suction, rinsed with ethanol, and the filter cake was dried to afford compound 46b (1.82 g). MS m/z (ESI): 242.9 $[M+H]^+$.

**Step 2: preparation of methyl 6-fluoro-4-oxo-3,4-dihydroquinazoline-8-carboxylate (compound 46c)**

Compound 46b (1.82 g, 7.49 mmol), Pd(dppf)$Cl_2$·DCM (611.56 mg, 748.87 μmol), methanol (25 mL) and triethylamine (3.79 g, 37.44 mmol, 5.20 mL) were sequentially added to a reaction kettle, purge with nitrogen was performed before CO was pumped in to 1.5 MPa, and then the reaction was heated to 120°C and allowed to proceed for 5hr. After the reaction was complete, the reaction solution was cooled to room temperature, concentrated, filtered with suction, and the filter cake was dried to afford compound 46c (800 mg). MS m/z (ESI): 222.9 $[M+H]^+$.

**Step 3: preparation of methyl 4-((2,4-dimethoxybenzyl)amino)-6-fluoroquinazoline-8-carboxylate (compound 46d)**

Compounds 46c (472 mg, 2.12 mmol), 1d (372.98 mg, 2.23 mmol, 335.12 μL) and DBU (642.09 mg, 2.55 mmol) were added to DMF (10 mL), PyBOP (1.22 g, 2.34 mmol) was then added, and after the addition, the reaction was stirred at 25°C for 16h. After the reaction was complete, the reaction was diluted by adding water, and extracted with ethyl acetate. The organic phase was washed with water, dried over anhydrous sodium sulfate, filtered, concentrated, and then purified through reversed-phase column chromatography on a C18 column (acetonitrile:0.05% formic acid in water=45:55), to afford compound 46d (340 mg). MS m/z (ESI): 372.1 $[M+H]^+$.

**Step 4: preparation of 4-((2,4-dimethoxybenzyl)amino)-6-fluoroquinazoline-8-carboxylic acid (compound 46e)**

Compound 46d (111 mg, 298.90 μmol) was added to water (5 mL) and methanol (5 mL), sodium hydroxide (35.87 mg, 896.70 μmol) was added, and after the addition, the reaction was stirred at 25°C for 16hr. After the reaction was complete, the reaction was evaporated under reduced pressure to remove the organic solvent, diluted by adding water, and adjusted to a pH of about 5 with 2M dilute hydrochloric acid. The precipitated solid was filtered with suction, and dried in vacuum to afford compound 46e (90 mg). MS m/z (ESI): 358.0 $[M+H]^+$.

**Step 5: preparation of N-(1-((3-chloro-2-fluorophenyl)amino)-6-methylisoquinolin-5-yl)-4-((2,4-dimethoxybenzyl)amino)-6-fluoroquinazoline-8-carboxamide (compound 46f)**

Compounds 46e (93 mg, 260.26 μmol), 1j (78.53 mg, 260.26 μmol) were dissolved in pyridine (4 mL), $T_3P$ (2 mL, a 50% solution in EA) was then added, and after the addition, the reaction was stirred at 25°C for 16hr. After the reaction was complete, the solvent was concentrated to dryness, the reaction was quenched by dropwise addition of a saturated solution of sodium bicarbonate, extracted with EA, washed with water and saturated brine, dried over anhydrous sodium sulfate, filtered, and concentrated, to afford compound 46f (100mg). MS m/z (ESI): 640.9 $[M+H]^+$.

**Step 6: preparation of 4-amino-N-(1-((3-chloro-2-fluorophenyl)amino)-6-methylisoquinolin-5-yl)-6-fluoroquinazoline-8-carboxamide (compound 46)**

Compound 46f (100 mg, 155.99 μmol) was dissolved in TFA (4 mL), and stirred at 90°C for 3hr. After the reaction was complete, the reaction solution was concentrated to dryness, adjusted to a pH of about 8 with a saturated solution of sodium bicarbonate, and extracted with EA twice. The organic phase was washed with a saturated saline solution, dried over anhydrous sodium sulfate, filtered, concentrated, and then separated and purified through Prep-HPLC, to afford compound 46 (2.09mg). MS m/z (ESI): 490.9 $[M+H]^+$.

$^1$H NMR (400 MHz, DMSO-$d_6$) δ 13.11 (s, 1H), 9.25 (s, 1H), 8.61 (s, 1H), 8.45-8.37 (m, 2H), 8.37 - 8.29 (m, 3H), 7.91 (d, $J$ = 6.0 Hz, 1H), 7.62 (d, $J$ = 8.8 Hz, 1H), 7.58 - 7.52 (m, 1H), 7.39 - 7.33 (m, 1H), 7.27 - 7.20 (m, 2H), 2.45 (s, 3H).

**Example 43: 4-amino-N-(1-((3-chloro-2-fluorophenyl)amino)-6-methylisoquinolin-5-yl)-6-morpholinoquinazoline-8-carboxamide (compound 53)**

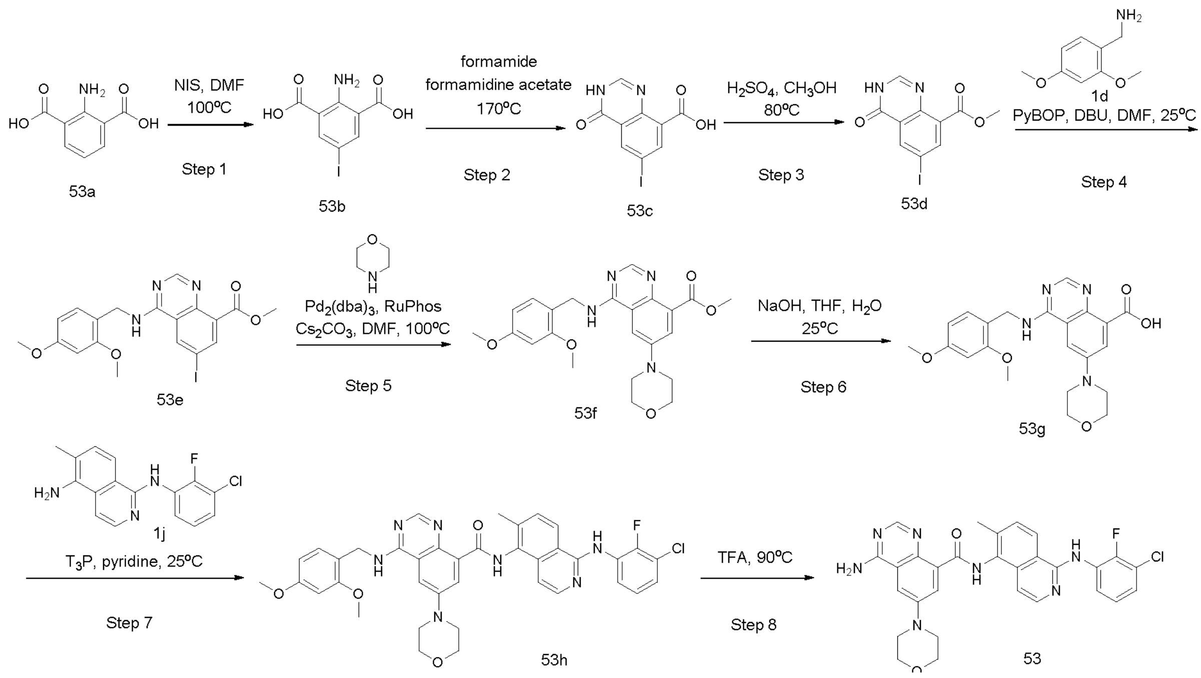


**Step 1: preparation of 2-amino-5-iodoisophthalic acid (compound 53b)**

Compound 53a (5000.00 mg, 27.33 mmol) was dissolved in N,N-dimethylformamide (50 mL), N-iodosuccinimide (6900.00 mg, 30.06 mmol) was added, and the reaction was heated to 100°C and allowed to proceed for 16 hours. After the reaction was complete, the reaction solution was cooled to room temperature, poured into ice water, the precipitated yellow solid was filtered with suction, the filter cake was washed with ice water twice, and dried in vacuum to afford compound 53b (8300 mg). MS m/z (ESI): 307.9 $[M+H]^+$.

**Step 2: preparation of 6-iodo-4-oxo-3,4-dihydroquinazoline-8-carboxylic acid (compound 53c)**

Compound 53b (9000.00 mg, 29.31 mmol) and formamidine acetate (9340.00 mg, 87.94 mmol) were added to single neck flask, and the reaction was vigorously stirred for 10 minutes. Formamide (1600.00 mg, 35.17 mmol) was then added, and the reaction was heated to 170°C and allowed to proceed for 1.5 hours. After the reaction was complete, the reaction solution was cooled to room temperature, continuously stirred after adding ice water, the solid was filtered with suction, the filter cake was rinsed with water, and then dried in vacuum to afford compound 53c (8800 mg). MS m/z (ESI): 317.0 $[M+H]^+$.

**Step 3: preparation of methyl 6-iodo-4-oxo-3,4-dihydroquinazoline-8-carboxylate (compound 53d)**

Compound 53c (8800.00 mg, 27.84 mmol) was added to methanol (100 mL), concentrated sulfuric acid (5570 mg, 55.69 μmol, 3.03 mL) was dropwise added under ice bath cooling, and after the dropwise addition, the reaction solution was heated to 80°C and allowed to proceed for 20 hours. After the reaction was complete, the reaction solution was cooled to 0°C, added with an aqueous solution of sodium bicarbonate for alkalization, diluted by adding water, the precipitated solid was filtered with suction, the filter cake was rinsed with water, and then dried in vacuum to afford compound 53d (9300 mg, purity 85%). MS m/z (ESI): 331.1 $[M+H]^+$.

**Step 4: preparation of methyl 4-((2,4-dimethoxybenzyl)amino)-6-iodoquinazoline-8-carboxylate (compound 53e)**

Compound 53d (9300.00 mg, 23.95 mmol, purity 85%) and PyBOP (13990 mg, 26.34 mmol) were dissolved in N,N-dimethylformamide (100 mL), DBU (6700 mg, 26.34 mmol) was then added at room temperature, and the reaction was stirred for 10 minutes. 2,4-dimethoxybenzylamine (6130 mg, 35.92 mmol) was then added, and after the addition, the reaction was allowed to proceed at 25°C for 1 hour. After the reaction was complete, the reaction solution was added to ice water, the precipitated solid was filtered with suction, the filter cake was rinsed with water, and then dried in vacuum to afford compound 53e (11000 mg). MS m/z (ESI): 480.0 $[M+H]^+$.

**Step 5: preparation of methyl 4-((2,4-dimethoxybenzyl)amino)-6-morpholinoquinazoline-8-carboxylate (compound 53f)**

Compound 53e (220 mg, 459.03 μmol), morpholine (47.99 mg, 550.84 μmol), $Pd_2(dba)_3$ (42.03 mg, 45.90 μmol), RuPhos (42.84 mg, 91.81 μmol) and $Cs_2CO_3$ (448.69 mg, 1.38 mmol) were dissolved in DMF (3 mL), purge with nitrogen was performed for three times, and the reaction was allowed to proceed at 100°C for 5 hours. After the reaction was complete, the reaction solution was cooled under ice-bath cooling, filtered through diatomaceous earth, diluted by adding 100 ml water, and extracted with EA. The organic phase was washed with a saturated saline solution, dried over anhydrous sodium sulfate, filtered and then concentrated, and the crude product was purified on a C18 reversed-phase column (acetonitrile:0.05% formic acid in water=32:68), to afford compound 53f (180 mg). MS m/z (ESI): 439.1 $[M+H]^+$.

**Step 6: preparation of 4-((2,4-dimethoxybenzyl)amino)-6-morpholinoquinazoline-8-carboxylic acid (compound 53g)**

Compound 53f (60 mg, 136.84 μmol) was dissolved in THF (3 mL) and $H_2O$ (1 mL), sodium hydroxide(16.42 mg, 410.51 μmol) was added, and the reaction was allowed to proceed at 25°C for 3 hours. After the reaction was complete, the mixture was purified on a C18 reversed-phase column (acetonitrile:0.05% formic acid in water=31:69), to afford compound 53g (35 mg). MS m/z (ESI): 425.0 $[M+H]^+$.

**Step 7: preparation of N-(1-((3-chloro-2-fluorophenyl)amino)-6-methylisoquinolin-5-yl)-4-((2,4-dimethoxybenzyl)amino)-6-morpholinoquinazoline-8-carboxamide (compound 53h)**

Compounds 53g (30 mg, 70.68 μmol) and 1j (21.33 mg, 70.68 μmol) were dissolved in pyridine (2 mL), 1-propylphosphonic anhydride (0.14 mL, a 50% solution in EA) was dropwise added, and the reaction was allowed to proceed at 25°C for 1 hour. After the reaction was complete, the solvent was removed under reduced pressure, the residue was added with a saturated aqueous solution of sodium bicarbonate (5 mL), and extracted with ethyl acetate. The organic phase was dried over anhydrous sodium sulfate, filtered, concentrated, and purified by Flash column chromatography on silica gel (DCM/MeOH=20/1), to afford compound 53h (30.00 mg). MS m/z (ESI): 707.9 $[M+H]^+$.

**Step 8: preparation of 4-amino-N-(1-((3-chloro-2-fluorophenyl)amino)-6-methylisoquinolin-5-yl)-6-morpholinoquinazoline-8-carboxamide (compound 53)**

Compound 53h (30.00 mg, 42.36 μmol) was dissolved in trifluoroacetic acid (2 mL), and the reaction was warmed to 90°C and allowed to proceed for 3 hours. After the reaction was complete, the solvent was removed under reduced pressure, and then the residue was purified through reversed-phase column chromatography on a C18 column (acetonitrile:0.05% formic acid in water=35:65), to afford compound 53 (18.00 mg). MS m/z (ESI): 558.1 $[M+H]^+$.

$^1H$ NMR (400 MHz, DMSO-$d_6$) δ 13.35 (s, 1H), 9.23 (s, 1H), 8.45 (s, 1H), 8.41 (d, *J* = 2.8 Hz, 1H), 8.33 (d, *J* = 8.8 Hz, 1H), 8.22-7.93 (m, 2H), 7.90 (d, *J* = 6.0 Hz, 1H), 7.78 (d, *J* = 2.8 Hz, 1H), 7.62 (d, *J* = 8.8 Hz, 1H), 7.58-7.52 (m, 1H), 7.38-7.33 (m, 1H), 7.28 - 7.19 (m, 2H), 3.84-3.80 (m, 4H), 3.33-3.28 (m, 4H), 2.44 (s, 3H).

**Example 44: 4-amino-N-(1-((3-chloro-2-fluorophenyl)amino)-6-methylisoquinolin-5-yl)-6-(4-methylpiperazin-1-yl)quinazoline-8-carboxamide (compound 54)**

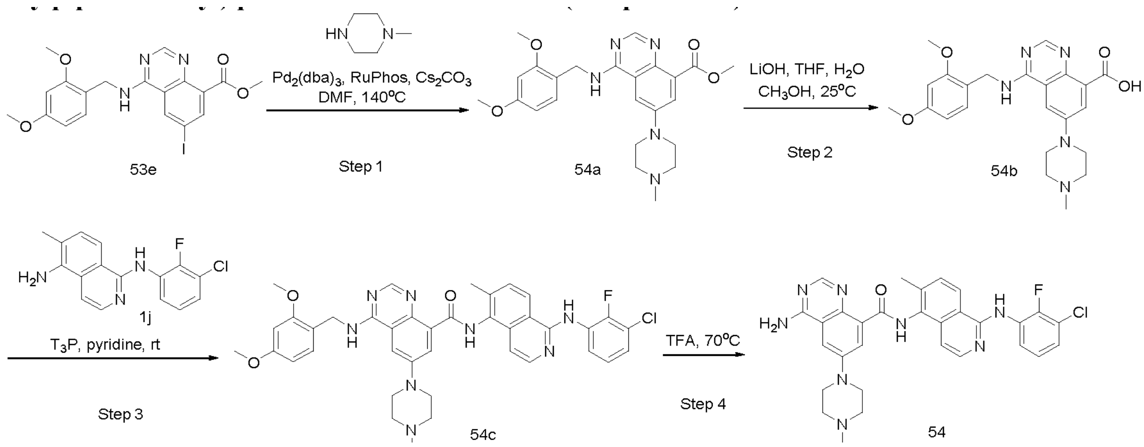


**Step 1: preparation of methyl 4-((2,4-dimethoxybenzyl)amino)-6-(4-methylpiperazin-1-yl)quinazoline-8-carboxylate (compound 54a)**

Compound 53e (250.00 mg, 521.63 μmol) and N-methylpiperazine (156.74 mg, 1.56 mmol) were dissolved in N,N-dimethylformamide (15 mL), tris(dibenzylideneacetone) dipalladium (23.89 mg, 26.09 μmol), 2-dicyclohexylphosphino-2',6'-dilsopropoxy-1,1'-biphenyl (24.34 mg, 52.16 μmol) and cesium carbonate (339.91 mg, 1.04 mmol) were then added, the reaction was warmed to 140°C under the protection of nitrogen and allowed to proceed for 2 hours. After the reaction was complete, the reaction solution was cooled to room temperature, diluted with ethyl acetate, and washed with water for 3 times. The organic phase was dried over anhydrous sodium sulfate, filtered, concentrated, and the crude product was purified by Flash column chromatography on silica gel (dichloromethane /methanol =19/1), to afford compound 54a (43 mg). MS m/z (ESI): 452.0 $[M+H]^+$.

**Step 2: preparation of 4-((2,4-dimethoxybenzyl)amino)-6-(4-methylpiperazin-1-yl)quinazoline-8-carboxylic acid (compound 54b)**

Compound 54a (43 mg, 95.23 μmol) was dissolved in a mixed solvent of THF (3 mL), MeOH (1 mL) and water (1 mL), lithium hydroxide monohydrate (5.99 mg, 142.85 μmol) was added, and after the addition, the reaction was allowed to proceed at 25°C for 16hr. After the reaction was complete, the reaction solution was concentrated to dryness, to afford compound 54b (41 mg). MS m/z (ESI): 438.0 $[M+H]^+$.

**Step 3: preparation of N-(1-((3-chloro-2-fluorophenyl)amino)-6-methylisoquinolin-5-yl)-4-((2,4-dimethoxybenzyl)amino)-6-(4-methylpiperazin-1-yl)quinazoline-8-carboxamide (compound 54c)**

Compound 54c (25 mg) was prepared according to the method for preparing compound 1k in Step 7 of Example 1, except that compound 1f was replaced with compound 54b. MS m/z (ESI): 721.0 $[M+H]^+$.

**Step 4: preparation of 4-amino-N-(1-((3-chloro-2-fluorophenyl)amino)-6-methylisoquinolin-5-yl)-6-(4-methylpiperazin-1-yl)quinazoline-8-carboxamide (compound 54)**

Compound 54 (17 mg) was prepared according to the method for preparing compound 1 in Step 8 of Example 1, except that compound 1k was replaced with compound 54c. MS m/z (ESI): 571.0 $[M+H]^+$.

$^1$H NMR (400 MHz, DMSO-$d_6$) δ 13.36 (s, 1H), 9.23 (s, 1H), 8.43 (s, 1H), 8.40 (d, $J$ = 2.4 Hz, 1H), 8.33 (d, $J$ = 8.4 Hz, 1H), 8.04 (brs, 2H), 7.90 (d, $J$ = 6.0 Hz, 1H), 7.76 (d, $J$ = 2.4 Hz, 1H), 7.62 (d, $J$ = 8.8 Hz, 1H), 7.55 (t, $J$ = 7.2 Hz, 1H), 7.36 (t, $J$ = 6.4 Hz, 1H), 7.24-7.20 (m, 2H), 2.55-2.50 (m, 8H), 2.44 (s, 3H), 2.26 (s, 3H).

**Example 45: 4-amino-N-(1-((4-chloro-2-fluorophenyl)amino)-6-methylisoquinolin-5-yl)imidazo[2,1-f][1,2,4]triazine-7-carboxamide (compound 55)**

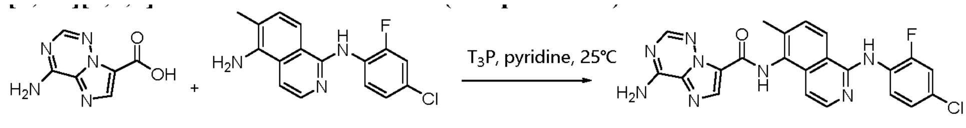


30c 41d 55

Compounds 41d (32.9 mg, 108.9 μmol) and 30c (15 mg, 83.7 μmol) were dissolved in pyridine (2.0 mL), $T_3P$ (3 mL, 50% in DMF) was dropwise added, and after the addition, the reaction was allowed to proceed at 25°C for 14 hours. After the reaction was complete, the solvent was removed by concentration under reduced pressure, the reaction solution was diluted with ethyl acetate, washed with a saturated aqueous solution of sodium bicarbonate for 3 times, the organic phase was dried over anhydrous sodium sulfate, filtered and then concentrated, and the crude product was separated and purified by Prep-HPLC, to afford a trifluoroacetate salt of compound 55 (3.3 mg). MS m/z (ESI): 463.1 $[M+H]^+$.

$^1H$ NMR (400 MHz, DMSO-$d_6$) δ 10.42 (s, 1H), 8.70 (s, 1H), 8.63 (s, 1H), 8.47 (d, *J* = 8.7Hz, 1H), 8.37 (s, 1H), 8.24 (s, 1H), 7.80 - 7.59 (m, 4H), 7.39 (d, *J* = 8.6Hz, 1H), 7.26 (d, *J* = 6.4Hz, 1H), 2.46 (s, 3H).

**Example 46: 4-amino-N-(1-((3-chloro-4-methoxyphenyl)amino)-6-methylisoquinolin-5-yl)imidazo[2,1-f][1,2,4]triazine-7-carboxamide (compound 56)**

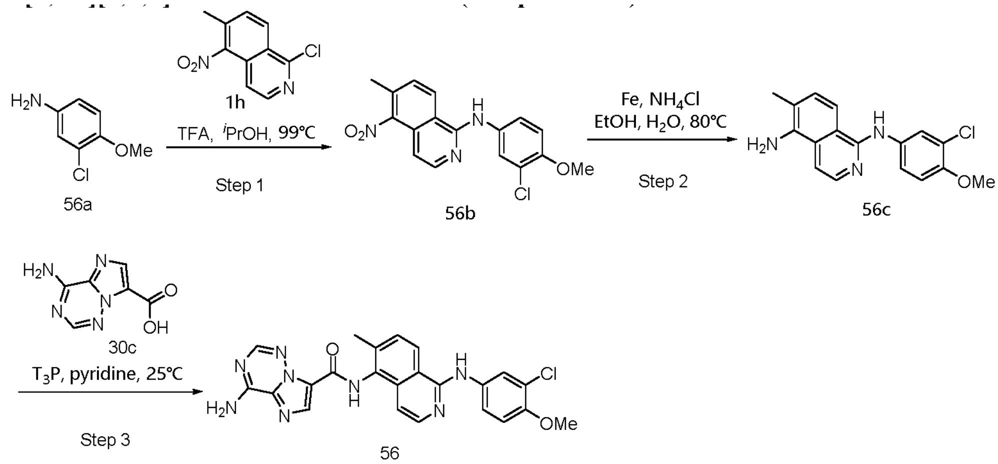


1h 56a 56b 56c 30c 56

**Step 1: preparation of N-(3-chloro-4-methoxyphenyl)-6-methyl-5-nitroisoquinolin-1-amine (compound 56b)**

Compounds 56a (128.7 mg, 800.4 μmol) and 1h (150.0 mg, 667.0 μmol) were dissolved in isopropanol (5 mL), followed by addition of TFA (93.1 mg, 800.4 μmol), the reaction was warmed to 99°C and allowed to proceed for 18 hours. After the reaction was complete, the mixture was directly concentrated, to afford compound 56b (200.0 mg). MS m/z (ESI): 344.1 $[M+H]^+$.

**Step 2: preparation of $N^1$-(3-chloro-4-methoxyphenyl)-6-methylisoquinoline-1,5-diamine (compound 56c)**

Compound 56b (200.0 mg, 581.8 μmol), reduced iron powder (82.1 mg, 1.45 mmol) and ammonium chloride (157.2 mg, 2.91 mmol) were added to a mixed solution of ethanol (20.0 mL) and water (5.0 mL), purge with nitrogen was performed for 3 times, and the reaction was warmed to 80°C and allowed to proceed for 2 hours. After the reaction was complete, the mixture was filtered with suction to remove the solid, the filtrate was concentrated, and the crude product was separated and purified by column chromatography on silica gel (DCM/MeOH=19/1), to afford compound 56c (150.0 mg). MS m/z (ESI): 314.0 $[M+H]^+$.

**Step 3: preparation of 4-amino-N-(1-((3-chloro-4-methoxyphenyl)amino)-6-methylisoquinolin-5-yl)imidazo[2,1-f][1,2,4]triazine-7-carboxamide (compound 56)**

Compounds 56c (26.3 mg, 83.7 μmol) and 30c (15.0 mg, 83.7 μmol) were dissolved in pyridine (5.0 mL), $T_3P$ (159.9 mg, 251.2 μmol) was then added, and after the addition, the reaction was allowed to proceed at 25°C for 16

hours. After the reaction was complete, the reaction was concentrated to remove pyridine, separated and purified through Prep-HPLC to afford compound 56 (15.0 mg). MS m/z (ESI): 475.1 [M+H]+.

$^1$H NMR (400 MHz, DMSO-$d_6$) δ 10.37 (s, 1H), 9.20 (s, 1H), 8.70 (s, 1H), 8.62 (s, 1H), 8.45 (d, *J* = 8.4 Hz, 1H), 8.38 (s, 1H), 8.24 (s, 1H), 8.08 (d, *J* = 2.4 Hz, 1H), 7.98 (d, *J* = 6.0 Hz, 1H), 7.77 (dd, *J* = 9.2, 2.8 Hz, 1H), 7.61 (d, *J* = 8.8 Hz, 1H), 7.18 - 7.12 (m, 2H), 3.84 (s, 3H), 2.43 (s, 3H).

**Example 47: 4-amino-N-(1-((4-methoxyphenyl)amino)-6-methylisoquinolin-5-yl)quinazoline-8-carboxamide (compound 57)**

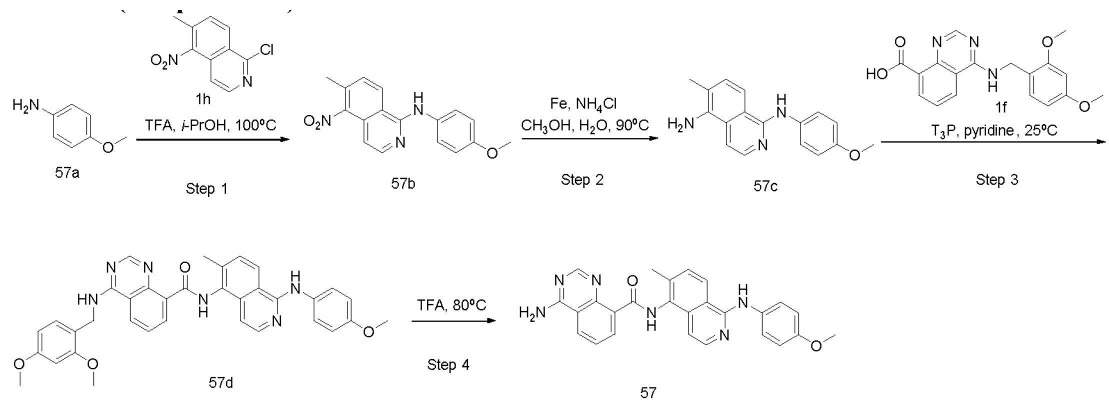


**Step 1: preparation of N-(4-methoxyphenyl)-6-methyl-5-nitroisoquinolin-1-amine (compound 57b)**

Compounds 57a (132.76 mg, 1.08 mmol) and 1h (200 mg, 898.36 μmol) were dissolved in isopropanol (5 mL), TFA (122.92 mg, 1.08 mmol, 80.08 μL) was added, and then the reaction was heated to 100°C and stirred for 18hr. After the reaction was complete, the reaction solution was cooled to room temperature, the solid was filtered with suction, washed with isopropanol, dried under reduced pressure to afford compound 57b (270 mg). MS m/z (ESI): 310.1 [M+H]+.

**Step 2: preparation of N$^1$-(4-methoxyphenyl)-6-methylisoquinoline-1,5-diamine (compound 57c)**

57b (220 mg, 711.24 μmol), iron powder (198.61 mg, 3.56 mmol) and $NH_4Cl$ (114.13 mg, 2.13 mmol) were added to methanol (12 mL) and water (3 mL), and stirred at 90°C for 6hr. After the reaction was complete, the reaction solution was concentrated to dryness, and purified by Flash column chromatography on silica gel (DCM/MeOH=85/15), to afford compound 57c (150 mg). MS m/z (ESI): 280.1 [M+H]+.

**Step 3: preparation of 4-((2,4-dimethoxybenzyl)amino)-N-(1-((4-methoxyphenyl)amino)-6-methylisoquinolin-5-yl)quinazoline-8-carboxamide (compound 57d)**

Compounds 57c (24.69 mg, 88.41 μmol) and 1f (30 mg, 88.41 μmol) were dissolved in pyridine (2 mL), $T_3P$ (0.5 mL, 50% in DMF) was then added, and after the addition, the reaction was stirred at 25°C for 16hr. After the reaction was complete, the reaction was evaporated under reduced pressure to remove pyridine, the reaction was quenched by dropwise addition of a saturated solution of sodium bicarbonate, and extracted with EA. The organic phase was washed with a saturated saline solution, dried over anhydrous sodium sulfate, filtered, and concentrated, to afford compound 57d (30 mg). MS m/z (ESI): 601.0 [M+H]+.

**Step 4: preparation of 4-amino-N-(1-((4-methoxyphenyl)amino)-6-methylisoquinolin-5-yl)quinazoline-8-carboxamide (compound 57)**

Compound 57d (30 mg, 49.94 μmol) was dissolved in TFA (4 mL), and stirred at 80°C for 3hr. After the reaction was complete, the reaction solution was concentrated to dryness, adjusted to a pH of about 8 by adding a saturated solution of sodium bicarbonate, and extracted with EA. The organic phase was washed with a saturated saline solution, dried over anhydrous sodium sulfate, filtered, concentrated, and then separated and purified through Prep-HPLC, to afford compound 57 (6 mg). MS m/z (ESI): 451.1 [M+H]+.

$^1$H NMR (400 MHz, DMSO-$d_6$) δ 13.17 (s, 1H), 9.07 (s, 1H), 8.66 (dd, *J* = 7.6, 1.2 Hz, 1H), 8.62 (s, 1H), 8.52

(dd, $J$ = 8.4, 1.2 Hz, 1H), 8.40 (d, $J$ = 8.8 Hz, 1H), 8.38 (br, 1H), 8.23 (br, 1H), 7.91 (d, $J$ = 6.0 Hz, 1H), 7.77 - 7.72 (m, 2H), 7.70 (t, $J$ = 7.6Hz, 1H), 7.57 (d, $J$ = 8.8 Hz, 1H), 7.16 (d, $J$ = 6.0 Hz, 1H), 6.94 - 6.90 (m, 2H), 3.75 (s, 3H), 2.42 (s, 3H).

**Example 48: 4-amino-N-(1-((3-chloro-4-fluorophenyl)amino)-6-methylisoquinolin-5-yl)imidazo[2,1-f][1,2,4]triazine-7-carboxamide (compound 58)**

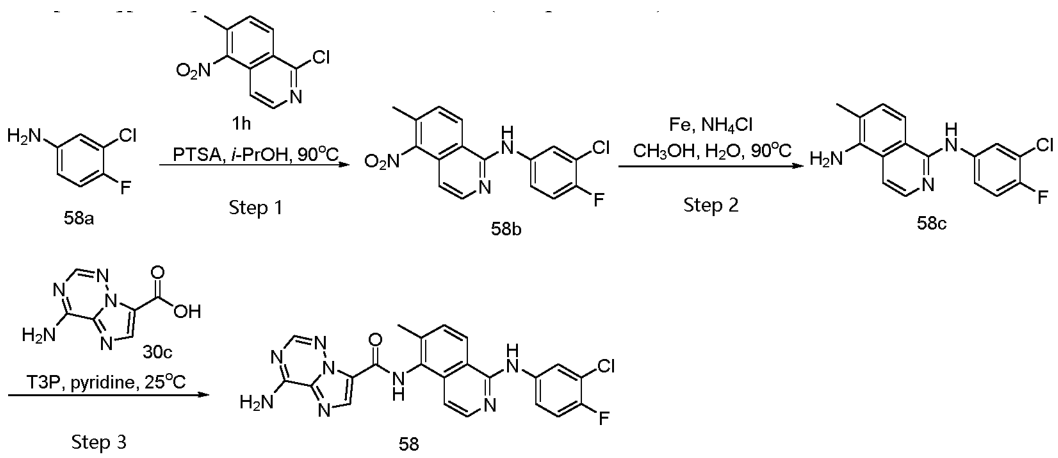


**Step 1: preparation of N-(3-chloro-4-fluorophenyl)-6-methyl-5-nitroisoquinolin-1-amine (compound 58b)**

Compounds 1h (400.00 mg, 1.80 mmol), 58a (313.84 mg, 2.16 mmol) and p-toluenesulfonic acid (30.94 mg, 179.67 μmol) were dissolved in isopropanol (20 mL), the reaction was heated to 90°C and allowed to proceed for 18 hours. After the reaction was complete, the reaction solution was cooled to room temperature, the precipitated solid was filtered with suction, the filter cake was rinsed with methyl *tert*-butyl ether, and dried to afford compound 58b (550 mg). MS m/z (ESI): 332.0 $[M+H]^+$.

**Step 2: preparation of $N^1$-(3-chloro-4-fluorophenyl)-6-methylisoquinoline-1,5-diamine (compound 58c)**

Compound 58c (300 mg) was prepared according to the method for preparing compound 57c in Step 2 of Example 47, except that compound 57b was replaced with compound 58b. MS m/z (ESI): 301.9 $[M+H]^+$.

**Step 3: preparation of 4-amino-N-(1-((3-chloro-4-fluorophenyl)amino)-6-methylisoquinolin-5-yl)imidazo[2,1-f][1,2,4]triazine-7-carboxamide (compound 58)**

Compound 58 (104 mg) was prepared according to the method for preparing compound 56 in Step 3 of Example 46, except that compound 56c was replaced with compound 58c. MS m/z (ESI): 462.9 $[M+H]^+$.

$^1$H NMR (400 MHz, DMSO-$d_6$) δ 11.58 (s, 1H), 10.51 (s, 1H), 8.82 (d, $J$ = 8.8 Hz, 1H), 8.74 (s, 1H), 8.67 (s, 1H), 8.37 (s, 1H), 8.27 (s, 1H), 7.98 (d, $J$ = 5.6 Hz, 1H), 7.88 (d, $J$ = 8.4 Hz, 1H), 7.65 (d, $J$ = 6.8, 2H), 7.61 (d, $J$ = 7.2, 1H), 7.33 (d, $J$ = 6.8 Hz, 1H), 2.51(s, 3H).

**Example 49: 4-amino-N-(1-((6-(dimethylamino)pyridin-3-yl)amino)-6-methylisoquinolin-5-yl)quinazoline-8-carboxamide (compound 59)**

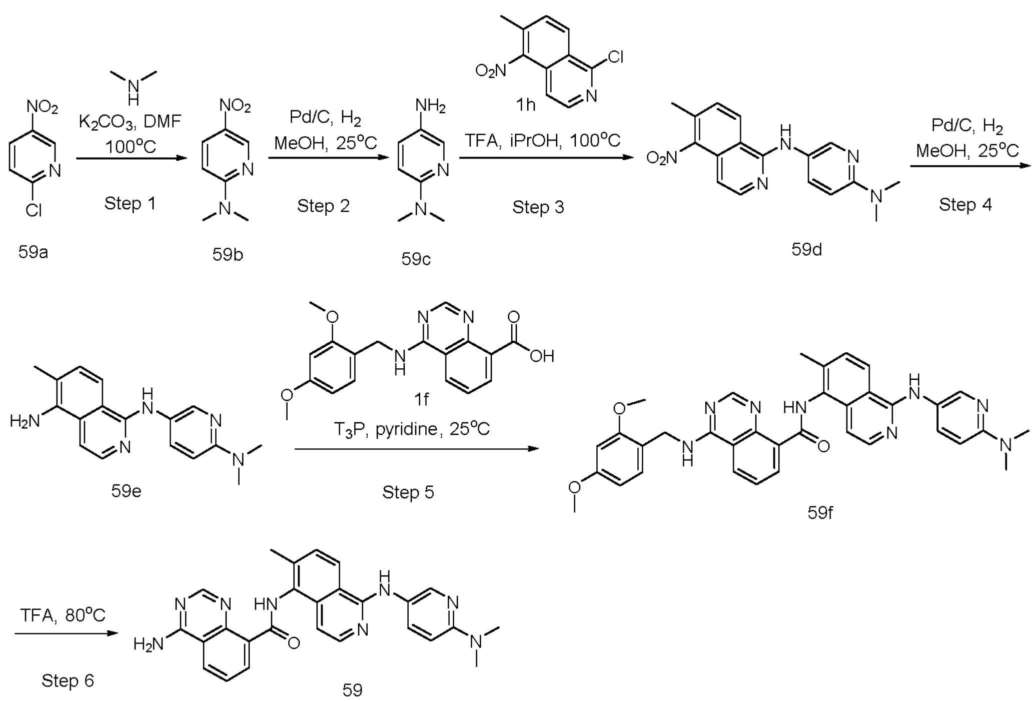


**Step 1: preparation of N,N-dimethyl-5-nitropyridin-2-amine (compound 59b)**

Compound 59a (720 mg, 4.54 mmol) and dimethylamine (555.49 mg, 6.81 mmol) were dissolved in DMF (3 mL), potassium carbonate (2.51 g, 18.17 mmol) was added, and the reaction was allowed to proceed at 100°C for 12 hours. After the reaction was complete, the reaction solution was added with water, the precipitated solid was filtered and collected, to afford compound 59b (530 mg). MS m/z (ESI): 168.1 $[M+H]^+$.

**Step 2: preparation of $N^2$,$N^2$-dimethylpyridine-2,5-diamine (compound 59c)**

Compound 59b (600 mg, 3.59 mmol) was dissolved in MeOH (5 mL), palladium on carbon (60 mg, 10% mass content) was added, purge with nitrogen was performed for three times, and then purge with hydrogen was performed for three times, the reaction was allowed to proceed at room temperature under hydrogen for 3 hours. After the reaction was complete, the reaction was filtered through diatomaceous earth, the filtrate was concentrated to dryness, to afford compound 59c (466 mg). MS m/z (ESI): 138.2 $[M+H]^+$.

**Step 3: preparation of $N^2$,$N^2$-dimethyl-$N^5$-(6-methyl-5-nitroisoquinolin-1-yl)pyridine-2,5-diamine (compound 59d)**

Compounds 59c (400 mg, 2.92 mmol) and 1h (540.95 mg, 2.43 mmol) were dissolved in isopropanol (15 mL), TFA (332.46 mg, 2.92 mmol) was added, and the reaction was allowed to proceed at 100°C for 12 hours. After the reaction was complete, the solvent was concentrated to dryness, the crude product was dissolved by adding DMF, followed by dropwise addition of 100 ml water, the precipitated solid was filtered and collected, to afford compound 59d (732 mg). MS m/z (ESI): 324.2 $[M+H]^+$.

**Step 4: preparation of $N^1$-(6-(dimethylamino)pyridin-3-yl)-6-methylisoquinoline-1,5-diamine (compound 59e)**

Compound 59d (600 mg, 1.86 mmol) was dissolved in MeOH (5 mL), palladium on carbon (60 mg, 10% mass content) was added, purge with nitrogen was performed for three times, and then purge with hydrogen was performed for three times. The reaction was allowed to proceed at room temperature under hydrogen for 12 hours. After the reaction was complete, the reaction was filtered through diatomaceous earth, the filtrate was concentrated to dryness, to afford compound 59e (365.00 mg). MS m/z (ESI): 294.1 $[M+H]^+$.

**Step 5: preparation of 4-((2,4-dimethoxybenzyl)amino)-N-(1-((6-(dimethylamino)pyridin-3-yl)amino)-6-methylisoquinolin-5-yl)quinazoline-8-carboxamide (compound 59f)**

Compounds 59e (31.12 mg, 106.09 μmol) and 1f (30 mg, 88.41 μmol) were dissolved in pyridine (3 mL), 1-propylphosphonic anhydride (0.17 mL, a 50% solution in EA) was dropwise added, and the reaction was allowed to proceed at 25°C for 1 hour. After the reaction was complete, the solvent was removed under reduced pressure, the reaction was quenched by adding a saturated aqueous solution of sodium bicarbonate, and extracted with ethyl acetate. The organic phase was dried over anhydrous sodium sulfate, filtered, concentrated, and the crude product was purified by silica gel column chromatography (DCM/MeOH=10/1), to afford compound 59f (25.00 mg). MS m/z (ESI): 615.2 $[M+H]^+$.

**Step 6: preparation of 4-amino-N-(1-((6-(dimethylamino)pyridin-3-yl)amino)-6-methylisoquinolin-5-yl)quinazoline-8-carboxamide (compound 59)**

Compound 59f (24.00 mg, 39.04 μmol) was dissolved in trifluoroacetic acid (3 mL), and the reaction was warmed to 80°C and allowed to proceed for 2 hours. After the reaction was complete, the solvent was removed under reduced pressure, and then the residue was purified through Prep-HPLC, to afford compound 59 (13.00 mg). MS m/z (ESI): 465.2 $[M+H]^+$.

$^1H$ NMR (400 MHz, DMSO-$d_6$) δ 13.17 (s, 1H), 9.01 (s, 1H), 8.66 (dd, *J* = 7.6, 1.6 Hz, 1H), 8.62 (s, 1H), 8.52 (dd, *J* = 8.4, 1.6 Hz, 1H), 8.46-8.34 (m, 3H), 8.23 (s, 1H), 7.89 (dd, *J* = 8.8, 2.4 Hz, 1H), 7.86 (d, *J* = 6.0 Hz, 1H), 7.69 (t, *J* = 8 Hz, 1H), 7.57 (d, *J* = 8.8 Hz, 1H), 7.13 (d, *J* = 6.0 Hz, 1H), 6.68 (d, *J* = 8.8 Hz, 1H), 3.02 (s, 6H), 2.42 (s, 3H).

**Example 50: 4-amino-N-(1-((2-fluoro-4-methoxyphenyl)amino)-6-methylisoquinolin-5-yl)quinazoline-8-carboxamide (compound 60)**

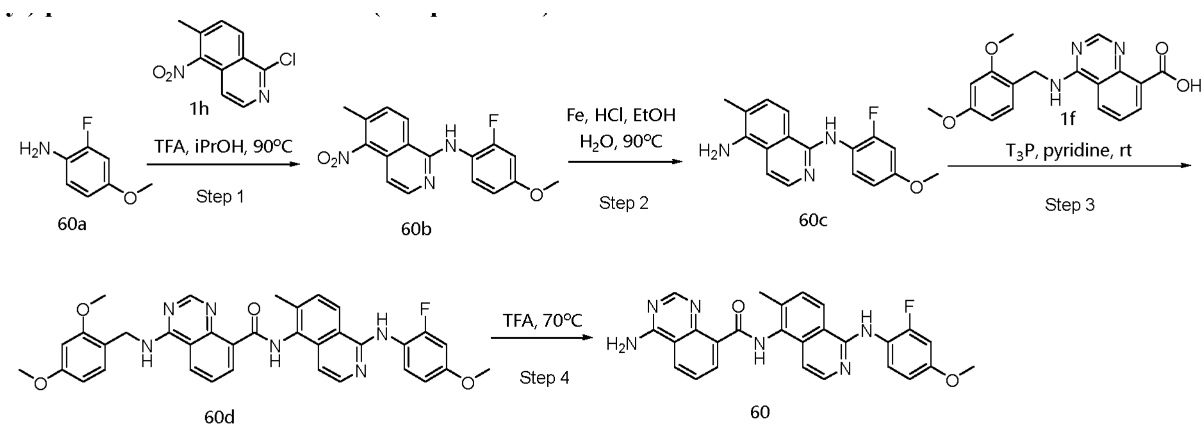

60a 60b 60c 60d 60

**Step 1: preparation of N-(2-fluoro-4-methoxyphenyl)-6-methyl-5-nitroisoquinolin-1-amine (compound 60b)**

Compounds 1h (150 mg, 673.77 μmol) and 60a (114 mg, 808.53 μmol) were added to isopropanol (6 mL), TFA (92 mg, 808.53 μmol) was dropwise added, and after the dropwise addition, the reaction was warmed to 90°C and allowed to proceed for 18hr. After the reaction was complete, the reaction solution was cooled to room temperature, the precipitated solid was filtered with suction, the filter cake was rinsed with isopropanol, and dried under reduced pressure to afford compound 60b (200 mg). ESI-MS (m/z): 328.1 $[M+H]^+$.

**Step 2: preparation of $N^1$-(2-fluoro-4-methoxyphenyl)-6-methylisoquinoline-1,5-diamine (compound 60c)**

60b (200 mg, 611.04 μmol), ethanol (8 mL) and water (1 mL) were added to a reaction flask, iron powder (170 mg, 3.06 mmol) and concentrated hydrochloric acid (12 M, 763.80 μL) were then added, and the reaction was stirred at 90°C for 2hr. After the reaction was complete, the reaction solution was concentrated, diluted by adding water, and then extracted with EA for three times. The organic phase was washed with a saturated saline solution, dried over anhydrous sodium sulfate, filtered, and concentrated, to afford compound 60c (190 mg). ESI-MS (m/z): 298.1 $[M+H]^+$.

**Step 3: preparation of 4-((2,4-dimethoxybenzyl)amino)-N-(1-((2-fluoro-4-methoxyphenyl)amino)-6-methylisoquinolin-5-yl)quinazoline-8-carboxamide (compound 60d)**

Compounds 1f (25 mg, 73.67 μmol), 60c (22 mg, 73.67 μmol) and pyridine (3 mL) were added to a reaction flask, and dissolved with stirring before addition of $T_3P$ (2 mL, a 50% solution in EA), and the reaction was allowed to proceed under the protection of nitrogen at room temperature overnight. After the reaction was complete, the reaction solution was rotary evaporated to dryness, diluted by adding water, adjusted to a pH of 7-8 with a saturated solution of $NaHCO_3$, and then extracted with EA. The organic phase was washed with a saturated saline solution, dried over anhydrous sodium sulfate, filtered, concentrated, the crude product was purified by Flash column chromatography on silica gel (DCM:MeOH=97:3), to afford compound 60c (30 mg). MS m/z (ESI): 619.3 $[M+H]^+$.

**Step 4: preparation of 4-amino-N-(1-((2-fluoro-4-methoxyphenyl)amino)-6-methylisoquinolin-5-yl)quinazoline-8-carboxamide (compound 60)**

Compound 60d (30 mg, 48.49 μmol) was dissolved in TFA (3 mL), and the reaction was allowed to proceed at 70°C under the protection of nitrogen for 2hr. After the reaction was complete, TFA was rotary evaporated to dryness, the residue was diluted by adding water, adjusted to a pH of 7-8 with a saturated solution of $NaHCO_3$, and then extracted with EA. The organic phase was washed with a saturated saline solution, dried over anhydrous sodium sulfate, concentrated under reduced pressure to afford a crude product, the crude product was purified by Prep-HPLC, to afford compound 60 (6 mg). MS m/z (ESI): 469.1 $[M+H]^+$.

$^1H$ NMR (400 MHz, DMSO-$d_6$) δ 13.17 (s, 1H), 8.91 (s, 1H), 8.65 (dd, *J* = 7.6, 1.6 Hz, 1H), 8.62 (s, 1H), 8.52 (dd, *J* = 8.0, 1.6 Hz, 1H), 8.45 - 8.30 (m, 2H), 8.23 (br, 1H), 7.80 (d, *J* = 6.0 Hz, 1H), 7.69 (t, *J* = 8.0 Hz, 1H), 7.57 (d, *J* = 8.8 Hz, 1H), 7.39 (t, *J* = 8.8 Hz, 1H), 7.12 (d, *J* = 6.0 Hz, 1H), 6.91 (dd, *J* = 12.4, 2.8 Hz, 1H), 6.83 - 6.78 (m, 1H), 3.79 (s, 3H), 2.43 (s, 3H).

**Example 51: preparation of 4-amino-N-(1-((2-fluoro-4-methoxyphenyl)amino)-6-methylisoquinolin-5-yl)imidazo[2,1-f][1,2,4]triazine-7-carboxamide (compound 61)**

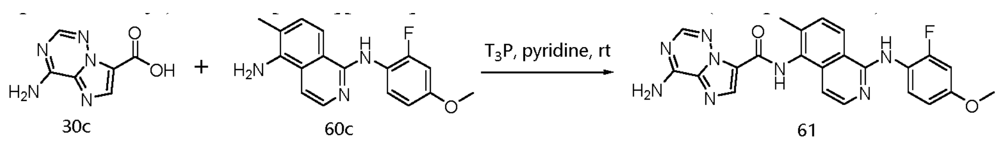


30c 60c 61

Compounds 30c (25 mg, 111.65 μmol), 60c (33 mg, 111.65 μmol) and pyridine (2 mL) were added to a reaction flask, and dissolved with stirring before addition of $T_3P$ (1.5 mL, a 50% solution in EA), and the reaction was allowed to proceed under the protection of nitrogen at room temperature overnight. After the reaction was complete, the reaction solution was concentrated to dryness, diluted by adding water, adjusted to a pH of 7-8 with a saturated solution of $NaHCO_3$, and then extracted with EA. The organic phase was washed with a saturated saline solution, dried over anhydrous sodium sulfate, filtered, concentrated, and the crude product was purified by Prep-HPLC, to afford compound 61 (20 mg). MS m/z (ESI): 459.1 $[M+H]^+$.

$^1H$ NMR (400 MHz, DMSO-$d_6$) δ 10.35 (s, 1H), 8.93 (s, 1H), 8.64 (d, *J* = 32.8 Hz, 2H), 8.39 - 8.34 (m, 2H), 8.24 (s, 1H), 7.81 (d, *J* = 6.0 Hz, 1H), 7.57 (d, *J* = 8.4 Hz, 1H), 7.38 (t, *J* = 9.2 Hz, 1H), 7.09 - 7.05 (m, 1H), 6.91 (dd, *J* = 12.4, 2.8 Hz, 1H), 6.83 - 6.78 (m, 1H), 3.79 (s, 3H), 2.42 (s, 3H).

**Example 52: 4-amino-N-(1-((4-((dimethylamino)methyl)-3-(trifluoromethyl)phenyl)amino)-6-methylisoquinolin-5-yl)imidazo[2,1-f][1,2,4]triazine-7-carboxamide (compound 62)**

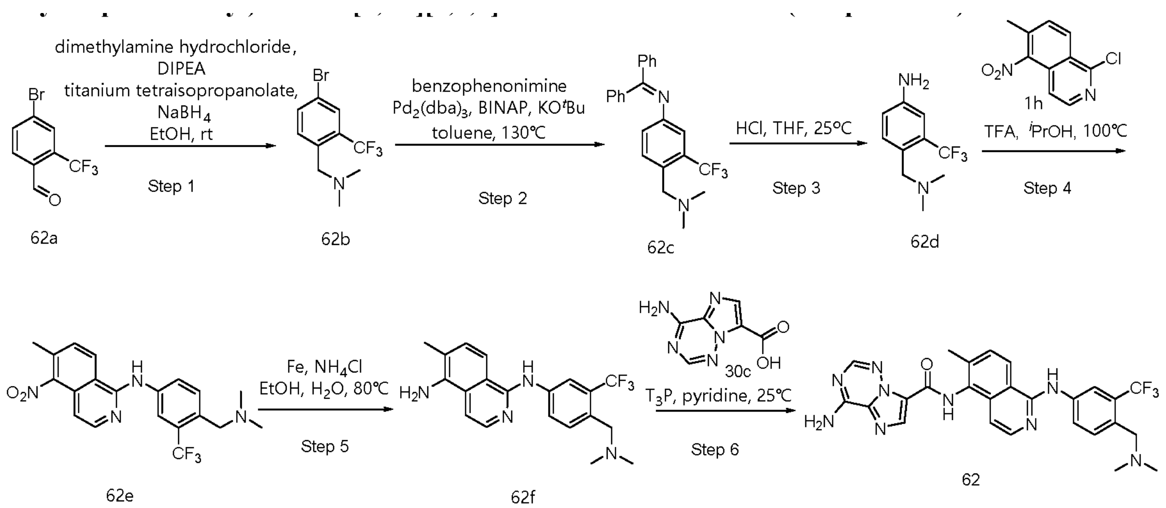


**Step 1: 1-(4-bromo-2-(trifluoromethyl)phenyl)-N,N-dimethylmethanamine (compound 62b)**

DIPEA (521.2 mg, 3.95 mmol), dimethylamine hydrochloride (322.3 mg, 3.95 mmol) and titanium tetraisopropanolate (1.13g, 3.95 mmol) were added to ethanol (10.0 mL) and thoroughly stirred, compound 62a (500 mg, 1.98 mmol) was then added, and the reaction was stirred at 25°C for 16 hours. $NaBH_4$ (114.4 mg, 1.96 mmol) was then added, and the reaction was continuously stirred for 1 hour. After the reaction was complete, the reaction was quenched by adding 2 ml water, directly concentrated, separated and purified through silica gel column chromatography (DCM/MeOH=19/1), to afford compound 62b (420.0 mg). MS m/z (ESI): 282.0 $[M+H]^+$.

**Step 2: preparation of 1-(4-((diphenylmethylene)amino)-2-(trifluoromethyl)phenyl-N,N-dimethylmethanamine (compound 62c)**

Compound 62b (320.0 mg, 2.30 mmol), benzophenonimine (1.05 g, 5.67 mmol), $Pd_2(dba)_3$ (106.0 mg, 113.4 μmol), BINAP (142.7 mg, 226.9 μmol) and KO$^t$Bu (642.9 mg, 5.67 mmol) were added to toluene (15.0 mL), purge with nitrogen was performed for three times, and the reaction was allowed to proceed at 130°C for 5 hours. After the reaction was complete, the mixture was directly concentrated, separated and purified by column chromatography on silica gel (PE/EA=4/1), to afford compound 62c (182 mg). MS m/z (ESI): 383.2 $[M+H]^+$.

**Step 3: preparation of 4-((dimethylamino)methyl)-3-(trifluoromethyl)aniline (compound 62d)**

Compound 62c (227.0 mg, 593.6 μmol) was dissolved in THF (5.0 mL), HCl (1 mL, 2.0 mol/L aqueous solution) was then added, and the reaction was allowed to proceed at 25°C for 2 hours. After the reaction was complete, the mixture was directly concentrated, separated and purified by column chromatography on silica gel (DCM/MeOH=19/1), to afford compound 62d (93 mg). MS m/z (ESI): 219.1 $[M+H]^+$.

**Step 4: preparation of N-(4-((dimethylamino)methyl)-3-(trifluoromethyl)phenyl)-6-methyl-5-nitroisoquinolin-1-amine (compound 62e)**

Compounds 62d (93.0 mg, 426.2 μmol), 1h (96.8 mg, 426.2 μmol) were dissolved in isopropanol (5.0 mL), TFA (49.6 mg, 426.2 μmol) was then added, and the reaction was warmed to 100°C and allowed to proceed for 18 hours. After the reaction was complete, the mixture was directly concentrated, and the crude product was separated and purified by column chromatography on silica gel (DCM/MeOH=19/1), to afford compound 62e (20.0 mg). MS m/z (ESI): 405.1 $[M+H]^+$.

**Step 5: preparation of $N^1$-(4-((dimethylamino)methyl)-3-(trifluoromethyl)phenyl)-6-methylisoquinoline-1,5-diamine (compound 62f)**

Compound 62e (20.0 mg, 49.5 μmol), reduced iron powder (8.5 mg, 148.4 μmol) and ammonium chloride (13.5 mg, 247.3 μmol) were added to a mixed solution of ethanol (6.0 mL) and water (2.0 mL), purge with nitrogen was performed for 3 times, and the reaction was warmed to 80°C and allowed to proceed for 3 hours. After the reaction was complete, the mixture was filtered with suction to remove the solid, the filtrate was concentrated, and the crude product

was separated and purified by column chromatography on silica gel (PE/EA=4/1), to afford compound 62f (16.0 mg). MS m/z (ESI): 375.2 [M+H]$^+$.

**Step 6: preparation of 4-amino-N-(1-((4-((dimethylamino)methyl)-3-(trifluoromethyl)phenyl)amino)-6-methylisoquinolin-5-yl)imidazo[2,1-f][1,2,4]triazine-7-carboxamide (compound 62)**

Compounds 62f (16.0 mg, 42.7 μmol) and 30c (7.7 mg, 42.7 μmol) were dissolved in pyridine (5.0 mL), $T_3P$ (0.16 mL, a 50% solution in EA) was added, and after the addition, the reaction was allowed to proceed at 25°C for 16 hours. After the reaction was complete, the solvent was removed by concentration, and the residue was separated and purified through Prep-HPLC to afford compound 62 (5.0 mg). MS m/z (ESI): 536.3 [M+H]$^+$.

$^1$H NMR (400 MHz, DMSO-$d_6$) δ 10.40 (s, 1H), 9.49 (s, 1H), 8.71 (s, 1H), 8.63 (s, 1H), 8.49 (d, *J* = 8.4 Hz, 1H), 8.38 (s, 1H), 8.32 (d, *J* = 2.0 Hz, 1H), 8.27 - 8.20 (m, 2H), 8.04 (d, *J* = 6.0 Hz, 1H), 7.65 (dd, *J* = 8.4, 2.0 Hz, 2H), 7.25 (d, *J* = 6.0 Hz, 1H), 3.49 (s, 2H), 2.44 (s, 3H), 2.19 (s, 6H).

**Example 53: N-(1-((4-acetylphenyl)amino)-6-methylisoquinolin-5-yl)-4-aminoquinazoline-8-carboxamide (compound 63)**

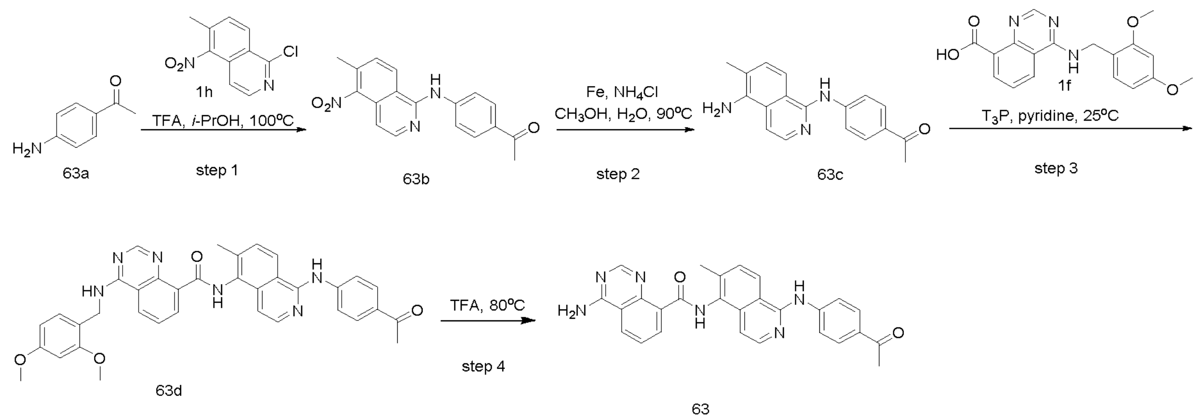


63a 63b 63c 63d 63

**Step 1: preparation of 1-(4-((6-methyl-5-nitroisoquinolin-1-yl)amino)phenyl)ethan-1-one (compound 63b)**

Compounds 63a (145.71 mg, 1.08 mmol), 1h (200 mg, 898.36 μmol) were dissolved in isopropanol (5 mL), TFA (122.92 mg, 1.08 mmol, 80.08 μL) was added, and the reaction was stirred in a sealed tube at 100°C for 18hr. After the reaction was complete, the reaction solution was cooled to room temperature, filtered with suction, rinsed with isopropanol, the filter cake was collected, dried under reduced pressure to afford compound 63b (200 mg). MS m/z (ESI): 322.2 [M+H]$^+$.

**Step 2: preparation of 1-(4-((5-amino-6-methylisoquinolin-1-yl)amino)phenyl)ethan-1-one (compound 63c)**

Compound 63b (200 mg, 622.41 μmol), iron powder (173.81 mg, 3.11 mmol) and $NH_4Cl$ (99.88 mg, 1.87 mmol) were added to water (4 mL) and methanol (16 mL), and the reaction was stirred at 90°C for 6hr. After the reaction was complete, the mixture was directly purified by Flash column chromatography on silica gel (DCM:MeOH=92:8), to afford compound 63c (150 mg). MS m/z (ESI): 292.1 [M+H]$^+$.

**Step 3: preparation of N-(1-((4-acetylphenyl)amino)-6-methylisoquinolin-5-yl)-4-((2,4-dimethoxybenzyl)amino)quinazoline-8-carboxamide (compound 63d)**

Compounds 1f (30 mg, 88.41 μmol) and 63c (25.76 mg, 88.41 μmol) were dissolved in pyridine (2 mL), $T_3P$ (0.5 mL, a 50% solution in EA) was then added, and the reaction was stirred at 25°C for 16hr. After the reaction was complete, the reaction solvent was removed by concentration, the reaction was quenched by dropwise addition of a saturated solution of sodium bicarbonate, extracted with EA, washed with water and saturated brine, dried over anhydrous sodium sulfate, filtered, and concentrated, to afford compound 63d (50 mg). MS m/z (ESI): 613.0 [M+H]$^+$.

**Step 4: preparation of N-(1-((4-acetylphenyl)amino)-6-methylisoquinolin-5-yl)-4-aminoquinazoline-8-carboxamide (compound 63)**

Compound 63d (24.78 mg, 40.45 μmol) was dissolved in TFA (4 mL), and stirred at 80°C for 3hr. After the reaction was complete, the solvent was removed by concentration, the residue was adjusted to a pH of about 8 with a saturated solution of sodium bicarbonate, and extracted with EA. The organic phase was washed with a saturated saline solution, dried over anhydrous sodium sulfate, filtered, concentrated, and then separated and purified through Prep-HPLC, to afford compound 63 (10mg). MS m/z (ESI): 463.2 $[M+H]^+$.

$^1H$ NMR (400 MHz, DMSO-$d_6$)δ 13.23 (s, 1H), 9.62 (s, 1H), 8.66 (dd, *J* = 7.6, 1.6 Hz, 1H), 8.63 (s, 1H), 8.53 (dd, *J* = 8.0, 1.2 Hz, 1H), 8.48 (d, *J* = 8.8 Hz, 1H), 8.36 (br, 1H), 8.24 (br, 1H), 8.14-8.02 (m, 3H), 7.98-7.90 (m, 2H), 7.74 - 7.62 (m, 2H), 7.38 (d, *J* = 6.0 Hz, 1H), 2.54 (s, 3H), 2.46 (s, 3H).

**Example 54: 4-amino-N-(1-((4-chloro-3-((dimethylamino)methyl)phenyl)amino)-6-methylisoquinolin-5-yl)quinazoline-8-carboxamide (compound 64)**

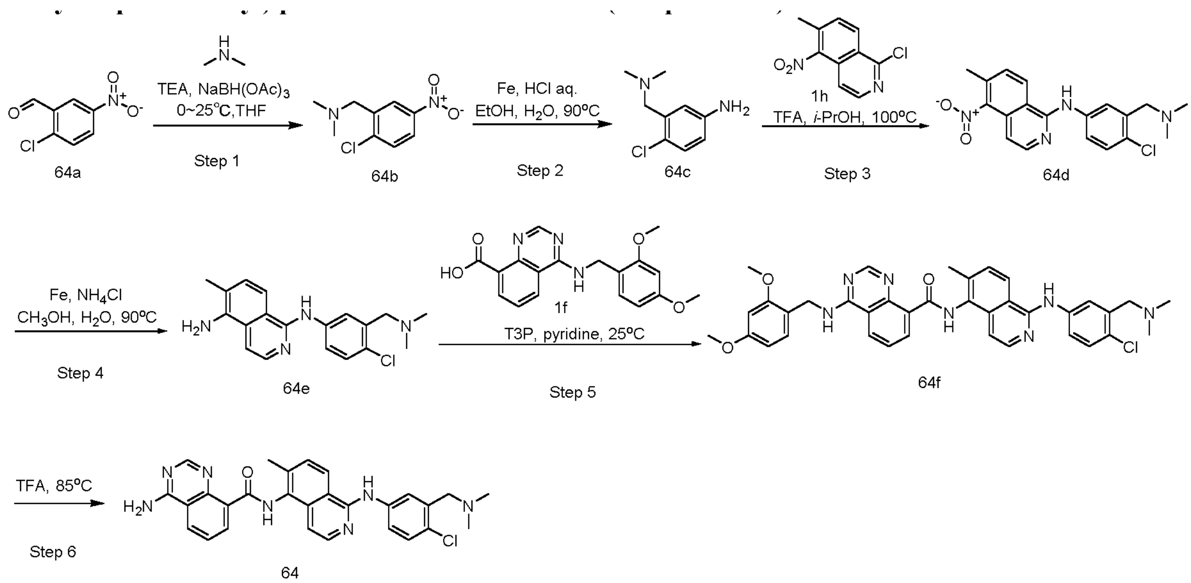


**Step 1: preparation of 1-(2-chloro-5-nitrophenyl)-N,N-dimethylmethanamine (compound 64b)**

Dimethylamine hydrochloride (1.32 g, 16.17 mmol) and triethylamine (1.64 g, 16.17 mmol, 2.25 mL) were added to THF (20 mL), 64a (2 g, 10.78 mmol) was then added, followed by addition of $NaBH(OAc)_3$ (3.43 g, 16.17 mmol) in batches under ice-bath cooling. After the addition, the reaction was naturally warmed to room temperature, and allowed to proceed for 16hr. After the reaction was complete, the reaction was quenched by adding water, and extracted with EA. The organic phase was dried over anhydrous sodium sulfate, filtered, and concentrated, to afford compound 64b (1.47g). MS m/z (ESI): 215.0 $[M+H]^+$.

**Step 2: preparation of 4-chloro-3-((dimethylamino)methyl)aniline (compound 64c)**

Compound 64b (470 mg, 2.19 mmol), iron powder (611.45 mg, 10.95 mmol), concentrated HCl (0.1 mL, 12N) were added to ethanol (10 mL) and water (10 mL), and the reaction was stirred at 90°C for 6hr. After the reaction was complete, the solvent was removed by concentration, and the residue was purified by Flash column chromatography on silica gel (DCM:MeOH=88:12), to afford compound 64c (150 mg). MS m/z (ESI): 185.1 $[M+H]^+$.

**Step 3: preparation of N-(4-chloro-3-((dimethylamino)methyl)phenyl)-6-methyl-5-nitroisoquinolin-1-amine (compound 64d)**

Compounds 64c (150 mg, 812.28 μmol) and 1h (180.84 mg, 812.28 μmol) were dissolved in isopropanol (8 mL), TFA (111.14 mg, 974.74 μmol, 72.40 μL) was then added, and after the addition, the reaction was stirred in a

sealed tube at 100°C for 18 hr. After the reaction was complete, the reaction solution was concentrated to dryness, to afford compound 64d (301mg). MS m/z (ESI): 371.1 [M+H]$^+$.

**Step 4: preparation of N$^1$-(4-chloro-3-((dimethylamino)methyl)phenyl)-6-methylisoquinoline-1,5-diamine (compound 64e)**

Compound 64d (150 mg, 404.50 μmol), iron powder (112.96 mg, 2.02 mmol) and $NH_4Cl$ (64.91 mg, 1.21 mmol) were added to water (2 mL) and methanol (8 mL), and the reaction was stirred at 90°C for 4hr. After the reaction was complete, the reaction solution was concentrated to dryness, purified by Flash column chromatography on silica gel (DCM:MeOH=85:15), to afford compound 64e (60 mg). MS m/z (ESI): 341.1 [M+H]$^+$.

**Step 5: preparation of N-(1-((4-chloro-3-((dimethylamino)methyl)phenyl)amino)-6-methylisoquinolin-5-yl)-4-((2,4-dimethoxybenzyl)amino)quinazoline-8-carboxamide (compound 64f)**

Compounds 64e (30.13 mg, 88.41 μmol) and 1f (30 mg, 88.41 μmol) were dissolved in pyridine (2 mL), $T_3P$ (0.5 mL, a 50% solution in EA) was then added, and the reaction was stirred at 25°C for 16hr. After the reaction was complete, the solvent was removed under reduced pressure, the reaction was quenched by dropwise addition of a saturated solution of sodium bicarbonate, extracted with EA, washed with water and saturated brine, dried over anhydrous sodium sulfate, filtered, and concentrated, to afford compound 64f (60 mg). MS m/z (ESI): 662.1 [M+H]$^+$.

**Step 6: preparation of 4-amino-N-(1-((4-chloro-3-((dimethylamino)methyl)phenyl)amino)-6-methylisoquinolin-5-yl)quinazoline-8-carboxamide (compound 64)**

Compound 64f (60 mg, 90.61 μmol) was dissolved in TFA (4 mL), and the reaction was stirred at 85°C for 3hr. After the reaction was complete, TFA was concentrated to dryness, the residue was adjusted to a pH of about 8 with a saturated solution of sodium bicarbonate, and extracted with EA. The organic phase was washed with a saturated saline solution, dried over anhydrous sodium sulfate, filtered, concentrated, and then separated and purified through Prep-HPLC, to afford compound 64 (7 mg). MS m/z (ESI): 512.2 [M+H]+.

$^1$H NMR (400 MHz, DMSO-$d_6$) δ 13.20 (s, 1H), 9.33 (s, 1H), 8.66 (dd, *J* = 7.2, 1.2 Hz, 1H), 8.63 (s, 1H), 8.53 (dd, *J* = 8.4, 1.2 Hz, 1H), 8.47 (d, *J* = 8.8 Hz, 1H), 8.37 (br, 1H), 8.23 (br, 1H), 8.05 - 7.97 (m, 2H), 7.92 (d, *J* = 2.4 Hz, 1H), 7.70 (t, *J* = 7.8 Hz, 1H), 7.61 (d, *J* = 8.8 Hz, 1H), 7.35 (d, *J* = 8.8 Hz, 1H), 7.26 (d, *J* = 6.0 Hz, 1H), 3.48 (s, 2H), 2.44 (s, 3H), 2.24 (s, 6H).

**Example 55: 4-amino-N-(6-methyl-1-(p-tolylamino)isoquinolin-5-yl)quinazoline-8-carboxamide (compound 65)**

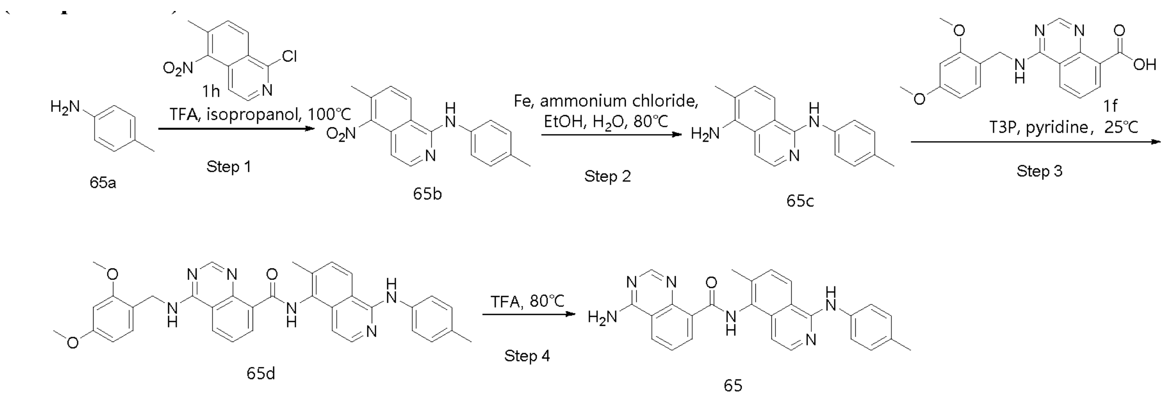


**Step 1: preparation of 6-methyl-5-nitro-N-(p-tolyl)isoquinolin-1-amine (compound 65b)**

Compounds 65a (86.6 mg, 808.5 μmol), 1h (150.0 mg, 673.8 μmol) and TFA (76.8 mg, 673.8 μmol) were added to isopropanol (5.0 mL), and the reaction was warmed to 100°C and allowed to proceed for 16 hours. After the reaction was complete, the reaction solution was adjusted to a basic pH with triethylamine, concentrated, and the crude product was purified by silica gel column chromatography (PE/EA=1/1), to afford compound 65b (140.0 mg). MS m/z (ESI): 294.1 [M+H]$^+$.

**Step 2: preparation of 6-methyl-N1-(p-tolyl)isoquinoline-1,5-diamine (compound 65c)**

Compound 65b (140.0 mg, 477.3 μmol) was dissolved in ethanol (6.0 mL) and water (2.0 mL), iron powder (133.3 mg, 2.4 mmol) and ammonium chloride (25.5 mg, 477.3 μmol) were then sequentially added, and the reaction was warmed to 80°C and allowed to proceed for 2 hours. After the reaction was complete, the mixture was filtered through diatomaceous earth, and the filtrate was concentrated, to afford compound 65c (113.6 mg). MS m/z (ESI): 264.1 $[M+H]^+$.

**Step 3: preparation of 4-((2,4-dimethoxybenzyl)amino)-N-(6-methyl-1-(p-tolylamino)isoquinolin-5-yl)quinazoline-8-carboxamide (compound 65d)**

Compounds 65c (38.8 mg, 147.3 μmol) and 1f (50.0 mg, 147.3 μmol) were dissolved in pyridine (3.0 mL), $T_3P$ (1 mL, 50% in DMF) was dropwise added, and after the addition, the reaction was allowed to proceed at 25°C for 3 hours. After the reaction was complete, the solvent was removed by concentration under reduced pressure, the reaction solution was diluted with ethyl acetate, washed with a saturated aqueous solution of sodium bicarbonate for 3 times, the organic phase was dried over anhydrous sodium sulfate, filtered and then concentrated, to afford compound 65d (50.0 mg). MS m/z (ESI): 585.3 $[M+H]^+$.

**Step 4: preparation of 4-amino-N-(6-methyl-1-(p-tolylamino)isoquinolin-5-yl)quinazoline-8-carboxamide (compound 65)**

Compound 65d (50.0 mg, 85.5 μmol) was added to trifluoroacetic acid (3.0 mL), and the reaction was allowed to proceed at 80°C for 2 hours. After the reaction was complete, the reaction solution was concentrated to dryness, alkalized by adding a saturated aqueous solution of sodium bicarbonate, and extracted with dichloromethane. The organic phase was dried over anhydrous sodium sulfate, filtered and then concentrated, and the crude product was separated and purified by Prep-HPLC, to afford compound 65 (2.0 mg). MS m/z (ESI): 435.0 $[M+H]^+$.

$^1$H NMR (400 MHz, DMSO-$d_6$) δ 13.18 (s, 1H), 9.11 (s, 1H), 8.66 (dd, *J* = 7.6, 1.6 Hz, 1H), 8.63 (s, 1H), 8.53 (dd, *J* = 8.0, 1.6 Hz, 1H), 8.44 (d, *J* = 8.4 Hz, 1H), 8.41 - 8.14 (m, 2H), 7.95 (d, *J* = 6.0 Hz, 1H), 7.80 - 7.73 (m, 2H), 7.69 (d, *J* = 8.0 Hz, 1H), 7.58 (d, *J* = 8.8 Hz, 1H), 7.20 (d, *J* = 6.0 Hz, 1H), 7.13 (d, *J* = 8.4 Hz, 2H), 2.43 (s, 3H), 2.29 (s, 3H).

**Example 56: 4-amino-N-(1-((3-chloro-2-fluorophenyl)amino)-6-methylisoquinolin-5-yl)pyrido[3,4-d]pyrimidine-8-carboxamide (compound 66)**

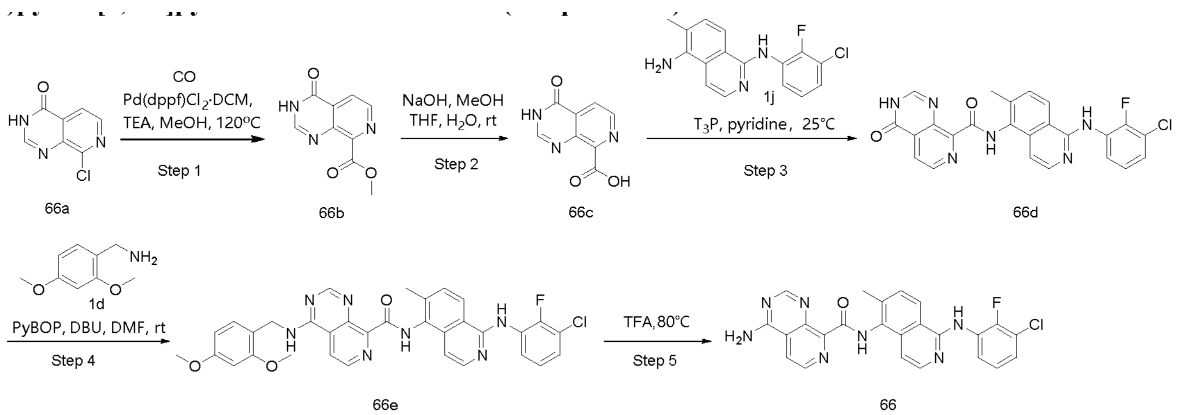


**Step 1: preparation of methyl 4-oxo-3,4-dihydropyrido[3,4-d]pyrimidine-8-carboxylate (compound 66b)**

Compound 66a (1.7 g, 9.4 mmol), TEA (9.5 g, 93.6 mmol, 13.0 mL), $Pd(dppf)Cl_2 \cdot DCM$ (764.6 mg, 936.2 μmol) and MeOH (20 mL) were added to an autoclave, after being sealed, carbon monoxide was pumped in to 1.0-1.2Mpa, and then the reaction was heated to 120°C and allowed to proceed for 5 hours. After the reaction was complete, the reaction solution was cooled to room temperature, and directly purified by Flash column chromatography on silica gel (DCM/MeOH=10/1), to afford compound 66b (1.82 g). MS m/z (ESI): 206.1 $[M+H]^+$.

**Step 2: preparation of 4-oxo-3,4-dihydropyrido[3,4-d]pyrimidine-8-carboxylic acid (compound 66c)**

Compound 66b (300 mg, 1.5 mmol) was dissolved in MeOH (2 mL) and THF (2 mL), water (2 mL) and NaOH (175.5 mg, 4.4 mmol) were added, and the reaction was allowed to proceed at 25°C for 3h. After the reaction was complete, the reaction solution was adjusted to a pH of 5-6 with 2N hydrochloric acid, the organic solvent was removed by evaporation under reduced pressure, the precipitated solid was filtered, the filter cake was rinsed with water, and dried in vacuum to afford compound 66c (220.0 mg). MS m/z (ESI): 192.1 $[M+H]^+$.

**Step 3: preparation of N-(1-(1-((3-chloro-2-fluorophenyl)amino)-6-methylisoquinolin-5-yl)-4-oxo-3,4-dihydropyrido[3,4-d]pyrimidine-8-carboxamide (compound 66d)**

Compounds 66c (220.0 mg,1.2 mmol) and 1j (347.3 mg, 1.2 mmol) were dissolved in pyridine (5.0 mL), $T_3P$ (2 mL, 50% in DMF) was dropwise added, and after the addition, the reaction was allowed to proceed at 25°C for 3 hours. After the reaction was complete, the solvent was removed by concentration under reduced pressure, the reaction solution was diluted with ethyl acetate, washed with a saturated aqueous solution of sodium bicarbonate for 3 times, the organic phase was dried over anhydrous sodium sulfate, filtered and then concentrated, to afford compound 66d (462.0 mg). MS m/z (ESI): 475.1 $[M+H]^+$.

**Step 4: preparation of N-(1-((3-chloro-2-fluorophenyl)amino)-6-methylisoquinolin-5-yl)-4-((2,4-dimethoxybenzyl)amino)pyrido[3,4-d]pyrimidine-8-carboxamide (compound 66e)**

Compounds 66d (200.0 mg, 421.6 μmol) and 1d (140.8 mg, 842.3 μmol, 126.5 μL) were dissolved in DMF (5 mL), DBU (159.1 mg, 631.8 μmol, 156.2 μL) was then dropwise added, the reaction was stirred for 10 min before addition of compound PyBOP (263.0 mg, 505.4 μmol), and the reaction was then stirred at 25°C for 3 hours. After the reaction was complete, the reaction was quenched by adding water, extracted with EA for three times, the organic phase was washed with a saturated saline solution, dried over anhydrous sodium sulfate, filtered, concentrated, purified by column chromatography on silica gel(DCM/MeOH= 10/1), to afford compound 66e (120.0 mg). MS m/z (ESI): 624.0 $[M+H]^+$.

**Step 5: preparation of 4-amino-N-(1-((3-chloro-2-fluorophenyl)amino)-6-methylisoquinolin-5-yl)pyrido[3,4-d]pyrimidine-8-carboxamide (compound 66)**

Compound 66e (50.0 mg, 80.1 μmol) was added to trifluoroacetic acid (3.0 mL), and the reaction was allowed to proceed at 80°C for 6 hours. After the reaction was complete, the reaction solution was concentrated to dryness, alkalized by adding a saturated aqueous solution of sodium bicarbonate, and extracted with dichloromethane. The organic phase was dried over anhydrous sodium sulfate, filtered, concentrated, and the crude product was separated and purified by Prep-HPLC, to afford compound 66 (9.0 mg). MS m/z (ESI): 474.1 $[M+H]^+$.

$^1H$ NMR (400 MHz, DMSO-$d_6$) δ 10.82 (s, 1H), 9.22 (s, 1H), 8.71 (d, *J* = 5.2 Hz, 1H), 8.67 (s, 1H), 8.48 - 8.29 (m, 3H), 8.27 (d, *J* = 5.2 Hz, 1H), 7.97 (d, *J* = 6.0 Hz, 1H), 7.65 - 7.59 (m, 2H), 7.59 - 7.53 (m, 1H), 7.41 - 7.31 (m, 1H), 7.30 - 7.17 (m, 1H), 2.54 (s, 3H).

**Example 57: 4-amino-N-(1-((2,4-difluorophenyl)amino)-6-methylisoquinolin-5-yl)quinazoline-8-carboxamide (compound 67)**

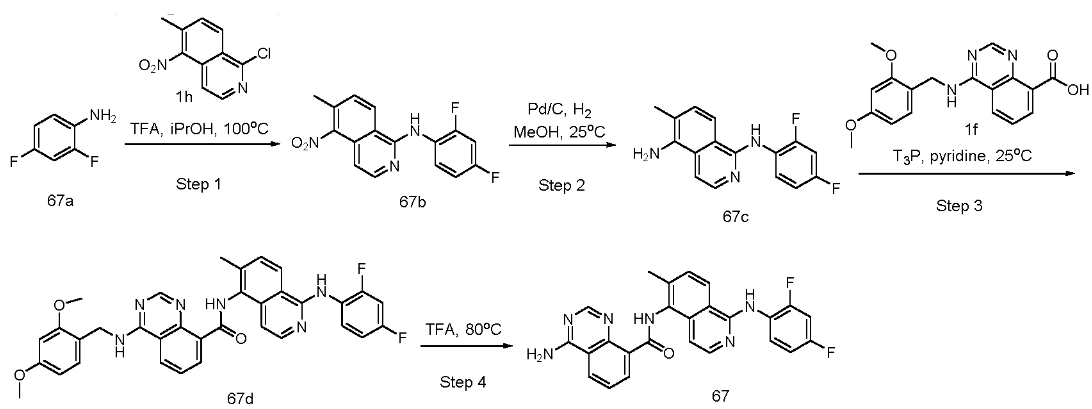

**Step 1: preparation of N-(2,4-difluorophenyl)-6-methyl-5-nitroisoquinolin-1-amine (compound 67b)**

Compound 67b (420.00 mg) was prepared according to the method for preparing compound 59d in Step 3 of Example 49, except that compound 59c was replaced with compound 67a. MS m/z (ESI): 316.1 $[M+H]^+$.

**Step 2: preparation of N1-(2,4-difluorophenyl)-6-methylisoquinoline-1,5-diamine (compound 67c)**

Compound 67c (450.00 mg) was prepared according to the method for preparing compound 59e in Step 4 of Example 49, except that compound 59d was replaced with compound 67b. MS m/z (ESI): 286.1 $[M+H]^+$.

**Step 3: preparation of N-(1-((2,4-difluorophenyl)amino)-6-methylisoquinolin-5-yl)-4-((2,4-dimethoxybenzyl)amino)quinazoline-8-carboxamide (compound 67d)**

Compound 67d (41.00 mg) was prepared according to the method for preparing compound 59f in Step 5 of Example 49, except that compound 59e was replaced with compound 67c. MS m/z (ESI): 607.1 $[M+H]^+$.

**Step 4: preparation of 4-amino-N-(1-((2,4-difluorophenyl)amino)-6-methylisoquinolin-5-yl)quinazoline-8-carboxamide (compound 67)**

Compound 67d (20.00 mg, 32.97 μmol) was dissolved in trifluoroacetic acid (3 mL), and the reaction was warmed to 80°C and allowed to proceed for 2 hours. After the reaction was complete, the solvent was removed under reduced pressure, and then purified by Prep-HPLC, to afford compound 67 (13.00 mg). MS m/z (ESI): 457.0 $[M+H]^+$.

$^1$H NMR (400 MHz, DMSO-$d_6$) δ 13.18 (s, 1H), 9.06 (s, 1H), 8.65 (d, *J* = 7.6 Hz, 1H), 8.62 (s, 1H), 8.53 (d, *J* = 8.4 Hz, 1H), 8.45-8.14 (m, 3H), 7.84 (d, *J* = 5.6 Hz, 1H), 7.69 (t, *J* = 8.0 Hz, 1H), 7.64-7.52 (m, 2H), 7.36-7.26 (m, 1H), 7.19 (d, *J* = 6.0 Hz, 1H), 7.15-7.05 (m, 1H), 2.44 (s, 3H).

**Example 58: 4-amino-N-(1-((3,4-dimethoxyphenyl)amino)-6-methylisoquinolin-5-yl)quinazoline-8-carboxamide (compound 68)**

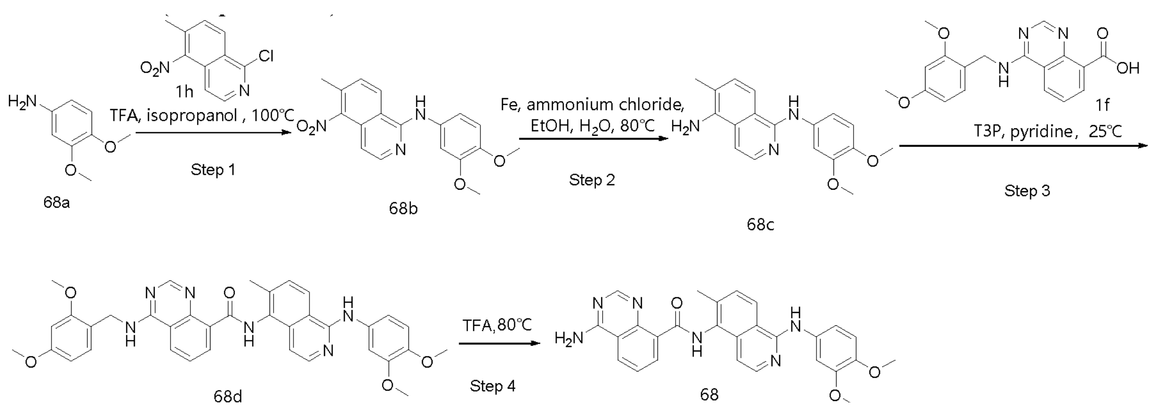


**Step 1: preparation of N-(3,4-dimethoxyphenyl)-6-methyl-5-nitroisoquinolin-1-amine (compound 68b)**

Compounds 68a (125.0 mg, 816.0 μmol), 1h (181.7 mg, 816.0 μmol) and TFA (93.1 mg, 816.0 μmol) were added to isopropanol (5.0 mL), and the reaction was warmed to 100°C and allowed to proceed for 16 hours. After the reaction was complete, the reaction solution was adjusted to a basic pH with triethylamine, concentrated, and the crude product was separated and purified by silica gel column chromatography (PE/EA=1/1), to afford compound 68b (120.0 mg). MS m/z (ESI): 340.0 $[M+H]^+$.

**Step 2: preparation of N1-(3,4-dimethoxyphenyl)-6-methylisoquinoline-1,5-diamine (compound 68c)**

Compound 68b (120.0 mg, 353.6 μmol) was dissolved in ethanol (6.0 mL) and water (2.0 mL), iron powder (98.8 mg, 1.8 mmol) and ammonium chloride (18.9 mg, 353.6 μmol) were then sequentially added, and the reaction was warmed to 80°C and allowed to proceed for 2 hours. After the reaction was complete, the reaction was filtered

through diatomaceous earth, the liquid was concentrated, to afford compound 68c (100.0 mg). MS m/z (ESI): 310.0 $[M+H]^+$.

**Step 3: preparation of 4-((2,4-dimethoxybenzyl)amino)-N-(1-((3,4-dimethoxyphenyl)amino)-6-methylisoquinolin-5-yl)quinazoline-8-carboxamide (compound 68d)**

Compounds 68c (50.0 mg, 161.6 μmol) and 1f (54.9 mg, 161.6 μmol) were dissolved in pyridine (3.0 mL), $T_3P$ (1 mL, 50% in DMF) was dropwise added, and after the addition, the reaction was allowed to proceed at 25°C for 3 hours. After the reaction was complete, the solvent was removed by concentration under reduced pressure, the reaction solution was diluted with ethyl acetate, washed with a saturated aqueous solution of sodium bicarbonate for 3 times, the organic phase was dried over anhydrous sodium sulfate, filtered and then concentrated, to afford compound 68d (53.6 mg). MS m/z (ESI): 632.0 $[M+H]^+$.

**Step 4: preparation of 4-amino-N-(1-((3,4-dimethoxyphenyl)amino)-6-methylisoquinolin-5-yl)quinazoline-8-carboxamide (compound 68)**

Compound 68d (53.6 mg, 85.0 μmol) was added to trifluoroacetic acid (3.0 mL), and the reaction was allowed to proceed at 80°C for 2 hours. After the reaction was complete, the reaction solution was concentrated to dryness, alkalized by adding a saturated aqueous solution of sodium bicarbonate, and extracted with dichloromethane. The organic phase was dried over anhydrous sodium sulfate, filtered, concentrated, and the crude product was separated and purified by Prep-HPLC, to afford compound 68 (7.3 mg). MS m/z (ESI): 481.0 $[M+H]^+$.

$^1H$ NMR (400 MHz, DMSO-$d_6$) δ 13.18 (s, 1H), 9.05 (s, 1H), 8.71 - 8.60 (m, 2H), 8.53 (dd, *J* = 8.4, 1.6 Hz, 1H), 8.48 - 8.10 (m, 3H), 7.95 (d, *J* = 6.0 Hz, 1H), 7.69 (d, *J* = 8.0 Hz, 1H), 7.58 (d, *J* = 8.4 Hz, 1H), 7.56 - 7.45 (m, 2H), 7.17 (d, *J* = 6.0 Hz, 1H), 6.92 (d, *J* = 8.8 Hz, 1H), 3.76 (d, *J* = 12.0 Hz, 6H), 2.43 (s, 3H).

**Example 59: 4-amino-N-(1-((3-chloro-2-fluorophenyl)amino)-6-methylisoquinolin-5-yl)pyrido[4,3-d]pyrimidine-8-carboxamide (compound 69)**

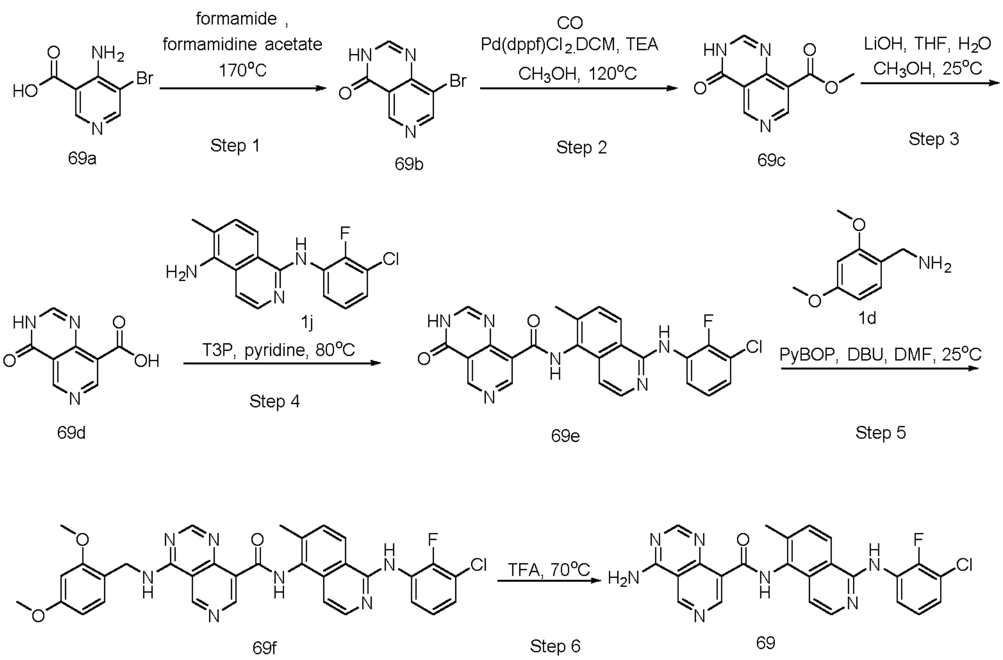


**Step 1: preparation of 8-bromopyrido[4,3-d]pyrimidin-4(3H)-one (compound 69b)**

Compound 69a (3000.00 mg, 13.82 mmol) and formamidine acetate (4320.00 mg, 41.47 mmol) were added to a single neck flask and heated to 170°C, formamide (1.10 mL) was dropwise added, and after the dropwise addition, the reaction was continued at 170°C for 3 hours. After the reaction was complete, the reaction solution was cooled to room temperature, poured into ice water, stirred at room temperature for 1hr, filtered with suction, the filter cake was

rinsed with water, and dried to afford compound 69b (800 mg). MS m/z (ESI): 225.9 [M+H]$^+$.

**Step 2: preparation of methyl 4-oxo-3,4-dihydropyrido[4,3-d]pyrimidine-8-carboxylate (compound 69c)**

Compound 69c (613 mg) was prepared according to the method for preparing compound 2c in Step 2 of Example 2, except that compound 2b was replaced with compound 69b. MS m/z (ESI): 205.9 [M+H]$^+$.

**Step 3: preparation of 4-oxo-3,4-dihydropyrido[4,3-d]pyrimidine-8-carboxylic acid (compound 69d)**

Compound 69d (390 mg) was prepared according to the method for preparing compound 54b in Step 2 of Example 44, except that compound 54a was replaced with compound 69c. MS m/z (ESI): 191.9 [M+H]$^+$.

**Step 4: preparation of N-(1-((3-chloro-2-fluorophenyl)amino)-6-methylisoquinolin-5-yl)-4-oxo-3,4-dihydropyrido[4,3-d]pyrimidine-8-carboxamide (compound 69e)**

Compound 69e (320 mg) was prepared according to the method for preparing compound 1k in Step 7 of Example 1, except that compound 1f was replaced with compound 69d, and the reaction temperature was changed to 80°C. MS m/z (ESI): 474.9 [M+H]$^+$.

**Step 5: preparation of N-(1-((3-chloro-2-fluorophenyl)amino)-6-methylisoquinolin-5-yl)-4-((2,4-dimethoxybenzyl)amino)pyrido[4,3-d]pyrimidine-8-carboxamide (compound 69f)**

Compound 69f (17 mg) was prepared according to the method for preparing compound 45f in Step 5 of Example 41, except that compound 45e was replaced with compound 69e. MS m/z (ESI): 623.9[M+H]$^+$.

**Step 6: preparation of 4-amino-N-(1-((3-chloro-2-fluorophenyl)amino)-6-methylisoquinolin-5-yl)pyrido[4,3-d]pyrimidine-8-carboxamide (compound 69)**

Compound 69 (4 mg) was prepared according to the method for preparing compound 60 in Step 4 of Example 50, except that compound 60d was replaced with compound 69f. MS m/z (ESI): 474.0 [M+H]$^+$.

$^1$H NMR (400 MHz, DMSO-$d_6$) δ 12.53 (s, 1H), 9.76 (s, 1H), 9.42 (s, 1H), 9.04-8.90 (m, 1H), 8.75 (s, 1H), 8.74(brs, 1H), 8.43 (d, *J* = 7.2 Hz, 1H), 7.84 (d, *J* = 6.0 Hz, 1H), 7.71 (d, *J* = 8.0 Hz, 1H), 7.60-7.37 (m, 3H), 7.35-7.25 (m, 2H), 2.48 (s, 3H).

**Example 60: 4-amino-N-(1-((4-chloro-2,3-difluorophenyl)amino)-6-methylisoquinolin-5-yl)imidazo[2,1-f][1,2,4]triazine-7-carboxamide (compound 70)**

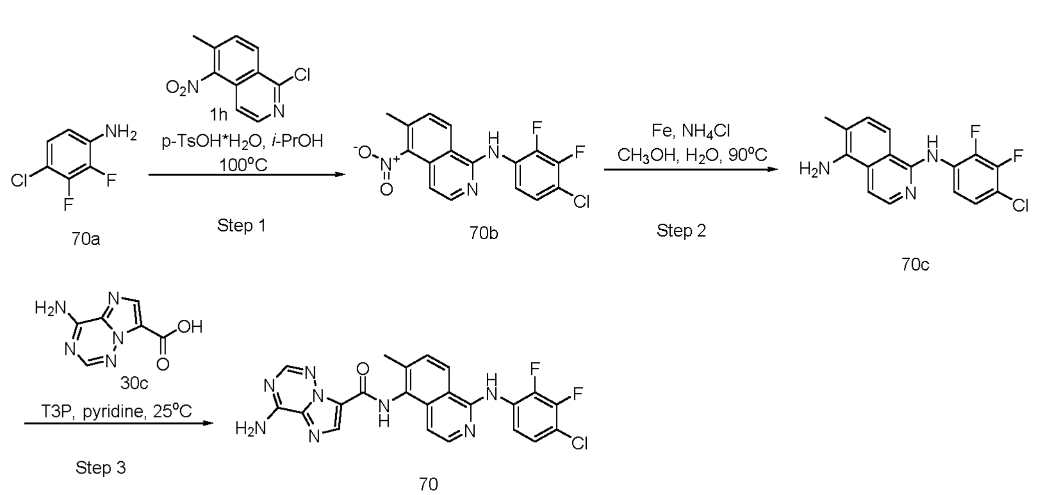


**Step 1: preparation of N-(4-chloro-2,3-difluorophenyl)-6-methyl-5-nitroisoquinolin-1-amine (compound 70b)**

Compounds 1h (220.39 mg, 1.35 mmol) and 70a (200 mg, 898.36 μmol) were dissolved in isopropanol (10

mL), p-toluenesulfonic acid monohydrate (68.35 mg, 359.34 μmol) was added, and the reaction was stirred at 100°C for18 hr. After the reaction was complete, the reaction solution was concentrated to dryness, to afford compound 70b (314 mg). MS m/z (ESI): 349.9 $[M+H]^+$.

**Step 2: preparation of N$^1$-(4-chloro-2,3-difluorophenyl)-6-methylisoquinoline-1,5-diamine (compound 70c)**

Compound 70b (314 mg, 897.86 μmol), iron powder (250.56 mg, 4.49 mmol), $NH_4Cl$ (143.98 mg, 2.69 mmol) were added to water (4 mL) and methanol (15 mL), and the reaction was stirred at 90°C for 4hr. After the reaction was complete, the mixture was directly purified by Flash column chromatography on silica gel (DCM:MeOH=88:12), to afford compound 70c (210 mg). MS m/z (ESI): 319.9 $[M+H]^+$.

**Step 3: preparation of 4-amino-N-(1-((4-chloro-2,3-difluorophenyl)amino)-6-methylisoquinolin-5-yl)imidazo[2,1-f][1,2,4]triazine-7-carboxamide (compound 70)**

Compounds 70c (35.70 mg, 111.65 μmol) and 30c (20 mg, 111.65 μmol) were dissolved in pyridine (2 mL), $T_3P$ (0.5 mL, a 50% solution in EA) was then added, and the reaction was stirred at 25°C for 16hr. After the reaction was complete, the solvent was removed by concentration, the reaction was quenched by dropwise addition of a saturated solution of sodium bicarbonate, and extracted with EA. The organic phase was washed with a saturated saline solution, dried over anhydrous sodium sulfate, filtered, concentrated, and then purified by Flash column chromatography on silica gel (DCM:MeOH=96:4), to afford compound 70 (6.88 mg). MS m/z (ESI): 480.9 $[M+H]^+$.

$^1H$ NMR (400 MHz, DMSO-$d_6$) δ 10.39 (s, 1H), 9.38 (s, 1H), 8.66 (d, *J* = 32.4 Hz, 2H), 8.41 - 8.32 (m, 2H), 8.24 (s, 1H), 7.92 (d, *J* = 6.0 Hz, 1H), 7.64 (d, *J* = 8.8Hz, 1H), 7.47 - 7.40 (m, 2H), 7.24 (d, *J* = 6.0 Hz, 1H), 2.44 (s, 3H).

**Example 61: 4-amino-N-(6-methyl-1-((2-(trifluoromethyl)pyridin-4-yl)amino)isoquinolin-5-yl)quinazoline-8-carboxamide (compound 71)**

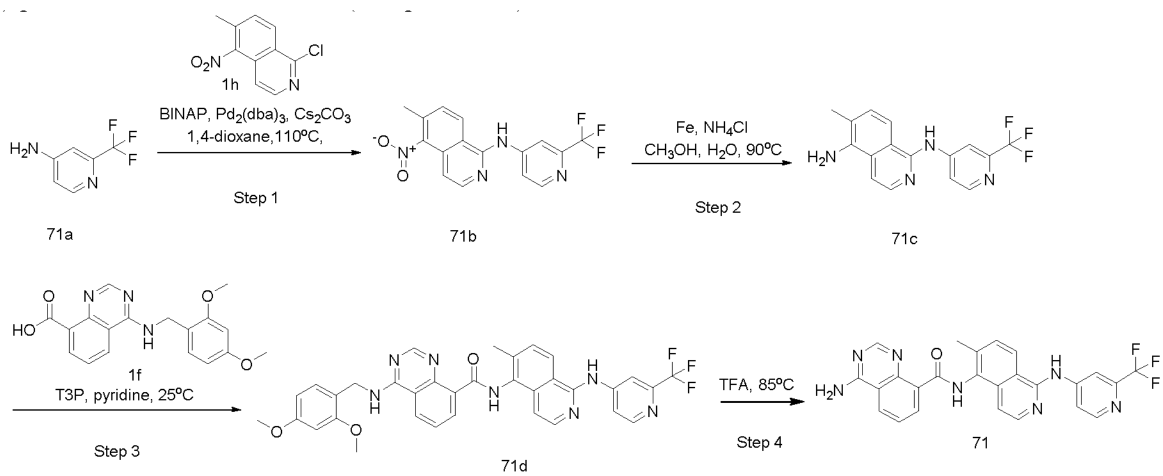


**Step 1: preparation of 6-methyl-5-nitro-N-(2-(trifluoromethyl)pyridin-4-yl)isoquinolin-1-amine (compound 71b)**

Compounds 70a (150 mg, 925.28 μmol), 1h (205.99 mg, 925.29 μmol), BINAP (115.23 mg, 185.06 μmol), $Pd_2(dba)_3$ (84.73 mg, 92.53 μmol) and $Cs_2CO_3$ (904.43 mg, 2.78 mmol) were added to 1,4-dioxane (10 mL), under the protection of nitrogen, the reaction was performed under microwave at 110°C for 3hr. After the reaction was complete, the reaction was diluted by adding water, extracted with EA, the organic phase was dried over anhydrous sodium sulfate, filtered, and concentrated, to afford compound 71b (300 mg). MS m/z (ESI): 349.0 $[M+H]^+$.

**Step 2: preparation of 6-methyl-N$^1$-(2-(trifluoromethyl)pyridin-4-yl)isoquinoline-1,5-diamine (compound 71c)**

Compound 71b (300 mg, 861.38 μmol), iron powder (240.54 mg, 4.31 mmol) and $NH_4Cl$ (138.23 mg, 2.58 mmol) were added to methanol (15 mL) and water (4 mL), and the reaction was stirred at 90°C for 4hr. After the reaction was complete, the mixture was directly purified by Flash column chromatography on silica gel (DCM:MeOH=88:12), to afford compound 71c (150mg). MS m/z (ESI): 319.0 $[M+H]^+$.

**Step 3: preparation of 4-((2,4-dimethoxybenzyl)amino)-N-(6-methyl-1-((2-(trifluoromethyl)pyridin-4-yl)amino)isoquinolin-5-yl)quinazoline-8-carboxamide (compound 71d)**

Compounds 1f (40.00 mg, 117.87 μmol) and 71c (37.52 mg, 117.87 μmol) were dissolved in pyridine (2 mL), $T_3P$ (0.5 mL, a 50% solution in EA) was then added, and the reaction was stirred at 25°C for 16hr. After the reaction was complete, the solvent was removed under reduced pressure, the reaction was quenched by dropwise addition of a saturated solution of sodium bicarbonate, and extracted with EA. The organic phase was washed with a saturated saline solution, dried over anhydrous sodium sulfate, filtered, and concentrated, to afford compound 71d (40 mg). MS m/z (ESI): 640.1 $[M+H]^+$.

**Step 4: preparation of 4-amino-N-(6-methyl-1-((2-(trifluoromethyl)pyridin-4-yl)amino)isoquinolin-5-yl)quinazoline-8-carboxamide (compound 71)**

Compound 71d (40 mg, 62.54 μmol) was dissolved in TFA (3 mL), and the reaction was stirred at 85°C for 3hr. After the reaction was complete, TFA was concentrated to dryness, the residue was adjusted to a pH of about 8 by adding a saturated solution of sodium bicarbonate. The reaction was extracted with EA, the organic phase was washed with a saturated saline solution, dried over anhydrous sodium sulfate, filtered, concentrated, and then separated and purified through Prep-HPLC, to afford a trifluoroacetate salt of compound 71 (18 mg). MS m/z (ESI): 490.1 $[M+H]^+$.

$^1H$ NMR (400 MHz, DMSO-$d_6$) δ 10.04 (s, 1H), 9.78 - 9.03 (m, 2H), 8.78 (d, *J* = 5.2 Hz, 1H), 8.68 (s, 1H), 8.64 (d, *J* = 8.0 Hz, 1H), 8.56 (d, *J* = 5.6 Hz, 1H), 8.52 (d, *J* = 8.4 Hz, 1H), 8.46 (d, *J* = 2.0 Hz, 1H), 8.24 (dd, *J* = 5.6, 1.6 Hz, 1H), 8.20 (d, *J* = 6.0 Hz, 1H), 7.89 (s, 1H), 7.74 (d, *J* = 8.8 Hz, 1H), 7.49 (d, *J* = 6.0 Hz, 1H), 2.48 (s, 3H).

**Example 62: 4-amino-N-(1-((2,5-difluorophenyl)amino)-6-methylisoquinolin-5-yl)quinazoline-8-carboxamide (compound 72)**

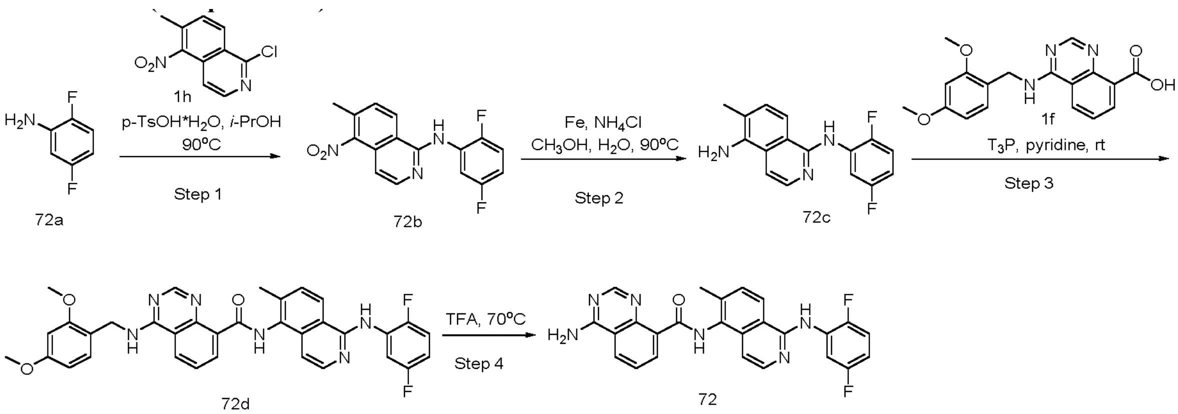


**Step 1: preparation of N-(2,5-difluorophenyl)-6-methyl-5-nitroisoquinolin-1-amine (compound 72b)**

Compound 72b (200 mg) was prepared according to the method for preparing compound 70b in Step 1 of Example 60, except that compound 70a was replaced with compound 72a, and the reaction temperature was changed to 90°C. MS m/z (ESI): 316.0 $[M+H]^+$.

**Step 2: preparation of $N^1$-(2,5-difluorophenyl)-6-methylisoquinoline-1,5-diamine (compound 72c)**

Compound 72c (150 mg) was prepared according to the method for preparing compound 70c in Step 2 of Example 60, except that compound 70b was replaced with compound 72b. MS m/z (ESI): 286.1 $[M+H]^+$.

**Step 3: preparation of N-(1-((2,5-difluorophenyl)amino)-6-methylisoquinolin-5-yl)-4-((2,4-dimethoxybenzyl)amino)quinazoline-8-carboxamide (compound 72d)**

Compound 72d (50 mg) was prepared according to the method for preparing compound 1k in Step 7 of Example 1, except that compound 1j was replaced with compound 72c. MS m/z (ESI): 607.2 $[M+H]^+$.

**Step 4: preparation of 4-amino-N-(1-((2,5-difluorophenyl)amino)-6-methylisoquinolin-5-yl)quinazoline-8-carboxamide (compound 72)**

Compound 72 (5 mg) was prepared according to the method for preparing compound 1 in Step 8 of Example 1, except that compound 1k was replaced with compound 72d. MS m/z (ESI): 457.1 $[M+H]^+$.

$^1$HNMR (400 MHz, DMSO-$d_6$) $\delta$ 13.21 (s, 1H), 9.13 (s, 1H), 8.67 (dd, *J* = 7.2, 1.6 Hz, 1H), 8.64 (s, 1H), 8.54 (dd, *J* = 8.4, 1.2 Hz, 1H), 8.41 (brs, 1H), 8.35 (d, *J* = 8.8 Hz, 1H), 8.25 (brs, 1H), 7.96 (d, *J* = 6.0 Hz, 1H), 7.71 (t, *J* = 8.0 Hz, 1H), 7.68-7.62 (m, 2H), 7.35-7.31 (m, 1H), 7.30(d, *J* = 6.0 Hz, 1H), 7.03-6.96 (m, 1H), 2.46 (s, 3H).

**Example 63: 4-amino-N-(1-((5-chloro-2-fluorophenyl)amino)-6-methylisoquinolin-5-yl)quinazoline-8-carboxamide (compound 73)**

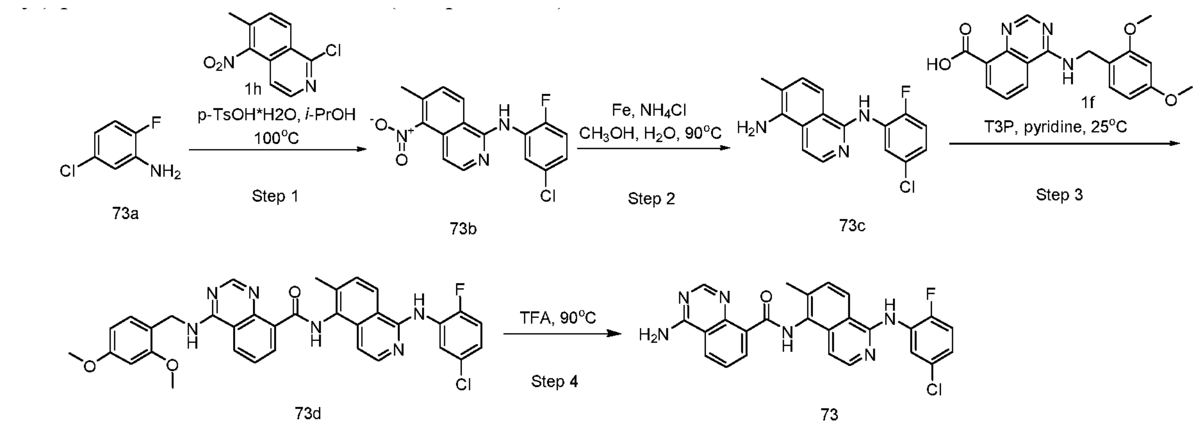


73a 73b 73c 73d 73

**Step 1: preparation of N-(5-chloro-2-fluorophenyl)-6-methyl-5-nitroisoquinolin-1-amine (compound 73b)**

Compounds 73a (156.92 mg, 1.08 mmol) and 1h (200 mg, 898.36 μmol) were dissolved in isopropanol (10 mL), p-toluenesulfonic acid monohydrate (68.35 mg, 359.34 μmol) was added, and the reaction was stirred at 100°C for 18 hr. After the reaction was complete, the solvent was removed by concentration, to afford compound 73b (298 mg). MS m/z (ESI): 332.0 $[M+H]^+$.

**Step 2: preparation of N$^1$-(5-chloro-2-fluorophenyl)-6-methylisoquinoline-1,5-diamine (compound 73c)**

Compound 73b (298 mg, 898.33 μmol), iron powder (250.86 mg, 4.49 mmol) and $NH_4Cl$ (144.15 mg, 2.69 mmol) were added to water (4 mL) and methanol (10 mL), and the reaction was stirred at 90°C for 4hr. After the reaction was complete, the mixture was directly purified by Flash column chromatography on silica gel (DCM:MeOH=90:10), to afford compound 73c (230 mg). MS m/z (ESI): 302.1 $[M+H]^+$.

**Step 3: preparation of N-(1-((5-chloro-2-fluorophenyl)amino)-6-methylisoquinolin-5-yl)-4-((2,4-dimethoxybenzyl)amino)quinazoline-8-carboxamide (compound 73d)**

Compounds 73c (44.46 mg, 147.34 μmol) and 1f (50 mg, 147.34 μmol) were dissolved in pyridine (2 mL), $T_3P$ (0.5 mL, a 50% solution in EA) was then added, and the reaction was stirred at 25°C for 16hr. After the reaction was complete, the solvent was removed under reduced pressure, the reaction was quenched by dropwise addition of a saturated solution of sodium bicarbonate, and extracted with EA. The organic phase was washed with a saturated saline solution, dried over anhydrous sodium sulfate, filtered, concentrated, and the crude product was purified by Flash column chromatography on silica gel (DCM:MeOH=92:8), to afford compound 73d (80 mg). MS m/z (ESI): 623.1 $[M+H]^+$.

**Step 4: preparation of 4-amino-N-(1-((5-chloro-2-fluorophenyl)amino)-6-methylisoquinolin-5-yl)quinazoline-8-carboxamide (compound 325)**

Compound 73d (80 mg, 128.40 μmol) was dissolved in TFA (4 mL), and the reaction was stirred at 90°C for 3hr. After the reaction was complete, the reaction was evaporated under reduced pressure to remove TFA, and separated

and purified through Prep-HPLC, to afford a trifluoroacetate salt of compound 73 (23.16 mg). MS m/z (ESI): 473.1 $[M+H]^+$.

$^1$H NMR (400 MHz, DMSO-$d_6$) δ 11.99 (br, 1H), 10.17 (br, 1H), 9.34 (br, 2H), 8.77 (d, *J* = 7.2 Hz, 1H), 8.70 (s, 1H), 8.65 (d, *J* = 8.4Hz, 1H), 8.51 (d, *J* = 8.4 Hz, 1H), 7.96 - 7.71 (m, 4H), 7.52 - 7.33 (m, 3H), 2.51 (s, 3H).

**Example 64: 4-amino-N-(1-((4-ethoxy-2-fluorophenyl)amino)-6-methylisoquinolin-5-yl)quinazoline-8-carboxamide (compound 74)**

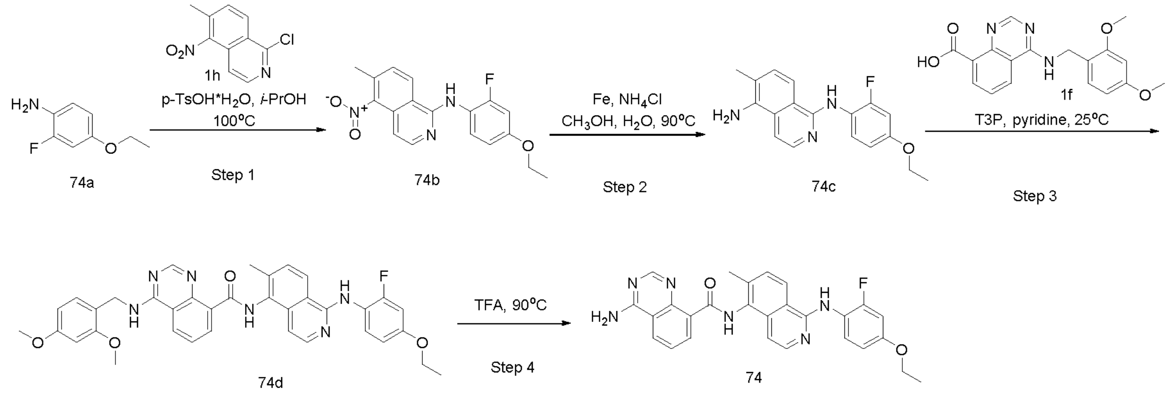


**Step 1: preparation of N-(4-ethoxy-2-fluorophenyl)-6-methyl-5-nitroisoquinolin-1-amine (compound 74b)**

Compounds 74a (125.46 mg, 808.53 μmol) and 1h (150 mg, 673.77 μmol) were dissolved in isopropanol (10 mL), p-toluenesulfonic acid monohydrate (51.27 mg, 269.51 μmol) was then added, and the reaction was stirred at 100°C for 18hr. After the reaction was complete, the reaction solution was concentrated to dryness, to afford compound 74b (229 mg). MS m/z (ESI): 342.1 $[M+H]^+$.

**Step 2: preparation of N$^1$-(4-ethoxy-2-fluorophenyl)-6-methylisoquinoline-1,5-diamine (compound 74c)**

Compound 74b (229 mg, 670.89 μmol), iron powder (187.35 mg, 3.35 mmol) and $NH_4Cl$ (107.66 mg, 2.01 mmol) were added to methanol (10 mL) and water (3 mL), and the reaction was stirred at 90°C for 4hr. After the reaction was complete, the mixture was directly purified by Flash column chromatography on silica gel (DCM:MeOH=90:10), to afford compound 74c (180 mg). MS m/z (ESI): 312.1 $[M+H]^+$.

**Step 3: preparation of 4-((2,4-dimethoxybenzyl)amino)-N-(1-((4-ethoxy-2-fluorophenyl)amino)-6-methylisoquinolin-5-yl)quinazoline-8-carboxamide (compound 74d)**

Compounds 74c (45.88 mg, 147.34 μmol) and 1f (50 mg, 147.34 μmol) were dissolved in pyridine (2 mL), $T_3P$ (0.5 mL, a 50% solution in EA) was then added, and the reaction was stirred at 25°C for 16hr. After the reaction was complete, the solvent was removed under reduced pressure, the reaction was quenched by dropwise addition of a saturated solution of sodium bicarbonate, and extracted with EA. The organic phase was washed with a saturated saline solution, dried over anhydrous sodium sulfate, filtered, concentrated, and then purified by Flash column chromatography on silica gel (DCM:MeOH=92:8), to afford compound 74d (80 mg). MS m/z (ESI): 633.3 $[M+H]^+$.

**Step 4: preparation of 4-amino-N-(1-((4-ethoxy-2-fluorophenyl)amino)-6-methylisoquinolin-5-yl)quinazoline-8-carboxamide (compound 74)**

Compound 74d (80 mg, 126.45 μmol) was dissolved in TFA (4 mL), and the reaction was stirred at 90°C for 3hr. After the reaction was complete, the reaction was evaporated under reduced pressure to remove TFA, adjusted to a pH of about 8 with a saturated solution of sodium bicarbonate, and extracted with ethyl acetate. The organic phase was washed with a saturated saline solution, dried over anhydrous sodium sulfate, filtered, concentrated, and the crude product was purified by Flash column chromatography on silica gel (DCM:MeOH=95:5), to afford compound 74 (11.29 mg). MS m/z (ESI): 483.0 $[M+H]^+$.

$^1$H NMR (400 MHz, DMSO-$d_6$) δ 13.18 (s, 1H), 8.92 (s, 1H), 8.67 (dd, *J* = 7.6, 1.2 Hz, 1H), 8.63 (s, 1H), 8.54 (dd, *J* = 8.4 Hz, 1.2 Hz, 1H), 8.41 (br, 1H), 8.35 (d, *J* = 8.4Hz, 1H), 8.24 (br, 1H), 7.81 (d, *J* = 6.0 Hz, 1H), 7.70 (t, *J* =

8.0Hz, 1H), 7.58 (d, *J* = 8.8 Hz, 1H), 7.39 (t, *J* = 8.8 Hz, 1H), 7.14 (d, *J* = 6.0 Hz, 1H), 6.91 (dd, *J* = 12.4, 2.8 Hz, 1H), 6.80 (dd, *J* = 8.4, 2.4 Hz, 1H), 4.07 (q, *J* = 6.8Hz, 2H), 2.44 (s, 3H), 1.36 (t, *J* = 7.0Hz, 3H).

**Example 65: 4-amino-N-(1-((6-methoxypyridin-3-yl)amino)-6-methylisoquinolin-5-yl)quinazoline-8-carboxamide (compound 75)**

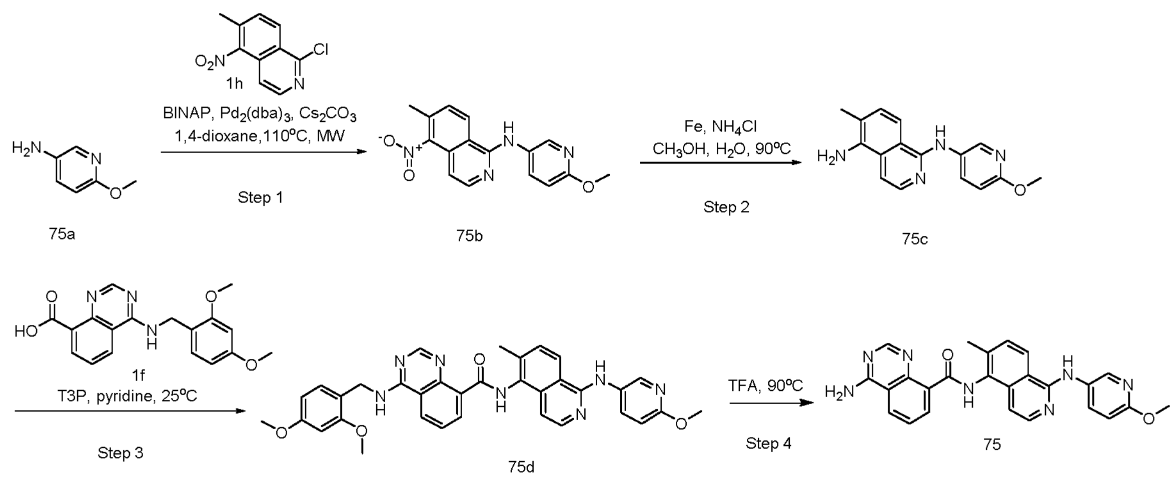


**Step 1: preparation of N-(6-methoxypyridin-3-yl)-6-methyl-5-nitroisoquinolin-1-amine (compound 75b)**

Compounds 75a (134.5 mg, 1.08 mmol), 1h (201 mg, 902.85 μmol), $Pd_2(dba)_3$ (82.68 mg, 90.29 μmol), BINAP (112.44 mg, 180.57 μmol) and $Cs_2CO_3$ (882.50 mg, 2.71 mmol) were added to 1,4-dioxane (5 mL), purge with nitrogen was performed for three times, and the reaction was performed under microwave at 110°C for 5 hours. After the reaction was complete, the reaction solution was cooled to room temperature, filtered through diatomaceous earth, diluted by adding water, and extracted with EA. The organic phase was washed with a saturated saline solution, dried over anhydrous sodium sulfate, filtered, concentrated, and then purified by Flash column chromatography on silica gel (DCM:MeOH=30:1), to afford compound 75b (115 mg). MS m/z (ESI): 311.0 $[M+H]^+$.

**Step 2: preparation of N$^1$-(6-methoxypyridin-3-yl)-6-methylisoquinoline-1,5-diamine (compound 75c)**

Compound 75b (100 mg, 1.86 mmol), iron powder (89.99 mg, 1.61mmol) and ammonium chloride (43.09 mg, 805.65 umol) were added to MeOH (8 mL) and $H_2O$ (2 mL), and the reaction was allowed to proceed at 90°C for 12 hours. After the reaction was complete, the mixture was directly purified by Flash column chromatography on silica gel (DCM/MeOH=10/1), to afford compound 75c (50.00 mg). MS m/z (ESI): 281.1 $[M+H]^+$.

**Step 3: preparation of 4-((2,4-dimethoxybenzyl)amino)-N-(1-((6-methoxypyridin-3-yl)amino)-6-methylisoquinolin-5-yl)quinazoline-8-carboxamide (compound 75d)**

Compounds 75c (31 mg, 110.59 μmol) and 1f (37.53 mg, 110.59 μmol) were dissolved in pyridine (3 mL), 1-propylphosphonic anhydride (0.2 mL, a 50% solution in EA) was dropwise added, and the reaction was allowed to proceed at 25°C for 3 hours. After the reaction was complete, the solvent was removed under reduced pressure, the residue was added with a saturated aqueous solution of sodium bicarbonate, and extracted with ethyl acetate. The organic phase was dried over anhydrous sodium sulfate, filtered, concentrated, and then purified by thin layer chromatography on silica gel (DCM:MeOH=20:1), to afford compound 75d (35.00 mg). MS m/z (ESI): 602.3 $[M+H]^+$.

**Step 4: preparation of 4-amino-N-(1-((6-methoxypyridin-3-yl)amino)-6-methylisoquinolin-5-yl)quinazoline-8-carboxamide (compound 75)**

Compound 75d (31.00 mg, 51.52 μmol) was dissolved in trifluoroacetic acid (5 mL), and the reaction was warmed to 90°C and allowed to proceed for 12 hours. After the reaction was complete, the solvent was removed under reduced pressure, and the residue was then purified by Prep-HPLC, to afford compound 75 (15.00 mg). MS m/z (ESI): 452.1 $[M+H]^+$.

$^1$H NMR (400 MHz, DMSO-$d_6$) δ 13.20 (s, 1H), 9.23 (s, 1H), 8.67 (dd, *J* = 7.6, 1.2 Hz, 1H), 8.64 (s, 1H), 8.55 (d, *J* = 2.4, 1H), 8.54 (d, *J* = 8.4, 1H) 8.42 (d, *J* = 12.4Hz, 2H), 8.28 (s, 1H), 8.15 (dd, *J* = 8.8, 2.8 Hz, 1H), 7.93 (d, *J* = 6.0 Hz, 1H), 7.71 (t, *J* = 8.0 Hz, 1H), 7.62 (d, *J* = 8.8 Hz, 1H), 7.22 (d, *J* = 5.6 Hz, 1H), 6.85 (d, *J* = 9.2 Hz, 1H), 3.86 (s, 3H), 2.45 (s, 3H).

**Example 66: 4-amino-N-(1-((3-chloro-2-fluorophenyl)amino)-6-methylisoquinolin-5-yl)-6-hydroxyquinazoline-8-carboxamide (compound 76)**

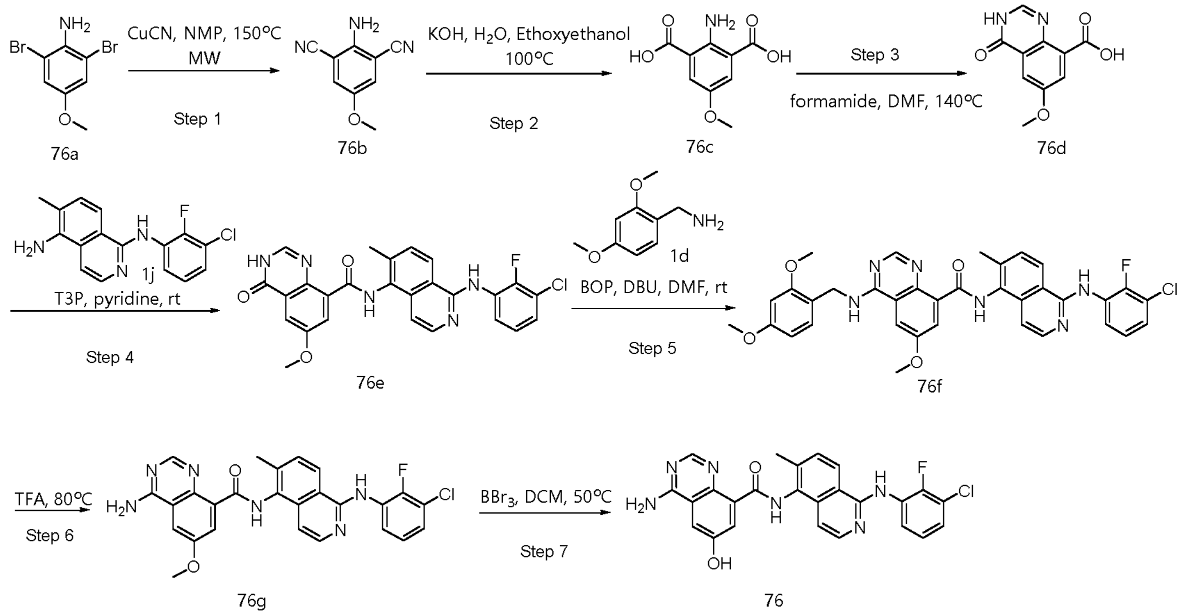


**Step 1: preparation of 2-amino-5-methoxyisophthalonitrile (compound 76b)**

Compound 76a (500 mg, 1.78 mmol) and CuCN (666 mg, 7.12 mmol) were added to NMP (5 mL), and the reaction was performed under microwave at 150°C for 3hr. After the reaction was complete, the reaction solution was diluted by EA, poured into a 50 mL aqueous solution of 10% ethylenediamine, vigorously stirred for 10 min, and filtered through diatomaceous earth. The filtrate was extracted with EA, the organic phase was washed with a saturated saline solution, dried over anhydrous sodium sulfate, filtered, concentrated, and then purified by Flash column chromatography on silica gel (mobile phase: 100% DCM), to afford compound 76b (210 mg). MS m/z (ESI): 174.0 [M+H]$^+$.

**Step 2: preparation of 2-amino-5-methoxyisophthalic acid (compound 76c)**

Compound 76b (210 mg, 1.21 mmol) was added to Ethoxyethanol (3 mL), a solution of KOH (408 mg, 7.28 mmol) in water (8 mL) was then added, and the reaction was allowed to proceed under the protection of nitrogen at 100°C for 16hr. After the reaction was complete, the pH was adjusted to 5-6 with 3M dilute hydrochloric acid, the precipitated solid was filtered, the filter cake was sequentially rinsed with water and MTBE, and dried under reduced pressure to afford compound 76c (210 mg). MS m/z (ESI): 211.9 [M+H]$^+$.

**Step 3: preparation of 6-methoxy-4-oxo-3,4-dihydroquinazoline-8-carboxylic acid (compound 76d)**

Compound 76c (210 mg, 994.45 μmol) and formamide (5 mL) were added to a reaction flask, and the reaction was performed under the protection of nitrogen at 140°C for 5hr. After the reaction was complete, the reaction was diluted by adding water, adjusted to a pH of 5-6 with 3M dilute hydrochloric acid, and extracted with EA. The organic phase was washed with a saturated saline solution, dried over anhydrous sodium sulfate, filtered, and concentrated, to afford compound 76d (150mg). MS m/z (ESI): 221.0 [M+H]$^+$.

**Step 4: preparation of N-(1-((3-chloro-2-fluorophenyl)amino)-6-methylisoquinolin-5-yl)-6-methoxy-4-oxo-3,4-dihydroquinazoline-8-carboxamide (compound 76e)**

Compounds 1j (205 mg, 681.26 μmol) and 76d (150 mg, 681.26 μmol) were dissolved in pyridine (9 mL), $T_3P$ (6 mL, a 50% solution in EA) was then added, and the reaction was allowed to proceed under the protection of nitrogen at room temperature overnight. After the reaction was complete, the reaction solution was directly concentrated to dryness, diluted by adding water, adjusted to a pH of 7-8 with a saturated solution of $NaHCO_3$, and then extracted with EA. The organic phase was washed with a saturated saline solution, dried over anhydrous sodium sulfate, filtered, and concentrated, to afford compound 76e (320mg). MS m/z (ESI): 504.1 $[M+H]^+$.

**Step 5: preparation of N-(1-((3-chloro-2-fluorophenyl)amino)-6-methylisoquinolin-5-yl)-4-((2,4-dimethoxybenzyl)amino)-6-methoxyquinazoline-8-carboxamide (compound 76f)**

Compound 76e (300 mg, 595.34 μmol) and BOP (395 mg, 893.01 μmol) were dissolved in DMF (15 mL), DBU (450 mg, 1.79 mmol) was dropwise added at room temperature, and then the reaction was stirred for 10 min. Compound 1d (149 mg, 893.01 μmol) was then added, and the reaction was stirred at room temperature for 14hr. After the reaction was complete, the reaction was diluted by adding water, and extracted with EA. The organic phase was washed with a saturated saline solution, dried over anhydrous sodium sulfate, filtered, concentrated, and the crude product was purified by Flash column chromatography on silica gel (mobile phase: 100% DCM), to afford compound 76f (350mg). MS m/z (ESI): 653.3 $[M+H]^+$.

**Step 6: preparation of 4-amino-N-(1-((3-chloro-2-fluorophenyl)amino)-6-methylisoquinolin-5-yl)-6-methoxyquinazoline-8-carboxamide (compound 76g)**

Compound 76f (350 mg, 535.91 μmol) and TFA (10 mL) were added to a reaction flask, and the reaction was allowed to proceed under the protection of nitrogen at 80°C for 3hr. After the reaction was complete, the reaction solution was directly concentrated to dryness, diluted by adding water, adjusted to a pH of 7-8 with a saturated solution of $NaHCO_3$, and extracted with EA. The organic phase was washed with a saturated saline solution, dried over anhydrous sodium sulfate, filtered, and concentrated, to afford compound 76g (250 mg). MS m/z (ESI): 503.2 $[M+H]^+$.

**Step 7: preparation of 4-amino-N-(1-((3-chloro-2-fluorophenyl)amino)-6-methylisoquinolin-5-yl)-6-hydroxyquinazoline-8-carboxamide (compound 76)**

Compound 76g (250 mg, 497.09 μmol) was dissolved in DCM (6.5 mL), $BBr_3$ (2 M, 2.49 mL) was then added, and the reaction was allowed to proceed under the protection of nitrogen at 50°C for 120hr. After the reaction was complete, the reaction was quenched by methanol, adjusted to a pH of 6-7 by adding a saturated solution of $NaHCO_3$, a large amount of solid precipitated, which was filtered. The filter cake was washed with water and MTBE, and dried in vacuum, to afford a crude product 270 mg. 20 mg of the crude product was purified through Pre-HPLC, to afford compound 76 (4.78 mg). MS m/z (ESI): 489.0 $[M+H]^+$.

$^1H$ NMR (400 MHz, DMSO-$d_6$) δ 13.36 (s, 1H), 10.28 (s, 1H), 9.26 (s, 1H), 8.47 (s, 1H), 8.35 (d, *J* = 8.8 Hz, 1H), 8.29 (d, *J* = 2.8 Hz, 1H), 8.02 (br, 2H), 7.91 (d, *J* = 6.0 Hz, 1H), 7.79 (d, *J* = 2.8 Hz, 1H), 7.63 (d, *J* = 8.8 Hz, 1H), 7.59 - 7.54 (m, 1H), 7.40 - 7.34 (m, 1H), 7.28 - 7.21 (m, 2H), 2.45 (s, 3H).

**Example 67: 4-amino-N-(1-((2,3-dihydrobenzo[b][1,4]dioxin-6-yl)amino)-6-methylisoquinolin-5-yl)quinazoline-8-carboxamide (compound 77)**

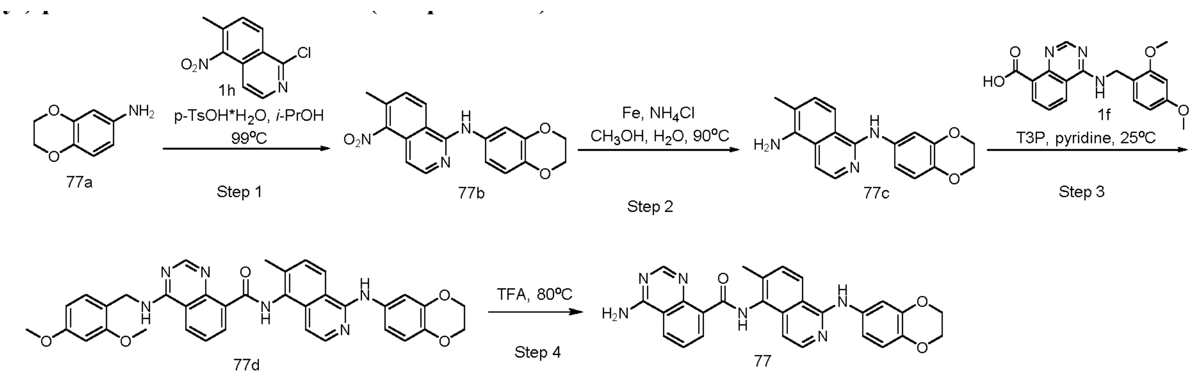


**Step 1: preparation of N-(2,3-dihydrobenzo[b][1,4]dioxin-6-yl)-6-methyl-5-nitroisoquinolin-1-amine (compound 77b)**

Compounds 77a (162.96 mg, 1.08 mmol) and 1h (200 mg, 898.36 umol) were dissolved in isopropanol (10 mL), p-toluenesulfonic acid monohydrate (15.47 mg, 89.84 umol) was then added, and the reaction was allowed to proceed at 99°C for 12 hours. After the reaction was complete, the mixture was filtered, and the solid was collected, to afford compound 77b (285.00 mg). MS m/z (ESI): 338.0 $[M+H]^+$.

**Step 2: preparation of $N^1$-(2,3-dihydrobenzo[b][1,4]dioxin-6-yl)-6-methylisoquinoline-1,5-diamine (compound 77c)**

Compound 77b (205.00 mg, 607.72 umol), iron powder (169.70 mg, 3.04 mmol) and ammonium chloride (81.27 mg, 1.52 mmol) were added to MeOH (10 mL) and $H_2O$ (2 mL), and the reaction was allowed to proceed at 90°C for 12 hours. After the reaction was complete, the reaction solution was concentrated to dryness, and purified by Flash column chromatography on silica gel (DCM:MeOH=10:1), to afford compound 77c (132.00 mg). MS m/z (ESI): 308.1 $[M+H]^+$.

**Step 3: preparation of N-(1-((2,3-dihydrobenzo[b][1,4]dioxin-6-yl)amino)-6-methylisoquinolin-5-yl)-4-((2,4-dimethoxybenzyl)amino)quinazoline-8-carboxamide (compound 77d)**

Compounds 77c (45.29 mg, 147.34 μmol) and 1f (50 mg, 147.34 μmol) were dissolved in pyridine (3 mL), 1-propylphosphonic anhydride (0.28 mL, a 50% solution in EA) was then dropwise added, and the reaction was allowed to proceed at 25°C for 3 hours. After the reaction was complete, the solvent was removed under reduced pressure, a saturated aqueous solution of sodium bicarbonate was added, and the mixture was extracted with ethyl acetate. The organic phase was dried over anhydrous sodium sulfate, filtered, concentrated, and then purified by thin layer chromatography on silica gel (DCM:MeOH=15:1), to afford compound 77d (20.00 mg). MS m/z (ESI): 629.3 $[M+H]^+$.

**Step 4: preparation of 4-amino-N-(1-((2,3-dihydrobenzo[b][1,4]dioxin-6-yl)amino)-6-methylisoquinolin-5-yl)quinazoline-8-carboxamide (compound 77)**

Compound 77d (20.00 mg, 31.81 μmol) was dissolved in trifluoroacetic acid (2 mL), the reaction was warmed to 80°C and allowed to proceed for 3 hours. After the reaction was complete, the solvent was removed under reduced pressure, and then the residue was purified by Prep-HPLC, to afford compound 77 (12.00 mg). MS m/z (ESI): 479.1 $[M+H]^+$.

$^1H$ NMR (400 MHz, DMSO-$d_6$) δ 13.19 (s, 1H), 9.04 (s, 1H), 8.67 (dd, *J* = 7.2, 1.2 Hz, 1H), 8.64 (s, 1H), 8.54 (dd, *J* = 8.4, 1.6 Hz, 1H), 8.48-8.15 (m, 3H), 7.95 (d, *J* = 6.0 Hz, 1H), 7.71 (t, *J* = 7.8 Hz, 1H), 7.59 (d, *J* = 8.8 Hz, 1H), 7.55 (d, *J* = 2.4 Hz, 1H), 7.29 (dd, *J* = 8.8, 2.4 Hz, 1H), 7.19 (d, *J* = 6.0 Hz, 1H), 6.82 (d, *J* = 8.8 Hz, 1H), 4.29-4.21 (m, 4H), 2.44 (s, 3H).

**Example 68: 4-amino-N-(1-((4-cyano-2-fluorophenyl)amino)-6-methylisoquinolin-5-yl)quinazoline-8-carboxamide (compound 78)**

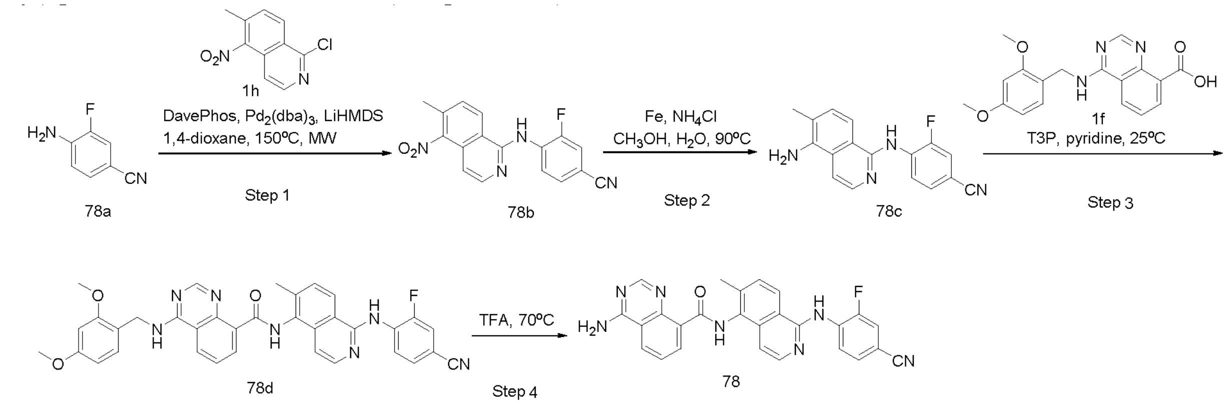


**Step 1: preparation of 3-fluoro-4-((6-methyl-5-nitroisoquinolin-1-yl)amino)benzonitrile (compound 78b)**

Compounds 1h (100.00 mg, 449.18 μmol), 78a (64.20 mg, 471.64 μmol), 2-dicyclohexylphosphino-2'-(N,N-dimethylamino)-biphenyl (7.07 mg, 17.97 μmol), tris(dibenzylideneacetone) dipalladium (8.23 mg, 8.98 μmol) and LiHMDS (898.36 μL) were added to dry 1,4-dioxane (4 mL), and under the protection of nitrogen, the reaction was performed under microwave at 150°C for 1.5 hours. After the reaction was complete, the reaction solution was cooled to room temperature, diluted with ethyl acetate, washed with water, the organic phase was dried over anhydrous sodium sulfate, filtered, concentrated, and then purified by Flash column chromatography on silica gel (PE/EA=3/1), to afford compound 78b (153 mg). MS m/z (ESI): 323.0 $[M+H]^+$.

**Step 2: preparation of 4-((5-amino-6-methylisoquinolin-1-yl)amino)-3-fluorobenzonitrile (compound 78c)**

Compound 78c (46 mg) was prepared according to the method for preparing compound 73c in Step 2 of Example 63, except that compound 73b was replaced with compound 78b. MS m/z (ESI): 293.0 $[M+H]^+$.

**Step 3: preparation of N-(1-((4-cyano-2-fluorophenyl)amino)-6-methylisoquinolin-5-yl)-4-((2,4-dimethoxybenzyl)amino)quinazoline-8-carboxamide (compound 78d)**

Compound 78d (33 mg) was prepared according to the method for preparing compound 73d in Step 3 of Example 63, except that compound 73c was replaced with compound 78c. MS m/z (ESI): 614.0 $[M+H]^+$.

**Step 4: preparation of 4-amino-N-(1-((4-cyano-2-fluorophenyl)amino)-6-methylisoquinolin-5-yl)quinazoline-8-carboxamide (compound 78)**

Compound 78 (2 mg) was prepared according to the method for preparing compound 1 in Step 8 of Example 1, except that compound 1k was replaced with compound 78d. MS m/z (ESI): 464.2 $[M+H]^+$.

$^1$HNMR (400 MHz, DMSO-$d_6$) δ 13.24 (s, 1H), 9.46 (s, 1H), 8.66 (d, *J* = 6.8 Hz, 1H), 8.64 (s, 1H), 8.54 (d, *J* = 6.8 Hz, 1H), 8.41 (brs, 1H), 8.34 (d, *J* = 8.4 Hz, 1H), 8.24 (brs, 1H), 8.03 (d, *J* = 6.0 Hz, 1H), 7.97 (t, *J* = 8.0 Hz, 1H), 7.88 (dd, *J* = 11.2, 1.6 Hz, 1H), 7.74-7.65 (m, 3H), 7.41 (d, *J* = 6.0 Hz, 1H), 2.47 (s, 3H).

**Example 69: 4-amino-N-(1-((3-chloro-2-fluorophenyl)amino)-6-methylisoquinolin-5-yl)-6-(2-morpholinoethoxy)quinazoline-8-carboxamide (compound 79)**

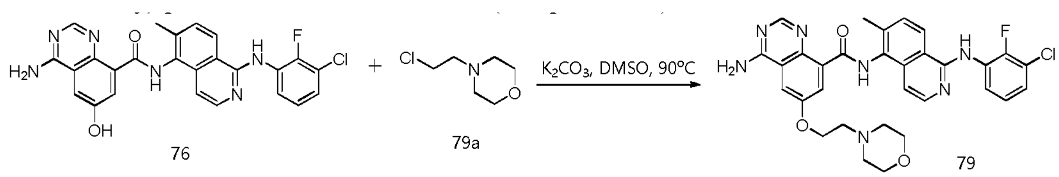


Compound 76 (10 mg, 20.45 μmol), a hydrochloride salt of 79a (4 mg, 22.50 μmol), $K_2CO_3$ (7 mg, 51.14 μmol) and DMSO (2 mL) were added to a reaction flask, and the reaction was allowed to proceed under the protection of

nitrogen at 90°C for 3hr. After the reaction was complete, the reaction was diluted by adding water, and extracted with EA. The organic phase was washed with a saturated saline solution, dried over anhydrous sodium sulfate, filtered, concentrated, and the crude product was purified through Pre-HPLC, to afford compound 79 (3.5 mg). MS m/z (ESI): 602.0 [M+H]$^+$.

$^1$H NMR (400 MHz, DMSO-$d_6$) $\delta$ 13.24 (s, 1H), 9.23 (s, 1H), 8.51 (s, 1H), 8.34 (d, *J* = 8.4 Hz, 1H), 8.22 (d, *J* = 2.8 Hz, 1H), 8.11 (br, 2H), 7.99 (d, *J* = 3.2 Hz, 1H), 7.90 (d, *J* = 6.0 Hz, 1H), 7.62 (d, *J* = 8.8 Hz, 1H), 7.59 - 7.53 (m, 1H), 7.39 - 7.32 (m, 1H), 7.26 - 7.20 (m, 2H), 4.28 (t, *J* = 6.0 Hz, 2H), 3.62 - 3.57 (m, 4H), 2.80 (t, *J* = 6.0 Hz, 2H), 2.55 - 2.52 (m, 4H), 2.44 (s, 3H).

**Example 70: 4-amino-N-(1-((5-cyano-2-fluorophenyl)amino)-6-methylisoquinolin-5-yl)quinazoline-8-carboxamide (compound 80)**

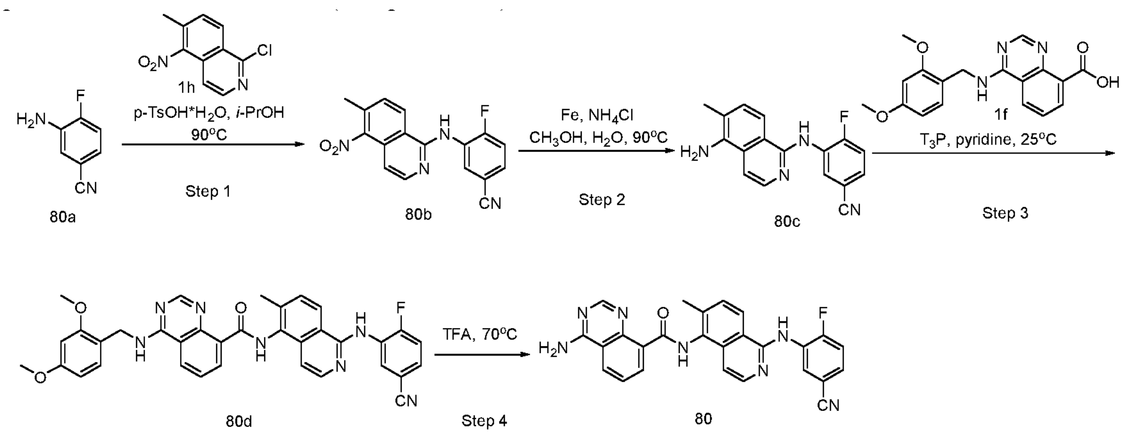


**Step 1: preparation of 4-fluoro-3-((6-methyl-5-nitroisoquinolin-1-yl)amino)benzonitrile (compound 80b)**

Compound 80b (33 mg) was prepared according to the method for preparing compound 73b in Step 1 of Example 63, except that compound 73a was replaced with compound 80a, and the reaction temperature was changed to 90°C. MS m/z (ESI): 323.0 [M+H]$^+$.

**Step 2: preparation of 3-((5-amino-6-methylisoquinolin-1-yl)amino)-4-fluorobenzonitrile (compound 80c)**

Compound 80c (26 mg) was prepared according to the method for preparing compound 73c in Step 2 of Example 63, except that compound 73b was replaced with compound 80b. MS m/z (ESI): 293.1 [M+H]$^+$.

**Step 3: preparation of N-(1-((5-cyano-2-fluorophenyl)amino)-6-methylisoquinolin-5-yl)-4-((2,4-dimethoxybenzyl)amino)quinazoline-8-carboxamide (compound 80d)**

Compound 80d (22 mg) was prepared according to the method for preparing compound 73d in Step 3 of Example 63, except that compound 73c was replaced with compound 80c. MS m/z (ESI): 614.1 [M+H]$^+$.

**Step 4: preparation of 4-amino-N-(1-((5-cyano-2-fluorophenyl)amino)-6-methylisoquinolin-5-yl)quinazoline-8-carboxamide (compound 80)**

Compound 80 (12 mg) was prepared according to the method for preparing compound 1 in Step 8 of Example 1, except that compound 1k was replaced with compound 80d. MS m/z (ESI): 464.1 [M+H]$^+$.

$^1$H NMR (400 MHz, DMSO-$d_6$) $\delta$ 13.22 (s, 1H), 9.31 (s, 1H), 8.67 (dd, *J* = 7.2, 1.2 Hz, 1H), 8.64 (s, 1H), 8.39 (brs, 1H), 8.35 (d, *J* = 8.8 Hz, 1H), 8.25 (brs, 1H), 8.19 (dd, *J* = 7.2, 2.0 Hz, 2H), 7.96 (d, *J* = 6.0 Hz, 1H), 7.73-7.68 (m, 2H), 7.66 (d, *J* = 8.8 Hz, 1H), 7.56-7.51 (m, 1H), 7.32 (d, *J* = 6.0 Hz, 1H), 2.47 (s, 3H).

**Example 71: 4-amino-N-(1-((3-chloro-2-fluorophenyl)amino)-6-methylisoquinolin-5-yl)pyrido[3,2-d]pyrimidine-8-carboxamide (compound 81)**

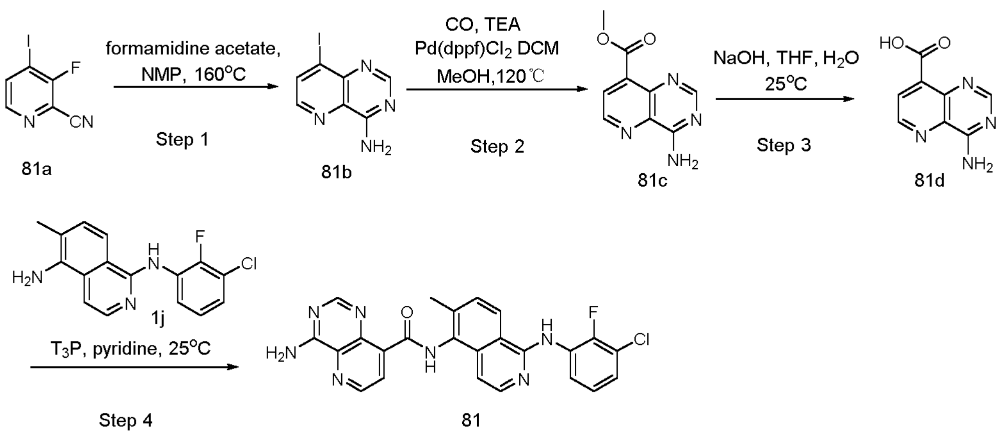


**Step 1: preparation of 8-iodopyrido[3,2-d]pyrimidin-4-amine (compound 81b)**

Compound 81a (250 mg, 1.01 mmol) and formamidine acetate (524.76 mg, 5.04 mmol) were dissolved in NMP (5 mL), purge with nitrogen was performed for three times, and the reaction was performed under microwave at 160°C for 0.5 hour. After the reaction was complete, the reaction solution was added with 100 ml water, and extracted with EA. The organic phase was washed with a saturated saline solution, dried over anhydrous sodium sulfate, filtered, and concentrated, to afford compound 81b (220 mg). MS m/z (ESI): 272.8 $[M+H]^+$.

**Step 2: preparation of methyl 4-aminopyrido[3,2-d]pyrimidine-8-carboxylate (compound 81c)**

Compound 81b (215 mg, 790.31 μmol), TEA (239.91 mg, 2.37 mmol), Pd(dppf)$Cl_2$ DCM (64.49 mg, 79.03 μmol) were dissolved in methanol (3 mL), and the reaction was allowed to proceed under an atmosphere of carbon monoxide at 120°C for 5 hours. After the reaction was complete, the reaction solution was cooled to room temperature, concentrated to dryness, and purified by Flash column chromatography on silica gel (DCM:MeOH=20:1), to afford compound 81c (60 mg). MS m/z (ESI): 205.2 $[M+H]^+$.

**Step 3: preparation of 4-aminopyrido[3,2-d]pyrimidine-8-carboxylic acid (compound 81d)**

Compound 81c (40 mg, 195.90 umol) was dissolved in THF (4 mL) and $H_2O$ (1 mL), sodium hydroxide (23.51 mg, 587.70 umol) was added, and the reaction was allowed to proceed at 25°C for 3 hours. After the reaction was complete, the pH was adjusted to about 4 with dilute hydrochloric acid, and the solvent was concentrated to dryness, to afford compound 81d (30.00 mg). MS m/z (ESI): 191.1 $[M+H]^+$.

**Step 4: preparation of 4-amino-N-(1-((3-chloro-2-fluorophenyl)amino)-6-methylisoquinolin-5-yl)pyrido[3,2-d]pyrimidine-8-carboxamide (compound 81)**

Compounds 81d (30 mg, 157.76 μmol) and 1j (47.60 mg, 157.76 μmol) were dissolved in pyridine (3 mL), 1-propylphosphonic anhydride (0.5 mL, a 50% solution in EA) was dropwise added, and the reaction was allowed to proceed at 25°C for 1 hour. After the reaction was complete, the solvent was removed under reduced pressure, the residue was added with a saturated aqueous solution of sodium bicarbonate, and extracted with ethyl acetate. The organic phase was dried over anhydrous sodium sulfate, filtered, concentrated, and then purified by Prep-HPLC, to afford compound 81 (20 mg). MS m/z (ESI): 474.0 $[M+H]^+$.

$^1H$ NMR (400 MHz, DMSO-$d_6$) δ 12.68 (s, 1H), 9.28 (s, 1H), 9.02 (d, *J* = 4.4 Hz, 1H), 8.64 (s, 1H), 8.51 (s, 1H), 8.46 (d, *J* = 4.4 Hz, 1H), 8.44 (s, 1H), 8.38 (d, *J* = 8.4 Hz, 1H), 7.93 (d, *J* = 6.0 Hz, 1H), 7.65 (d, *J* = 8.4 Hz, 1H), 7.60-7.54 (m, 1H), 7.42 - 7.35 (m, 1H), 7.31 (d, *J* = 6.0 Hz, 1H), 7.27-7.21 (m, 1H), 2.48 (s, 3H).

**Example 72: 4-amino-N-(6-methyl-1-((2-methyl-1,2,3,4-tetrahydroisoquinolin-7-yl)amino)isoquinolin-5-yl)quinazoline-8-carboxamide (compound 82)**

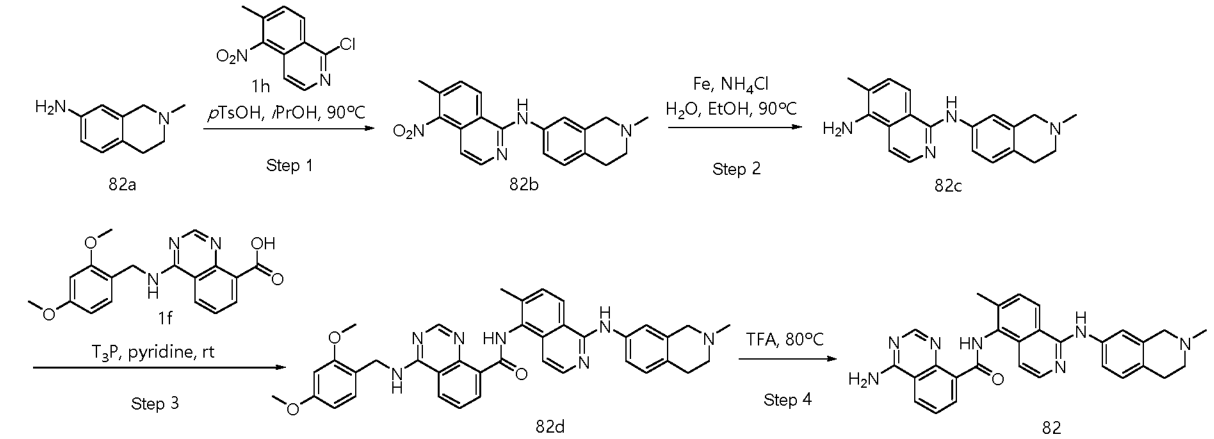


**Step 1: preparation of 6-methyl-N-(2-methyl-1,2,3,4-tetrahydroisoquinolin-7-yl)-5-nitroisoquinolin-1-amine (compound 82b)**

Compounds 82a (115 mg, 707.46 μmol), 1h (150 mg, 673.77 μmol) and p-toluenesulfonic acid (29 mg, 336.88 μmol) were added to isopropanol (5 mL), and under the protection of nitrogen, the reaction was performed under microwave at 90°C for 2.5 hr. After the reaction was complete, the reaction was diluted by adding water, and extracted with ethyl acetate. The organic phase was dried over anhydrous sodium sulfate, filtered, and concentrated, to afford compound 82b (230 mg). MS m/z (ESI): 349.2 $[M+H]^+$.

**Step 2: preparation of 6-methyl-$N^1$-(2-methyl-1,2,3,4-tetrahydroisoquinolin-7-yl)isoquinoline-1,5-diamine (compound 82c)**

Compound 82b (230 mg, 660.17 μmol) was dissolved in EtOH (10 mL), iron powder (368 mg, 6.60 mmol) and a solution of $NH_4Cl$ (88 mg, 1.65 mmol) in water (2.5 mL) were then added , and the reaction was refluxed at 90°C for 3 hr. After the reaction was complete, the reaction solution was filtered, the filtrate was diluted by adding water, and then extracted with EA. The organic phase was washed with a saturated saline solution, dried over anhydrous sodium sulfate, filtered and concentrated, to afford compound 82c (81 mg). MS m/z (ESI): 319.2 $[M+H]^+$.

**Step 3: preparation of 4-((2,4-dimethoxybenzyl)amino)-N-(6-methyl-1-((2-methyl-1,2,3,4-tetrahydroisoquinolin-7-yl)amino)isoquinolin-5-yl)quinazoline-8-carboxamide (compound 82d)**

Compounds 1f (70 mg, 206.28 μmol) and 82c (66 mg, 206.28 μmol) were added to pyridine (3 mL), $T_3P$ (2 mL, a 50% solution in EA) was then added, and then the reaction was allowed to proceed at room temperature for 14 hr. After the reaction was complete, the solvent was removed by concentration under reduced pressure, the residue was added with a saturated aqueous solution of sodium bicarbonate (5 mL), and extracted with ethyl acetate. The organic phase was dried over anhydrous sodium sulfate, filtered, concentrated, and then purified by Flash column chromatography on silica gel (mobile phase DCM:MeOH=96:4), to afford compound 82d (25 mg). MS m/z (ESI): 640.4 $[M+H]^+$.

**Step 4: preparation of 4-amino-N-(6-methyl-1-((2-methyl-1,2,3,4-tetrahydroisoquinolin-7-yl)amino)isoquinolin-5-yl)quinazoline-8-carboxamide (compound 82)**

Compound 82d (25 mg, 39.08 μmol) was added to TFA (3 mL), and the reaction was allowed to proceed under the protection of nitrogen at 80°C for 3 hr. After the reaction was complete, the solvent was removed under reduced pressure, then the residue was diluted by adding water, adjusted to a pH of 7-8 with a saturated solution of $NaHCO_3$, and extracted with EA. The organic phase was washed with a saturated saline solution, dried over anhydrous sodium sulfate, filtered, concentrated, and the crude product was purified through Pre-HPLC, to afford compound 82 (5 mg). MS m/z (ESI): 490.0 $[M+H]^+$.

$^1H$ NMR (400 MHz, DMSO-$d_6$) δ 13.18 (s, 1H), 9.12 (s, 1H), 8.66 (dd, *J* = 7.6, 1.6 Hz, 1H), 8.62 (s, 1H), 8.53

(dd, *J* = 8.0, 1.6 Hz, 1H), 8.46 - 8.21 (m, 3H), 7.96 (d, *J* = 6.0 Hz, 1H), 7.69 (t, *J* = 8.0 Hz, 1H), 7.66 - 7.61 (m, 2H), 7.59 (d, *J* = 8.4 Hz, 1H), 7.20 (d, *J* = 6.0 Hz, 1H), 7.08 (d, *J* = 8.4 Hz, 1H), 3.78 - 3.57 (m, 2H), 3.32 (s, 3H), 2.90 - 2.72 (m, 4H), 2.43 (s, 3H).

**Example 73: 4-amino-N-(1-((3-cyano-2-fluorophenyl)amino)-6-methylisoquinolin-5-yl)quinazoline-8-carboxamide (compound 83)**

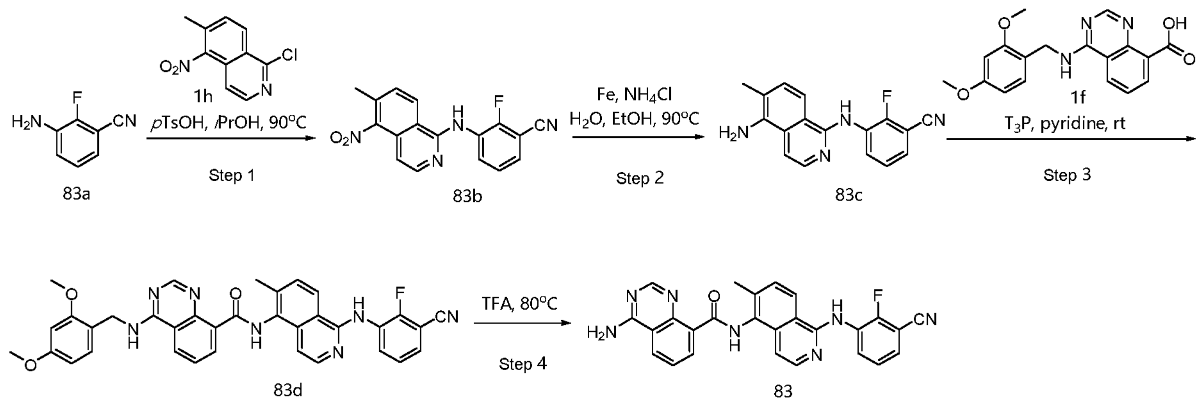


**Step 1: preparation of 2-fluoro-3-((6-methyl-5-nitroisoquinolin-1-yl)amino)benzonitrile (compound 83b)**

Compounds 1h (130 mg, 583.94 μmol), 83a (87 mg, 642.33 μmol) and p-toluenesulfonic acid (50 mg, 291.97 μmol) were added to isopropanol (5 mL), and the reaction was allowed to proceed at 90°C for 18 hr. After the reaction was complete, the reaction solution was cooled to room temperature, the solid was filtered with suction, the filter cake was washed with MTBE, dried under reduced pressure, to afford compound 83b (120 mg). MS m/z (ESI): 323.1 $[M+H]^+$.

**Step 2: preparation of 3-((5-amino-6-methylisoquinolin-1-yl)amino)-2-fluorobenzonitrile (compound 83c)**

Compound 83b (120 mg, 372.33 μmol) was dissolved in EtOH (8 mL), iron powder (208 mg, 3.72 mmol) and a solution of $NH_4Cl$ (50 mg, 930.83 μmol) in water (2.5 mL) were then added, and the reaction was stirred at 90°C for 3 hr. After the reaction was complete, the reaction solution was filtered, the filtrate was diluted by adding water, and then extracted with EA. The organic phase was washed with a saturated saline solution, dried over anhydrous sodium sulfate, filtered, and concentrated, to afford compound 83c (100 mg). MS m/z (ESI): 293.1 $[M+H]^+$.

**Step 3: preparation of N-(1-((3-cyano-2-fluorophenyl)amino)-6-methylisoquinolin-5-yl)-4-((2,4-dimethoxybenzyl)amino)quinazoline-8-carboxamide (compound 83d)**

Compound 1f (35 mg, 103.14 μmol) was added to pyridine (3 mL), compound 83c (30 mg, 103.14 μmol) and $T_3P$ (2 mL, a 50% solution in EA) were then sequentially added, and after the addition, the reaction was allowed to proceed at room temperature for 14 hr. After the reaction was complete, the solvent was removed by concentration under reduced pressure, the residue was added with a saturated aqueous solution of sodium bicarbonate (5 mL), and extracted with ethyl acetate. The organic phase was dried over anhydrous sodium sulfate, filtered, concentrated, and the crude product was purified by Flash column chromatography on silica gel (mobile phase DCM/MeOH=96/4), to afford compound 83d (40 mg). MS m/z (ESI): 614.4 $[M+H]^+$.

**Step 4: preparation of 4-amino-N-(1-((3-cyano-2-fluorophenyl)amino)-6-methylisoquinolin-5-yl)quinazoline-8-carboxamide (compound 83)**

Compound 83d (40 mg, 65.18 μmol) was added to TFA (3 mL), and the reaction was allowed to proceed under the protection of nitrogen at 80°C for 3 hr. After the reaction was complete, the solvent was removed by evaporation under reduced pressure, the residue was diluted by adding water, adjusted to a pH of 7-8 with a saturated solution of $NaHCO_3$, and then extracted with EA. The organic phase was washed with a saturated saline solution, dried over anhydrous sodium sulfate, filtered, concentrated, and the crude product was purified through Pre-HPLC, to afford compound 83 (8 mg). MS m/z (ESI): 464.2 $[M+H]^+$.

$^1$H NMR (400 MHz, DMSO-$d_6$) δ 13.21 (s, 1H), 9.38 (s, 1H), 8.65 (dd, $J$ = 7.6, 1.6 Hz, 1H), 8.62 (s, 1H), 8.53 (dd, $J$ = 8.0, 1.6 Hz, 1H), 8.41 - 8.18 (m, 3H), 7.97 - 7.91 (m, 2H), 7.72 - 7.62 (m, 3H), 7.42 (t, $J$ = 8.0 Hz, 1H), 7.30 (d, $J$ = 6.0 Hz, 1H), 2.45 (s, 3H).

**Example 74: 4-amino-N-(1-((5-chloro-2-fluorophenyl)amino)-6-methylisoquinolin-5-yl)-6-fluoroquinazoline-8-carboxamide (compound 84)**

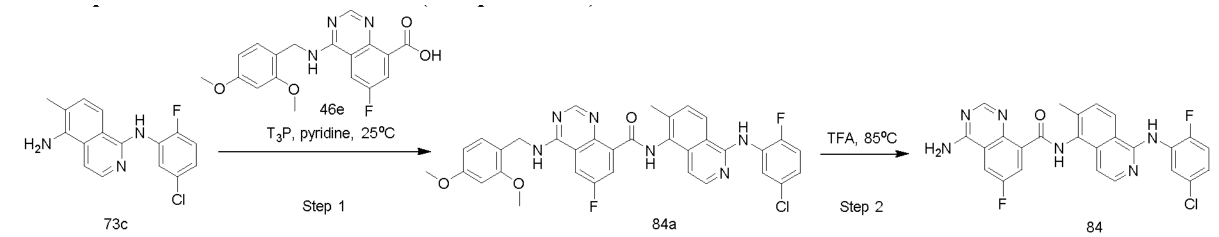


73c 84a 84

**Step 1: preparation of N-(1-((5-chloro-2-fluorophenyl)amino)-6-methylisoquinolin-5-yl)-4-((2,4-dimethoxybenzyl)amino)-6-fluoroquinazoline-8-carboxamide (compound 84a)**

Compounds 46e (30 mg, 83.95 μmol) and 73c (24.33 mg, 80.62 μmol) were dissolved in pyridine (2 mL), $T_3P$ (0.5 mL, a 50% solution in EA) was then added, and after the addition, the reaction was stirred at 25°C for 16 hr. After the reaction was complete, the solvent was removed under reduced pressure, the reaction was quenched by dropwise addition of a saturated solution of sodium bicarbonate, and extracted with EA. The organic phase was washed with a saturated saline solution, dried over anhydrous sodium sulfate, filtered, and concentrated, to afford compound 84a (50 mg). MS m/z (ESI): 641.2 $[M+H]^+$.

**Step 2: preparation of 4-amino-N-(1-((5-chloro-2-fluorophenyl)amino)-6-methylisoquinolin-5-yl)-6-fluoroquinazoline-8-carboxamide (compound 84)**

Compound 84a (50 mg, 78.00 μmol) was dissolved in TFA (3 mL), and the reaction was stirred at 85°C for 16 hr. After the reaction was complete, the solvent was removed under reduced pressure, and the crude product was separated and purified by Prep-HPLC, to afford a trifluoroacetate salt of compound 84 (22 mg). MS m/z (ESI): 491.2 $[M+H]^+$.

$^1$H NMR (400 MHz, DMSO-$d_6$) δ 12.70 (s, 1H), 10.14 (s, 1H), 8.74 (br, 1H), 8.65 (s, 1H), 8.55 - 8.42 (m, 3H), 7.87 - 7.76 (m, 3H), 7.54 - 7.39 (m, 3H), 2.51 (s, 3H).

**Example 75: 4-amino-N-(1-((2,4-difluorophenyl)amino)-6-methylisoquinolin-5-yl)-6-fluoroquinazoline-8-carboxamide (compound 85)**

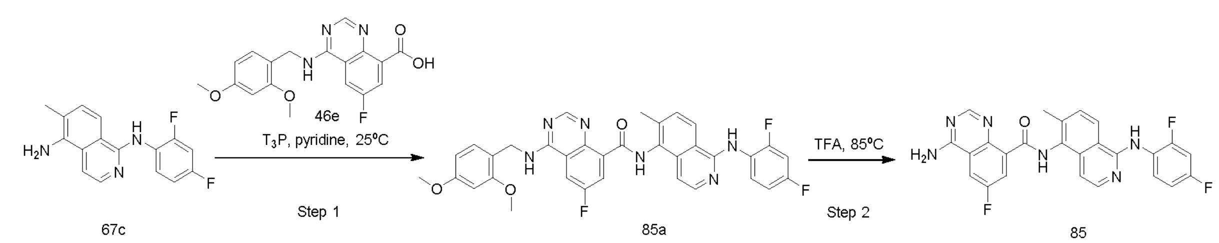


67c 85a 85

**Step 1: preparation of N-(1-((2,4-difluorophenyl)amino)-6-methylisoquinolin-5-yl)-4-((2,4-dimethoxybenzyl)amino)-6-fluoroquinazoline-8-carboxamide (compound 85a)**

Compounds 46e (28.81 mg, 80.62 μmol) and 67c (23 mg, 80.62 μmol) were dissolved in pyridine (2 mL), $T_3P$ (0.5 mL, a 50% solution in EA) was then added, and after the addition, the reaction was stirred at 25°C for 16 hr. After the reaction was complete, the solvent was removed under reduced pressure, the reaction was quenched by dropwise

addition of a saturated solution of sodium bicarbonate, and extracted with EA. The organic phase was washed with a saturated saline solution, dried over anhydrous sodium sulfate, filtered, and concentrated, to afford compound 85a (50 mg). MS m/z (ESI): 625.3 $[M+H]^+$.

**Step 2: preparation of 4-amino-N-(1-((2,4-difluorophenyl)amino)-6-methylisoquinolin-5-yl)-6-fluoroquinazoline-8-carboxamide (compound 85)**

Compound 85a (50 mg, 80.05 μmol) was dissolved in TFA (3 mL), and the reaction was stirred at 85°C for 16 hr. After the reaction was complete, the solvent was removed under reduced pressure, and the crude product was separated and purified by Prep-HPLC, to afford a trifluoroacetate salt of compound 85 (27 mg). MS m/z (ESI): 475.2 $[M+H]^+$.

$^1$H NMR (400 MHz, DMSO-$d_6$) δ 12.85 (s, 1H), 10.70 (s, 1H), 8.65 (s, 1H), 8.63 (br, 2H), 8.58 (d, *J* = 8.8 Hz, 1H), 8.45 (d, *J* = 9.2Hz, 2H), 7.87 (d, *J* = 8.4 Hz, 1H), 7.79-7.66 (m, 2H), 7.59 (t, *J* = 8.4 Hz, 1H), 7.44 (d, *J* = 6.8 Hz, 1H), 7.33 (t, *J* = 7.8 Hz, 1H), 2.54 (s, 3H).

**Example 76: 4-amino-N-(1-((3-chloro-2-fluorophenyl)amino)-6-methylisoquinolin-5-yl)-6-(2-hydroxyethoxy)quinazoline-8-carboxamide (compound 86)**

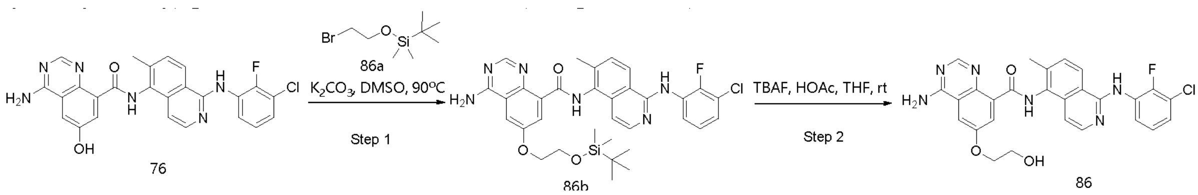


**Step 1: preparation of 4-amino-6-(2-((*tert*-butyldimethylsilyl)oxy)ethoxy)-N-(1-((3-chloro-2-fluorophenyl)amino)-6-methylisoquinolin-5-yl)quinazoline-8-carboxamide (compound 86b)**

Compounds 76 (40 mg, 81.82 μmol), 86a (39 mg, 163.63 μmol) and $K_2CO_3$ (28 mg, 204.57 μmol) were added to DMSO (3 mL), and the reaction was allowed to proceed under the protection of nitrogen at 90°C for 3 hr. After the reaction was complete, the reaction solution was diluted by adding water, and extracted with EA. The organic phase was washed with a saturated saline solution, dried over anhydrous sodium sulfate, filtered, and concentrated, to afford compound 86b (50 mg). MS m/z (ESI): 647.3 $[M+H]^+$.

**Step 2: preparation of 4-amino-N-(1-((3-chloro-2-fluorophenyl)amino)-6-methylisoquinolin-5-yl)-6-(2-hydroxyethoxy)quinazoline-8-carboxamide (compound 86)**

Compound 86b (50 mg, 77.25 μmol) was dissolved in THF (3 mL), acetic acid (2.32 mg, 38.63 μmol) and TBAF (115.88 μL, 1M THF solution) were then added, and the reaction was allowed to proceed under the protection of nitrogen at room temperature for 16 hr. After the reaction was complete, the reaction solution was diluted by adding water, and extracted with EA. The organic phase was washed with a saturated saline solution, dried over anhydrous sodium sulfate, filtered, concentrated, and the crude product was purified through Pre-HPLC, to afford compound 86 (8 mg). MS m/z (ESI): 532.9 $[M+H]^+$.

$^1$H NMR (400 MHz, DMSO-$d_6$) δ 13.25 (s, 1H), 9.24 (s, 1H), 8.51 (s, 1H), 8.34 (d, *J* = 8.8 Hz, 1H), 8.26 - 8.01 (m, 3H), 7.98 (d, *J* = 3.2 Hz, 1H), 7.90 (d, *J* = 6.0 Hz, 1H), 7.62 (d, *J* = 8.8 Hz, 1H), 7.58 - 7.53 (m, 1H), 7.39 - 7.33 (m, 1H), 7.27 - 7.19 (m, 2H), 4.98 (br, 1H), 4.19 (t, *J* = 4.8 Hz, 2H), 3.81 (t, *J* = 4.8 Hz, 2H), 2.44 (s, 3H).

**Example 77: 4-amino-N-(1-((2-fluoro-5-methoxyphenyl)amino)-6-methylisoquinolin-5-yl)quinazoline-8-carboxamide (compound 87)**

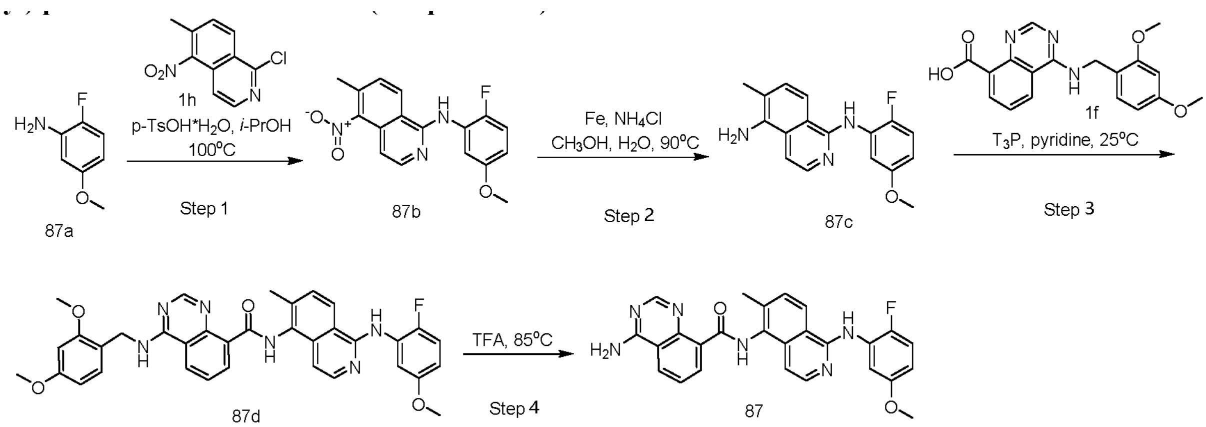


**Step 1: preparation of N-(2-fluoro-5-methoxyphenyl)-6-methyl-5-nitroisoquinolin-1-amine (compound 87b)**

Compounds 87a (114.12 mg, 808.53 μmol) and 1h (150 mg, 673.77 μmol) were dissolved in isopropanol (5 mL), p-toluenesulfonic acid monohydrate (51.27 mg, 269.51 μmol) was then added, and then the reaction was stirred at 100°C for 18 hr. After the reaction was complete, the reaction solution was concentrated to dryness, to afford compound 87b (220 mg). MS m/z (ESI): 328.1 $[M+H]^+$.

**Step 2: preparation of $N^1$-(2-fluoro-5-methoxyphenyl)-6-methylisoquinoline-1,5-diamine (compound 87c)**

Compound 87b (220 mg, 672.15 μmol), iron powder (187.78 mg, 3.36 mmol) and ammonium chloride (107.90 mg, 2.02 mmol) were added to methanol (10 mL) and water (3 mL), and the reaction was stirred at 90°C for 4 hr. After the reaction was complete, the reaction solution was concentrated to dryness, and purified by Flash column chromatography on silica gel (DCM/MeOH=96/4), to afford compound 87c (150 mg). MS m/z (ESI): 298.0 $[M+H]^+$.

**Step 3: preparation of 4-((2,4-dimethoxybenzyl)amino)-N-(1-((2-fluoro-5-methoxyphenyl)amino)-6-methylisoquinolin-5-yl)quinazoline-8-carboxamide (compound 87d)**

Compounds 87c (26.29 mg, 88.41 μmol) and 1f (30 mg, 88.41 μmol) were dissolved in pyridine (2 mL), $T_3P$ (0.5 mL, a 50% solution in EA) was then added, and the reaction was stirred at 25°C for 16 hr. After the reaction was complete, the solvent was removed under reduced pressure, the reaction was quenched by dropwise addition of a saturated solution of sodium bicarbonate, and extracted with EA. The organic phase was washed with a saturated saline solution, dried over anhydrous sodium sulfate, filtered, concentrated, and the crude product was purified by Flash column chromatography on silica gel (DCM/MeOH=94/6), to afford compound 87d (30 mg). MS m/z (ESI): 619.0 $[M+H]^+$.

**Step 4: preparation of 4-amino-N-(1-((2-fluoro-5-methoxyphenyl)amino)-6-methylisoquinolin-5-yl)quinazoline-8-carboxamide (compound 87)**

Compound 87d (30 mg, 48.49 μmol) was dissolved in TFA (3 mL), and the reaction was stirred at 85°C for 3hr. After the reaction was complete, the solvent was removed under reduced pressure, the residue was diluted by adding water, and adjusted to a pH of about 8 with a saturated solution of sodium bicarbonate. The solution was extracted with EA, the organic phase was washed with a saturated saline solution, dried over anhydrous sodium sulfate, filtered, concentrated, and then separated and purified through Prep-HPLC, to afford compound 87 (6mg). MS m/z (ESI): 469.3 $[M+H]^+$.

$^1H$ NMR (400 MHz, DMSO-$d_6$) δ 13.19 (s, 1H), 9.01 (s, 1H), 8.65 (dd, *J* = 8.8, 1.2 Hz, 1H), 8.62 (s, 1H), 8.52 (dd, *J* = 8.4, 1.2 Hz, 1H), 8.37 (br, 1H), 8.33 (d, *J* = 8.4 Hz, 1H), 8.23 (br, 1H), 7.89 (d, *J* = 6.0 Hz, 1H), 7.69 (t, *J* = 7.8 Hz, 1H), 7.60 (d, *J* = 8.4 Hz, 1H), 7.27-7.20 (m, 2H), 7.17 (dd, *J* = 10.4, 9.2 Hz, 1H), 6.72 (dt, *J* = 9.0, 3.4 Hz, 1H), 3.75 (s, 3H), 2.44 (s, 3H).

**Example 78: 4-amino-N-(1-((2-fluoro-4-methylphenyl)amino)-6-methylisoquinolin-5-yl)quinazoline-8-carboxamide (compound 88)**

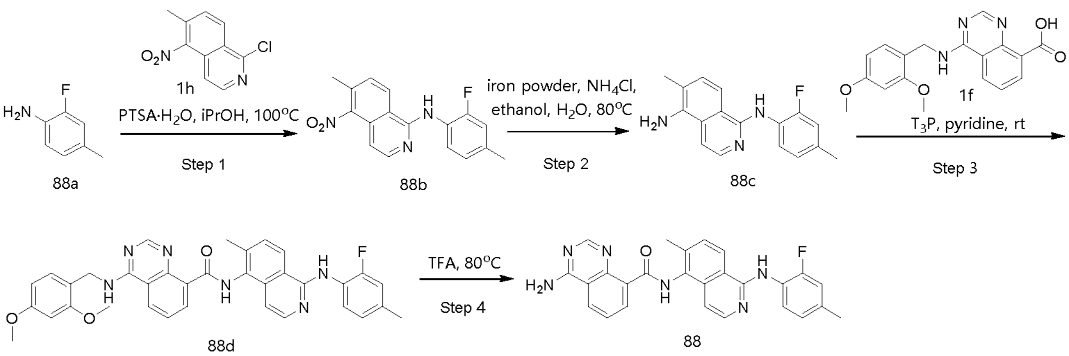


**Step 1: preparation of N-(2-fluoro-4-methylphenyl)-6-methyl-5-nitroisoquinolin-1-amine (compound 88b)**

Compounds 88a (101.18 mg, 0.81 mmol) and 1h (150 mg, 0.67 mmol) were added to isopropanol (5 mL), p-toluenesulfonic acid monohydrate (51.27 mg, 0.051 mmol) was then added, and the reaction was stirred at a temperature of 100°C for 18 hours. After the reaction was complete, the reaction solution was cooled to room temperature, filtered with suction, the filter cake was rinsed with isopropanol, and the resulting solid was dried under reduced pressure to afford compound 88b (200 mg). MS m/z (ESI): 312.0 $[M+H]^+$.

**Step 2: preparation of $N^1$-(2-fluoro-4-methylphenyl)-6-methylisoquinoline-1,5-diamine (compound 88c)**

Compound 88b (200 mg, 0.64 mmol) was added to ethanol (12 mL) and water (4 mL), iron powder (179.40 mg, 3.21 mmol) and ammonium chloride (34.36 mg, 0.64 mmol) were then added, and the reaction was heated to 80°C and stirred for 3 hours. After the reaction was complete, the reaction was immediately filtered through diatomaceous earth, the filtrate was concentrated under reduced pressure, to afford compound 88c (180 mg). MS m/z (ESI): 282.1 $[M+H]^+$.

**Step 3: preparation of 4-((2,4-dimethoxybenzyl)amino)-N-(1-((2-fluoro-4-methylphenyl)amino)-6-methylisoquinolin-5-yl)quinazoline-8-carboxamide (compound 88d)**

Compounds 1f (40 mg, 117.87 μmol) and 88c (43.11 mg, 153.24 μmol) were added to pyridine (3 mL), $T_3P$ (3 mL, 50% in DMF) was then added, and the reaction was allowed to proceed at room temperature overnight. After the reaction was complete, the reaction solution was directly rotary evaporated to dryness, and the crude product was separated and purified by column chromatography on silica gel (PE/EA=1/1), to afford compound 88d (28.0 mg). MS m/z (ESI): 603.7 $[M+H]^+$.

**Step 4: preparation of 4-amino-N-(1-((2-fluoro-4-methylphenyl)amino)-6-methylisoquinolin-5-yl)quinazoline-8-carboxamide (compound 88)**

Compound 88d (30 mg, 49.78 μmol) was added to trifluoroacetic acid (5.0 mL), and the reaction was allowed to proceed at 80°C for 16 hours. After the reaction was complete, the reaction solution was concentrated to dryness, the crude product was separated and purified by Prep-HPLC, to afford compound 88 (5.0 mg). MS m/z (ESI): 454.0 $[M+H]^+$.

$^1H$ NMR (400 MHz, DMSO-$d_6$) δ 13.18 (s, 1H), 9.04 (s, 1H), 8.67 (dd, *J* = 7.2, 0.8 Hz, 1H), 8.63 (s, 1H), 8.54 (dd, *J* = 8.4, 1.2 Hz, 1H), 8.45 - 8.20 (m, 3H), 7.83 (d, *J* = 6.0, 1H), 7.71 (t, *J* = 8.0, 1H), 7.61 (d, *J* = 8.4, 1H), 7.45 (t, *J* = 8.0, 1H), 7.19 (d, *J* = 6.0, 1H), 7.11 (d, *J* = 11.2, 1H), 7.04 (d, *J* = 8.0, 1H), 2.45 (s, 3H), 2.36 (s, 3H).

**Example 79: 4-amino-N-(1-((2-fluoro-5-(methylsulfonyl)phenyl)amino)-6-methylisoquinolin-5-yl)quinazoline-8-carboxamide (compound 89)**

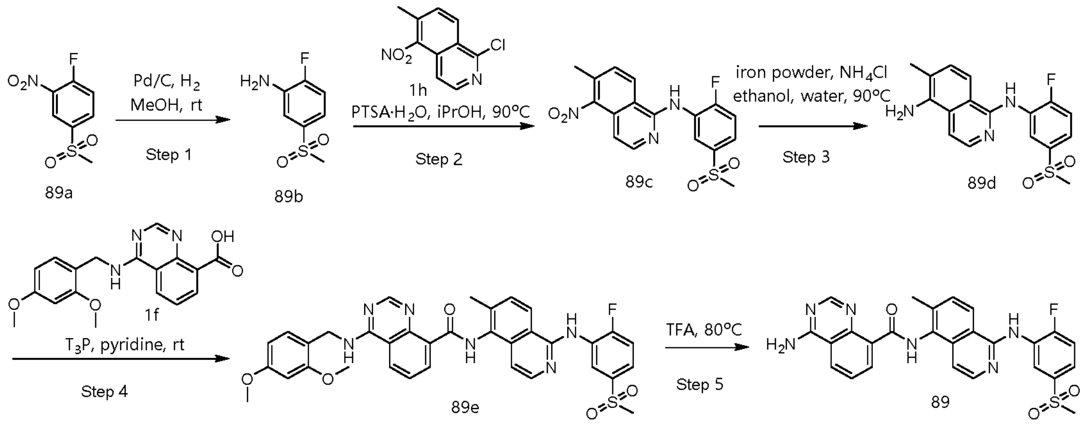


**Step 1: preparation of 2-fluoro-5-(methylsulfonyl)aniline (compound 89b)**

Compound 89a (210 mg, 958 μmol) and Pd/C (116 mg, content 5%) were added to methanol (5 mL), purge with hydrogen was then performed, and the reaction was allowed to proceed at room temperature under an atmosphere of hydrogen for 2hr. After the reaction was complete, the reaction solution was filtered through diatomaceous earth, the filter cake was washed with methanol, the resulting filtrate was concentrated under reduced pressure to afford compound 89b (180 mg). MS m/z (ESI): 207.0 $[M+NH_4]^+$.

**Step 2: preparation of N-(2-fluoro-5-(methylsulfonyl)phenyl)-6-methyl-5-nitroisoquinolin-1-amine (compound 89c)**

Compounds 89b (180 mg, 951 μmol) and 1h (212 mg, 951 μmol) were added to isopropanol (10 mL), p-toluenesulfonic acid monohydrate (164 mg, 951 μmol) was then added, and the reaction was stirred at 90°C for 16 hours. After the reaction was complete, the reaction solution was naturally cooled to room temperature, filtered with suction, the filter cake was rinsed with isopropanol, the resulting solid was dried under reduced pressure to afford compound 89c (250 mg). MS m/z (ESI): 376.1 $[M+H]^+$.

**Step 3: preparation of $N^1$-(2-fluoro-5-(methylsulfonyl)phenyl)-6-methylisoquinoline-1,5-diamine (compound 89d)**

Compound 89c (250 mg, 666 μmol) was added to ethanol (10 mL) and water (2 mL), iron powder (186 mg, 3.33 mmol) and $NH_4Cl$ (89 mg, 1.67 mmol) were then added, and the reaction was heated to 90°C and stirred for 3 hours. After the reaction was complete, the reaction was immediately filtered through diatomaceous earth, the filtrate was concentrated under reduced pressure to remove most of the solvent, and then extracted with EA. The organic phase was dried over anhydrous sodium sulfate, filtered, concentrated, and then separated and purified by column chromatography on silica gel (DCM/MeOH=96/4), to afford compound 89d (140 mg). MS m/z (ESI): 346.1 $[M+H]^+$.

**Step 4: preparation of 4-((2,4-dimethoxybenzyl)amino)-N-(1-((2-fluoro-5-(methylsulfonyl)phenyl)amino)-6-methylisoquinolin-5-yl)quinazoline-8-carboxamide (compound 89e)**

Compounds 1f (30 mg, 88.41 μmol) and 89d (30 mg, 88.41 μmol) were added to pyridine (3 mL), $T_3P$ (2 mL, a 50% solution in EA) was then added, and the reaction was allowed to proceed under the protection of nitrogen at room temperature overnight. After the reaction was complete, the reaction solution was directly concentrated to dryness, diluted by adding 30 ml water, and extracted with EA (30 mL x3). The organic phases were combined, washed with saturated brine, dried over anhydrous sodium sulfate, filtered, and concentrated, to afford compound 89e (50 mg). MS m/z (ESI): 667.3 $[M+H]^+$.

**Step 5: preparation of 4-amino-N-(1-((2-fluoro-5-(methylsulfonyl)phenyl)amino)-6-methylisoquinolin-5-yl)quinazoline-8-carboxamide (compound 89)**

Compound 89e (50 mg, 75 μmol) was added to trifluoroacetic acid (5.0 mL), and the reaction was allowed to proceed at 80°C for 3 hours. After the reaction was complete, the reaction solution was concentrated to dryness, added with a saturated solution of sodium bicarbonate for alkalization, and extracted with ethyl acetate. The organic phase

was dried over anhydrous sodium sulfate, filtered, concentrated, and the crude product was separated and purified by Prep-HPLC, to afford compound 89 (16 mg). MS m/z (ESI): 517.2 $[M+H]^+$.

$^1$H NMR (400 MHz, DMSO-$d_6$) δ 13.21 (s, 1H), 9.40 (s, 1H), 8.65 (dd, *J* = 7.6, 1.6 Hz, 1H), 8.62 (s, 1H), 8.53 (dd, *J* = 8.4, 1.6 Hz, 1H), 8.44 - 8.17 (m, 4H), 7.93 (d, *J* = 6.0 Hz, 1H), 7.77 - 7.67 (m, 2H), 7.64 (d, *J* = 8.4 Hz, 1H), 7.59 - 7.52 (m, 1H), 7.32 - 7.28 (m, 1H), 3.27 (s, 3H), 2.45 (s, 3H).

**Example 80: 4-amino-N-(6-methyl-1-((2,4,5-trifluorophenyl)amino)isoquinolin-5-yl)quinazoline-8-carboxamide (compound 90)**

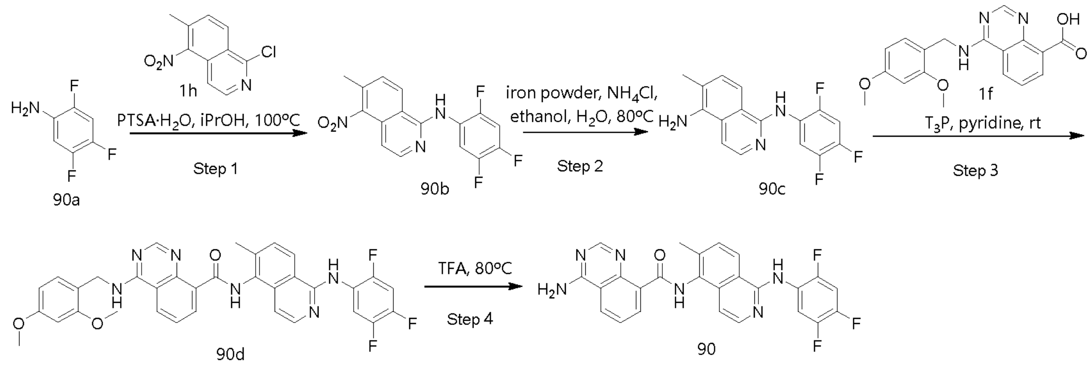


**Step 1: preparation of 6-methyl-5-nitro-N-(2,4,5-trifluorophenyl)isoquinolin-1-amine (compound 90b)**

Compounds 90a (118.93 mg, 0.81 mmol) and 1h (150 mg, 0.67 mmol) were added to isopropanol (5 mL), p-toluenesulfonic acid monohydrate (51.27 mg, 0.051 mmol) was then added, and the reaction was stirred at a temperature of 100°C for 18 hours. After the reaction was complete, the reaction solution was cooled to room temperature, filtered with suction, the filter cake was rinsed with isopropanol, and the resulting solid was dried under reduced pressure to afford compound 90b (200 mg). MS m/z (ESI): 334.1 $[M+H]^+$.

**Step 2: preparation of 6-methyl-N$^1$-(2,4,5-trifluorophenyl)isoquinoline-1,5-diamine (compound 90c)**

Compound 90b (200 mg, 0.60 mmol) was added to ethanol (12 mL) and water (4 mL), iron powder (167.58 mg, 3.00 mmol) and ammonium chloride (32.10 mg, 0.60 mmol) were then added, and the reaction was heated to 80°C and stirred for 3 hours. After the reaction was complete, the reaction was immediately filtered through diatomaceous earth, and the filtrate was concentrated under reduced pressure, to afford compound 90c (150 mg). MS m/z (ESI): 304.8 $[M+H]^+$.

**Step 3: preparation of 4-((2,4-dimethoxybenzyl)amino)-N-(6-methyl-1-((2,4,5-trifluorophenyl)amino)isoquinolin-5-yl)quinazoline-8-carboxamide (compound 90d)**

Compounds 1f (40 mg, 117.87 μmol) and 90c (46.47 mg, 153.24 μmol) were added to pyridine (3 mL), $T_3P$ (3 mL, 50% in DMF) was then added, and the reaction was allowed to proceed at room temperature overnight. After the reaction was complete, the reaction solution was directly rotary evaporated to dryness, and the crude product was separated and purified by column chromatography on silica gel (PE/EA=1/1), to afford compound 90d (15.0 mg). MS m/z (ESI): 625.2 $[M+H]^+$.

**Step 4: preparation of 4-amino-N-(6-methyl-1-((2,4,5-trifluorophenyl)amino)isoquinolin-5-yl)quinazoline-8-carboxamide (compound 90)**

Compound 90d (15 mg, 24.01 μmol) was added to trifluoroacetic acid (5.0 mL), and the reaction was allowed to proceed at 80°C for 16 hours. After the reaction was complete, the reaction solution was concentrated to dryness, and the crude product was separated and purified by Prep-HPLC, to afford a trifluoroacetate salt of compound 90 (8.46 mg). MS m/z (ESI): 475.0 $[M+H]^+$.

$^1$H NMR (400 MHz, DMSO-$d_6$) δ 12.46 (s, 1H), 9.75 (s, 1H), 9.04 (s, 1H), 8.73 (d, *J* = 7.2 Hz, 1H), 8.68 (s, 1H), 8.61 (d, *J* = 8.0 Hz, 1H), 8.46 (d, *J* = 8.4 Hz, 1H), 7.90 - 7.67 (m, 5H), 7.33 (d, *J* = 6.0 Hz, 1H), 2.50 (s, 3H).

**Example 81: preparation of 4-amino-N-(1-((5-bromo-2-fluorophenyl)amino)-6-methylisoquinolin-5-yl)quinazoline-8-carboxamide (compound 91)**

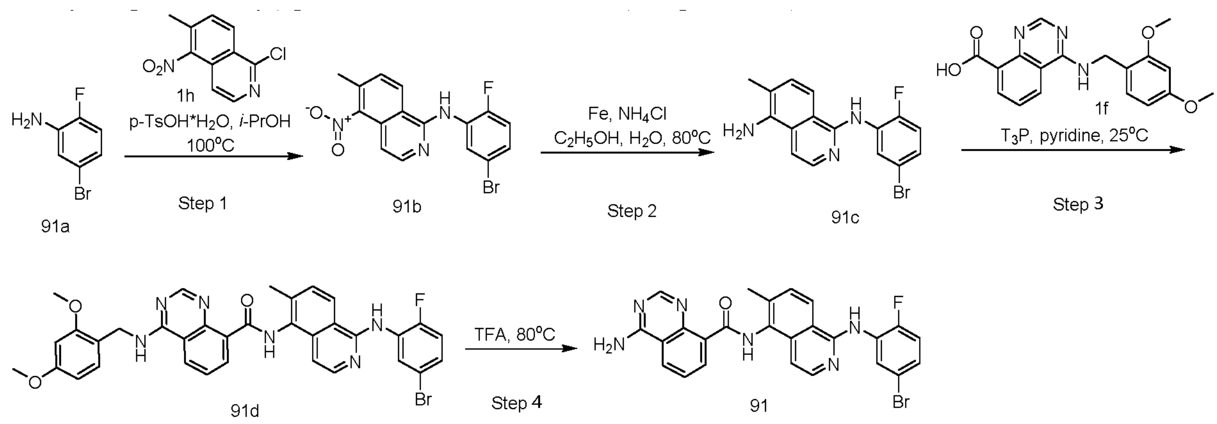


**Step 1: N-(5-bromo-2-fluorophenyl)-6-methyl-5-nitroisoquinolin-1-amine (compound 91b)**

Compounds 91a (187.77 mg, 988.20 μmol) and 1h (200 mg, 898.36 μmol) were dissolved in isopropanol (5 mL), p-toluenesulfonic acid monohydrate (34.18 mg, 179.67 μmol) was then added, and then the reaction was stirred at 100°C for 18 hr. After the reaction was complete, the reaction solution was concentrated to dryness, to afford compound 91b (250 mg). MS m/z (ESI): 376.1 $[M+H]^+$.

**Step 2: preparation of $N^1$-(5-bromo-2-fluorophenyl)-6-methylisoquinoline-1,5-diamine (compound 91c)**

Compound 91b (250 mg, 664.58 μmol), iron powder (185.58 mg, 3.32 mmol) and ammonium chloride (35.55 mg, 664.58 mmol) were added to ethanol (6 mL) and water (2 mL), and the reaction was stirred at 80°C for 3hr. After the reaction was complete, the reaction solution was filtered, and the filtrate was concentrated to dryness, to afford compound 91c (200 mg). MS m/z (ESI): 346.0 $[M+H]^+$.

**Step 3: preparation of N-(1-((5-bromo-2-fluorophenyl)amino)-6-methylisoquinolin-5-yl)-4-((2,4-dimethoxybenzyl)amino)quinazoline-8-carboxamide (compound 91d)**

Compounds 91c (40.81 mg, 117.87 μmol) and 1f (40 mg, 117.87 μmol) were dissolved in pyridine (3 mL), $T_3P$ (2 mL, a 50% solution in EA) was then added, and the reaction was stirred at 25°C for 16 hr. After the reaction was complete, the solvent was removed under reduced pressure, the reaction was quenched by dropwise addition of a saturated solution of sodium bicarbonate, and extracted with EA. The organic phase was washed with a saturated saline solution, dried over anhydrous sodium sulfate, filtered, and concentrated, to afford compound 91d (60 mg). MS m/z (ESI): 667.2 $[M+H]^+$.

**Step 4: preparation of 4-amino-N-(1-((5-bromo-2-fluorophenyl)amino)-6-methylisoquinolin-5-yl)quinazoline-8-carboxamide (compound 91)**

Compound 91d (60 mg, 89.88 μmol) was dissolved in TFA (3 mL), and stirred at 80°C for 3hr. After the reaction was complete, the solvent was removed under reduced pressure, the residue was diluted by adding water, adjusted to a pH of about 8 with a saturated solution of sodium bicarbonate, and extracted with EA. The organic phase was washed with a saturated saline solution, dried over anhydrous sodium sulfate, filtered, concentrated, and then separated and purified through Prep-HPLC, to afford compound 91 (8.25 mg). MS m/z (ESI): 517.1 $[M+H]^+$.

$^1H$ NMR (400 MHz, DMSO-$d_6$) δ 13.20 (s, 1H), 9.15 (s, 1H), 8.65 (dd, J=7.5, 1.3, 1H), 8.62 (s, 1H), 8.53 (dd, J=8.3, 1.4, 1H), 8.43 - 8.21 (m, 3H), 7.93 (d, J=6.0, 1H), 7.89 (dd, J=7.0, 2.5, 1H), 7.69 (t, J=7.8, 1H), 7.62 (d, *J*=8.7, 1H), 7.34 (M, 1H), 7.27 (M, 2H), 2.44 (s, 3H).

**Example 82: 4-amino-N-(1-((3-chloro-2-fluorophenyl)amino)-6-fluoroisoquinolin-5-yl)quinazoline-8-carboxamide (compound 92)**

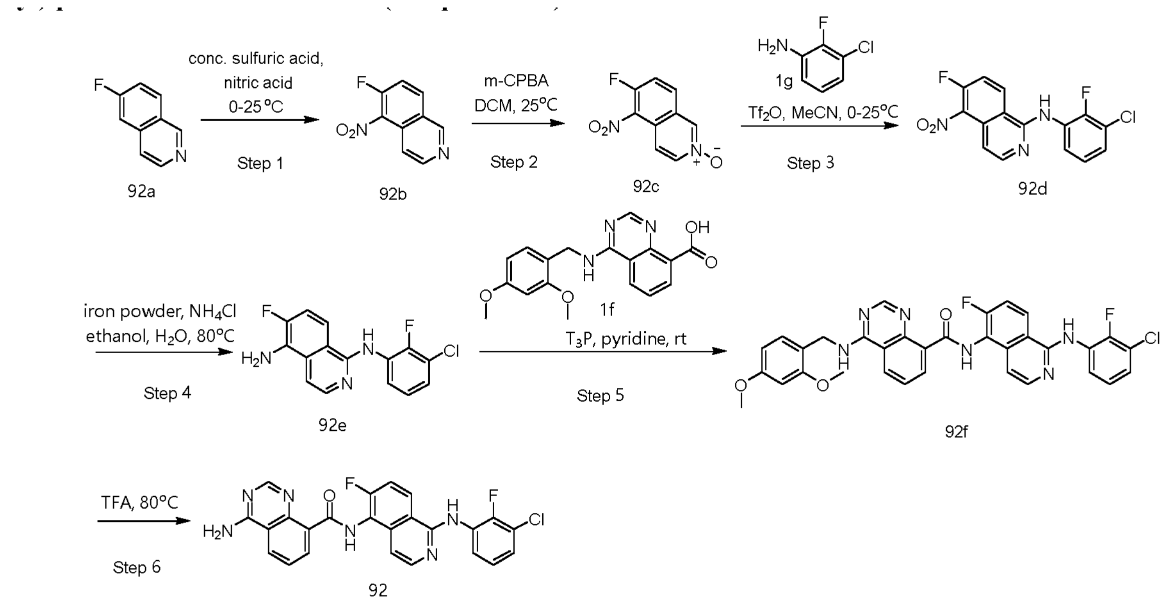

92a 92b 92c 92d 92e 92f 92

**Step 1: preparation of 6-fluoro-5-nitroisoquinoline (compound 92b)**

Nitric acid (2 mL) was added to concentrated sulfuric acid (2.8 mL) at 0°C, compound 92a (350 mg, 2.38 mmol) was then added, and after the addition, the reaction was warmed to room temperature and stirred for 16 hours. After the reaction was complete, the reaction solution poured into ice water, adjusted to a pH of 9~10 with a 4N solution of NaOH, extracted with ethyl acetate, washed with saturated brine, dried over anhydrous sodium sulfate, filtered, concentrated, and the crude product was separated and purified by column chromatography on silica gel (PE/EA=2/1), to afford compound 92b (140 mg). MS m/z (ESI): 193.1 $[M+H]^+$.

**Step 2: preparation of 6-fluoro-5-nitroisoquinoline 2-oxide (compound 92c)**

Compound 92b (150 mg, 0.78 mmol) was added to dichloromethane (10 mL), m-chloroperbenzoic acid (336.79 mg, 1.56 mmol) was then added, and after the addition, the reaction was stirred at room temperature for 16 hours. After the reaction was complete, the reaction solution was concentrated, and the crude product was separated and purified by column chromatography on silica gel (DCM/MeOH=93/7), to afford compound 92c (150 mg). MS m/z (ESI): 209.1 $[M+H]^+$.

**Step 3: preparation of N-(3-chloro-2-fluorophenyl)-6-fluoro-5-nitroisoquinolin-1-amine (compound 92d)**

Compounds 92c (200.00 mg, 0.96 mmol) and 1g (167.84 mg, 1.15 mmol) were added to acetonitrile (10 mL), then trifluoromethanesulfonic anhydride (406.65 mg, 1.44 mmol) was added at 0°C, and the reaction was warmed to room temperature and stirred for 8 hours. After the reaction was complete, the reaction was adjusted to a pH of 8~9 by adding a saturated solution of sodium carbonate, and extracted with ethyl acetate. The organic phase was washed with a saturated saline solution, dried over anhydrous sodium sulfate, filtered, concentrated, and the crude product was separated and purified by column chromatography on silica gel (PE/EA=3/1), to afford compound 92d (160 mg). MS m/z (ESI): 336.0 $[M+H]^+$.

**Step 4: preparation of $N^1$-(3-chloro-2-fluorophenyl)-6-fluoroisoquinoline-1,5-diamine (compound 92e)**

Compound 92d (100 mg, 0.60 mmol) was added to ethanol (12 mL) and water (4 mL), iron powder (83.19 mg, 1.49 mmol) and ammonium chloride (31.87mg, 0.60 mmol) were then added, and the reaction was heated to 80°C and stirred for 3 hours. After the reaction was complete, the reaction was immediately filtered through diatomaceous earth, and the filtrate was concentrated under reduced pressure, to afford compound 92e (90.0 mg). MS m/z (ESI): 306.0 $[M+H]^+$.

**Step 5: preparation of N-(1-((3-chloro-2-fluorophenyl)amino)-6-fluoroisoquinolin-5-yl)-4-((2,4-dimethoxybenzyl)amino)quinazoline-8-carboxamide** (compound 92f)

Compounds 1f (50 mg, 147.34 μmol) and 92e (45.04 mg, 147.34 μmol) were added to pyridine (1 mL), $T_3P$ (0.5 mL, 50% in DMF) was then added, and the reaction was allowed to proceed at room temperature overnight. After the reaction was complete, the reaction solution was directly rotary evaporated to dryness, and the crude product was separated and purified by column chromatography on silica gel (PE/EA=1/1), to afford compound 92f (30.0 mg). MS m/z (ESI): 627.1 $[M+H]^+$.

**Step 6: preparation of 4-amino-N-(1-((3-chloro-2-fluorophenyl)amino)-6-fluoroisoquinolin-5-yl)quinazoline-8-carboxamide (compound 92)**

Compound 92f (30 mg, 47.84 μmol) was added to trifluoroacetic acid (5.0 mL), and the reaction was allowed to proceed at 80°C for 16 hours. After the reaction was complete, the reaction solution was concentrated to dryness, and the crude product was separated and purified by Prep-HPLC, to afford compound 92 (15.0 mg). MS m/z (ESI): 477.1 $[M+H]^+$.

$^1H$ NMR (400 MHz, DMSO-$d_6$) δ 13.38 (s, 1H), 9.39 (s, 1H), 8.66 (dd, *J* = 7.6, 1.6 Hz, 1H), 8.63 (s, 1H), 8.56 - 8.47 (m, 2H), 8.45 - 8.21 (m, 2H), 7.98 (d, *J* = 6.0 Hz, 1H), 7.74 - 7.65 (m, 2H), 7.57 - 7.50 (m, 1H), 7.43 - 7.35 (m, 1H), 7.29 (d, *J* = 6.0 Hz, 1H), 7.24 (td, *J* = 8.1, 1.2 Hz, 1H).

**Example 83: 4-amino-N-(1-((3-chloro-2-fluorophenyl)amino)-6-methylisoquinolin-5-yl)-5-fluoroquinazoline-8-carboxamide (compound 93)**

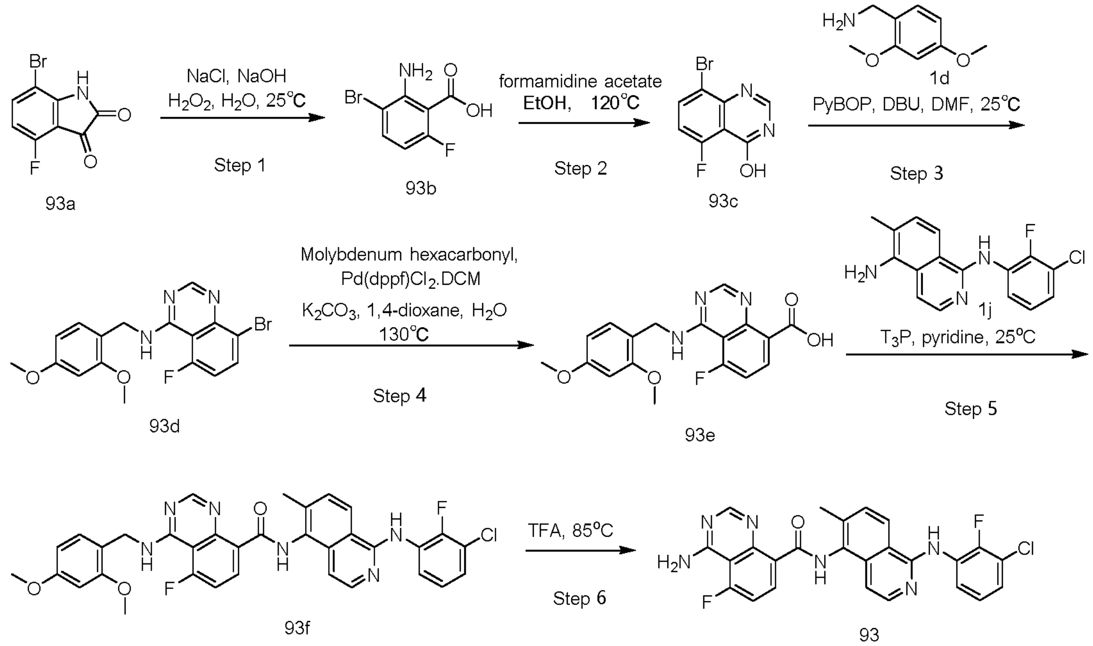


**Step 1: preparation of 2-amino-3-bromo-6-fluorobenzoic acid (compound** 93b)

Compound 93a (1 g, 4.10 mmol), NaCl (574.78 mg, 9.84 mmol), NaOH (393.41 mg, 9.84 mmol) were added to 50 ml water, and the reaction was stirred at 25°C for 0.5hr. A 30% aqueous solution of $H_2O_2$ (2.5 mL) was then dropwise added, followed by addition of a solution of NaOH (393.41 mg, 9.84 mmol) in water (50 mL), and the reaction was continuously stirred at 25°C for 3.5hr. After the reaction was complete, the solution was adjusted to a pH of about 3 with 1M dilute hydrochloric acid, and extracted with EA for three times. The organic phase was dried over anhydrous sodium sulfate, filtered, and concentrated, to afford compound 93b (900 mg). MS m/z (ESI): 233.9 $[M+H]^+$.

**Step 2: preparation of 8-bromo-5-fluoroquinazolin-4-ol (compound 93c)**

Compound 93b (900 mg, 3.85 mmol) and formamidine acetate (2.00 g, 19.23 mmol) were added to anhydrous ethanol (15 mL), and the reaction was stirred at 120 °C for 16hr. After the reaction was complete, the reaction solution was cooled to room temperature, filtered with suction, the filter cake was washed with ethanol, and the filter cake was dried in vacuum, to afford compound 93c (800 mg). MS m/z (ESI): 243.0 $[M+H]^+$.

**Step 3: preparation of 8-bromo-N-(2,4-dimethoxybenzyl)-5-fluoroquinazolin-4-amine (compound 93d)**

Compounds 93c (400 mg, 1.65 mmol), 1d (288.96 mg, 1.73 mmol) and DBU (497.43 mg, 1.98 mmol) were dissolved in DMF (8 mL), PyBOP (899.32 mg, 1.73 mmol) was then added, and after the addition, the reaction was stirred at 25°C for 16 hr. After the reaction was complete, the reaction was diluted by adding water, and extracted with EA. The organic phase was washed with water for three times, dried over anhydrous sodium sulfate, filtered, concentrated, and purified by Flash column chromatography on silica gel (DCM/MeOH=93/7), to afford compound 93d (300 mg). MS m/z (ESI): 392.0 $[M+H]^+$.

**Step 4: preparation of 4-((2,4-dimethoxybenzyl)amino)-5-fluoroquinazoline-8-carboxylic acid (compound 93e)**

Compound 93d (100 mg, 254.96 μmol), molybdenum hexacarbonyl (33.65 mg, 127.48 μmol), $K_2CO_3$ (105.71 mg, 764.87 μmol) and Pd(dppf)$Cl_2$·DCM (31.23 mg, 38.24 μmol) were added to 1,4-dioxane (4 mL) and water (1 mL), and the reaction was stirred under the protection of nitrogen at 130°C for 1hr. After the reaction was complete, the reaction solution was cooled to room temperature, adjusted to a pH of about 12 with a 4M aqueous solution of NaOH, washed with DCM twice, the aqueous phase was adjusted to a pH of about 5 with 3M dilute hydrochloric acid, and then the solution was concentrated to dryness. The resulting solid was added with 20 mL of a mixed solvent (methanol/dichloromethane =10/1), stirred for 0.5hr, filtered with suction, and the filtrate was concentrated, to afford compound 93e (80 mg). MS m/z (ESI): 358.1 $[M+H]^+$.

**Step 5: preparation of N-(1-((3-chloro-2-fluorophenyl)amino)-6-methylisoquinolin-5-yl)-4-((2,4-dimethoxybenzyl)amino)-5-fluoroquinazoline-8-carboxamide (compound 93f)**

Compounds 1j (25.33 mg, 83.95 μmol) and 93e (30 mg, 83.95 μmol) were dissolved in pyridine (2 mL), $T_3P$ (0.5 mL, a 50% solution in EA) was then added, and the reaction was stirred at 25°C for 16hr. After the reaction was complete, the solvent was removed under reduced pressure, the reaction was quenched by dropwise addition of a saturated solution of sodium bicarbonate, and extracted with EA. The organic phase was washed with a saturated saline solution, dried over anhydrous sodium sulfate, filtered, concentrated, and purified by Flash column chromatography on silica gel (DCM/MeOH=9/1), to afford compound 93f (30 mg). MS m/z (ESI): 641.0 $[M+H]^+$.

**Step 6: preparation of 4-amino-N-(1-((3-chloro-2-fluorophenyl)amino)-6-methylisoquinolin-5-yl)-5-fluoroquinazoline-8-carboxamide (compound 93)**

Compound 93f (30 mg, 46.80 μmol) was dissolved in TFA (3 mL), and the reaction was stirred at 85°C for 3hr. After the reaction was complete, the solvent was concentrated to dryness, the reaction was adjusted to a pH of about 8 with a saturated solution of sodium bicarbonate, and extracted with EA twice. The organic phase was washed with a saturated saline solution, dried over anhydrous sodium sulfate, filtered, concentrated, and then separated and purified through Prep-HPLC, to afford compound 93 (10 mg). MS m/z (ESI): 491.0 $[M+H]^+$.

$^1H$ NMR (400 MHz, DMSO-$d_6$) δ 12.97 (s, 1H), 9.24 (s, 1H), 8.70 - 8.62 (m, 2H), 8.58 (s, 1H), 8.34 (d, *J* = 8.4 Hz, 1H), 7.90 (d, *J* = 6.0 Hz, 1H), 7.84 (d, *J* = 8.8 Hz, 1H), 7.62 (d, *J* = 8.8 Hz, 1H), 7.59 - 7.53 (m, 1H), 7.50 (dd, *J*= 11.2, 8.8 Hz, 1H), 7.39 - 7.32 (m, 1H), 7.27 - 7.19 (m, 2H), 2.44 (s, 3H).

**Example 84: 4-amino-N-(6-methyl-1-((2,4,6-trifluorophenyl)amino)isoquinolin-5-yl)quinazoline-8-carboxamide (compound 94)**

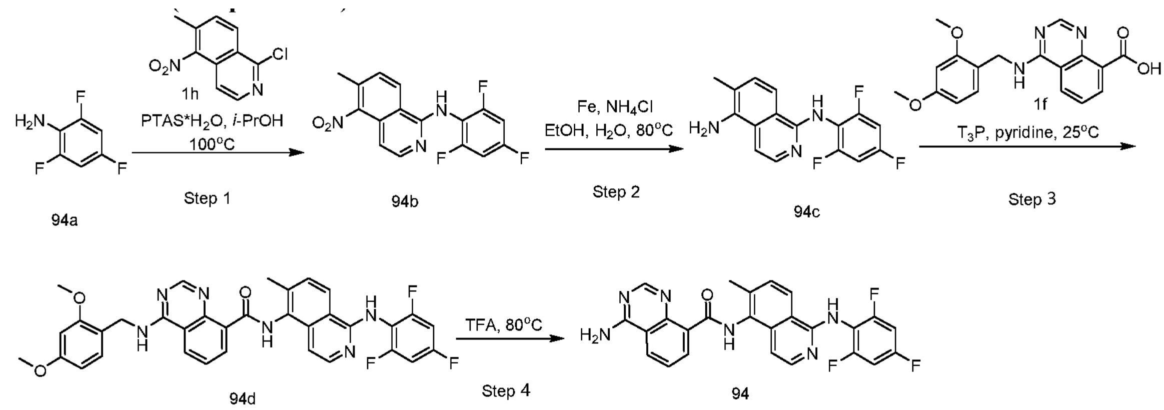


**Step 1: preparation of 6-methyl-5-nitro-N-(2,4,6-trifluorophenyl)isoquinolin-1-amine (compound 94b)**

Compounds 94a (50 mg, 339.91 μmol) and 1h (63.06 mg, 283.26 μmol) were dissolved in isopropanol (3 mL), p-toluenesulfonic acid monohydrate (24.39 mg, 141.63 μmol) was then added, and then the reaction was stirred at 100°C for 18hr. After the reaction was complete, the reaction solution was concentrated to dryness, to afford compound 94b (60 mg). MS m/z (ESI): 334.0 $[M+H]^+$.

**Step 2: preparation of 6-methyl-$N^1$-(2,4,6-trifluorophenyl)isoquinoline-1,5-diamine (compound 94c)**

Compound 94b (60 mg, 180.04 μmol), iron powder (50.28 mg, 900.19 μmol) and ammonium chloride (9.63 mg, 180.04 mmol) were added to ethanol (3 mL) and water (1 mL), and the reaction was stirred at 80°C for 3hr. After the reaction was complete, the reaction solution was filtered, the filtrate was concentrated to dryness, to afford compound 94c (50 mg). MS m/z (ESI): 304.1 $[M+H]^+$.

**Step 3: preparation of 4-((2,4-dimethoxybenzyl)amino)-N-(6-methyl-1-((2,4,6-trifluorophenyl)amino)isoquinolin-5-yl)quinazoline-8-carboxamide (compound 94d)**

Compounds 94c (50 mg, 164.86 μmol) and 1f (61.54 mg, 181.35 μmol) were dissolved in pyridine (3 mL), $T_3P$ (2 mL, a 50% solution in EA) was then added, and the reaction was stirred at 25°C for 16hr. After the reaction was complete, the solvent was removed under reduced pressure, the reaction was quenched by dropwise addition of a saturated solution of sodium bicarbonate, and extracted with EA. The organic phase was washed with a saturated saline solution, dried over anhydrous sodium sulfate, filtered, and concentrated, to afford compound 94d (60 mg). MS m/z (ESI): 625.3 $[M+H]^+$.

**Step 4: preparation of 4-amino-N-(6-methyl-1-((2,4,6-trifluorophenyl)amino)isoquinolin-5-yl)quinazoline-8-carboxamide (compound 94)**

Compound 94d (60 mg, 96.06 μmol) was dissolved in TFA (3 mL), and stirred at 80°C for 3hr. After the reaction was complete, the solvent was removed under reduced pressure, the residue was diluted by adding water, and adjusted to a pH of about 8 with a saturated solution of sodium bicarbonate. The solution was extracted with EA, the organic phase was washed with a saturated saline solution, dried over anhydrous sodium sulfate, filtered, concentrated, and then separated and purified through Prep-HPLC, to afford compound 94 (20.35 mg). MS m/z (ESI): 475.1 $[M+H]^+$.

$^1H$ NMR (400 MHz, DMSO-$d_6$) δ 13.18 (s, 1H), 8.95 (s, 1H), 8.65 (dd, *J*=7.5, 1.4, 1H), 8.61 (s, 1H), 8.52 (dd, *J*=8.3, 1.4, 1H), 8.42-8.14 (m, 3H), 7.81 (d, *J*=6.0, 1H), 7.72-7.66 (m, 1H), 7.61 (d, *J*=8.7, 1H), 7.36-7.26 (m, 2H), 7.19 (d, *J*=6.0, 1H), 2.44 (s, 3H).

**Example 85: 4-amino-N-(6-methyl-1-((2,3,5,6-tetrafluorophenyl)amino)isoquinolin-5-yl)quinazoline-8-carboxamide (compound 95)**

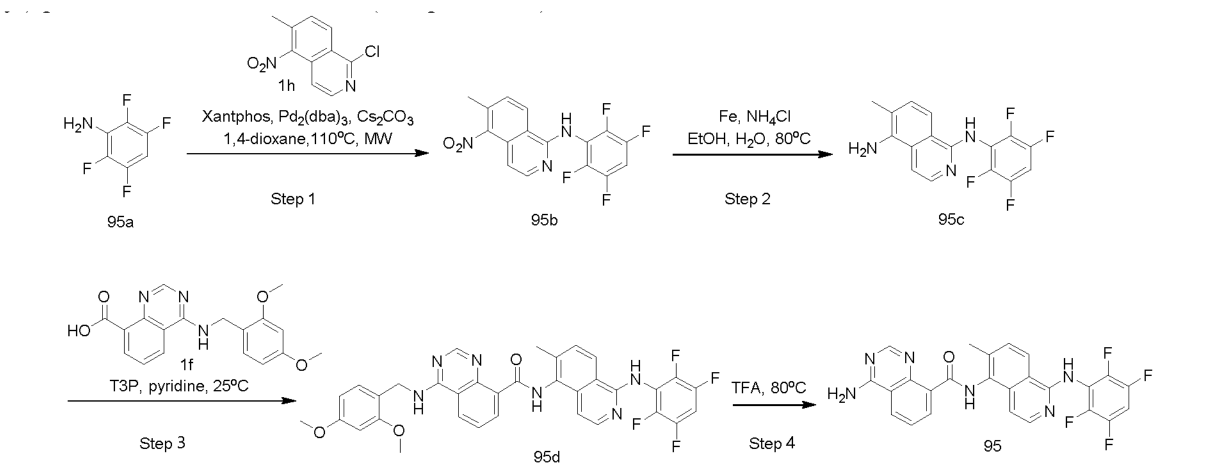


95a 95b 95c 95d 95

**Step 1: preparation of 6-methyl-5-nitro-N-(2,3,5,6-tetrafluorophenyl)isoquinolin-1-amine (compound 95b)**

Compounds 95a (150 mg, 908.61 μmol), 1h (168.57 mg, 757.17 μmol), $Pd_2(dba)_3$ (43.54 mg, 75.72 μmol), 4,5-bis(diphenylphosphino)-9,9-dimethylxanthene (87.62 mg, 151.43 μmol) and $Cs_2CO_3$ (740.10 mg, 2.27 mmol) were added to 1,4-dioxane (5 mL), purge with nitrogen was performed for three times, and the reaction was performed under microwave at 110°C for 2 hours. After the reaction was complete, the reaction solution was cooled to room temperature, filtered through diatomaceous earth, diluted by adding water, and extracted with EA. The organic phase was washed with a saturated saline solution, dried over anhydrous sodium sulfate, filtered, concentrated, and then purified by Flash column chromatography on silica gel (DCM/MeOH=20/1), to afford compound 95b (120 mg). MS m/z (ESI): 352.1 $[M+H]^+$.

**Step 2: preparation of 6-methyl-$N^1$-(2,3,5,6-tetrafluorophenyl)isoquinoline-1,5-diamine (compound 95c)**

Compound 95b (120 mg, 341.63 μmol), iron powder (190.80 mg,3.42 mmol) and ammonium chloride (17.93 mg, 341.63 mmol) were added to ethanol (6 mL) and water (2 mL), and the reaction was stirred at 80°C for 3hr. After the reaction was complete, the reaction solution was filtered, and the filtrate was concentrated to dryness, to afford compound 95c (100 mg). MS m/z (ESI): 322.1 $[M+H]^+$.

**Step 3: preparation of 4-((2,4-dimethoxybenzyl)amino)-N-(6-methyl-1-((2,3,5,6-tetrafluorophenyl)amino)isoquinolin-5-yl)quinazoline-8-carboxamide (compound 95d)**

Compounds 95c (30 mg, 93.38 μmol) and 1f (34.86 mg, 102.72 μmol) were dissolved in pyridine (3 mL), $T_3P$ (2 mL, a 50% solution in EA) was then added, and the reaction was stirred at 25°C for 16hr. After the reaction was complete, the solvent was removed under reduced pressure, the reaction was quenched by dropwise addition of a saturated solution of sodium bicarbonate, and extracted with EA. The organic phase was washed with a saturated saline solution, dried over anhydrous sodium sulfate, filtered, and concentrated, to afford compound 95d (30 mg). MS m/z (ESI): 643.2 $[M+H]^+$.

**Step 4: preparation of 4-amino-N-(6-methyl-1-((2,3,5,6-tetrafluorophenyl)amino)isoquinolin-5-yl)quinazoline-8-carboxamide (compound 95)**

Compound 95d (30 mg, 46.69 μmol) was dissolved in TFA (3 mL), and the reaction was stirred at 80°C for 3hr. After the reaction was complete, the solvent was removed under reduced pressure, the residue was diluted by adding water, and adjusted to a pH of about 8 with a saturated solution of sodium bicarbonate. The solution was extracted with EA, the organic phase was washed with a saturated saline solution, dried over anhydrous sodium sulfate, filtered, concentrated, and then separated and purified through Prep-HPLC, to afford compound 95 (4.65 mg). MS m/z (ESI): 493.2 $[M+H]^+$.

$^1H$ NMR (400 MHz, DMSO-$d_6$) δ 13.21 (s, 1H), 9.38 (s, 1H), 8.67-8.60 (m, 2H), 8.52 (dd, *J*=8.3, 1.4 Hz, 1H), 8.43-8.18 (m, 3H), 7.89 (d, *J*=6.0 Hz, 1H), 7.84-7.72 (m, 1H), 7.72-7.63 (m, 2H), 7.29 (d, *J*=6.2 Hz, 1H), 2.46 (s, 3H).

**Example 86: 4-amino-N-(6-methyl-1-((2,3,4,5-tetrafluorophenyl)amino)isoquinolin-5-yl)quinazoline-8-carboxamide (compound 96)**

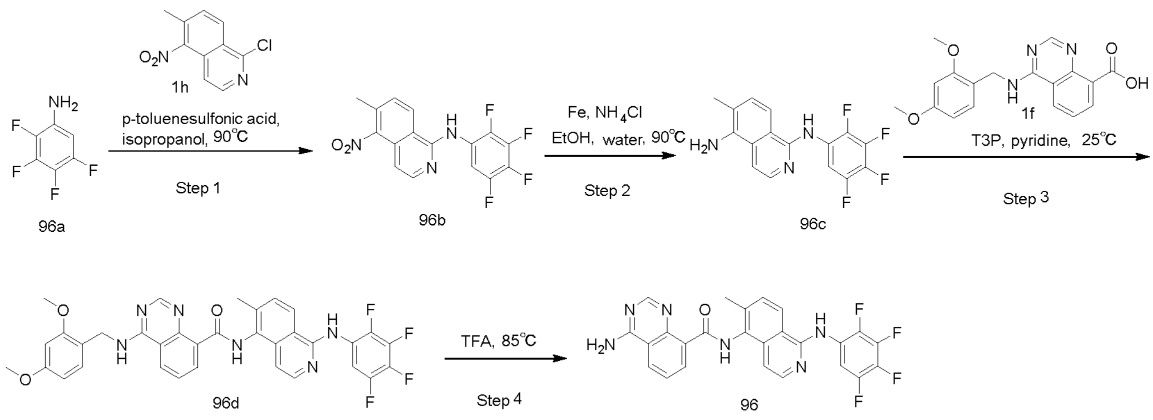


**Step 1: preparation of 6-methyl-5-nitro-N-(2,3,4,5-tetrafluorophenyl)isoquinolin-1-amine (compound 96b)**

Compounds 1h (600 mg, 2.70 mmol), 96a (489 mg, 2.96 mmol) were added to isopropanol (8 mL), p-toluenesulfonic acid (232 mg, 1.35 mmol) was then added, and the reaction was heated to 90°C and stirred for 16 hours. After the reaction was complete, the reaction solution was cooled to room temperature, filtered with suction, the filter cake was rinsed with isopropanol, and the resulting solid was dried under reduced pressure to afford compound 96b (919 mg). MS m/z (ESI): 352.0 $[M+H]^+$.

**Step 2: preparation of 6-methyl-$N^1$-(2,3,4,5-tetrafluorophenyl)isoquinoline-1,5-diamine (compound 96c)**

Compound 96b (300 mg, 0.85 mmol) was added to ethanol (5 mL) and water (1 mL), iron powder (238 mg, 4.27 mmol) and ammonium chloride (114 mg, 2.14 mmol) were then added, and the reaction was heated to 90°C and stirred for 4 hours. After the reaction was complete, the reaction was immediately filtered through diatomaceous earth, the filtrate was diluted by adding 60 ml water, and extracted with EA. The organic phase was washed with a saturated saline solution, dried over anhydrous sodium sulfate, filtered, concentrated, and the crude product was separated and purified by column chromatography on silica gel (PE/EA=4/1), to afford compound 96c (135 mg). MS m/z (ESI): 322.1 $[M+H]^+$.

**Step 3: preparation of 4-((2,4-dimethoxybenzyl)amino)-N-(6-methyl-1-((2,3,4,5-tetrafluorophenyl)amino)isoquinolin-5-yl)quinazoline-8-carboxamide (compound 96d)**

Compounds 96c (70 mg, 0.22 mmol) and 1f (74 mg, 0.22 mmol) were dissolved in pyridine (4 mL), $T_3P$ (0.5 mL, a 50% solution in EA) was then dropwise added, and the reaction was allowed to proceed at room temperature for 16 hours. After the reaction was complete, the solvent was removed by concentration under reduced pressure, the reaction solution was diluted with ethyl acetate, adjusted to pH of about 13 with a saturated aqueous solution of sodium bicarbonate, and extracted with ethyl acetate. The organic phase was washed with water and saturated brine, dried over anhydrous sodium sulfate, filtered, and concentrated, to afford compound 96d (123 mg). MS m/z (ESI): 643.3 $[M+H]^+$.

**Step 4: preparation of 4-amino-N-(6-methyl-1-((2,3,4,5-tetrafluorophenyl)amino)isoquinolin-5-yl)quinazoline-8-carboxamide (compound 96)**

Compound 96d (123 mg, 0.19 mmol) was added to trifluoroacetic acid (4 mL), and the reaction was allowed to proceed at 85°C for 4 hours. After the reaction was complete, the reaction solution was concentrated to dryness, added with a saturated solution of sodium bicarbonate, and extracted with EA. The organic phase was washed with a saturated saline solution, dried over anhydrous sodium sulfate, filtered, concentrated, and the crude product was separated and purified by Prep-HPLC, to afford compound 96 (15.2 mg). MS m/z (ESI): 493.1 $[M+H]^+$.

$^1H$ NMR (400 MHz, DMSO-$d_6$) $\delta$ 13.21 (s, 1H), 9.35 (s, 1H), 8.70 - 8.60 (m, 2H), 8.53 (d, *J*=8.2, 1H), 8.46 - 8.16 (m, 3H), 7.94 (d, *J*=6.0, 1H), 7.76 - 7.57 (m, 3H), 7.31 (d, *J*=6.0, 1H), 2.45 (s, 3H).

**Example 87: 4-amino-N-(1-((2-fluoro-4-(2-morpholinoethoxy)phenyl)amino)-6-methylisoquinolin-5-yl)quinazoline-8-carboxamide (compound 97)**

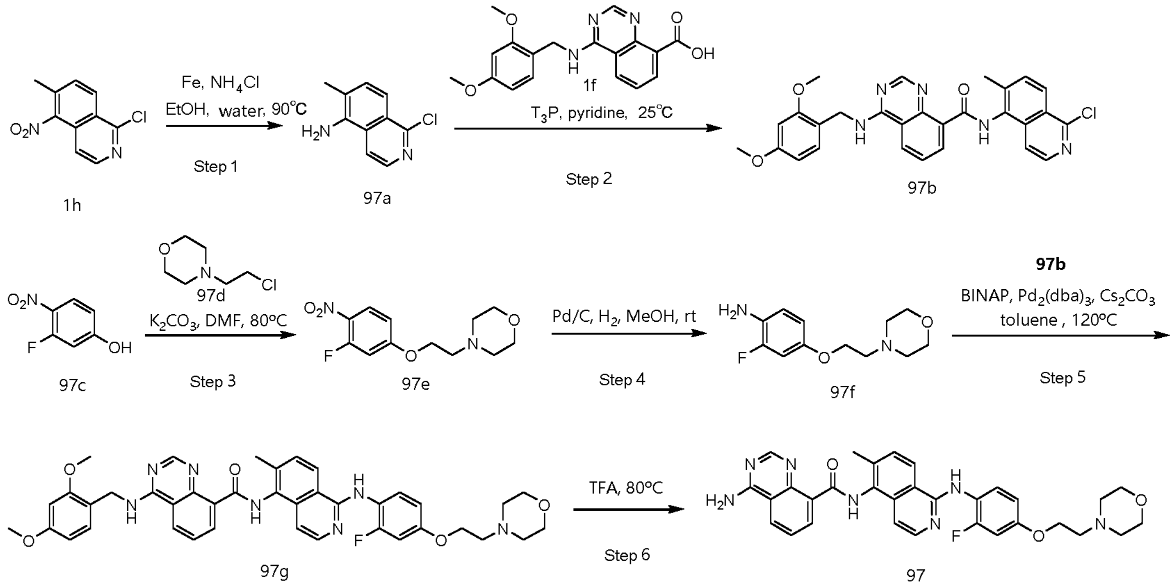


**Step 1: preparation of 1-chloro-6-methylisoquinolin-5-amine (compound 97a)**

Compound 1h (5 g, 22.46 mmol) was dissolved in EtOH (90 mL), reduced iron powder (6.27 g, 112.29 mmol) was added, followed by dropwise addition of a solution of ammonium chloride (3.00 g, 56.15 mmol) in water (15 mL), and after the addition, the reaction was allowed to proceed under the protection of nitrogen at 90°C for 3 hours. After the reaction was complete, the solvent was removed by concentration under reduced pressure, the residue was then diluted by adding water, and extracted with EA. The organic phase was washed with a saturated saline solution, dried over anhydrous sodium sulfate, filtered, concentrated, and the crude product was purified by Flash column chromatography on silica gel (PE/EA=1/1), to afford compound 97a (3.7 g). MS m/z (ESI): 193.1 $[M+H]^+$.

**Step 2: preparation of N-(1-chloro-6-methylisoquinolin-5-yl)-4-((2,4-dimethoxybenzyl)amino)quinazoline-8-carboxamide (compound 97b)**

Compounds 1f (200 mg, 589.37 μmol) and 97a (113.54 mg, 589.37 μmol) were added to pyridine (6 mL), $T_3P$ (3 mL, a 50% solution in EA) was then added, and the reaction was allowed to proceed at 25°C for 16hr. After the reaction was complete, the solvent was removed by concentration under reduced pressure, the residue was added with a saturated aqueous solution of sodium bicarbonate (5 mL), and extracted with ethyl acetate. The organic phase was dried over anhydrous sodium sulfate, filtered, and concentrated, to afford compound 97b (210 mg, 408.58 μmol). MS m/z (ESI): 514.2 $[M+H]^+$.

**Step 3: preparation of 4-(2-(3-fluoro-4-nitrophenoxy)ethyl)morpholine (compound 97e)**

Compounds 97c (200 mg, 1.27 mmol), 97d (474 mg, 2.55 mmol), $K_2CO_3$ (528 mg, 3.82 mmol) and DMF (10 mL) were sequentially added to a reaction flask, and the reaction was performed at 80°C under the protection of nitrogen for 4hr. After the reaction was complete, the reaction solution was diluted by adding water, and extracted with EA. The organic phase was washed with a saturated saline solution, dried over anhydrous sodium sulfate, filtered, concentrated, and the crude product was purified by Flash column chromatography on silica gel (EA/PE=9/1), to afford compound 97e (335 mg). MS m/z (ESI): 271.1 $[M+H]^+$.

**Step 4: preparation of 2-fluoro-4-(2-morpholinoethoxy)aniline (compound 97f)**

Compound 97e (120 mg, 444.02 μmol) and MeOH (5 mL) were added to a reaction flask, 10% Pd/C (54 mg) was then added, and the reaction was stirred under an atmosphere of hydrogen at 25°C for 3hr. After the reaction was

complete, the reaction solution was filtered, and the filtrate was concentrated under reduced pressure to afford compound 97f (90 mg). MS m/z (ESI): 241.1 [M+H]+.

**Step 5: preparation of 4-((2,4-dimethoxybenzyl)amino)-N-(1-((2-fluoro-4-(2-morpholinoethoxy)phenyl)amino)-6-methylisoquinolin-5-yl)quinazoline-8-carboxamide (compound 97g)**

Compounds 97b (30 mg, 58.37 μmol) and 97f (17 mg, 70.04 μmol) were added to toluene (3 mL), 1,1'-binaphthalene-2,2'-bisdiphenylphosphine (7 mg, 11.67 μmol), $Pd_2(dba)_3$ (5 mg, 5.84 μmol) and $Cs_2CO_3$ (57 mg, 175.11 μmol) were then sequentially added, and then the reaction was allowed to proceed under the protection of nitrogen at 120°C overnight. After the reaction was complete, the reaction solution was diluted by adding water, and extracted with EA. The organic phase was washed with a saturated saline solution, dried over anhydrous sodium sulfate, filtered, concentrated, and the crude product was purified by preparative thin layer chromatography (DCM/MeOH=10/1), to afford compound 97g (25 mg). MS m/z (ESI): 718.3 [M+H]+.

**Step 6: preparation of 4-amino-N-(1-((2-fluoro-4-(2-morpholinoethoxy)phenyl)amino)-6-methylisoquinolin-5-yl)quinazoline-8-carboxamide (compound 97**)

Compound 97g (25 mg, 34.83 μmol) and TFA (3 mL) were added to a reaction flask, and the reaction was heated to 80°C and allowed to proceed for 3hr. After the reaction was complete, the solvent was removed under reduced pressure, the residue was diluted by adding water, then adjusted to a pH of 7-8 with a saturated solution of $NaHCO_3$, followed by extraction with EA. The organic phase was washed with a saturated saline solution, dried over anhydrous sodium sulfate, filtered, concentrated, and the crude product purified through Pre-HPLC to afford compound 97 (2.5 mg). MS m/z (ESI): 568.3 [M+H]+.

$^1H$ NMR (400 MHz, DMSO-$d_6$) δ 13.16 (s, 1H), 8.90 (s, 1H), 8.65 (dd, *J* = 7.2, 1.6 Hz, 1H), 8.61 (s, 1H), 8.52 (dd, *J* = 8.4, 1.6 Hz, 1H), 8.42 - 8.16 (m, 3H), 7.80 (d, *J* = 6.0 Hz, 1H), 7.69 (t, *J* = 8.0 Hz, 1H), 7.57 (d, *J* = 8.8 Hz, 1H), 7.38 (d, *J* = 8.8 Hz, 1H), 7.12 (d, *J* = 6.0 Hz, 1H), 6.93 (dd, *J* = 12.4, 2.8 Hz, 1H), 6.83 - 6.78 (m, 1H), 4.12 (t, *J* = 5.6 Hz, 2H), 3.62 - 3.57 (m, 4H), 2.71 (t, *J* = 5.6 Hz, 2H), 2.49 - 2.47 (m, 4H), 2.43 (s, 3H).

**Example 88: 4-amino-N-(1-((2-fluoro-3-(2-morpholinoethoxy)phenyl)amino)-6-methylisoauinolin-5-yl)quinazoline-8-carboxamide (compound 98)**

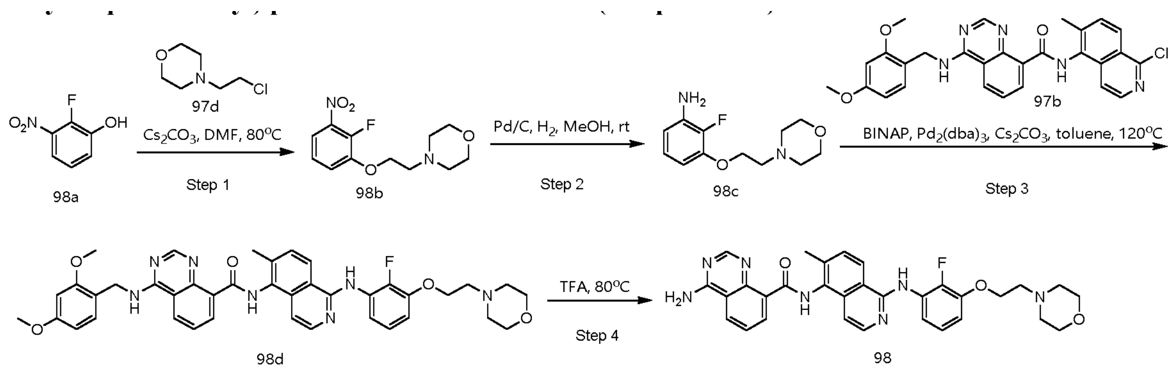


**Step 1: preparation of 4-(2-(2-fluoro-3-nitrophenoxy)ethyl)morpholine (compound 98b)**

98a (100 mg, 0.636 mmol), 97d (190 mg, 1.27 mmol), $Cs_2CO_3$ (622 mg, 1.91 mmol) and DMF (5 mL) were sequentially added to a reaction flask, protection of nitrogen was applied, and the reaction was allowed to proceed at 80°C for 4hr. After the reaction was complete, the reaction solution was diluted by adding water, and extracted with EA. The organic phase was washed with a saturated saline solution, dried over anhydrous sodium sulfate, filtered, and concentrated. The crude product was purified by Flash column chromatography on silica gel (EA/PE=9/1), to afford compound 98b (60 mg). MS m/z (ESI): 271.1 [M+H]+.

**Step 2: preparation of 2-fluoro-3-(2-morpholinoethoxy)aniline (compound 98c)**

Compound 98b (120 mg, 444.02 μmol) was dissolved in MeOH (6 mL), 10% Pd/C (54 mg) was then added,

and the reaction was stirred under an atmosphere of hydrogen at 25°C for 3hr. After the reaction was complete, the reaction solution was filtered, and the filtrate was concentrated under reduced pressure to afford compound 98c (90 mg). MS m/z (ESI): 241.1 [M+H]$^+$.

**Step 3: preparation of 4-((2,4-dimethoxybenzyl)amino)-N-(1-((2-fluoro-3-(2-morpholinoethoxy)phenyl)amino)-6-methylisoquinolin-5-yl)quinazoline-8-carboxamide (compound 98d)**

Compounds 97b (30 mg, 58.37 μmol) and 98c (17 mg, 70.04 μmol) were dissolved in toluene (3 mL), 1,1'-binaphthalene-2,2'-bisdiphenylphosphine (7 mg, 11.67 μmol), $Pd_2(dba)_3$ (5 mg, 5.84 μmol) and $Cs_2CO_3$ (57 mg, 175.11 μmol) were then sequentially added, purge with nitrogen was performed, and the reaction was allowed to proceed at 120°C overnight. After the reaction was complete, the reaction solution was diluted by adding water, and extracted with EA. The organic phase was washed with a saturated saline solution, dried over anhydrous sodium sulfate, filtered, and concentrated. The crude product was purified by preparative thin layer chromatography (DCM/MeOH=10/1), to afford compound 98d (25 mg). MS m/z (ESI): 718.3 [M+H]$^+$.

**Step 4: preparation of 4-amino-N-(1-((2-fluoro-3-(2-morpholinoethoxy)phenyl)amino)-6-methylisoquinolin-5-yl)quinazoline-8-carboxamide (compound 98)**

Compound 98d (25 mg, 34.83 μmol) and TFA (3 mL) were added to a reaction flask, and the reaction was allowed to proceed under the protection of nitrogen at 80°C for 3hr. After the reaction was complete, the solvent was removed under reduced pressure, diluted by adding water, adjusted to a pH of 7-8 with a saturated solution of $NaHCO_3$, and then extracted with EA. The organic phase was washed with a saturated saline solution, dried over anhydrous sodium sulfate, filtered, concentrated, and the crude product was purified through Pre-HPLC, to afford compound 98 (2 mg). MS m/z (ESI): 568.3 [M+H]$^+$.

$^1$H NMR (400 MHz, DMSO-$d_6$) δ 13.18 (s, 1H), 9.01 (s, 1H), 8.65 (dd, *J* = 7.6, 1.6 Hz, 1H), 8.62 (s, 1H), 8.52 (dd, *J* = 8.4, 1.6 Hz, 1H), 8.43 - 8.17 (m, 3H), 7.87 (d, *J* = 6.0 Hz, 1H), 7.69 (t, *J* = 8.0 Hz, 1H), 7.59 (d, *J* = 8.8 Hz, 1H), 7.22 - 7.18 (m, 1H), 7.16 - 7.06 (m, 2H), 7.02 - 6.96 (m, 1H), 4.22 - 4.15 (m, 2H), 3.60 - 3.56 (m, 4H), 2.76 - 2.69 (m, 2H), 2.49 - 2.47 (m, 4H), 2.43 (s, 3H).

**Example 89: 4-amino-N-(1-((4-(2-(dimethylamino)ethoxy)-2-fluorophenyl)amino)-6-methylisoquinolin-5-yl)quinazoline-8-carboxamide (compound 99)**

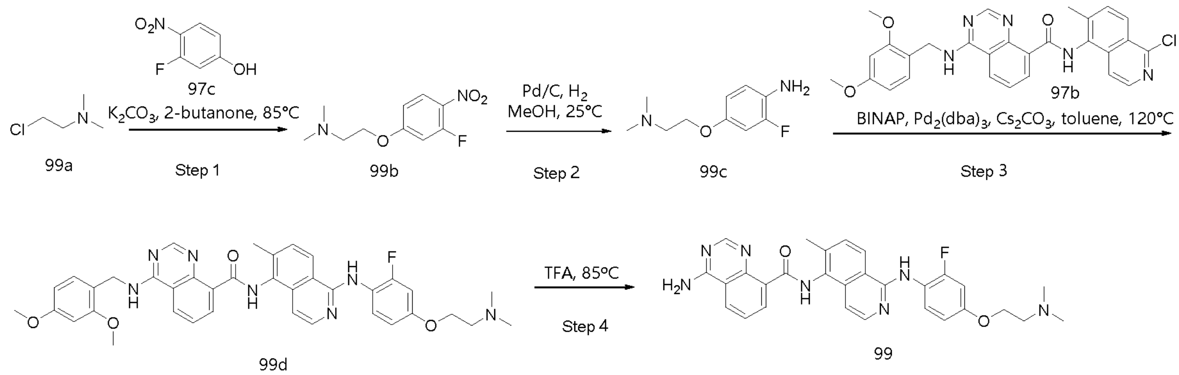

99a 99b 99c 99d 99

**Step 1: preparation of 2-(3-fluoro-4-nitrophenoxy)-N,N-dimethylethylamine (compound 99b)**

Compounds 97c (500 mg, 3.18 mmol), 99a (1008.58 mg, 7.00 mmol, hydrochloride salt) and $K_2CO_3$ (879.76 mg, 6.37 mmol) were added to 2-butanone (10 mL), and the reaction was stirred at 85°C for 21hr. After the reaction was complete, the solvent was removed under reduced pressure, and the crude product was purified by Flash column chromatography on silica gel (DCM/MeOH=10/1), to afford compound 99b (500mg). MS m/z (ESI): 229.1 [M+H]$^+$.

**Step 2: preparation of 4-(2-(dimethylamino)ethoxy)-2-fluoroaniline (compound 99c)**

Compound 99b (500 mg, 2.19 mmol) was dissolved in MeOH (10 mL), 10% Pd/C (100 mg) was added, purge with hydrogen was performed, and the reaction was stirred at 25°C for 16hr. After the reaction was complete, the reaction

was filtered with suction, and the filtrate was concentrated, to afford compound 99c (420mg). MS m/z (ESI): 199.1 [M+H]+.

**Step 3: preparation of 4-((2,4-dimethoxybenzyl)amino)-N-(1-((4-(2-(dimethylamino)ethoxy)-2-fluorophenyl)amino)-6-methylisoquinolin-5-yl)quinazoline-8-carboxamide (compound 99d)**

Compounds 99c (17.36 mg, 87.55 μmol), 97b (30 mg, 58.37 μmol), 1,1'-binaphthalene-2,2'-bisdiphenylphosphine (7.27 mg, 11.67 μmol), $Pd_2(dba)_3$ (5.34 mg, 5.84 μmol) and $Cs_2CO_3$ (57.05 mg, 175.11 μmol) were added to toluene (3 mL), and the reaction was stirred under the protection of nitrogen at 120°C for 16hr. After the reaction was complete, the mixture was directly separated and purified by Flash column chromatography on silica gel (DCM/MeOH=10/1), to afford compound 99d (35mg). MS m/z (ESI): 676.3 [M+H]+.

**Step 4: preparation of 4-amino-N-(1-((4-(2-(dimethylamino)ethoxy)-2-fluorophenyl)amino)-6-methylisoquinolin-5-yl)quinazoline-8-carboxamide (compound 99)**

Compound 99d (35 mg, 51.79 μmol) was dissolved in TFA (4 mL), and the reaction was stirred at 85°C for 4hr. After the reaction was complete, the reaction solution was concentrated to dryness, added with a saturated solution of sodium bicarbonate for alkalization, and extracted with ethyl acetate. The organic phase was dried over anhydrous sodium sulfate, filtered, concentrated, and the crude product was separated and purified by Prep-HPLC, to afford compound 99 (4.85mg). MS m/z (ESI): 526.2 [M+H]+.

$^1H$ NMR (400 MHz, DMSO-$d_6$) δ 13.17 (s, 1H), 8.90 (s, 1H), 8.65 (dd, *J* = 7.2, 1.2 Hz, 1H), 8.62 (s, 1H), 8.52 (dd, *J* = 8.4, 1.2 Hz, 1H), 8.44-8.15 (m, 3H), 7.80 (d, *J* = 6.0 Hz, 1H), 7.69 (t, *J* = 7.8 Hz, 1H), 7.57 (d, *J* = 8.8 Hz, 1H), 7.38 (t, *J* = 9.0 Hz, 1H), 7.13 (d, *J* = 6.0 Hz, 1H), 6.92 (dd, *J* = 12.4, 2.8 Hz, 1H), 6.80 (dd, *J* = 8.8, 2.4 Hz, 1H), 4.08 (t, *J* = 5.8 Hz, 2H), 2.64 (t, *J* = 5.8 Hz, 2H), 2.43 (s, 3H), 2.23 (s, 6H).

**Example 90: 4-amino-N-(6-methyl-1-((perfluorophenyl)amino)isoquinolin-5-yl)quinazoline-8-carboxamide (compound 100)**

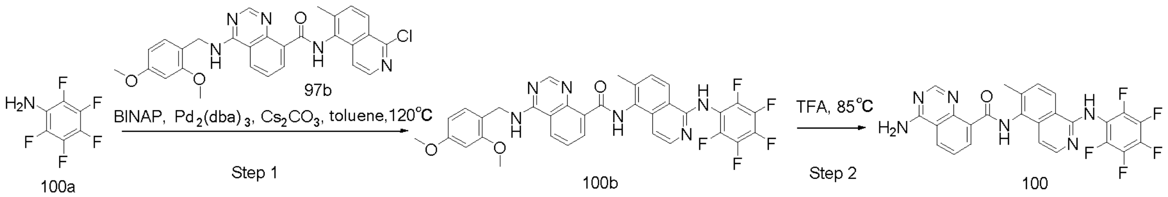


**Step 1: preparation of 4-((2,4-dimethoxybenzyl)amino)-N-(6-methyl-1-((perfluorophenyl)amino)isoquinolin-5-yl)quinazoline-8-carboxamide (compound 100b)**

Compounds 97b (40 mg, 0.08 mmol), 100a (17 mg, 0.09 mmol), 1,1'-binaphthalene-2,2'-bisdiphenylphosphine (10 mg, 0.015 mmol), $Pd_2(dba)_3$ (7 mg, 0.007 mmol) and $Cs_2CO_3$ (76 mg, 0.23 mmol) were added to toluene (3 mL), and the reaction was allowed to proceed under the protection of nitrogen at 120°C for 8 hours. After the reaction was complete, the reaction solution was filtered through diatomaceous earth, the filtrate the reaction was diluted by adding water, and extracted with EA. The organic phase was washed with a saturated saline solution, dried over anhydrous sodium sulfate, filtered, and concentrated, to afford compound 100b (50 mg). MS m/z (ESI): 661.2 [M+H]+.

**Step 2: preparation of 4-amino-N-(6-methyl-1-((perfluorophenyl)amino)isoquinolin-5-yl)quinazoline-8-carboxamide (compound 100)**

Compound 100b (50 mg, 0.075 mmol) was added to trifluoroacetic acid (4 mL), and the reaction was allowed to proceed at 85°C for 4 hours. After the reaction was complete, the reaction solution was concentrated to dryness, added with a saturated solution of sodium bicarbonate, and extracted with EA. The organic phase was washed with a saturated saline solution, dried over anhydrous sodium sulfate, filtered, concentrated, and the crude product was separated and purified by Prep-HPLC, to afford compound 100 (2.89 mg). MS m/z (ESI): 511.1 [M+H]+.

$^1H$ NMR (400 MHz, DMSO-$d_6$) δ 13.21 (s, 1H), 9.36 (s, 1H), 8.69 - 8.58 (m, 2H), 8.52 (d, *J* = 8.3 Hz, 1H), 8.44 - 8.18 (m, 3H), 7.88 (d, *J* = 5.9 Hz, 1H), 7.74 - 7.61 (m, 2H), 7.29 (d, *J* = 5.9 Hz, 1H), 2.46 (s, 3H).

**Example 91: 4-amino-N-(1-((3-chloro-2-fluorophenyl)amino)-6-methylisoquinolin-5-yl)-5-methyl-5H-pyrrolo[3,2-d]pyrimidine-7-carboxamide (compound 101)**

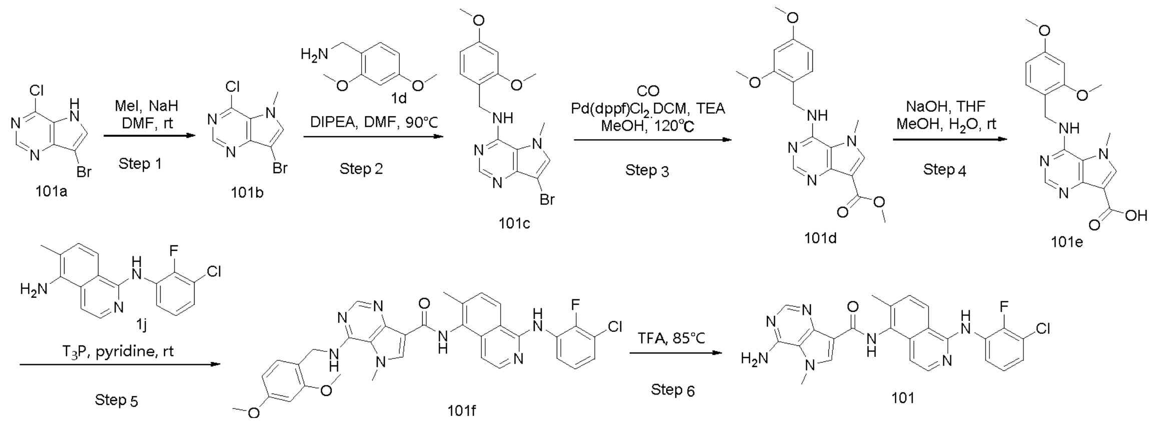


**Step 1: preparation of 7-bromo-4-chloro-5-methyl-5H-pyrrolo[3,2-d]pyrimidine (compound 101b)**

Compound 101a (500 mg, 2.15 mmol) was dissolved in DMF (6 mL), NaH (258.10 mg, 6.45 mmol, content 60%) was added, and then the reaction was stirred at room temperature for 30 min. Iodomethane (915.87 mg, 6.45 mmol) was then added, and the reaction was stirred at room temperature for 16hr. After the reaction was complete, the reaction solution was diluted by adding ethyl acetate, and washed with water for 3 times. The organic phase was dried over anhydrous sodium sulfate and then filtered, and concentrated under reduced pressure to afford compound 101b (530 mg). MS m/z (ESI): 245.9 $[M+H]^+$.

**Step 2: preparation of 7-bromo-N-(2,4-dimethoxybenzyl)-5-methyl-5H-pyrrolo[3,2-d]pyrimidin-4-amine (compound 101c)**

Compound 101b (530 mg, 2.15 mmol) was dissolved in DMF (4 mL), compound 1d (431.42 mg, 2.58 mmol) and DIPEA (833.67 mg, 6.45 mmol) were then sequentially added, and after the addition, the reaction was stirred at 90°C for 16hr. After the reaction was complete, the reaction solution was diluted by adding ethyl acetate, and washed with water for 3 times. The organic phase was dried over anhydrous sodium sulfate, filtered and then concentrated under reduced pressure, separated and purified by Flash column chromatography on silica gel (DCM/MeOH=94/6), to afford compound 101c (620 mg). MS m/z (ESI): 377.0 $[M+H]^+$.

**Step 3: preparation of methyl 4-((2,4-dimethoxybenzyl)amino)-5-methyl-5H-pyrrolo[3,2-d]pyrimidine-7-carboxylate (compound 101d)**

Compound 101c (400 mg, 1.06 mmol), Pd(dppf)$Cl_2$·DCM (86.59 mg, 106.03 μmol), MeOH (10 mL) and TEA (536.48 mg, 5.30 mmol) were sequentially added to a reaction kettle, purge with nitrogen was performed, CO was pumped in to 1.5MPa, and then the reaction was heated to 120°C and stirred for 5hr. After the reaction was complete, the reaction solution was cooled to room temperature, separated and purified by Flash column chromatography on silica gel (DCM/MeOH=92/8), to afford compound 101d (377 mg). MS m/z (ESI): 357.1 $[M+H]^+$.

**Step 4: preparation of 4-((2,4-dimethoxybenzyl)amino)-5-methyl-SH-pyrrolo[3,2-d]pyrimidine-7-carboxylic acid (compound 101e)**

Compound 101d (400 mg, 1.12 mmol) was dissolved in THF (10 mL) and MeOH (5 mL), a solution of sodium hydroxide(224.48 mg, 5.61 mmol) in water (5 mL) was added, and the reaction was stirred at 25°C for 6hr. After the reaction was complete, the reaction was evaporated under reduced pressure to remove the organic solvent, diluted by adding water, washed with DCM twice, the aqueous phase was adjusted to a pH of about 5 with 3M dilute hydrochloric acid, and then the solution was concentrated to dryness. The remaining solid was redissolved in dichloromethane and methanol (1:10), the insoluble solid was filtered off, and the filtrate was concentrated under reduced pressure, to afford compound 101e (300 mg). MS m/z (ESI): 343.1 $[M+H]^+$.

**Step 5: preparation of N-(1-((3-chloro-2-fluorophenyl)amino)-6-methylisoquinolin-5-yl)-4-((2,4-dimethoxybenzyl)amino)-5-methyl-5H-pyrrolo[3,2-d]pyrimidine-7-carboxamide (compound 101f)**

Compounds 1j (26.44 mg, 87.63 μmol) and 101e (30 mg, 87.63 μmol) were dissolved in pyridine (2 mL), $T_3P$ (0.5 mL, a 50% solution in EA) was then added, and the reaction was stirred at 25°C for 16hr. After the reaction was complete, the solvent was removed under reduced pressure, the reaction was quenched by adding a saturated solution of sodium bicarbonate, and extracted with EA. The organic phase was dried over anhydrous sodium sulfate, filtered, and concentrated, to afford compound 101f (50 mg). MS m/z (ESI): 626.1 $[M+H]^+$.

**Step 6: preparation** of **4-amino-N-(1-((3-chloro-2-fluorophenyl)amino)-6-methylisoquinolin-5-yl)-5-methyl-5H-pyrrolo[3,2-d]pyrimidine-7-carboxamide (compound 101)**

Compound 101f (50 mg, 79.86 μmol) was dissolved in TFA (4 mL), and the reaction was stirred at 85°C for 4hr. After the reaction was complete, the reaction solution was concentrated to dryness, added with a saturated solution of sodium bicarbonate for alkalization, and extracted with ethyl acetate. The organic phase was dried over anhydrous sodium sulfate, filtered, concentrated, and the crude product was separated and purified by Prep-HPLC, to afford compound 101 (3.68 mg). MS m/z (ESI): 476.1 $[M+H]^+$.

$^1H$ NMR (400 MHz, DMSO-$d_6$) δ 10.52 (s, 1H), 9.23 (s, 1H), 8.36 - 8.29 (m, 2H), 8.13 (s, 1H), 7.92 - 7.85 (m, 1H), 7.60 (d, *J* = 8.8 Hz, 1H), 7.57 - 7.50 (m, 1H), 7.38 - 7.31 (m, 1H), 7.22 (td, *J* = 8.4, 1.2 Hz, 1H), 7.18 - 7.06 (m, 3H), 4.11 (s, 3H), 2.42 (s, 3H).

**Example 92: 4-amino-N-(4-((3-chloro-2-fluorophenyl)amino)-7-methylquinazolin-8-yl)quinazoline-8-carboxamide (compound 102)**

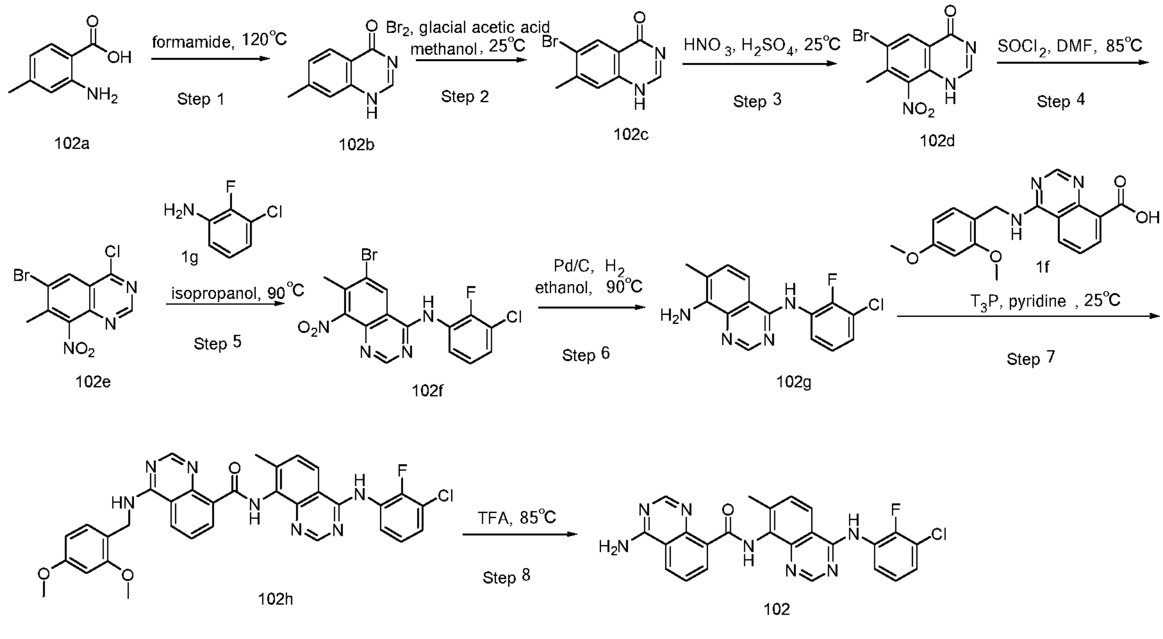


**Step 1: preparation of 7-methylquinazolin-4(1H)-one (compound 102b**)

Compound 102a (3 g, 19.85 mmol) was added to formamide (20 mL), and heated to 120°C for 5 hours. After the reaction was complete, ice water was added to the reaction solution under stirring, a large amount of white solid precipitated, which was filtered. The filter cake was rinsed with water, dried to afford compound 102b (2g). MS m/z (ESI): 161.1 $[M+H]^+$.

**Step 2: preparation of 6-bromo-7-methylquinazolin-4(1H)-one (compound 102c)**

Compound 102b (2 g, 12.49 mmol) was added to glacial acetic acid (12 mL) and methanol (12 mL), liquid bromine (1 mL) was slowly dropwise added at ambient temperature under stirring, and then the reaction was allowed

to proceed at 25°C for 3 hours. After the reaction was complete, a saturated aqueous solution of sodium thiosulfate was added to the reaction solution to quench the reaction. The solution was extracted with EA for three times, the organic phases were combined, washed with water for three times, washed with saturated brine once, dried over anhydrous sodium sulfate, filtered, and concentrated to afford compound 102c (2.7 g). MS m/z (ESI): 239.0 $[M+H]^+$.

**Step 3: preparation of 6-bromo-7-methyl-8-nitroquinazolin-4(1H)-one (compound 102d)**

Compound 102c (500 mg, 2.09 mmol) was added to concentrated sulfuric acid (10 mL), fuming nitric acid (0.5 mL) was slowly dropwise added at 0°C, and then the reaction was allowed to proceed at 25°C for 5 hours. After the reaction was complete, the reaction solution was poured into ice water, and extracted with EA. The organic phase was washed with water and saturated brine, dried over anhydrous sodium sulfate, filtered, and concentrated to afford compound 102d (353 mg). MS m/z (ESI): 283.9 $[M+H]^+$.

**Step 4: preparation of 6-bromo-4-chloro-7-methyl-8-nitroquinazoline (compound 102e**)

Compound 102d (230 mg, 0.81 mmol) was added to thionyl chloride(4 mL), DMF (0.5 mL) was then added, and the reaction was heated to 85°C for 1 hour. After the reaction was complete, the reaction solution was concentrated under reduced pressure to dryness, to afford compound 102e (244 mg).

**Step 5: preparation of 6-bromo-N-(3-chloro-2-fluorophenyl)-7-methyl-8-nitroquinazolin-4-amine (compound 102f)**

Compounds 102e (244mg, 0.79 mmol) and 1g (347 mg, 2.38 mmol) were added to isopropanol (8 mL), and then the reaction was heated to 90°C and allowed to proceed for 1 hour. After the reaction was complete, the reaction solution was concentrated to dryness, added ethyl acetate, washed with water and saturated brine, dried over anhydrous sodium sulfate, filtered, concentrated, and the crude product was separated and purified by column chromatography on silica gel (PE/EA=7/3), to afford compound 102f (130 mg).

**Step 6: preparation of $N^4$-(3-chloro-2-fluorophenyl)-7-methylquinazoline-4,8-diamine (compound 102g**)

Compound 102f (85 mg, 0.21 mmol) was added to ethanol (4 mL), 10% Pd/C (25 mg, 0.21 mmol) was then added, and the reaction was heated to 85°C under an atmosphere of hydrogen, and allowed to proceed for 1 hour. After the reaction was complete, the reaction solution was filtered through diatomaceous earth, and the filtrate was concentrated to dryness, to afford compound 102g (60 mg). MS m/z (ESI): 303.0 $[M+H]^+$.

**Step 7: preparation of N-(4-((3-chloro-2-fluorophenyl)amino)-7-methylquinazolin-8-yl)-4-((2,4-dimethoxybenzyl)amino)quinazoline-8-carboxamide (compound 102h)**

Compounds 102g (15 mg, 49.55 μmol) and 1f (17 mg, 49.55 μmol) were dissolved in pyridine (4.0 mL), $T_3P$ (0.5 mL, a 50% solution in EA) was dropwise added, and the reaction was allowed to proceed at room temperature for 16 hours. After the reaction was complete, the solvent was removed by concentration under reduced pressure, the residue was adjusted to pH of about 13 with a saturated aqueous solution of sodium bicarbonate, and extracted with ethyl acetate. The organic phase was washed with a saturated saline solution, dried over anhydrous sodium sulfate, filtered, and concentrated, to afford compound 102h (30 mg). MS m/z (ESI): 624.2 $[M+H]^+$.

**Step 8: preparation of 4-amino-N-(4-((3-chloro-2-fluorophenyl)amino)-7-methylquinazolin-8-yl)quinazoline-8-carboxamide (compound 102)**

Compound 102h (30 mg, 48.07 μmol) was added to trifluoroacetic acid (3.0 mL), and the reaction was allowed to proceed at 85°C for 4 hours. After the reaction was complete, the reaction solution was concentrated to dryness, added with a saturated solution of sodium bicarbonate, and extracted with ethyl acetate. The organic phase was washed with a saturated saline solution, dried over anhydrous sodium sulfate, filtered, concentrated, and the crude product was separated and purified by Prep-HPLC, to afford compound 102 (0.31 mg). MS m/z (ESI): 474.0 $[M+H]^+$.

$^1H$ NMR (400 MHz, DMSO-$d_6$) δ 13.51 (s, 1H), 10.01 (s, 1H), 8.70 (d, J=7.4, 1H), 8.59 (s, 1H), 8.51 (s, 1H), 8.31 (d, J=8.7, 1H), 7.73 - 7.60 (m, 2H), 7.60 - 7.47 (m, 2H), 7.30 (t, J=8.6, 1H), 7.22 (s, 1H), 6.67 (s, 2H), 2.44 (s, 3H).

**Comparative Example 1: preparation of 1-amino-N-(1-((3-chloro-2-fluorophenyl)amino)-6-methylisoquinolin-5-yl)isoquinoline-5-carboxamide (comparative compound 1)**

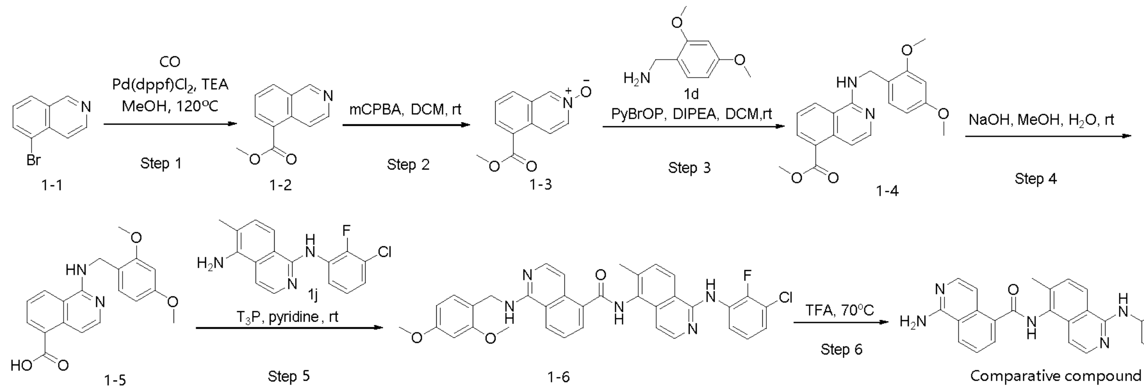


**Step 1: preparation of methyl isoquinoline-5-carboxylate (compound 1-2)**

Compound 1-1 (2 g, 9.61 mmol), TEA (4.86 g, 48.06 mmol), Pd(dppf)$Cl_2$·DCM (785.03 mg, 961.29 μmol) and MeOH (8.3 mL) were added to an autoclave, purge with nitrogen was performed twice, carbon monoxide was then pumped in to a pressure of 1.0-1.2MPa, and then the reaction was warmed to 120°C and allowed to proceed for 5 hours. After the reaction was complete, the reaction solution was directly concentrated under reduced pressure to dryness, and the crude product was separated and purified by column chromatography on silica gel (EA/PE=1/4), to afford compound 1-2 (1.6 g). MS m/z (ESI): 188.1 $[M+H]^+$.

**Step 2: preparation of 5-(methoxycarbonyl)isoquinoline 2-oxide(compound 1-3)**

Compound 1-2 (1.5 g, 8.01 mmol) was dissolved in DCM (30 mL), mCPBA (2.77 g, 16.03 mmol) was then added, and the reaction was allowed to proceed at room temperature under the protection of nitrogen for 15 hours. After the reaction was complete, the reaction solution was directly concentrated under reduced pressure to dryness, and the crude product was separated and purified by column chromatography on silica gel (MeOH/DCM=3/97), to afford compound 1-3 (1.05g). MS m/z (ESI): 204.0 $[M+H]^+$.

**Step 3: preparation of methyl 1-((2,4-dimethoxybenzyl)amino)isoquinoline-5-carboxylate (compound 1-4)**

Compounds 1-3 (1.05 g, 5.17 mmol), 1d (1.73 g,10.33 mmol) were dissolved in DCM (70 mL), PyBrOP (4.82 g, 10.33 mmol) and DIPEA (2.67 g, 20.67 mmol) were then added, and then the reaction was allowed to proceed at room temperature under the protection of nitrogen for 16 hours. After the reaction was complete, the reaction solution was directly concentrated under reduced pressure to dryness, and the crude product was separated and purified by column chromatography on silica gel (EA/PE=1/3), to afford compound 1-4 (1.26 g). MS m/z (ESI): 353.1 $[M+H]^+$.

**Step 4: preparation of 1-((2,4-dimethoxybenzyl)amino)isoquinoline-5-carboxylic acid (compound 1-5)**

Compound 1-4 (1.26 g, 3.58 mmol) was dissolved in MeOH (30 mL), a solution of NaOH (572.10 mg, 14.30 mmol) in water (6 mL) was then added, and the reaction was allowed to proceed under the protection of nitrogen at room temperature overnight. After the reaction was complete, the reaction solution was adjusted to a pH of 5-6 with 2N hydrochloric acid, and evaporated under reduced pressure to remove the organic solvent. The precipitated solid was filtered, the filter cake was washed with water, and dried under reduced pressure, to afford compound 1-5 (800 mg). MS m/z (ESI): 339.1 $[M+H]^+$.

**Step 5: preparation of N-(1-((3-chloro-2-fluorophenyl)amino)-6-methylisoquinolin-5-yl)-1-((2,4-dimethoxybenzyl)amino)isoquinoline-5-carboxamide (compound 1-6)**

Compounds 1-5 (140 mg, 413.76 μmol) and 1j (124.85 mg, 413.76 μmol) were added to pyridine (6 mL), $T_3P$ (4 mL, 50% in DMF) was then added, and the reaction was allowed to proceed under the protection of nitrogen at room

temperature overnight. After the reaction was complete, the reaction solution was directly rotary evaporated to dryness, added with a saturated solution of sodium bicarbonate for alkalization, and extracted with EA. The organic phase was washed with brine, dried over anhydrous sodium sulfate, filtered, concentrated, and the crude product was separated and purified by column chromatography on silica gel (DCM/MeOH=10/1), to afford compound 1-6 (170.0 mg). MS m/z (ESI): 622.2 $[M+H]^+$.

**Step 6: preparation of 1-amino-N-(1-((3-chloro-2-fluorophenyl)amino)-6-methylisoquinolin-5-yl)isoquinoline-5-carboxamide (comparative compound 1)**

Compound 1-6 (190 mg, 305.42 μmol) was added to trifluoroacetic acid (7.0 mL), and the reaction was allowed to proceed at 70°C for 2 hours. After the reaction was complete, the reaction solution was concentrated to dryness, added with a saturated solution of sodium bicarbonate for alkalization, and extracted with ethyl acetate. The organic phase was dried over anhydrous sodium sulfate, filtered, concentrated, and the crude product was separated and purified by Prep-HPLC, to afford comparative compound 1 (45.0 mg). MS m/z (ESI): 472.0 $[M+H]^+$.

$^1H$ NMR (400 MHz, DMSO-$d_6$) δ 10.37 (s, 1H), 9.24 (s, 1H), 8.41 - 8.33 (m, 2H), 8.12 - 8.08 (m, 1H), 7.95 (d, *J* = 6.0 Hz, 1H), 7.89 (d, *J* = 6.0 Hz, 1H), 7.65 - 7.59 (m, 2H), 7.57 - 7.52 (m, 1H), 7.39 - 7.34 (m, 1H), 7.28 - 7.21 (m, 3H), 6.96 (s, 2H), 2.51 (s, 3H).

**Comparative Example 2: 1-amino-N-(6-methyl-1-((3-(trifluoromethyl)phenyl)amino)isoquinolin-5-yl)isoquinoline-5-carboxamide (comparative compound 2)**

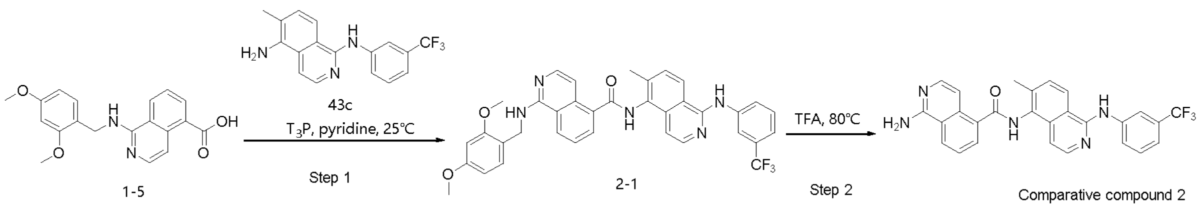


1-5 2-1 Comparative compound 2

**Step 1: preparation of 1-((2,4-dimethoxybenzyl)amino)-N-(6-methyl-1-((3-(trifluoromethyl)phenyl)amino)isoquinolin-5-yl)isoquinoline-5-carboxamide (compound 2-1)**

Compounds 43c (300 mg, 945.45 μmol) and 1-5 (383.38 mg, 1.13 mmol) were added to pyridine (2.0 mL), $T_3P$ (902.47 mg, 2.84 mmol) was then added, and the reaction was allowed to proceed at 25°C for 2 hours. After the reaction was complete, the reaction was added with 100 ml water, solid precipitated, which was filtered and collected, and dried to afford compound 2-1 (325 mg). MS m/z (ESI): 638.3 $[M+H]^+$.

**Step 2: preparation of 1-amino-N-(6-methyl-1-((3-(trifluoromethyl)phenyl)amino)isoquinolin-5-yl)isoquinoline-5-carboxamide (comparative compound 2)**

Compound 2-1 (300 mg, 470.48 μmol) was added to trifluoroacetic acid (10.0 mL), and the reaction was allowed to proceed at 80°C for 12 hours. After the reaction was complete, the reaction solution was concentrated to dryness, added with a saturated solution of sodium bicarbonate for alkalization, and extracted with ethyl acetate. The organic phase was dried over anhydrous sodium sulfate, filtered, concentrated, and the crude product was separated and purified by Prep-HPLC, to afford comparative compound 2 (195 mg). MS m/z (ESI): 488.1 $[M+H]^+$.

$^1H$ NMR (400 MHz, DMSO-$d_6$) δ 10.40 (s, 1H), 9.52 (s, 1H), 8.50 (d, *J* = 9.2 Hz, 1H), 8.40 (d, *J* = 8.4 Hz, 1H), 8.37 (s, 1H), 8.26 (d, *J* = 8.8 Hz, 1H), 8.14-8.10 (m, 2H), 7.90 (d, *J* = 6.0 Hz, 1H), 7.69-7.61 (m, 2H), 7.56 (t, *J* = 8.0 Hz, 1H), 7.37-7.26 (m, 3H), 7.04 (s, 2H), 2.52 (s, 3H).

**Separation Method**

Prep-HPLC purification of the compounds in the Examples were performed on Aglient Model 1260 or Waters Model 2489 HPLC, separation column model was Waters SunFire Prep $C_{18}$ OBD (19 mm×150 mm×5.0 μm), Waters Xbridge Prep $C_{18}$ OBD (19 mm×150 mm×5.0 μm) or YMC Actus Triart $C_{18}$ (20 mm×150 mm×5.0 μm), column temperature was always 25°C, detecting wavelength was 214 nm, 254 nm or 280 nm, mobile phase A was acetonitrile, mobile phase B was a 0.05% aqueous solution of formic acid or a 0.05% aqueous solution of ammonium bicarbonate

or a 0.05% aqueous solution of TFA, the volume ratio of the mobile phase was adjusted according to the polarity of the compounds; the flow rate of the mobile phase was 28 mL/min.

## Biological evaluation

### Experimental Example 1: RAF inhibition assay

Assay protocol: the inhibitory effects of the compounds of the present invention on the wild-type BRAF enzyme, mutant CRAF enzyme (RAF1 Y340D and Y341D) and mutant BRAF enzyme (BRAF-V600E) activity were determined according to the instructions of the TB-PMAP2K1 (PSER217/221) kit (ThermoFisher). Different RAF enzymes and substrates (FLUORESCEIN-MAP2K1) were pre-incubated with test compounds at various concentrations at room temperature for 15 min, and then the adenosine triphosphate (ATP) was added to initiate the reaction. After incubation at room temperature for 60 min, EDTA and Tb-labeled anti-pMAP2K1 [pS217/221] antibody solutions were added, and incubation was performed at room temperature for 60 min before the detection of the fluorescence values of the compounds in each group. With the vehicle group (DMSO) as the negative control and the buffer group (without RAF enzyme) as the blank control, the relative inhibitory activity percentage (i.e., the inhibition rate) of a compound at different concentrations was calculated according to the following formula:

$$\text{Relative inhibitory activity percentage} = 1 - (\text{compound group at various concentrations} - \text{blank control})/(\text{negative control} - \text{blank control}) * 100\%$$

The relative inhibitory activity percentages of the compounds at different concentrations were plotted against the compound concentrations, the curve was fitted according to a four-parameter model, and the $IC_{50}$ value was calculated according to the following formula:

$$y=\min+(\max-\min)/(1+(x/IC_{50})^{\wedge}(-\text{Hillslope}))$$

wherein y is the relative inhibitory activity percentage, max and min are respectively the maximum and minimum values of the fitted curve, x is the logarithm concentration of the compound, and Hillslope is the slope of the curve. The test results are shown in Table 1-Table 3.

Table 1: the inhibitory activity of the compounds of the present invention on the BRAF enzyme

| Compound No. | BRAF $IC_{50}$ (nM) |
|---|---|
| 1 | 18.69±2.71 |
| 19 | 21.64±4.85 |
| 22 | 50.17±8.47 |
| 23 | 51.44±1.64 |
| 28 | 9.43±0.76 |
| 35 | 124.9±3.5 |
| 38 | 23.17±2.67 |
| 39 | 8.44±2.46 |
| 41 | 31.76±5.36 |
| 43 | 20.74±5.71 |
| 46 | 79.31±19.51 |
| 58 | 10.81±1.09 |
| 60 | 8.48±0.90 |
| 67 | 13.21±1.67 |
| 73 | 36.23±16.50 |

(continued)

| Compound No. | BRAF $IC_{50}$ (nM) |
|---|---|
| 85 | 12.92±0.90 |
| 89 | 20.42±1.30 |
| 90 | 26.50±2.18 |
| 92 | 21.78±3.89 |
| 93 | 13.48±1.02 |
| 94 | 10.30±1.00 |

Table 2: the inhibitory activity of the compounds on the BRAF V600E enzyme

| Compound No. | BRAF V600E $IC_{50}$ (nM) |
|---|---|
| 1 | 4.71±2.62 |
| 2 | 35.6±2.4 |
| 4 | 7.0±0.3 |
| 5 | 8.90±1.03 |
| 6 | 25.2±4.08 |
| 7 | 28.3±1.6 |
| 8 | 8.63±0.99 |
| 10 | 4.73±1.82 |
| 11 | 33.1±7.2 |
| 12 | 1.50±0.20 |
| 13 | 32.8±0.6 |
| 14 | 6.3±0.7 |
| 15 | 11.1±1.5 |
| 16 | 6.27±1.15 |
| 17 | 5.73±1.05 |
| 18 | 8.33±1.25 |
| 19 | 13.91±3.24 |
| 20 | 10.65±1.93 |
| 21 | 5.99±1.82 |
| 22 | 26.82±6.82 |
| 23 | 13.25±3.17 |
| 24 | 7.10±0.70 |
| 25 | 6.31±0.99 |
| 26 | 9.00±0.77 |
| 27 | 6.48±0.93 |
| 28 | 4.58±0.72 |
| 30 | 4.1±0.2 |
| 33 | 33.52±6.13 |

(continued)

| Compound No. | BRAF V600E $IC_{50}$ (nM) |
|---|---|
| 34 | 26.90±2.58 |
| 35 | 50.41±12.59 |
| 36 | 22.52±4.41 |
| 37 | 20.38±3.03 |
| 38 | 9.50±2.68 |
| 39 | 1.74±0.23 |
| 40 | 8.51±1.02 |
| 41 | 12.70±2.28 |
| 42 | 35.78±9.02 |
| 43 | 4.84±0.97 |
| 44 | 0.88±0.15 |
| 45 | 18.21±7.52 |
| 46 | 16.17±5.93 |
| 53 | 17.32±3.43 |
| 54 | 10.98±2.02 |
| 55 | 2.86±0.24 |
| 56 | 3.16±0.67 |
| 57 | 2.89±0.34 |
| 58 | 4.47±0.60 |
| 59 | 13.03±1.35 |
| 60 | 6.31±1.09 |
| 61 | 2.88±0.74 |
| 62 | 19.67±4.67 |
| 63 | 5.39±0.80 |
| 64 | 12.95±3.58 |
| 65 | 4.80±0.78 |
| 66 | 2.57±0.32 |
| 67 | 8.66±0.95 |
| 68 | 15.01±2.65 |
| 69 | 4.30±1.14 |
| 70 | 10.47±1.72 |
| 71 | 22.97±1.76 |
| 72 | 6.9±1.3 |
| 73 | 11.9±2.3 |
| 74 | 13.8±0.9 |
| 75 | 5.58±0.77 |
| 76 | 29.27±3.32 |
| 77 | 7.90±0.73 |

(continued)

| Compound No. | BRAF V600E $IC_{50}$ (nM) |
|---|---|
| 78 | 12.36±0.94 |
| 79 | 14.67±1.66 |
| 80 | 13.49±12.12 |
| 81 | 22.00±3.69 |
| 82 | 13.18±1.69 |
| 83 | 15.08±1.62 |
| 84 | 23.82±4.41 |
| 85 | 9.02±0.86 |
| 86 | 11.89±1.30 |
| 87 | 4.64±0.70 |
| 88 | 11.72±0.99 |
| 89 | 7.58±0.51 |
| 90 | 18.65±2.25 |
| 91 | 20.89±2.81 |
| 92 | 5.34±0.75 |
| 93 | 5.03±0.54 |
| 94 | 3.46±0.22 |
| 95 | 13.93±1.48 |
| 98 | 21.95±6.38 |
| 101 | 5.58±0.57 |
| 102 | 12.39±3.05 |
| Comparative Example 1 | >1000 |
| Comparative Example 2 | >1000 |

Table 3: the inhibitory activity of the compounds of the present invention on the CRAF enzyme (RAF1 Y340D and Y341D)

| Compound No. | CRAF (RAF1 Y340D and Y341D) $IC_{50}$ (nM) |
|---|---|
| 1 | 5.4±0.6 |
| 19 | 10.34±2.29 |
| 22 | 14.48±9.94 |
| 23 | 3.93±1.00 |
| 28 | 3.48±0.48 |
| 35 | 15.43±1.89 |
| 38 | 10.78±2.02 |
| 39 | 2.60±0.21 |
| 41 | 24.96±9.32 |
| 43 | 4.49±0.95 |
| 46 | 38.1±21.9 |

(continued)

| Compound No. | CRAF (RAF1 Y340D and Y341D) $IC_{50}$ (nM) |
|---|---|
| 58 | 2.68±0.13 |
| 60 | 3.14±0.09 |
| 67 | 5.76±1.04 |
| 73 | 8.78±1.48 |
| 85 | 4.43±0.32 |
| 89 | 4.26±0.16 |
| 90 | 14.25±6.59 |
| 92 | 10.83±4.78 |
| 93 | 5.73±0.36 |
| 94 | 4.24±0.17 |

The test results show that the compounds of the present invention have strong inhibitory effects on BRAF, BRAF V600E and CRAF (RAF1 Y340D and Y341D) enzymes.

**Experimental Example 2: Cancer cell proliferation inhibition assay**

The inhibitory effects of the compounds of the present invention on cancer cell proliferation were further evaluated by testing the effects of the compounds of the present invention on the growth of cancer cells.
In this example, human melanoma cell A375 (BRAF-V600E mutation, purchased from the Cell Bank of the Chinese Academy of Sciences) and human hepatocellular carcinoma cell HepG2 (NRAS-Q61K mutation, purchased from ATCC, USA) were employed.
In this example, the test compounds at various concentrations (100000 nM, 30000 nM, 10000 nM, 3000 nM, 1000 nM, 300 nM, 100 nM, 30 nM, 10 nM) were added to the above cells, and the incubation was performed for 72 h. The inhibitory activity of the compounds was tested by measuring ATP in living cells using the Cell Titer Glo Kit (Promega).
The $IC_{50}$ values of the compounds of the present invention for each tested cell were determined, and the test results are as follows:

Table 4: Inhibitory activity of the compounds of the present invention on human hepatocellular carcinoma cell HepG2

| Compound No. | HepG2 ($IC_{50}$ nM) |
|---|---|
| 1 | 71.3±5.5 |
| 12 | 13.9±1.7 |
| 19 | 136.1±21.3 |
| 30 | 13.3±0.9 |
| 38 | 38.1±3.6 |
| 39 | 27.1±2.5 |
| 41 | 55.6±4.8 |
| 43 | 32.9±3.3 |
| 45 | 64.30±5.08 |
| 46 | 74.43±7.91 |
| 53 | 113.48±3.35 |
| 54 | 95.64±8.18 |
| 55 | 30.4±4.3 |
| 56 | 22.2±2.9 |

(continued)

| Compound No. | HepG2 ($IC_{50}$ nM) |
|---|---|
| 57 | 24.3±3.3 |
| 58 | 37.57±3.34 |
| 59 | 210.95±16.60 |
| 60 | 34.15±4.32 |
| 61 | 21.34±4.88 |
| 62 | 82.25±10.08 |
| 63 | 28.6±2.7 |
| 64 | 75.2±6.9 |
| 65 | 38.8±3.0 |
| 66 | 46.1±9.6 |
| 67 | 56.7±9.3 |
| 68 | 109.1±12.2 |
| 69 | 49.6±9.7 |
| 70 | 59.9±6.8 |
| 71 | 53.0±9.7 |
| 72 | 64.3±12.3 |
| 73 | 59.6±2.2 |
| 74 | 62.3±9.2 |
| 75 | 34.0±7.6 |
| 76 | 147.3±19.2 |
| 77 | 51.4±8.5 |
| 78 | 56.4±5.8 |
| 79 | 144.7±12.3 |
| 80 | 75.4±6.0 |
| 81 | 222.1±29.8 |
| 82 | 244.8±27.3 |
| 83 | 50.46±8.48 |
| 84 | 67.15±9.93 |
| 85 | 40.14±6.97 |
| 86 | 47.99±5.36 |
| 87 | 37.72±7.05 |
| 88 | 70.2±9.8 |
| 89 | 105.6±25.2 |
| 90 | 118.3±12.6 |
| 91 | 139.7±17.0 |
| 92 | 49.8±4.3 |
| 93 | 62.9±11.3 |
| 94 | 47.4±6.5 |

(continued)

| Compound No. | HepG2 ($IC_{50}$ nM) |
|---|---|
| 95 | 69.0±18.9 |
| 96 | 119.0±29.3 |
| 98 | 63.7±4.4 |
| 101 | 81.1±4.5 |

Table 5: Inhibitory activity of the compounds of the present invention on human melanoma cell A375

| Compound No. | A375 ($IC_{50}$ nM) |
|---|---|
| 1 | 29.3±2.3 |
| 19 | 162.6±21.3 |
| 23 | 64.67±4.22 |
| 24 | 70.68±6.15 |
| 28 | 98.75±10.17 |
| 30 | 89.3±11.3 |
| 38 | 96.7±14.3 |
| 41 | 22.2±1.86 |
| 43 | 61.7±3.0 |
| 45 | 74.2±3.5 |
| 46 | 89.3±5.2 |
| 53 | 77.7±2.3 |
| 54 | 107.7±19.9 |
| 56 | 67.0±7.3 |
| 57 | 66.1±17.0 |
| 59 | 85.0±5.2 |
| 60 | 28.02±3.12 |
| 61 | 141.43±4.88 |
| 62 | 49.13±2.74 |
| 63 | 46.33±1.79 |
| 64 | 70.33±8.50 |
| 65 | 47.1±9.4 |
| 66 | 166.3±6.1 |
| 67 | 184.3±16.9 |
| 68 | 103.5±20.6 |
| 69 | 94.6±4.0 |
| 70 | 190.1±23.8 |
| 71 | 170.0±13.8 |
| 73 | 109.3±22.8 |
| 74 | 62.7±12.5 |

(continued)

| Compound No. | A375 ($IC_{50}$ nM) |
|---|---|
| 75 | 67.72±15.10 |
| 76 | 201.71±5.74 |
| 77 | 31.58±7.42 |
| 78 | 63.53±2.60 |
| 79 | 72.16±13.01 |
| 80 | 166.69±8.47 |
| 82 | 141.41±44.10 |
| 83 | 61.9±6.7 |
| 84 | 165.2±6.5 |
| 85 | 148.3±3.8 |
| 86 | 60.6±2.8 |
| 87 | 56.0±2.5 |
| 88 | 55.4±8.0 |
| 89 | 110.9±5.2 |
| 90 | 74.0±6.3 |
| 91 | 87.1±10.4 |
| 92 | 127.6±8.0 |
| 93 | 61.7±3.6 |
| 94 | 58.8±5.6 |

The test results show that the compounds of the present invention have strong inhibitory effects on the cell line A375 expressing BRAF-V600E and the cell line HepG2 expressing NRAS-Q61K.

**Experimental Example 3: Pharmacokinetic test of the compound in balb/c mice**

The compounds were administered to female Balb/c mice by gavage (PO) to investigate the pharmacokinetic profile.

Compound 1 of the present invention and HM95573 (prepared according to Example 116 in CN104039798B) were administered via PO; the administration dosage was 10 mg/kg, and the administration vehicle was a 20% aqueous solution of hydroxypropyl-β-cyclodextrin. Blood samples were collected before the administration (0 h) and at 0.25, 0.5, 1, 2, 4, 6, 8, 24, 48, and 72 h after the administration; 50-70 μL of blood was drawn from the orbit and spotted on Whatman 903 card, which was air-dried at room temperature for testing. After the testing, the samples were placed in a sealed dry box and stored at -80°C (DBS samples). The DBS samples were punched with a 6 mm punch, then 80 μL of 50% acetonitrile-water was added and shaken (800 rpm, 10 min), followed by sonication for 10 min, then 350 μL of acetonitrile containing internal standard was added and shaken (700 rpm, 10 min). After sonication for 10 min, the samples were centrifuged at 4000 rpm and 4°C for 10 min, and the supernatant was collected for LC-MS/MS analysis. The pharmacokinetic parameters were calculated using the WinNonlin 6.3 software and employing a non-compartmental model, and the results are shown in Table 6.

Table 6: Pharmacokinetic parameters of the compounds in mice

| Compound No. | Route of administration | Dosage mg/kg | $AUC_{last}$ h*ng/mL | $C_{max}$ ng/mL | $T_{1/2}$ h | $T_{max}$ h |
|---|---|---|---|---|---|---|
| 1 | PO | 10 | 171613±18260 | 8240±586 | 8.33±0.95 | 1.67±0.58 |

(continued)

| Compound No. | Route of administration | Dosage mg/kg | $AUC_{last}$ h*ng/mL | $C_{max}$ ng/mL | $T_{1/2}$ h | $T_{max}$ h |
|---|---|---|---|---|---|---|
| HM95573 | PO | 10 | 110760±9636 | 4710±806 | 9.57±0.58 | 1.67±0.58 |
| Conclusion: compound 1 of the present invention has a high maximum plasma concentration and a high exposure in mice after PO administration. | | | | | | |

**Experimental Example 4: Pharmacokinetic test of the compound in Beagle dogs**

The compounds were administered to male Beagle dogs by gavage (PO) to investigate the pharmacokinetic profile.

Compound 1 of the present invention and HM95573 were administered via PO; the administration dosage was 2.5 mg/kg, and the solvent was a 20% aqueous solution of hydroxypropyl-β-cyclodextrin. Blood samples were collected before the administration (0 h) and at 0.25, 0.5, 1, 2, 4, 6, 8, 24, 48, and 72 hours after the administration, and 1 mL blood was collected from the limbs vein and placed in a $K_2$-EDTA anticoagulation tube. After centrifugation at 4000 rpm for 5 min (4 °C), the plasma was separated and stored at -80°C for testing. The plasma samples were processed by precipitating proteins, and then subjected to LC-MS/MS analysis. The pharmacokinetic parameters were calculated using the WinNonlin 6.3 software and employing a non-compartmental model, and the results are shown in Table 7.

Table 7: Pharmacokinetic parameters of the compounds in Beagle dogs

| Compound No. | Route of administration | Dosage mg/kg | $AUC_{last}$ h*ng/mL | $C_{max}$ ng/mL | $T_{1/2}$ h | $T_{max}$ h |
|---|---|---|---|---|---|---|
| 1 | PO | 2.5 | 31009±4241 | 1803±127 | 15.4±4.1 | 1.00±0.00 |
| HM95573 | PO | 2.5 | 13080±6254 | 989±411 | 8.43±1.76 | 1.33±0.58 |
| Conclusion: Compound 1 of the present invention has a high maximum plasma concentration, high exposure, long half-life and good overall PK properties in dogs after PO administration. | | | | | | |

In addition to those described herein, according to the foregoing description, various modifications to the present invention would be apparent to those skilled in the art. Such modifications are intended to fall within the scope of the appended claims. Each reference cited herein (including all patents, patent applications, journal articles, books and any other disclosures) are incorporated herein by reference in its entirety.

**Claims**

1. A compound of Formula I', or a pharmaceutically acceptable salt, ester, stereoisomer, tautomer, polymorph, cocrystal, solvate, metabolite, isotopically labeled compound, N-oxide or prodrug thereof:

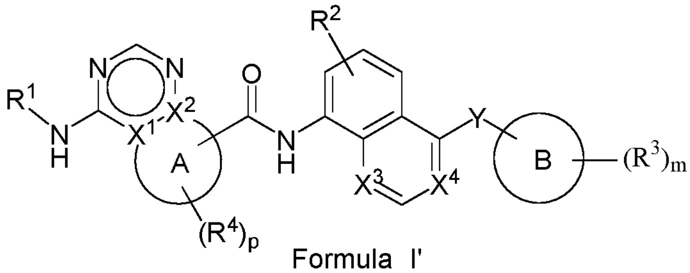

Formula I'

wherein:

ring A is selected from the group consisting of benzene ring and 5-6-membered heteroaromatic ring;
ring B is selected from the group consisting of $C_{6-10}$ aromatic ring, 5-10-membered heteroaromatic ring and 4-10-membered heterocycle;
$X^1$ and $X^2$ are each independently selected from the group consisting of C and N;
$X^3$ and $X^4$ are each independently selected from the group consisting of CH and N;

Y is selected from the group consisting of -O-, -NH-, -C(=O)-, $-CR^5R^6-$, $-CR^5R^6O-$ and $-CR^5R^6NH-$; provided that, when ring A is a thiophene ring, Y is not -O- or -NH-;

$R^1$ is selected from the group consisting of H, $C_{1-6}$ alkyl and $C_{3-6}$ cycloalkyl, the alkyl and cycloalkyl are each optionally substituted with one or more halogens;

$R^2$ is selected from the group consisting of H, halogen, $C_{1-6}$ alkyl and $C_{1-6}$ alkoxy, the alkyl and alkoxy are each optionally substituted with one or more halogens;

$R^3$ is $L-R^{3'}$;

L, at each occurrence, is each independently a direct bond or $-(CH_2)_n-$;

$R^{3'}$, at each occurrence, is each independently selected from the group consisting of H, hydroxyl, halogen, CN, NOz, $C_{1-6}$ alkyl, $C_{1-6}$ heteroalkyl (e.g., $C_{1-6}$ alkoxy), $C_{2-6}$ alkenyl, $C_{2-6}$ alkynyl, $C_{3-8}$ cycloalkyl, $C_{3-8}$ cycloalkoxy, 4-10-membered heterocyclyl, $C_{6-12}$ aryl, 5-10-membered heteroaryl, $-NR^{20a}R^{20b}$, $-SR^{21}$, - $S(=O)_2R^{22}$, $-S(=O)_2NR^{20a}R^{20b}$, $-NR^{20a}S(=O)_2R^{20b}$, $-C(=O)R^{21}$, $-C(=O)NR^{23a}R^{23b}$, $-NR^{23a}C(=O)R^{23b}$ and - $NR^{24a}C(=O)NR^{25a}R^{25b}$, the alkyl, heteroalkyl (e.g., alkoxy), alkenyl, alkynyl, cycloalkyl, cycloalkoxy, heterocyclyl, aryl and heteroaryl are each optionally substituted with one or more substituents independently selected from the group consisting of: hydroxyl, halogen, CN, NOz, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl and 4-10-membered heterocyclyl; or when L is a direct bond, and m is greater than 1, two $R^{3'}$ together with the group to which they are attached form a 4-10-membered heterocycle;

$R^4$, at each occurrence, is each independently selected from the group consisting of H, hydroxyl, halogen, CN, NOz, $C_{1-6}$ alkyl, $C_{1-6}$ heteroalkyl (e.g., $C_{1-6}$ alkoxy), $C_{2-6}$ alkenyl, $C_{2-6}$ alkynyl, $C_{3-8}$ cycloalkyl, $C_{3-8}$ cycloalkoxy, 4-10-membered heterocyclyl, $C_{6-12}$ aryl, 5-10-membered heteroaryl, $-NR^{20a}R^{20b}$, $-SR^{21}$, - $S(=O)_2R^{22}$, $-S(=O)_2NR^{20a}R^{20b}$, $-NR^{20a}S(=O)_2R^{20b}$, $-C(=O)R^{21}$, $-C(=O)NR^{23a}R^{23b}$, $-NR^{23a}C(=O)R^{23b}$ and - $NR^{24a}C(=O)NR^{25a}R^{25b}$, the alkyl, heteroalkyl (e.g., alkoxy), alkenyl, alkynyl, cycloalkyl, cycloalkoxy, heterocyclyl, aryl and heteroaryl are each optionally substituted with one or more substituents independently selected from the group consisting of: hydroxyl, halogen, CN, $NH_2$, NOz, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl and 4-10-membered heterocyclyl;

$R^5$ and $R^6$ are each independently selected from the group consisting of H, hydroxyl, halogen, $-NH_2$, - $NHCH_3$, $-N(CH_3)_2$, CN, $C_{1-6}$ alkyl, $C_{1-6}$ heteroalkyl (e.g., $C_{1-6}$ alkoxy), $C_{3-8}$ cycloalkyl, $C_{3-8}$ cycloalkoxy and 4-10-membered heterocyclyl, or $R^5$ and $R^6$ together with the atom to which they are attached form $C_{3-8}$ cycloalkyl or 3-8-membered heterocyclyl, the alkyl, heteroalkyl (e.g., alkoxy), cycloalkyl, cycloalkoxy and heterocyclyl are each optionally substituted with one or more halogens;

$R^{20a}$, $R^{20b}$, $R^{23a}$, $R^{23b}$, $R^{24a}$, $R^{25a}$ and $R^{25b}$ are each independently selected from the group consisting of H, OH, $-NHCH_3$, $-N(CH_3)_2$, $C_{1-6}$ alkyl, $C_{1-6}$ alkoxy, $C_{3-8}$ cycloalkyl and 4-10-membered heterocyclyl; the alkyl, alkoxy, cycloalkyl and heterocyclyl are each optionally substituted with one or more substituents independently selected from the group consisting of: halogen, $C_{1-6}$ alkyl and 4-10-membered heterocyclyl;

$R^{21}$ and $R^{22}$ are each independently selected from the group consisting of $C_{1-6}$ alkyl, $C_{1-6}$ alkoxy, $C_{3-8}$ cycloalkyl and 4-10-membered heterocyclyl, the alkyl, alkoxy, cycloalkyl and heterocyclyl are each optionally substituted with one or more halogens;

m is 0, 1, 2, 3, 4 or 5;

n is 1 or 2; and

p is 0, 1, 2 or 3.

**2.** The compound according to claim 1 or a pharmaceutically acceptable salt, ester, stereoisomer, tautomer, polymorph, cocrystal, solvate, metabolite, isotopically labeled compound, N-oxide or prodrug thereof, wherein $R^4$, at each occurrence, is each independently selected from the group consisting of H, hydroxyl, halogen, CN, NOz, $C_{1-4}$ alkyl, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-6-membered heterocyclyl, $C_{6-10}$ aryl, 5-6-membered heteroaryl, - $NR^{20a}R^{20b}$, $-SR^{21}$, $-S(=O)_2R^{22}$, $-S(=O)_2NR^{20a}R^{20b}$, $-NR^{20a}S(=O)_2R^{20b}$, $-C(=O)R^{21}$, $-C(=O)NR^{23a}R^{23b}$, - $NR^{23a}C(=O)R^{23b}$ and $-NR^{24a}C(=O)NR^{25a}R^{25b}$, the alkyl, heteroalkyl (e.g., alkoxy), alkenyl, alkynyl, cycloalkyl, cycloalkoxy, heterocyclyl, aryl and heteroaryl are each optionally substituted with one or more substituents independently selected from the group consisting of: hydroxyl, halogen, CN, $NH_2$, NOz, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl and 4-6-membered heterocyclyl;

preferably, $R^4$, at each occurrence, is each independently selected from the group consisting of H, hydroxyl, halogen, CN, NOz, $C_{1-4}$ alkyl, $C_{1-4}$ heteroalkyl (e.g., $C_{1-4}$ alkoxy), $C_{3-6}$ cycloalkyl, $C_{3-6}$ cycloalkoxy, 4-6-membered heterocyclyl, 5-6-membered heteroaryl, $-NR^{20a}R^{20b}$, $-S(=O)_2NR^{20a}R^{20b}$, - $NR^{20a}S(=O)_2R^{20b}$, $-C(=O)R^{21}$, $-C(=O)NR^{23a}R^{23b}$ and $-NR^{23a}C(=O)R^{23b}$, the alkyl, heteroalkyl (e.g., alkoxy), cycloalkyl, cycloalkoxy, heterocyclyl and heteroaryl are each optionally substituted with one or more substituents independently selected from

the group consisting of: hydroxyl, halogen, $NH_2$, $C_{1-3}$ alkyl, $C_{1-3}$ haloalkyl, $C_{1-3}$ haloalkoxy and 4-6-membered heterocyclyl;

more preferably, $R^4$, at each occurrence, is each independently selected from the group consisting of H, hydroxyl, halogen, $C_{1-4}$ alkyl, $C_{1-4}$ heteroalkyl (e.g., $C_{1-4}$ alkoxy), 4-6-membered heterocyclyl, 5-6-membered heteroaryl and $-NR^{20a}R^{20b}$, the alkyl, heteroalkyl (e.g., alkoxy), heterocyclyl and heteroaryl are each optionally substituted with one or more substituents independently selected from the group consisting of: hydroxyl, halogen, $NH_2$, $C_{1-3}$ alkyl, $C_{1-3}$ haloalkyl, $C_{1-3}$ haloalkoxy and 4-6-membered heterocyclyl; and p is 0 or 1;

most preferably, $R^4$, at each occurrence, is each independently selected from the group consisting of hydroxyl, F, methyl, $-CH_2CH_2NH_2$,

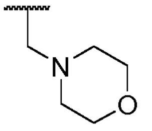 , 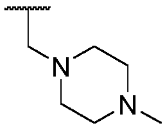 , 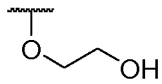 , 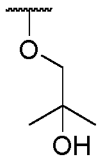 ,

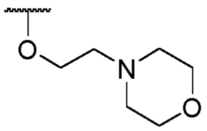 , 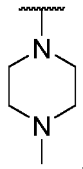 , 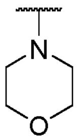 , 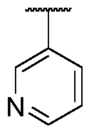 , 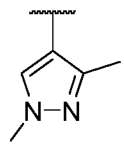 , 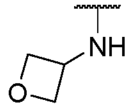 and 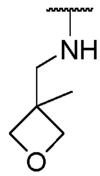 .

3. The compound according to claim 1 or 2, or a pharmaceutically acceptable salt, ester, stereoisomer, tautomer, polymorph, cocrystal, solvate, metabolite, isotopically labeled compound, N-oxide or prodrug thereof, wherein the compound has the structure of Formula I:

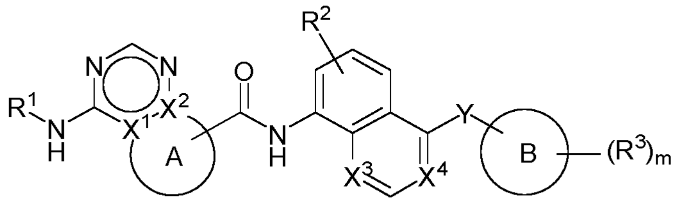

Formula **I**

wherein:

ring A is selected from the group consisting of benzene ring and 5-6-membered heteroaromatic ring;

ring B is selected from the group consisting of $C_{6-10}$ aromatic ring, 5-10-membered heteroaromatic ring and 4-10-membered heterocycle;

$X^1$ and $X^2$ are each independently selected from the group consisting of C and N;

$X^3$ and $X^4$ are each independently selected from the group consisting of CH and N;

Y is selected from the group consisting of -O-, -NH-, -C(=O)-, $-CR^5R^6-$, $-CR^5R^6O-$ and $-CR^5R^6NH-$; provided that, when ring A is a thiophene ring, Y is not -O- or -NH-;

$R^1$ is selected from the group consisting of H, $C_{1-6}$ alkyl and $C_{3-6}$ cycloalkyl, the alkyl and cycloalkyl are each optionally substituted with one or more halogens;

$R^2$ is selected from the group consisting of H, halogen, $C_{1-6}$ alkyl and $C_{1-6}$ alkoxy, the alkyl and alkoxy are each optionally substituted with one or more halogens;

$R^3$ is $L-R^{3'}$;

L, at each occurrence, is each independently a direct bond or $-(CH_2)_n-$;

$R^{3'}$, at each occurrence, is each independently selected from the group consisting of H, hydroxyl, halogen, CN, NOz, $C_{1-6}$ alkyl, $C_{1-6}$ heteroalkyl (e.g., $C_{1-6}$ alkoxy), $C_{2-6}$ alkenyl, $C_{2-6}$ alkynyl, $C_{3-8}$ cycloalkyl, $C_{3-8}$ cycloalkoxy, 4-10-membered heterocyclyl, $C_{6-12}$ aryl, 5-10-membered heteroaryl, $-NR^{20a}R^{20b}$, $-SR^{21}$, - $S(=O)_2R^{22}$, $-S(=O)_2NR^{20a}R^{20b}$, $-NR^{20a}S(=O)_2R^{20b}$, $-C(=O)R^{21}$, $-C(=O)NR^{23a}R^{23b}$, $-NR^{23a}C(=O)R^{23b}$ and $-NR^{24a}C(=O)NR^{25a}R^{25b}$, the alkyl, heteroalkyl (e.g., alkoxy), alkenyl, alkynyl, cycloalkyl, cycloalkoxy, heterocyclyl, aryl and heteroaryl are each optionally substituted with one or more substituents independently selected

from the group consisting of: hydroxyl, halogen, CN, NOz, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl and 4-10-membered heterocyclyl; or when L is a direct bond, and m is greater than 1, two $R^{3'}$ together with the group to which they are attached form a 4-10-membered heterocycle;

$R^5$ and $R^6$ are each independently selected from the group consisting of H, hydroxyl, halogen, $-NH_2$, - $NHCH_3$, $-N(CH_3)_2$, CN, $C_{1-6}$ alkyl, $C_{1-6}$ heteroalkyl (e.g., $C_{1-6}$ alkoxy), $C_{3-8}$ cycloalkyl, $C_{3-8}$ cycloalkoxy and 4-10-membered heterocyclyl, or $R^5$ and $R^6$ together with the atom to which they are attached form $C_{3-8}$ cycloalkyl or 3-8-membered heterocyclyl, the alkyl, heteroalkyl (e.g., alkoxy), cycloalkyl, cycloalkoxy and heterocyclyl are each optionally substituted with one or more halogens;

$R^{20a}$, $R^{20b}$, $R^{23a}$, $R^{23b}$, $R^{24a}$, $R^{25a}$ and $R^{25b}$ are each independently selected from the group consisting of H, OH, $-NHCH_3$, $-N(CH_3)_2$, $C_{1-6}$ alkyl, $C_{1-6}$ alkoxy, $C_{3-8}$ cycloalkyl and 4-10-membered heterocyclyl; the alkyl, alkoxy, cycloalkyl and heterocyclyl are each optionally substituted with one or more substituents independently selected from the group consisting of: halogen, $C_{1-6}$ alkyl and 4-10-membered heterocyclyl;

$R^{21}$ and $R^{22}$ are each independently selected from the group consisting of $C_{1-6}$ alkyl, $C_{1-6}$ alkoxy, $C_{3-8}$ cycloalkyl and 4-10-membered heterocyclyl, the alkyl, alkoxy, cycloalkyl and heterocyclyl are each optionally substituted with one or more halogens;

m is 0, 1, 2, 3, 4 or 5; and

n is 1 or 2.

**4.** The compound according to any one of claims 1 to 3, or a pharmaceutically acceptable salt, ester, stereoisomer, tautomer, polymorph, cocrystal, solvate, metabolite, isotopically labeled compound, N-oxide or prodrug thereof, wherein ring A is a benzene ring, thiophene ring, pyrrole ring, pyrazole ring, imidazole ring or pyridine ring; preferably, ring A is a benzene ring, thiophene ring, pyrrole ring, imidazole ring or pyridine ring; or
ring A is a benzene ring or 5-membered heteroaromatic ring; preferably, ring A is a benzene ring, thiophene ring, pyrrole ring, pyrazole ring or imidazole ring; more preferably, ring A is a benzene ring, thiophene ring, pyrrole ring or imidazole ring.

**5.** The compound according to any one of claims 1 to 4 or a pharmaceutically acceptable salt, ester, stereoisomer, tautomer, polymorph, cocrystal, solvate, metabolite, isotopically labeled compound, N-oxide or prodrug thereof, wherein ring B is a $C_{6-10}$ aromatic ring or 5-10-membered heteroaromatic ring; preferably, ring B is a $C_6$ aromatic ring or 6-membered heteroaromatic ring; more preferably, ring B is a benzene ring or pyridine ring.

**6.** The compound according to any one of claims 1 to 5 or a pharmaceutically acceptable salt, ester, stereoisomer, tautomer, polymorph, cocrystal, solvate, metabolite, isotopically labeled compound, N-oxide or prodrug thereof, wherein $X^1$ is C and $X^2$ is C; or $X^1$ is C and $X^2$ is N; or $X^1$ is N and $X^2$ is C;
preferably, $X^1$ is C and $X^2$ is C; or $X^1$ is C and $X^2$ is N.

**7.** The compound according to any one of claims 1 to 6 or a pharmaceutically acceptable salt, ester, stereoisomer, tautomer, polymorph, cocrystal, solvate, metabolite, isotopically labeled compound, N-oxide or prodrug thereof, wherein $X^3$ is CH and $X^4$ is N.

**8.** The compound according to any one of claims 1 to 7 or a pharmaceutically acceptable salt, ester, stereoisomer, tautomer, polymorph, cocrystal, solvate, metabolite, isotopically labeled compound, N-oxide or prodrug thereof, wherein Y is selected from the group consisting of -NH-, -C(=O)-, $-CR^5R^6-$, - $CR^5R^6O-$ and $-CR^5R^6NH-$; provided that, when ring A is a thiophene ring, Y is not -NH-;

preferably, Y is selected from the group consisting of -NH-, -C(=O)-, $-CH_2-$, -CHOH- and - $CH(CH_3)O-$; provided that, when ring A is a thiophene ring, Y is not -NH-;

more preferably, Y is selected from the group consisting of -NH-, -C(=O)-, $-CH_2-$ and -CHOH-; provided that, when ring A is a thiophene ring, Y is not -NH-.

**9.** The compound according to any one of claims 1 to 8 or a pharmaceutically acceptable salt, ester, stereoisomer, tautomer, polymorph, cocrystal, solvate, metabolite, isotopically labeled compound, N-oxide or prodrug thereof, wherein $R^1$ is H or $C_{1-3}$ alkyl; preferably, $R^1$ is H.

**10.** The compound according to any one of claims 1 to 9 or a pharmaceutically acceptable salt, ester, stereoisomer, tautomer, polymorph, cocrystal, solvate, metabolite, isotopically labeled compound, N-oxide or prodrug thereof, wherein $R^2$ is selected from the group consisting of H, halogen, $C_{1-3}$ alkyl and $C_{1-3}$ alkoxy, the alkyl and alkoxy are

each optionally substituted with one or more halogens; preferably, $R^2$ is F or $-CH_3$.

**11.** The compound according to any one of claims 1 to 10 or a pharmaceutically acceptable salt, ester, stereoisomer, tautomer, polymorph, cocrystal, solvate, metabolite, isotopically labeled compound, N-oxide or prodrug thereof, wherein L, at each occurrence, is each independently a direct bond or $-CH_2-$.

**12.** The compound according to any one of claims 1 to 11 or a pharmaceutically acceptable salt, ester, stereoisomer, tautomer, polymorph, cocrystal, solvate, metabolite, isotopically labeled compound, N-oxide or prodrug thereof, wherein $R^{3'}$, at each occurrence, is each independently selected from the group consisting of H, hydroxyl, halogen, CN, NOz, $C_{1-4}$ alkyl, $C_{1-4}$ heteroalkyl (e.g., $C_{1-4}$ alkoxy), $C_{2-6}$ alkenyl, $C_{2-6}$ alkynyl, $C_{3-6}$ cycloalkyl, $C_{3-6}$ cycloalkoxy, 4-6-membered heterocyclyl, $C_{6-10}$ aryl, 5-6-membered heteroaryl, $-NR^{20a}R^{20b}$, $-SR^{21}$, $-S(=O)_2R^{22}$, $-S(=O)_2NR^{20a}R^{20b}$, $-NR^{20a}S(=O)_2R^{20b}$, $-C(=O)R^{21}$, - $C(=O)NR^{23a}R^{23b}$, $-NR^{23a}C(=O)R^{23b}$ and $-NR^{24a}C(=O)NR^{25a}R^{25b}$, the alkyl, heteroalkyl (e.g., alkoxy), alkenyl, alkynyl, cycloalkyl, cycloalkoxy, heterocyclyl, aryl and heteroaryl are each optionally substituted with one or more substituents independently selected from the group consisting of: hydroxyl, halogen, CN, NOz, $C_{1-4}$ alkyl, $C_{1-4}$ haloalkyl, $C_{1-4}$ hydroxyalkyl, $C_{1-4}$ haloalkoxy, $C_{1-4}$ heteroalkyl (e.g., $C_{1-4}$ alkoxy), $C_{3-6}$ cycloalkyl and 4-6-membered heterocyclyl;

preferably, $R^{3'}$, at each occurrence, is each independently selected from the group consisting of H, hydroxyl, halogen, CN, NOz, $C_{1-4}$ alkyl, $C_{1-4}$ heteroalkyl (e.g., $C_{1-4}$ alkoxy), $C_{3-6}$ cycloalkyl, $C_{3-6}$ cycloalkoxy, 4-6-membered heterocyclyl, $C_{6-10}$ aryl, 5-6-membered heteroaryl, $-NR^{20a}R^{20b}$, $-S(=O)_2R^{22}$, - $S(=O)_2NR^{20a}R^{20b}$, $-NR^{20a}S(=O)_2R^{20b}$, $-C(=O)R^{21}$, $-C(=O)NR^{23a}R^{23b}$ and $-NR^{23a}C(=O)R^{23b}$, the alkyl, heteroalkyl (e.g., alkoxy), cycloalkyl, cycloalkoxy, heterocyclyl, aryl and heteroaryl are each optionally substituted with one or more substituents independently selected from the group consisting of: hydroxyl, halogen, CN, NOz, $C_{1-3}$ alkyl, $C_{1-3}$ haloalkyl, $C_{1-3}$ hydroxyalkyl, $C_{1-3}$ haloalkoxy, $C_{1-3}$ heteroalkyl (e.g., $C_{1-3}$ alkoxy), $C_{3-6}$ cycloalkyl and 4-6-membered heterocyclyl;
more preferably, $R^{3'}$, at each occurrence, is each independently selected from the group consisting of H, hydroxyl, halogen, CN, NOz, $C_{1-4}$ alkyl, $C_{1-4}$ heteroalkyl (e.g., $C_{1-4}$ alkoxy), $C_{3-6}$ cycloalkyl, $C_{3-6}$ cycloalkoxy, 4-6-membered heterocyclyl, $-NR^{20a}R^{20b}$, $-S(=O)_2R^{22}$, $-S(=O)_2NR^{20a}R^{20b}$, $-NR^{20a}S(=O)_2R^{20b}$, - $C(=O)R^{21}$, $-C(=O)NR^{23a}R^{23b}$ and $-NR^{23a}C(=O)R^{23b}$, the alkyl, heteroalkyl (e.g., alkoxy), cycloalkyl, cycloalkoxy and heterocyclyl are each optionally substituted with one or more substituents independently selected from the group consisting of: hydroxyl, halogen, $C_{1-3}$ alkyl, $C_{1-3}$ haloalkyl, $C_{1-3}$ haloalkoxy, $C_{1-3}$ heteroalkyl and 4-6-membered heterocyclyl;
more preferably, $R^{3'}$, at each occurrence, is each independently selected from the group consisting of H, halogen, CN, $C_{1-4}$ alkyl, $C_{1-4}$ heteroalkyl (e.g., $C_{1-4}$ alkoxy), $-NR^{20a}R^{20b}$, $-S(=O)_2R^{22}$ and $C(=O)R^{21}$, the alkyl and heteroalkyl (e.g., alkoxy) are each optionally substituted with one or more substituents independently selected from the group consisting of halogen, $C_{1-3}$ heteroalkyl and 4-6-membered heterocyclyl;
most preferably, $R^{3'}$, at each occurrence, is each independently F, Cl, Br, CN, methyl, trifluoromethyl, methoxy, ethoxy, $-C(=O)CH_3$, $-N(CH_3)_2$, $-S(=O)_2CH_3$,

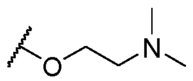,
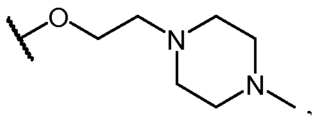,
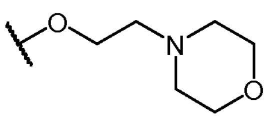 or 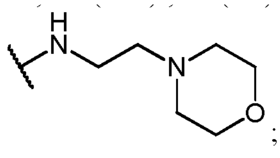;

or when L is a direct bond, and m is greater than 1, any two $R^{3'}$ together with the group to which they are attached form a 4-6-membered heterocycle, preferably, together form

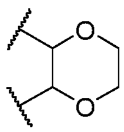 or 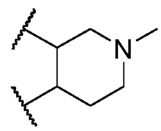.

**13.** The compound according to any one of claims 1 to 12 or a pharmaceutically acceptable salt, ester, stereoisomer, tautomer, polymorph, cocrystal, solvate, metabolite, isotopically labeled compound, N-oxide or prodrug thereof, wherein the compound has the following structure:

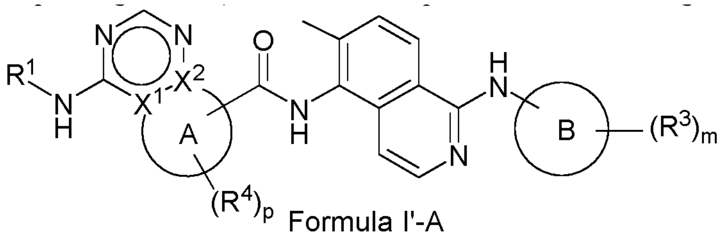

Formula I'-A

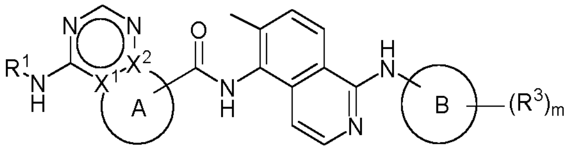

I-A

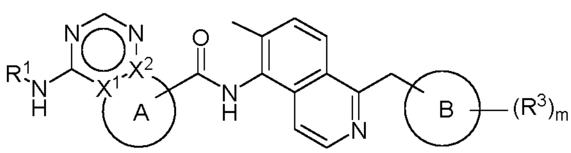

I-B

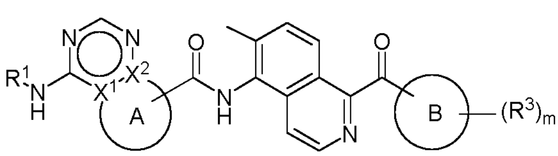

I-C

or

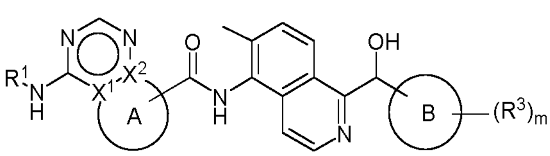

I-D

wherein each group is as defined in any one of claims 1-12.

**14.** The compound according to any one of claims 1 to 13 or a pharmaceutically acceptable salt, ester, stereoisomer, tautomer, polymorph, cocrystal, solvate, metabolite, isotopically labeled compound, N-oxide or prodrug thereof, wherein the compound is selected from the group consisting of:

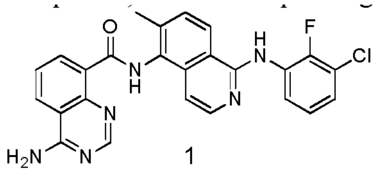

1

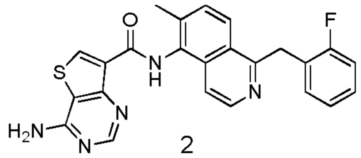

2

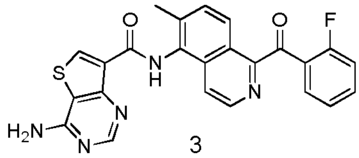

3

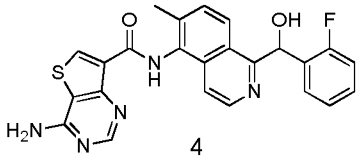

4

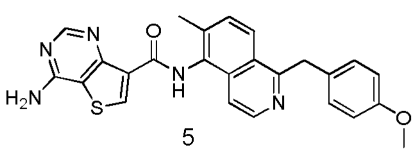

5

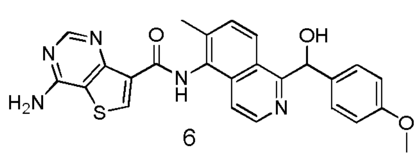

6

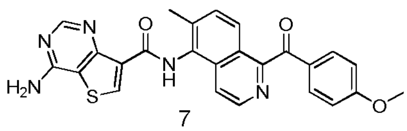

7

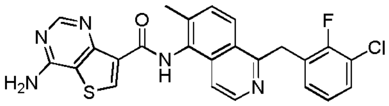

8

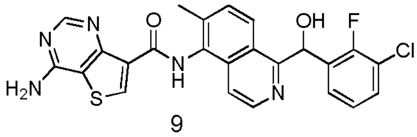

9

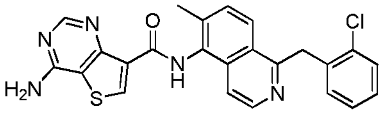
10

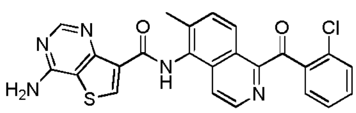
11

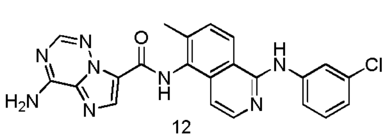
12

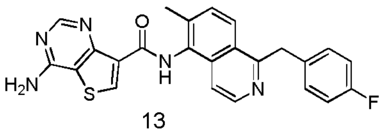
13

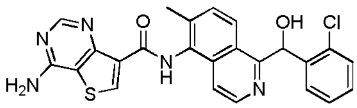
14

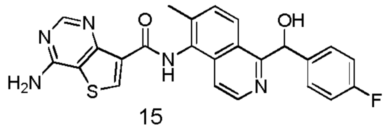
15

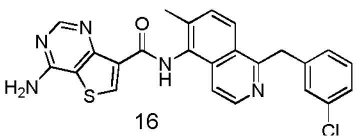
16

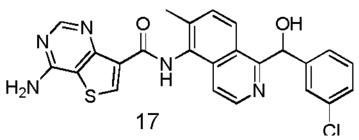
17

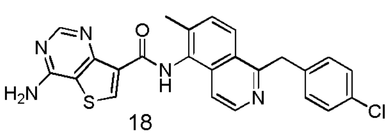
18

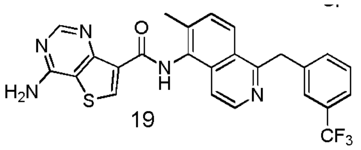
19

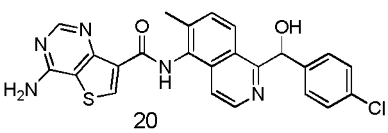
20

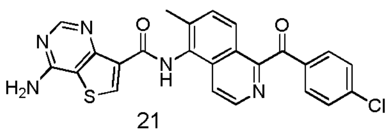
21

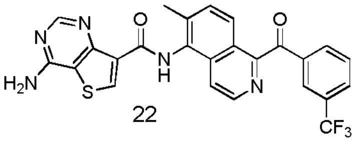
22

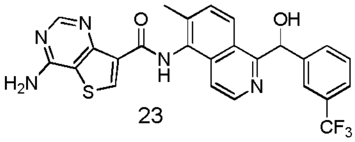
23

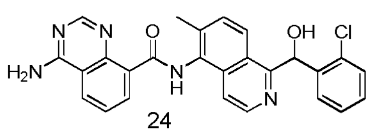
24

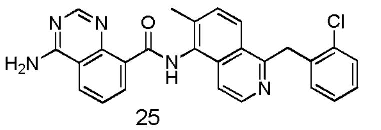
25

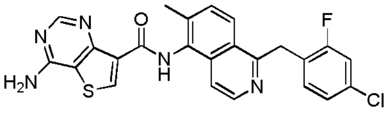
26

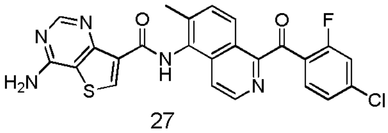
27

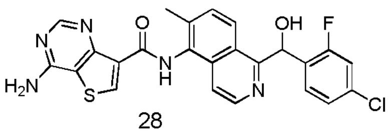
28

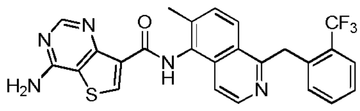
29

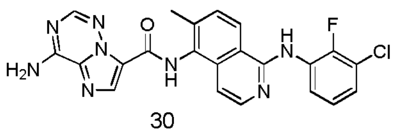
30

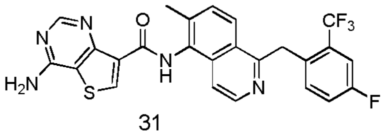
31

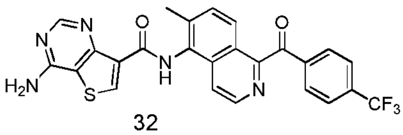
32

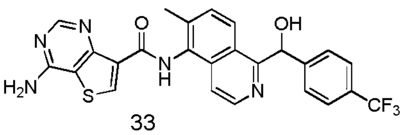
33

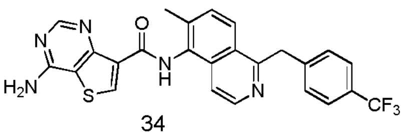
34

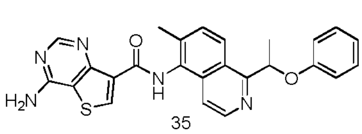
35

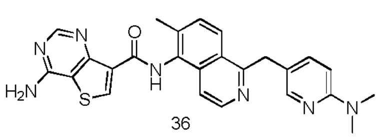
36

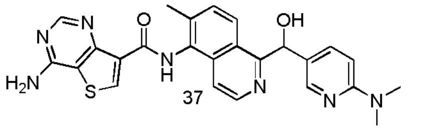
37

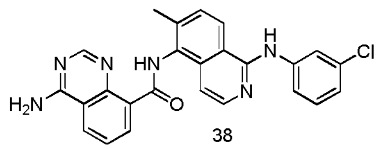
38

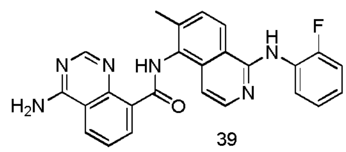
39

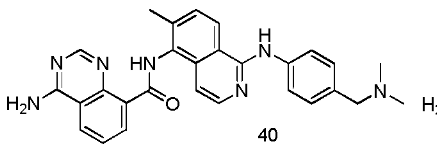
40

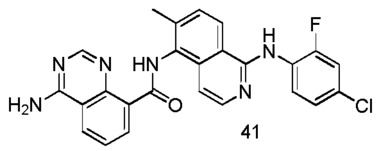
41

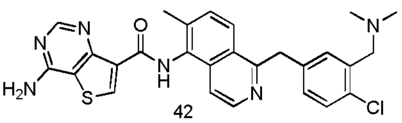
42

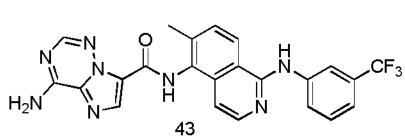
43

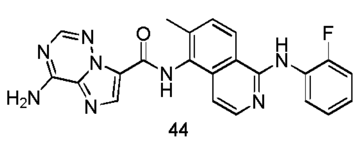
44

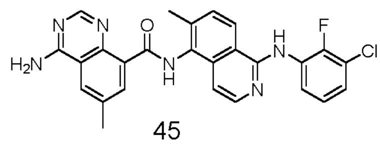
45

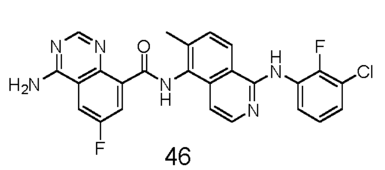
46

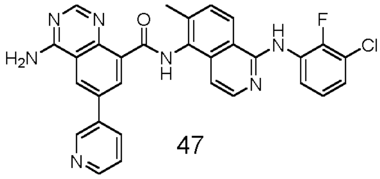
47

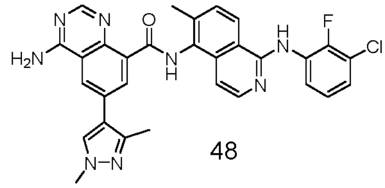
48

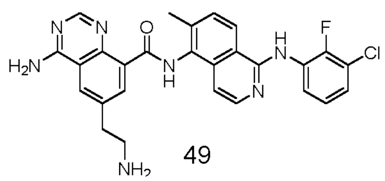
49

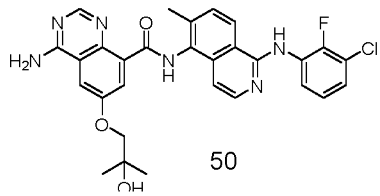
50

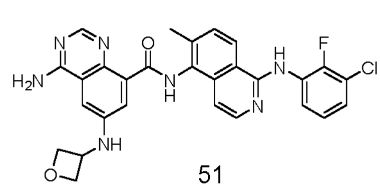
51

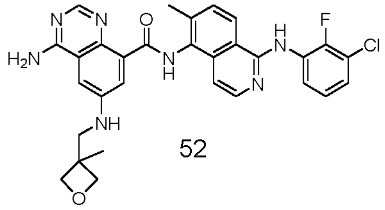
52

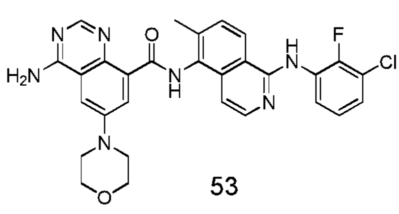
53

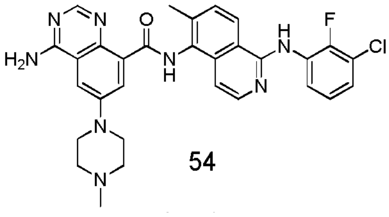
54

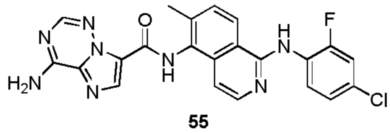
**55**

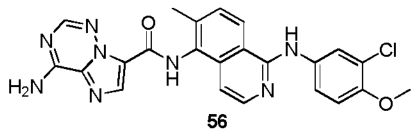
**56**

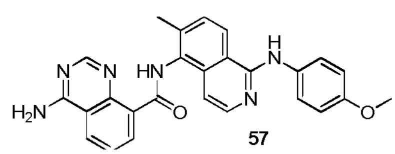
**57**

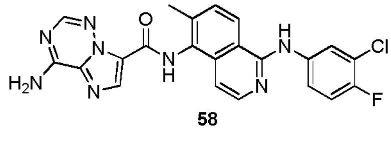
**58**

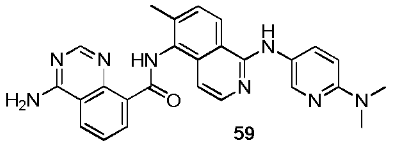
**59**

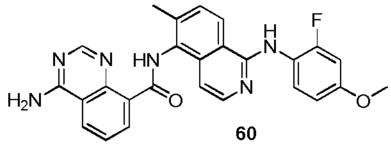
**60**

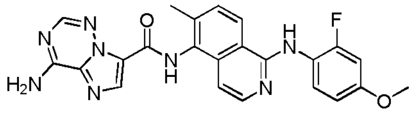
61
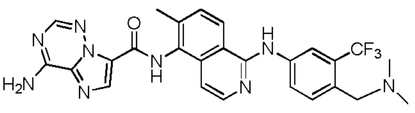
62
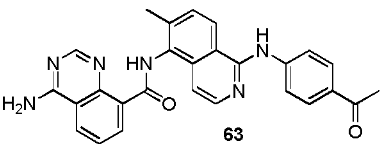
63
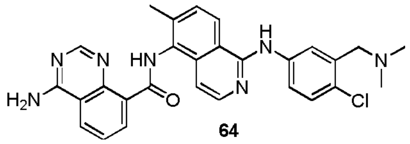
64
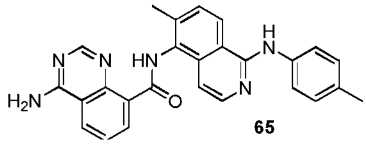
65
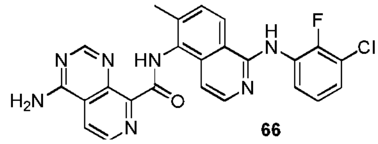
66
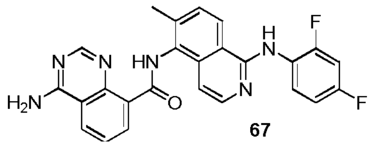
67
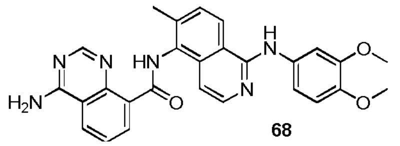
68
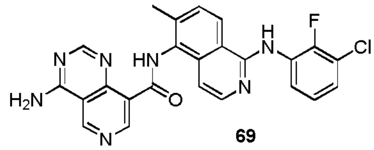
69
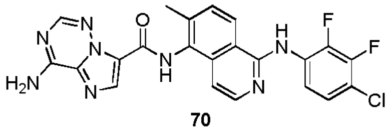
70
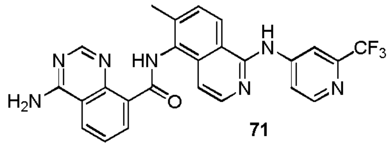
71
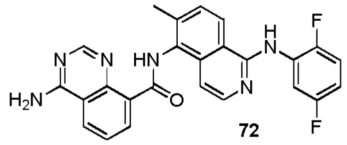
72
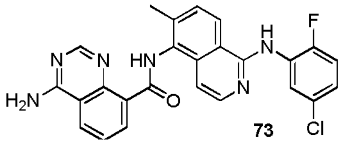
73
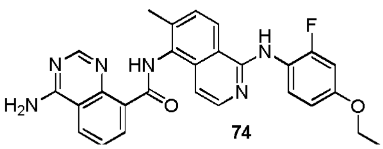
74
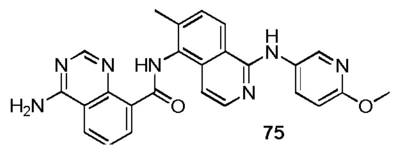
75
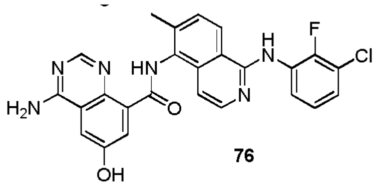
76
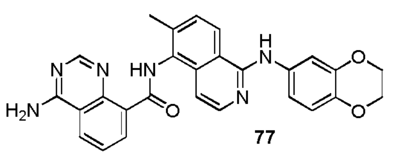
77
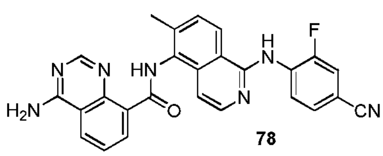
78
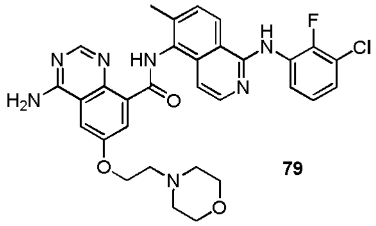
79
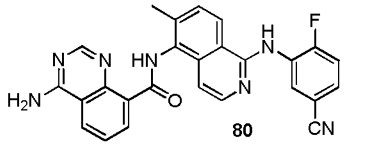
80
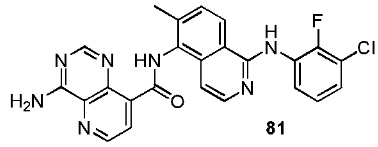
81
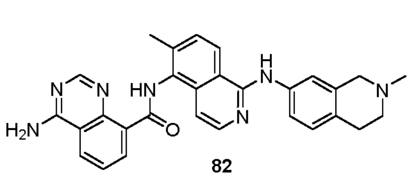
82
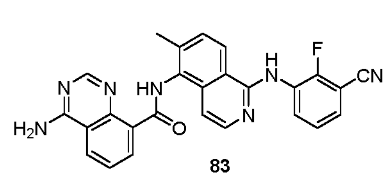
83
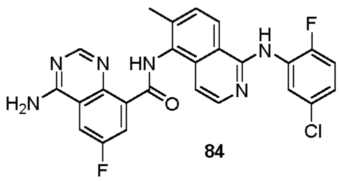
84

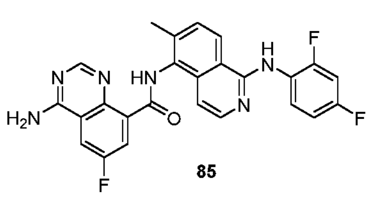
85

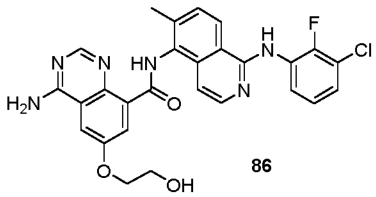
86

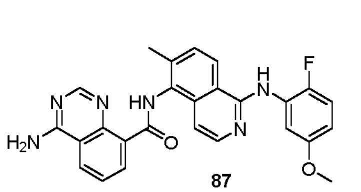
87

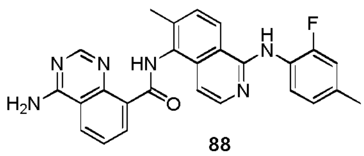
88

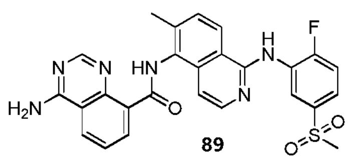
89

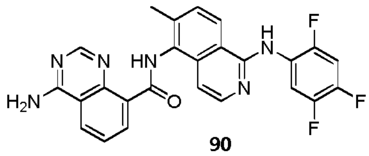
90

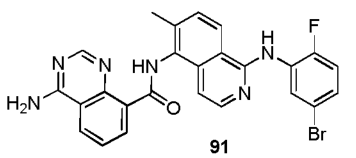
91

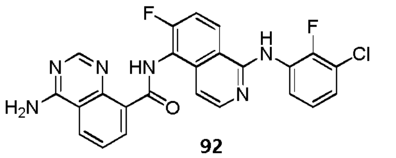
92

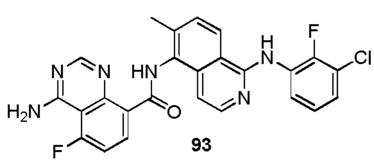
93

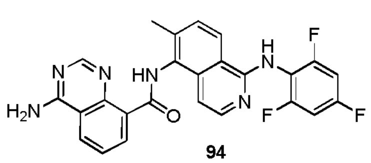
94

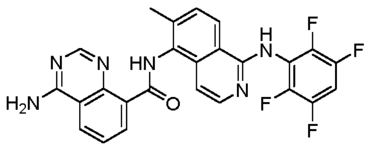
95

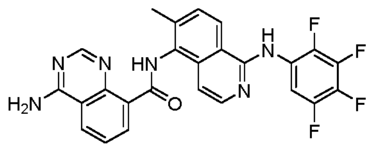
96

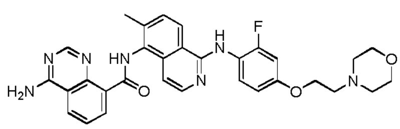
97

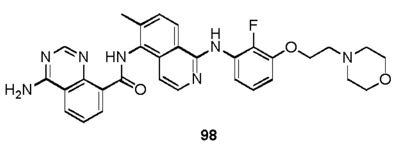
98

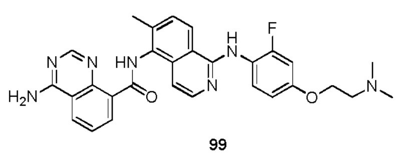
99

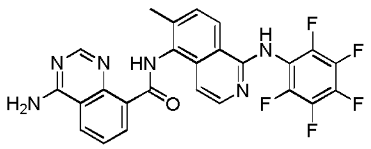
100

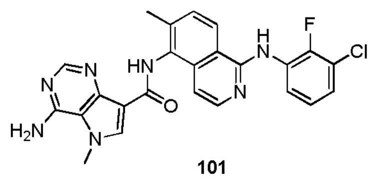
101

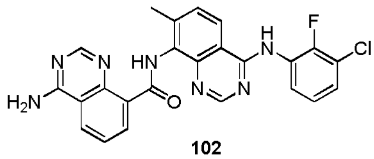
102

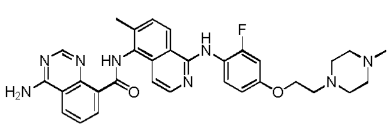
103

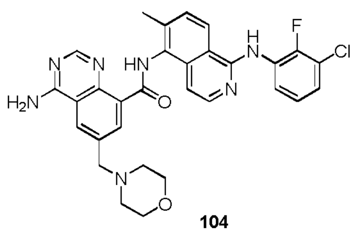
104

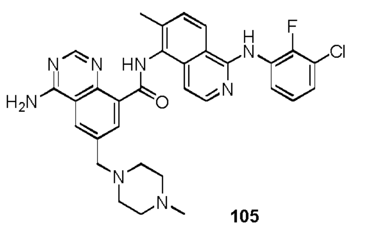
105

.

**15.** A pharmaceutical composition comprising a prophylactically or therapeutically effective amount of the compound of any one of claims 1-14, or a pharmaceutically acceptable salt, ester, stereoisomer, tautomer, polymorph, cocrystal, solvate, metabolite, isotopically labeled compound, N-oxide or prodrug thereof, and one or more pharmaceutically acceptable carriers.

**16.** Use of the compound of any one of claims 1-14 or a pharmaceutically acceptable salt, ester, stereoisomer, tautomer, polymorph, cocrystal, solvate, metabolite, isotopically labeled compound, N-oxide or prodrug thereof, or the pharmaceutical composition of claim 15 in the manufacture of a medicament for the prophylaxis or treatment of a disease or disorder associated with RAF and/or RAS kinase activity; the disease or disorder associated with RAF and/or RAS kinase activity is preferably cancer or tumor; the cancer or tumor is preferably lung cancer (e.g., non-small cell lung cancer), breast cancer, ovarian cancer, gastric cancer, liver cancer, kidney cancer, bone cancer, colorectal cancer, intestinal cancer, pancreatic cancer, head and neck cancer, uterine cancer, esophageal cancer, thyroid cancer, bladder cancer, blood cancer, lymphoma, multiple myeloma, melanoma, glioma, brain tumor or sarcoma.

**17.** The compound of any one of claims 1-14 or a pharmaceutically acceptable salt, ester, stereoisomer, tautomer, polymorph, cocrystal, solvate, metabolite, isotopically labeled compound, N-oxide or prodrug thereof, or the pharmaceutical composition of claim 15, for use in the prophylaxis or treatment of a disease or disorder associated with RAF and/or RAS kinase activity; the disease or disorder associated with RAF and/or RAS kinase activity is preferably cancer or tumor; the cancer or tumor is preferably lung cancer (e.g., non-small cell lung cancer), breast cancer, ovarian cancer, gastric cancer, liver cancer, kidney cancer, bone cancer, colorectal cancer, intestinal cancer, pancreatic cancer, head and neck cancer, uterine cancer, esophageal cancer, thyroid cancer, bladder cancer, blood cancer, lymphoma, multiple myeloma, melanoma, glioma, brain tumor or sarcoma.

**18.** A method for the prophylaxis or treatment of a disease or disorder associated with RAF and/or RAS kinase activity, wherein the method comprises administering to a subject in need thereof an effective amount of the compound of any one of claims 1-14 or a pharmaceutically acceptable salt, ester, stereoisomer, tautomer, polymorph, cocrystal, solvate, metabolite, isotopically labeled compound, N-oxide or prodrug thereof, or the pharmaceutical composition of claim 15.

**19.** The method of claim 18, wherein the disease or disorder associated with RAF and/or RAS kinase activity is preferably cancer or tumor; the cancer or tumor is preferably lung cancer (e.g., non-small cell lung cancer), breast cancer, ovarian cancer, gastric cancer, liver cancer, kidney cancer, bone cancer, colorectal cancer, intestinal cancer, pancreatic cancer, head and neck cancer, uterine cancer, esophageal cancer, thyroid cancer, bladder cancer, blood cancer, lymphoma, multiple myeloma, melanoma, glioma, brain tumor or sarcoma.

**20.** A preparation method, wherein

the method is a method for preparing a compound of Formula I-A, comprising the following steps:

Scheme A

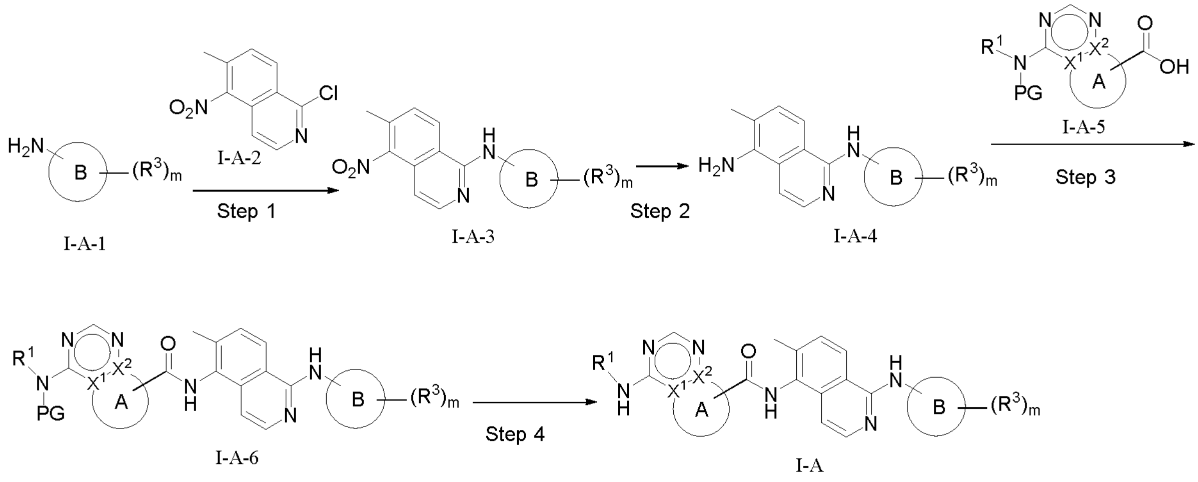


wherein:

PG is an amino-protecting group, preferably 2,4-dimethoxybenzyl;
the remaining groups are each as defined in any one of claims 1-13;
the reaction conditions of each step are as follows:

Step 1: subjecting compounds I-A-1 and I-A-2 to a substitution reaction or a coupling reaction to afford compound I-A-3;

Step 2: subjecting compound I-A-3 to a reduction reaction to afford compound I-A-4;
Step 3: subjecting compounds I-A-4 and I-A-5 to a condensation reaction to afford compound I-A-6;
Step 4: removing the protecting group from compound I-A-6 under an acidic condition to afford a compound of Formula I-A;

alternatively, the method is a method for preparing a compound of Formula I-A, comprising the following steps:

Scheme A-1

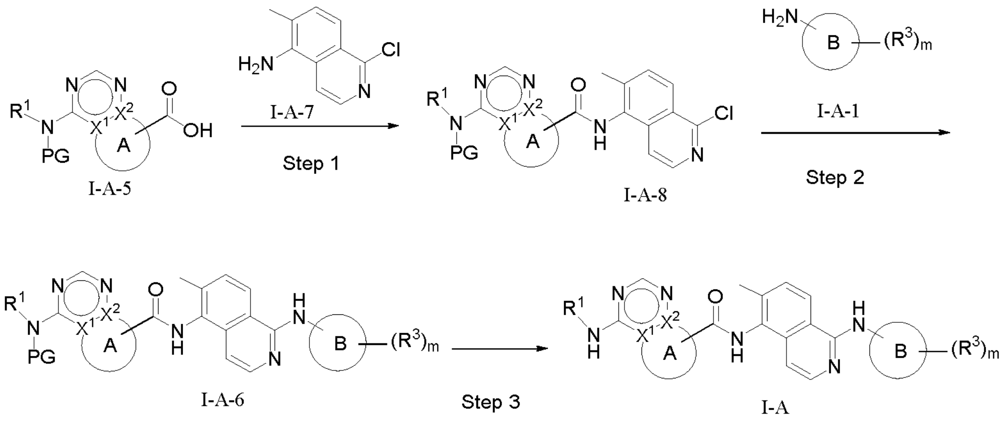


wherein:

PG is an amino-protecting group, preferably 2,4-dimethoxybenzyl;
the remaining groups are each as defined in any one of claims 1-13;
the reaction conditions of each step are as follows:

Step 1: subjecting compounds I-A-5 and I-A-7 to a condensation reaction to afford compound I-A-8;
Step 2: subjecting compounds I-A-1 and I-A-8 to a substitution reaction or a coupling reaction to afford compound I-A-6;
Step 3: removing the protecting group from compound I-A-6 under an acidic condition to afford a compound of Formula I-A;

alternatively, the method is a method for preparing a compound of Formula I-B, comprising the following steps:

Scheme B-1

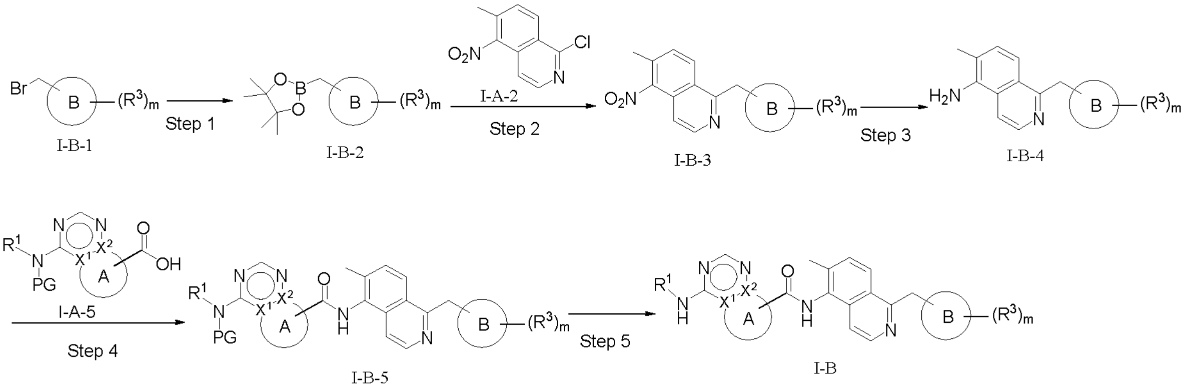


wherein:

PG is an amino-protecting group, preferably 2,4-dimethoxybenzyl;
the remaining groups are each as defined in any one of claims 1-13;
the reaction conditions of each step are as follows:

Step 1: reacting compound I-B-1 with a boron-containing reagent to afford compound I-B-2;
Step 2: subjecting compounds I-B-2 and I-A-2 to a coupling reaction to afford compound I-B-3;
Step 3: subjecting compound I-B-3 to a reduction reaction to afford compound I-B-4;
Step 4: subjecting compounds I-B-4 and I-A-5 to a condensation reaction to afford compound I-B-5;
Step 5: removing the protecting group from compound I-B-5 under an acidic condition to afford a compound of Formula I-B;

alternatively, the method is a method for preparing a compound of Formula I-B, comprising the following steps:

Scheme B-2

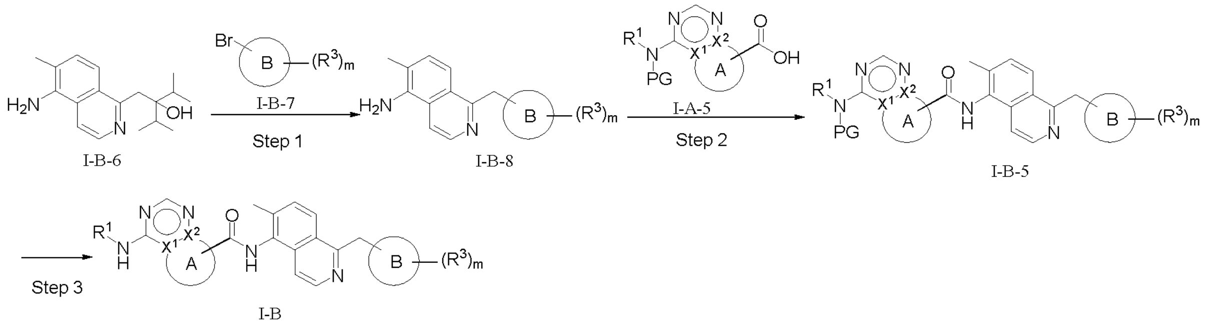


wherein:

PG is an amino-protecting group, preferably 2,4-dimethoxybenzyl;
the remaining groups are each as defined in any one of claims 1-13;
the reaction conditions of each step are as follows:

Step 1: subjecting compounds I-B-6 and I-B-7 to a coupling reaction to afford compound I-B-8;
Step 2: subjecting compounds I-B-8 and I-A-5 to a condensation reaction to afford compound I-B-5;
Step 3: removing the protecting group from compound I-B-5 under an acidic condition to afford a compound of Formula I-B;

alternatively, the method is a method for preparing a compound of Formula I-C, comprising the following steps:

Scheme C

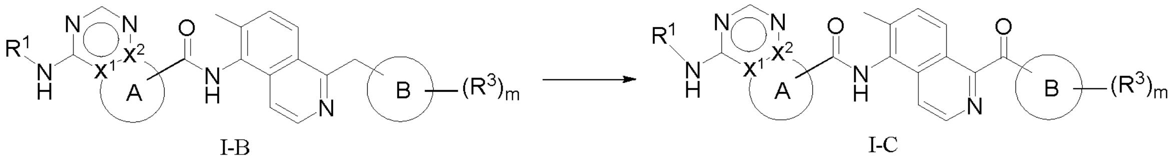


wherein:

the groups are each as defined in any one of claims 1-13;
the method comprises subjecting compound I-B to a catalytic oxidation reaction to afford a compound of Formula I-C;
alternatively, the method is a method for preparing a compound of Formula I-D, comprising the following steps:

Scheme D-1

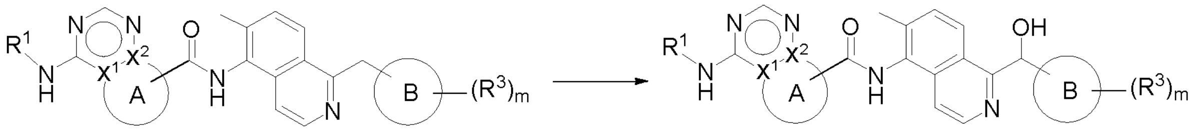

I-B I-D

wherein:

the groups are each as defined in any one of claims 1-13;
the method comprises subjecting compound I-B to a catalytic oxidation reaction to afford a compound of Formula I-D;
alternatively, the method is a method for preparing a compound of Formula I-D, comprising the following steps:

Scheme D-2

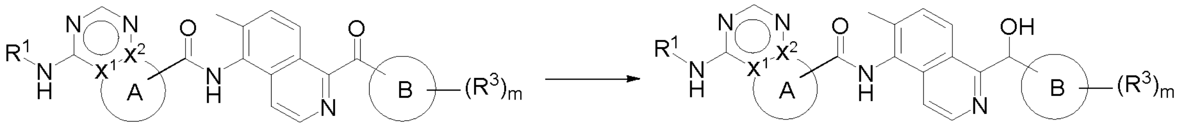

I-C I-D

wherein:

the groups are each as defined in any one of claims 1-13;
the method comprises subjecting compound I-C to a reduction reaction to afford a compound of Formula I-D.

## INTERNATIONAL SEARCH REPORT

International application No. **PCT/CN2021/123949**

**A. CLASSIFICATION OF SUBJECT MATTER**

C07D 401/12(2006.01)i； C07D 471/02(2006.01)i； A61K 31/519(2006.01)i； A61P 35/00(2006.01)i

According to International Patent Classification (IPC) or to both national classification and IPC

**B. FIELDS SEARCHED**

Minimum documentation searched (classification system followed by classification symbols)

C07D;A61K;A61P

Documentation searched other than minimum documentation to the extent that such documents are included in the fields searched

Electronic data base consulted during the international search (name of data base and, where practicable, search terms used)

CNTXT; ENTXT; WPABS; VEN; STN(CAPLUS, REGISTRY, MARPAT): 苯并嘧啶, RAF, RAS, 苯并吡啶, RAS INHIBITOR?, RAF INHIBITOR?, benzopyridine, benzopyrimidine, structural formula search

**C. DOCUMENTS CONSIDERED TO BE RELEVANT**

| Category* | Citation of document, with indication, where appropriate, of the relevant passages | Relevant to claim No. |
|---|---|---|
| X | CN 104039798 A (HANMI PHARMACEUTICAL CO., LTD.) 10 September 2014 (2014-09-10)<br>entire document, in particular claims 1, 6, 9-10, experiment embodiment 1, description, paragraphs [0218]-[0226] | 1-17,20 |
| A | US 2019367489 A1 (ARAXES PHARMA LLC.) 05 December 2019 (2019-12-05)<br>entire document | 1-17,20 |
| A | US 2020010454 A1 (ARAXES PHARMA LLC.) 09 January 2020 (2020-01-09)<br>entire document | 1-17,20 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **24 December 2021** | **13 January 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/123949**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **18-19**
because they relate to subject matter not required to be searched by this Authority, namely:

[1] Claims 18-19 relate to a treatment or prevention method for diseases, which is practiced on the human or animal body, and therefore, the subject matters of claims 18-19 belong to subject matter that the International Search Authority is not required to search (PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/123949**

| Patent document cited in search report | Publication date (day/month/year) | Patent family member(s) | Publication date (day/month/year) |
|---|---|---|---|
| CN 104039798 A | 10 September 2014 | CA 2859668 A1 | 04 July 2013 |
| | | CA 2859668 C | 15 January 2019 |
| | | ZA 201405583 B | 28 October 2015 |
| | | PE 20141404 A1 | 28 October 2014 |
| | | CL 2014001729 A1 | 26 September 2014 |
| | | SG 11201402765 T A | 27 June 2014 |
| | | AR 089489 A1 | 27 August 2014 |
| | | PT 2797927 T | 30 October 2019 |
| | | HR P20191840 T1 | 27 December 2019 |
| | | HR P20191840 T8 | 07 February 2020 |
| | | ES 2751608 T3 | 01 April 2020 |
| | | IL 266285 D0 | 30 June 2019 |
| | | US 2014371219 A1 | 18 December 2014 |
| | | US 9156852 B2 | 13 October 2015 |
| | | BR 112014015720 A2 | 13 June 2017 |
| | | BR 112014015720 A8 | 04 July 2017 |
| | | BR 112014015720 B1 | 17 March 2020 |
| | | JP 2015503553 A | 02 February 2015 |
| | | JP 5797345 B2 | 21 October 2015 |
| | | MX 2014007230 A | 15 May 2015 |
| | | MX 363659 B | 28 March 2019 |
| | | EP 3617213 A1 | 04 March 2020 |
| | | NZ 628013 A | 29 July 2016 |
| | | PL 2797927 T3 | 31 December 2019 |
| | | UA 109614 C2 | 10 September 2015 |
| | | DK 2797927 T3 | 28 October 2019 |
| | | SG 10201701999 Q A | 27 April 2017 |
| | | AU 2012363558 A1 | 21 August 2014 |
| | | AU 2012363558 B2 | 19 November 2015 |
| | | AU 2012363558 C1 | 29 August 2019 |
| | | MX 2019003468 A | 06 June 2019 |
| | | KR 20130079256 A | 10 July 2013 |
| | | KR 101490761 B1 | 09 February 2015 |
| | | RS 59420 B1 | 29 November 2019 |
| | | IL 233441 D0 | 31 August 2014 |
| | | IL 233441 A | 30 May 2019 |
| | | BR 122019019582 B1 | 13 July 2021 |
| | | WO 2013100632 A1 | 04 July 2013 |
| | | IN 6101DEN2014 A | 14 August 2015 |
| | | HK 1200833 A1 | 14 August 2015 |
| | | RU 2014131390 A | 20 February 2016 |
| | | RU 2625799 C2 | 19 July 2017 |
| | | CO 7010840 A2 | 31 July 2014 |
| | | EP 2797927 A1 | 05 November 2014 |
| | | EP 2797927 A4 | 08 July 2015 |
| | | EP 2797927 B1 | 25 September 2019 |
| | | TW 201331209 A | 01 August 2013 |
| | | TW I462927 B | 01 December 2014 |
| | | PH 12014501504 B1 | 08 October 2014 |
| US 2019367489 A1 | 05 December 2019 | WO 2018140512 A1 | 02 August 2018 |

Form PCT/ISA/210 (patent family annex) (January 2015)

**INTERNATIONAL SEARCH REPORT**
**Information on patent family members**

International application No.
**PCT/CN2021/123949**

| Patent document cited in search report | Publication date (day/month/year) | Patent family member(s) | Publication date (day/month/year) |
|---|---|---|---|
| | | EP 3573954 A1 | 04 December 2019 |
| US 2020010454 A1 | 09 January 2020 | WO 2018140514 A1 | 02 August 2018 |
| | | EP 3573970 A1 | 04 December 2019 |
| | | US 11136308 B2 | 05 October 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 104039798 B


### Non-patent literature cited in the description


- **KAROULIA Z et al.** *Nat Rev Cancer.,* November 2017, vol. 17 (11), 676-691
- **STAHL ; WERMUTH.** Hand book of Pharmaceutical Salts: Properties, Selection, and Use. Wiley-VCH, 2002
- **T. L. GILCHRIST.** Comprehensive Organic Synthesis. Academic Press, vol. 7, 748-750
- **G. W. H. CHEESEMAN ; E. S. G. WERSTIUK.** Advances in Heterocyclic Chemistry. Academic Press, vol. 22, 390-392
- **T. HIGUCHI ; V STELLA.** Pro-drugs as Novel Delivery Systems. *ACS Symposium Series,* vol. 14
- Design of Prodrugs. Elsevier, 1985
- **T. W. GREENE ; P. G. M. WUTS.** Protective Groups in Organic Synthesis. John Wiley & Sons, 1991
- *Remington's Pharmaceutical Sciences,* 1990